# EUROPEAN PATENT APPLICATION

(11) **EP 4 509 505 A1**
(43) Date of publication of application: **19.02.2025**
(21) Application number: 23787851.7
(22) Date of filing: 14.04.2023
(51) Int. Cl.: C07D 413/14, C07D 403/14, A61P 35/00, A61K 31/395

(54) **KIF18A INHIBITOR AND USE THEREOF**

(30) Priority: 15.04.2022 CN 202210400226; 29.08.2022 CN 202211042750; 07.02.2023 CN 202310104625; 03.03.2023 CN 202310231297; 07.04.2023 CN 202310387827
(71) Applicant: Wuhan Humanwell Innovative Drug Research and Development Center Limited Company, Wuhan, Hubei 430075 (CN)
(72) Inventor: ZHANG, Xuejun, Wuhan, Hubei 430075 (CN); ZANG, Yang, Wuhan, Hubei 430075 (CN); LI, Qun, Wuhan, Hubei 430075 (CN); DING, Xiaohua, Wuhan, Hubei 430075 (CN); SUN, Xiaochuan, Wuhan, Hubei 430075 (CN); FU, Haoliang, Wuhan, Hubei 430075 (CN); ZHAO, Xin, Wuhan, Hubei 430075 (CN); CHENG, Zhenqi, Wuhan, Hubei 430075 (CN); LI, Li'e, Wuhan, Hubei 430075 (CN); YANG, Jun, Wuhan, Hubei 430075 (CN)
(74) Representative: Gille Hrabal Partnerschaftsgesellschaft mbB Patentanwälte
(86) International application number: PCT/CN2023/088503
(87) International publication number: WO 2023/198209

(57) **Abstract**

The present invention provides a heterocyclic compound as represented by formula V, or a tautomer, a stereoisomer, a hydrate, a solvate, a pharmaceutically acceptable salt or a prodrug thereof. The compound has a good KIF18A inhibitory effect.

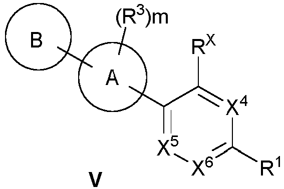

## Description

The present disclosure claims priority to:
a prior application with the application No. 2022104002265 entitled "KIF18A INHIBITOR AND USE THEREOF" and filed with China National Intellectual Property Administration on Apr. 15, 2022;
a prior application with the application No. 2022110427506 entitled "KIF18A INHIBITOR AND USE THEREOF" and filed with China National Intellectual Property Administration on Aug. 29, 2022;
a prior application with the application No. 2023101046251 entitled "KIF18A INHIBITOR AND USE THEREOF" and filed with China National Intellectual Property Administration on Feb. 7, 2023;
a prior application with the application No. 202310231297.1 entitled "KIF18A INHIBITOR AND USE THEREOF" and filed with China National Intellectual Property Administration on Mar. 3, 2023; and
a prior application with the application No. 202310387827.1 entitled "KIF18A INHIBITOR AND USE THEREOF" and filed with China National Intellectual Property Administration on Apr. 7, 2023,
which are incorporated herein by reference in their entirety.

### TECHNICAL FIELD

The present disclosure belongs to the field of pharmaceuticals, and particularly relates to a KIF18A inhibitor and use thereof.

### BACKGROUND

Cancer is one of the most prevalent diseases afflicting humans and is a leading cause of death worldwide. However, to date, only a few of the available cancer treatments and therapies have achieved considerable success. Cancer is generally characterized by unregulated cell proliferation. Cellular pathways control the proliferation process through the cell cycle and centrosome cycle, and damage to one or more genes responsible for the cellular pathways can result in loss of normal regulation of cell proliferation. These dysregulated genes can encode a variety of tumor suppressor proteins or oncogene proteins that are involved in a series of events leading to unexamined cell cycle progression and cell proliferation. Various kinases and kinesins have been identified, which play key roles in the regulation and progression of the cell cycle and mitosis in both normally dividing cells and cancer cells.

Kinesins are molecular motors that play important roles in cell division and intracellular vesicle and organelle trafficking. Mitotic kinesins function in multiple aspects of spindle assembly, chromosome segregation, centrosome segregation, and dynamics (O. Rath and F. Kozielski, Nature Review Cancer, 12:527-39, 2012). Human kinesins are categorized into 14 subfamilies based on sequence homology within the so-called "motor domain". The ATPase activity of this domain drives unidirectional movement along microtubules (MTs). The non-motor domains of these proteins are responsible for cargo attachment. The "cargo" may include any one of a variety of different membranous organelles, signal transduction scaffolding systems, and chromosomes. Kinesins use ATP hydrolysis energy to move cargo along polarized microtubules. Therefore, kinesins are often referred to as "plus-end" or "minus-end" directed motors.

The KIF18A gene belongs to the kinesin-8 subfamily and is a plus-end directed motor. KIF18A is thought to influence the dynamics of the plus ends of kinetochore microtubules to control proper chromosome positioning and spindle tension. The deletion of human KIF18A results in spindle elongation, increased metaphase chromosome oscillation, and activation of the mitotic spindle assembly checkpoint in HeLa cervical cancer cells. KIF18A appears to be a viable target for cancer treatment. KIF18A is overexpressed in various types of cancers, including but not limited to colon cancer, breast cancer, lung cancer, pancreatic cancer, prostate cancer, bladder cancer, head cancer, neck cancer, cervical cancer, and ovarian cancer. Furthermore, in cancer cell lines, the deletion or knock-out or inhibition of the KIF18A gene affects the mitotic spindle of cancer cell lines. In particular, it has been found that the inhibition of KIF18A induces mitotic cell arrest, a known weakness that can promote mitotic cell death by apoptosis, mitotic catastrophe, or multiphase-driven lethality, or death following mitotic slippage during the interphase. Therefore, there is a strong interest in finding inhibitors of the KIF18A protein.

Currently, no drugs that treat numerous conditions including cancer through inhibition of KIF18A are available on the market. Therefore, the development of new compounds capable of inhibiting the activity of KIF18A is of great significance for treating diseases.

### SUMMARY

The object of the present disclosure is to provide a new class of compounds having MT-based KIF18A regulatory activity, in particular, KIF18A inhibitory activity. To this end, the present disclosure further provides use of these compounds and pharmaceutically acceptable salts thereof in the preparation and manufacture of pharmaceutical compositions or medicaments for the therapeutic, prophylactic, acute, or chronic treatment of KIF18A-mediated diseases and disorders (including but not limited to cancer). Therefore, the compounds of the present disclosure can be used for manufacturing anti-cancer drugs. The present disclosure further provides a method for preparing a compound represented by formula V, and intermediates useful in the method.

In a first aspect of the present disclosure, provided is a compound represented by formula V, or a tautomer, a stereoisomer, a hydrate, a solvate, a pharmaceutically acceptable salt, or a prodrug thereof: 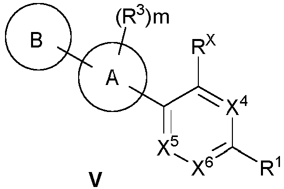 wherein
ring A is a 5- to 6-membered heteroaryl ring;
ring B is selected from 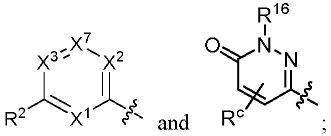
X¹ is N or CR⁵;
X² is N or CR⁶;
X³ is N or CR⁷;
X⁴ is N or CR⁸;
X⁵ is N or CR⁹;
X⁶ is N or CR¹⁰;
X⁷ is N or CR⁴;
R¹ is selected from cyano and a -ZR¹² group, wherein Z is independently selected from -C₀₋₆ alkyl-, -C₀₋₆ alkyl-NR¹¹-C₀₋₆ alkyl-, -C₀₋₆ alkyl-NR¹¹SO₂-C₀₋₆ alkyl-, -C₀₋₆ alkyl-SO₂NR¹¹-C₀₋₆ alkyl-, -C₀₋₆ alkyl-NR¹¹SO₂NR¹¹-C₀₋₆ alkyl-,-C₀₋₆ alkyl-NR¹¹SO₂NR¹¹-C(=O)-O-C₀₋₆ alkyl-, -C₀₋₆ alkyl-NR¹¹-S(=O)(=NH)-C₀₋₆ alkyl-, -C₀₋₆ alkyl-S(=O)(=NH)-C₀₋₆ alkyl-, -C₀₋₆ alkyl-S-C₀₋₆ alkyl-, -C₀₋₆ alkyl-S(=O)-C₀₋₆ alkyl-, -C₀₋₆ alkyl-SO₂-C₀₋₆ alkyl-, -C₀₋₆ alkyl-O-C₀₋₆ alkyl-, -P-, -C₀₋₆ alkyl-P(=O)(R¹¹)-, -C₀₋₆ alkyl-P(=O)₂, -C₀₋₆ alkyl-(C=O)-C₀₋₆ alkyl-, -C₀₋₆ alkyl-(C=O)-O-C₀₋₆ alkyl-, -C₀₋₆ alkyl-(C=O)NR¹¹-C₀₋₆ alkyl-, -C₀₋₆ alkyl-NR¹¹(C=O)-C₀₋₆ alkyl-, and -C(=N-OH)-; or the -ZR¹² group is -N=S(=O)-(R¹²)₂, wherein two R¹² are alternatively combined with the sulfur atom to which they are attached to form a saturated or partially saturated 3-, 4-, 5-, or 6-membered monocyclic ring comprising 0, 1, 2, or 3 N atoms and 0, 1, or 2 atoms selected from O and S;
R² is halogen or a -Y-R¹³ group, wherein Y is -C₀₋₆ alkyl-, -C₀₋₆ alkyl-NR¹³-C₀₋₆ alkyl-, -C₀₋₆ alkyl-O-C₀₋₆ alkyl-, -C₀₋₆ alkyl-S-C₀₋₆ alkyl-, -C₀₋₆ alkyl-S(=O)-C₀₋₆ alkyl-, -C₀₋₆ alkyl-S(=O)₂-C₀₋₆ alkyl-, -C₀₋₆ alkyl-SO₂NR¹³-C₀₋₆ alkyl-,-C₀₋₆ alkyl-S(=O)(=NH)-, -C₀₋₆ alkyl-NR¹³-SO₂-C₀₋₆ alkyl-, -C₀₋₆ alkyl-NR¹³SO₂NR¹³-C₀₋₆ alkyl-, -C₀₋₆ alkyl-C(=O)-C₀₋₆ alkyl-, -C₀₋₆ alkyl-NR¹³C(=O)-C₀₋₆ alkyl-, -C₀₋₆ alkyl-(C=O)NR¹³-C₀₋₆ alkyl-, or -C₀₋₆ alkyl-C(=O)-O-C₀₋₆ alkyl-; or -Y-R¹³ is -N=S(=O)-(R¹³)₂, wherein two R¹³ are alternatively combined with the sulfur atom to which they are attached to form a saturated or partially saturated 3-, 4-, 5-, or 6-membered monocyclic ring comprising 0, 1, 2, or 3 N atoms and 0, 1, or 2 atoms selected from O and S;
R³ is selected from halogen, hydroxyl, cyano, amino, C₁₋₆ alkyl, C₁₋₆ alkoxy, C₁₋₆ haloalkyl, -OC₁₋₆ alkyl, and -OC₁₋₆ haloalkyl;
m is selected from 0, 1, 2, 3, and 4;
R⁴ is selected from hydrogen, halogen, cyano, C₁₋₈ alkyl, -OC₁₋₈ alkyl, C₁₋₄ haloalkyl, -OR^{4a}, -OR^{4b}, -C₀₋₆ alkyl-(C=O)-N-(C₀₋₆ alkyl)₂, -C₀₋₆ alkyl-SO₂N-(C₀₋₆ alkyl)₂, R^{4a}, and R^{4b};
R⁵ is selected from hydrogen, halogen, cyano, C₁₋₆ alkyl, C₁₋₄ haloalkyl, C₃₋₈ cycloalkyl, halogenated C₃₋₈ cycloalkyl, -O-C₁₋₈ alkyl, and -O-R^{5a}, wherein R^{5a} is a saturated, partially saturated, or unsaturated 3-, 4-, 5-, 6-, or 7-membered monocyclic ring comprising 0, 1, 2, or 3 N atoms and 0, 1, or 2 atoms selected from O and S; or
alternatively, R² and R⁵ may be combined with the carbon atom to which they are each attached to form a saturated or partially saturated 5- or 6-membered monocyclic ring fused to the phenyl ring, wherein the 5- or 6-membered monocyclic ring comprises 0, 1, 2, or 3 N atoms and 0 or 1 atom selected from O and S, and further wherein the 5- or 6-membered monocyclic ring is substituted with 0, 1, 2, or 3 groups selected from: halogen, C₁₋₆ alkyl, C₁₋₄ haloalkyl, -OR^{a}, -OC₁₋₄ haloalkyl, cyano, -NR^{a}R^{a}, and oxo;
R⁷ is selected from hydrogen, halogen, cyano, C₁₋₈ alkyl, C₃₋₈ cycloalkyl, C₃₋₈ halocycloalkyl, C₁₋₄ haloalkyl, -O-C₁₋₈ alkyl, and R^{7a}; or alternatively, R² and R⁷ may be combined with the carbon atom to which they are each attached to form a saturated or partially saturated 5- or 6-membered monocyclic ring fused to the phenyl ring, wherein the 5- or 6-membered monocyclic ring comprises 0, 1, 2, or 3 N atoms and 0 or 1 atom selected from O and S, and further wherein the 5- or 6-membered monocyclic ring is substituted with 0, 1, 2, or 3 groups selected from: halogen, C₁₋₆ alkyl, C₁₋₄ haloalkyl, -OR^{a}, -OC₁₋₄ haloalkyl, CN, -NR^{a}R^{a}, and oxo;
R⁶, R⁸, and R⁹ are each independently selected from hydrogen, halogen, cyano, C₁₋₈ alkyl, -O-C₁₋₈ alkyl, C₃₋₈ cycloalkyl, C₃₋₈ halocycloalkyl, and C₁₋₄ haloalkyl;
R¹⁰ is hydrogen, halogen, hydroxyl, cyano, C₁₋₈ alkyl, C₃₋₈ cycloalkyl, C₃₋₈ halocycloalkyl, C₁₋₄ haloalkyl, -O-R^{10a}, or -O-R^{10b};
R¹¹ is hydrogen, R^{11a}, or R^{11b};
R¹² is hydrogen, halogen, hydroxyl, cyano, R^{12a}, or R^{12b};
R¹³ is hydrogen, halogen, cyano, R^{13a}, or R^{13b};
R¹⁶ is hydrogen, R^{16a}, or R^{16b};
R^{4a}, R^{7a}, R^{10a}, R^{11a}, R^{12a}, R^{13a}, and R^{16a} are each independently selected from the group consisting of: a saturated, partially saturated, or unsaturated 3-, 4-, 5-, 6-, or 7-membered monocyclic ring or 4-, 5-, 6-, 7-, 8-, 9-, 10-, 11-, or 12-membered bicyclic ring comprising 0, 1, 2, or 3 N atoms and 0, 1, or 2 atoms selected from O and S, wherein the monocyclic or bicyclic ring is substituted with 0, 1, 2, or 3 groups selected from: halogen, cyano, amino, C₁₋₆ alkyl, C₁₋₆ haloalkyl, -OR^{a}, -OC₁₋₆ haloalkyl, -C(=O)R^{b}, -C(=O)OR^{a}, -C(=O)NR^{a}R^{a}, -C(=NR^{a})NR^{a}R^{a}, -OC(=O)R^{b},-OC(=O)NR^{a}R^{a}, -OC₂₋₆ alkyl NR^{a}R^{a}, -OC₂₋₆ alkyl OR^{a}, -SR^{a}, -S(=O)R^{b}, -S(=O)₂R^{b}, -S(=O)₂NR^{a}R^{a}, -NR^{a}R^{a},-N(R^{a})C(=O)R^{b}, -N(R^{a})C(=O)OR^{b}, -N(R^{a})C(=O)NR^{a}R^{a}, -N(R^{a})C(=NR^{a})NR^{a}R^{a}, -N(R^{a})S(=O)₂R^{b},-N(R^{a})S(=O)₂NR^{a}R^{a}, -NR^{a}C₂₋₆ alkyl NR^{a}R^{a}, -NR^{a}C₂₋₆ alkyl OR^{a}, -C₁₋₆ alkyl NR^{a}R^{a}, -C₁₋₆ alkyl OR^{a}, -C₁₋₆ alkyl N(R^{a})C(=O)R^{b}, -C₁₋₆ alkyl OC(=O)R^{b}, -C₁₋₆ alkyl C(=O)NR^{a}R^{a}, -C₁₋₆ alkyl C(=O)OR^{a}, R¹⁴, and oxo;
R^{4b}, R^{10b}, R^{11b}, R^{12b}, R^{13b}, and R^{16b} are each independently selected from the group consisting of: C₁₋₆ alkyl substituted with 0, 1, 2, 3, 4, or 5 groups selected from the group consisting of: halogen, cyano, C₁₋₆ alkyl, C₁₋₆ haloalkyl, -C₀₋₆NR^{a}R^{a}, -OR^{a}, -C(=O)OR^{a}, and a saturated, partially saturated, or unsaturated 3-, 4-, 5-, or 6-membered monocyclic ring;
R¹⁴ is independently selected from the group consisting of: a saturated, partially saturated, or unsaturated 3-, 4-, 5-, 6-, or 7-membered monocyclic ring or 4-, 5-, 6-, 7-, 8-, 9-, 10-, 11-, or 12-membered bicyclic ring comprising 0, 1, 2, or 3 N atoms and 0, 1, or 2 atoms selected from O and S, wherein the monocyclic or bicyclic ring is substituted with 0, 1, 2, or 3 groups selected from: halogen, cyano, amino, C₁₋₆ alkyl, C₁₋₆ haloalkyl, -OR^{a}, -OC₁₋₆ haloalkyl,-C(=O)R^{b}, -C(=O)OR^{a}, -C(=O)NR^{a}R^{a}, -C(=NR^{a})NR^{a}R^{a}, -OC(=O)R^{b}, -OC(=O)NR^{a}R^{a}, -OC₂₋₆ alkyl NR^{a}R^{a}, -OC₂-C₆ alkyl OR^{a}, -SR^{a}, -S(=O)R^{b}, -S(=O)₂R^{b}, -S(=O)₂NR^{a}R^{a}, -NR^{a}R^{a}, -N(R^{a})C(=O)R^{b}, -N(R^{a})C(=O)OR^{b},-N(R^{a})C(=O)NR^{a}R^{a}, -N(R^{a})C(=NR^{a})NR^{a}R^{a}, -N(R^{a})S(=O)₂R^{b}, -N(R^{a})S(=O)₂NR^{a}R^{a}, -NR^{a}C₂₋₆ alkyl NR^{a}R^{a}, -NR^{a}C₂₋₆ alkyl OR^{a}, -C₁₋₆ alkyl NR^{a}R^{a}, -C₁₋₆ alkyl OR^{a}, -C₁₋₆ alkyl N(R^{a})C(=O)R^{b}, -C₁₋₆ alkyl OC(=O)R^{b}, -C₁₋₆ alkyl C(=O)NR^{a}R^{a}, -C₁₋₆ alkyl C(=O)OR^{a}, and oxo;
R^{x} is selected from the group consisting of: hydrogen, 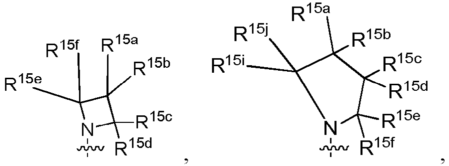 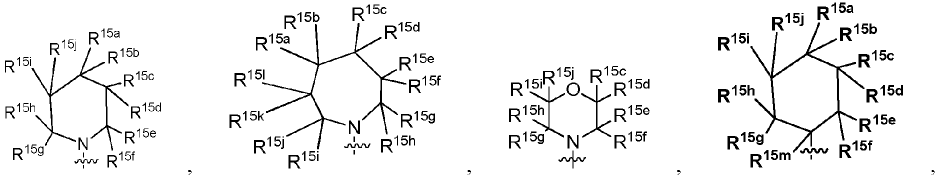 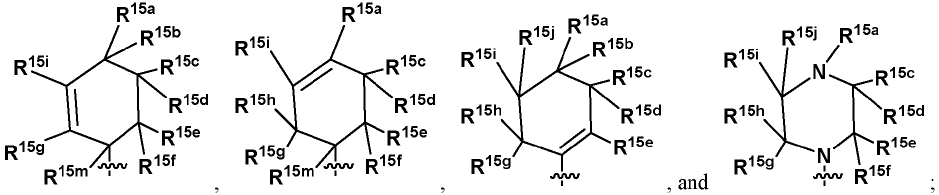 or
R^{x} is C₂₋₈ alkyl substituted with 0, 1, 2, 3, 4, or 5 groups selected from the group consisting of: halogen, cyano, amino, C₁₋₆ alkyl, C₁₋₆ haloalkyl, -OR^{a}, -NR^{a}R^{a}, -C(=O)OR^{a}, -OC₁₋₄ haloalkyl, and R¹⁵ⁿ; or
R^{x} is phenyl, or phenyl or an unsaturated 5-membered monocyclic ring substituted with 0, 1, 2, 3, 4, or 5 groups selected from the group consisting of: halogen, cyano, amino, C₁₋₆ alkyl, C₁₋₆ haloalkyl, -OR^{a}, -NR^{a}R^{a}, -C(=O)OR^{a}, -OC₁₋₄ haloalkyl, and R¹⁵ⁿ;
the 5-membered monocyclic ring comprises 0, 1, 2, or 3 N atoms and 0 or 1 atom selected from O and S;
R^{15a}, R^{15b}, R^{15c}, R^{15d}, R^{15e}, R^{15f}, R^{15g}, R^{15h}, R¹⁵ⁱ, R^{15j}, R^{15k}, R^{15l}, and R^{15m} are each independently hydrogen, halogen, R^{15o}, or R^{15p}; or
alternatively, each of the pairs of R^{15a} and R^{15b}, R^{15c} and R^{15d}, R^{15e} and R^{15f}, R^{15g} and R^{15h}, R¹⁵ⁱ and R^{15j}, and R^{15k} and R^{15l} may be independently combined with the carbon atom to which they are attached to form a saturated or partially saturated 3-, 4-, 5-, or 6-membered monocyclic ring spiro-attached to the R^{x} ring, wherein the 3-, 4-, 5-, or 6-membered monocyclic ring comprises 0, 1, 2, or 3 N atoms and 0, 1, or 2 atoms selected from O and S, and further wherein the 3-, 4-, 5-, or 6-membered monocyclic ring is substituted with 0, 1, 2, or 3 groups selected from: halogen, C₁₋₆ alkyl, C₁₋₄ haloalkyl, -OR^{a}, -OC₁₋₄ haloalkyl, CN, -NR^{a}R^{a}, and oxo; or
yet alternatively, each of the pairs of R^{15a} and R^{15b}, R^{15c} and R^{15d}, R^{15e} and R^{15f}, R^{15g} and R^{15h}, R¹⁵ⁱ and R^{15j}, and R^{15k} and R^{15l} may be independently combined to form a double bond;
R¹⁵ⁿ and R^{15o} may be independently selected from a saturated, partially saturated, or unsaturated 3-, 4-, 5-, 6-, or 7-membered monocyclic ring or 8-, 9-, 10-, 11-, or 12-membered bicyclic ring, wherein the monocyclic or bicyclic ring comprises 0, 1, 2, or 3 N atoms and 0, 1, or 2 atoms selected from O and S, and further wherein the monocyclic or bicyclic ring is substituted with 0, 1, 2, 3, or 4 groups selected from: halogen, C₁₋₆ alkyl, C₁₋₄ haloalkyl, -OR^{a}, -OC₁₋₄ haloalkyl, CN, -C(=O)OR^{a}, -C(=O)R^{b}, -C(=O)NR^{a}R^{a}, -C(=NR^{a})NR^{a}R^{a}, -OC(=O)R^{b}, -OC(=O)NR^{a}R^{a}, -OC₂₋₆ alkyl NR^{a}R^{a}, -NR^{a}R^{a}, -OC₂₋₆OR^{a}, -SR^{a}, -S(=O)R^{b}, -S(=O)₂R^{b}, -S(=O)₂NR^{a}R^{a}, -NR^{a}C(=O)R^{b}, -NR^{a}C(=O)OR^{b},-NR^{a}C(=O)NR^{a}R^{a}, -NR^{a}C(=NR^{a})NR^{a}R^{a}, -NR^{a}S(=O)₂NR^{b}, -NR^{a}S(=O)₂NR^{a}R^{a}, -R^{a}C₂₋₆ alkyl NR^{a}R^{a}, -NR^{a}C₂₋₆ alkyl OR^{a}, -C₁₋₆ alkyl NR^{a}R^{a}, -C₁₋₆ alkyl OR^{a}, -C₁₋₆ alkyl OC(=O)R^{b}, -C₁₋₆ alkyl NR^{a}C(=O)R^{b}, -C₁₋₆ alkyl NR^{a}C(=O)NR^{a}R^{a}, -C₁₋₆ alkyl-C(=O)OR^{a}, and oxo;
R^{15p} may be independently selected from C₁₋₈ alkyl substituted with 0, 1, 2, 3, 4, or 5 groups selected from halogen, C₁₋₄ haloalkyl, CN, -C(=O)OR^{a}, -OR^{a}, -OC₁₋₄ haloalkyl, and -NR^{a}R^{a};
R^{a} and R^{c} may be independently selected from H and R^{b};
R^{b} is independently selected from C₁₋₆ alkyl, phenyl, and benzyl, wherein the C₁₋₆ alkyl is substituted with 0, 1, 2, or 3 substituents selected from: halogen, hydroxyl, -OC₁₋₆ alkyl, -NH₂, -NHC₁₋₆ alkyl, -OC(=O)C₁₋₆ alkyl, and -N(C₁₋₆ alkyl)C₁₋₆ alkyl; and the phenyl or benzyl is substituted with 0, 1, 2, or 3 substituents selected from: halogen, C₁₋₆ alkyl, C₁₋₄ haloalkyl, -OH, -OC₁₋₆ alkyl, -NH₂, -NHC₁₋₆ alkyl, -OC(=O)C₁₋₆ alkyl, and -N(C₁₋₆ alkyl)C₁₋₆ alkyl.

In one preferred embodiment of the present disclosure, the compound represented by formula V, or the tautomer, the stereoisomer, the hydrate, the solvate, the pharmaceutically acceptable salt, or the prodrug thereof has structural formula II: 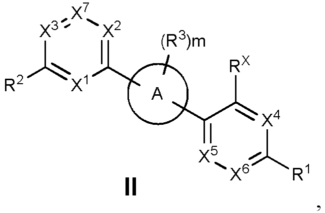 wherein
X¹ is N or CR⁵;
X² is N or CR⁶;
X³ is N or CR⁷;
X⁴ is N or CR⁸;
X⁵ is N or CR⁹;
X⁶ is N or CR¹⁰;
X⁷ is N or CR⁴;
ring A is a 5- to 6-membered heteroaryl ring;
R¹ is selected from cyano and a -ZR¹² group, wherein Z is selected from -C₀₋₆ alkyl-, -C₀₋₆ alkyl-NR¹¹-C₀₋₆ alkyl-,-C₀₋₆ alkyl-NR¹¹SO₂-C₀₋₆ alkyl-, -C₀₋₆ alkyl-SO₂NR¹¹-C₀₋₆ alkyl-, -C₀₋₆ alkyl-NR¹¹SO₂NR¹¹-C₀₋₆ alkyl-, -C₀₋₆ alkyl-NR¹¹SO₂NR¹¹-C(=O)-O-C₀₋₆ alkyl-, -C₀₋₆ alkyl-NR¹¹-S(=O)(=NC₀₋₆ alkyl)-, -C₀₋₆ alkyl-S(=O)(=NC₀₋₆ alkyl)-, -C₀₋₆ alkyl-S-C₀₋₆ alkyl-, -C₀₋₆ alkyl-S(=O)-C₀₋₆ alkyl-, -C₀₋₆ alkyl-SO₂-C₀₋₆ alkyl-, -C₀₋₆ alkyl-O-C₀₋₆ alkyl-, -P-, -C₀₋₆ alkyl-P(=O)(R¹¹)-, -C₀₋₆ alkyl-P(=O)₂, -C₀₋₆ alkyl-(C=O)-C₀₋₆ alkyl-, -C₀₋₆ alkyl-(C=O)-O-C₀₋₆ alkyl-, -C₀₋₆ alkyl-(C=O)NR¹¹-C₀₋₆ alkyl-, -C₀₋₆ alkyl-NR¹¹(C=O)-C₀₋₆ alkyl-, and -C(=N-OH)-; or the -ZR¹² group is -N=S(=O)-(R¹²)₂, wherein two R¹² are alternatively combined with the sulfur atom to which they are attached to form a saturated or partially saturated 3-, 4-, 5-, or 6-membered monocyclic ring comprising 0, 1, 2, or 3 N atoms and 0, 1, or 2 atoms selected from O and S;
R² is halogen or a -Y-R¹³ group, wherein Y is -C₀₋₆ alkyl-, -C₀₋₆ alkyl-NR¹³-C₀₋₆ alkyl-, -C₀₋₆ alkyl-O-C₀₋₆ alkyl-, -C₀₋₆ alkyl-S-C₀₋₆ alkyl-, -C₀₋₆ alkyl-S(=O)-C₀₋₆ alkyl-, -C₀₋₆ alkyl-S(=O)₂-C₀₋₆ alkyl-, -C₀₋₆ alkyl-SO₂NR¹³-C₀₋₆ alkyl-,-C₀₋₆ alkyl-S(=O)(=NH)-, -C₀₋₆ alkyl-NR¹³-SO₂-C₀₋₆ alkyl-, -C₀₋₆ alkyl-NR¹³SO₂NR¹³-C₀₋₆ alkyl-, -C₀₋₆ alkyl-C(=O)-C₀₋₆ alkyl-, -C₀₋₆ alkyl-NR¹³C(=O)-C₀₋₆ alkyl-, -C₀₋₆ alkyl-(C=O)NR¹³-C₀₋₆ alkyl-, or -C₀₋₆ alkyl-C(=O)-O-C₀₋₆ alkyl-; or -Y-R¹³ is -N=S(=O)-(R¹³)₂, wherein two R¹³ are alternatively combined with the sulfur atom to which they are attached to form a saturated or partially saturated 3-, 4-, 5-, or 6-membered monocyclic ring comprising 0, 1, 2, or 3 N atoms and 0, 1, or 2 atoms selected from O and S;
R³ is selected from halogen, hydroxyl, cyano, amino, C₁₋₆ alkyl, C₁₋₆ alkoxy, C₁₋₆ haloalkyl, -OC₁₋₆ alkyl, and -OC₁₋₆ haloalkyl;
m is selected from 0, 1, 2, 3, and 4;
R⁴ is selected from hydrogen, halogen, cyano, C₁₋₈ alkyl, -OC₁₋₈ alkyl, C₁₋₄ haloalkyl, -OR^{4a}, -OR^{4b}, C₀₋₆ alkyl-(C=O)-N-(C₀₋₆ alkyl)₂, -C₀₋₆ alkyl-SO₂N-(C₀₋₆ alkyl)₂, R^{4a}, and R^{4b};
R⁵ is selected from hydrogen, halogen, cyano, C₁₋₆ alkyl, C₁₋₄ haloalkyl, C₃₋₈ cycloalkyl, C3-8 halocycloalkyl, -O-C₁₋₈ alkyl, and -O-R^{5a}, wherein R^{5a} is a saturated, partially saturated, or unsaturated 3-, 4-, 5-, 6-, or 7-membered monocyclic ring comprising 0, 1, 2, or 3 N atoms and 0, 1, or 2 atoms selected from O and S; or
alternatively, R² and R⁵ may be combined with the carbon atom to which they are each attached to form a saturated or partially saturated 5- or 6-membered monocyclic ring fused to the phenyl ring, wherein the 5- or 6-membered monocyclic ring comprises 0, 1, 2, or 3 N atoms and 0 or 1 atom selected from O and S, and further wherein the 5- or 6-membered monocyclic ring is substituted with 0, 1, 2, or 3 groups selected from: halogen, C₁₋₆ alkyl, C₁₋₄ haloalkyl, -OR^{a}, -OC₁₋₄ haloalkyl, cyano, -NR^{a}R^{a}, and oxo;
R⁷ is selected from hydrogen, halogen, cyano, C₁₋₈ alkyl, C₃₋₈ cycloalkyl, C₃₋₈ halocycloalkyl, C₁₋₄ haloalkyl, -O-C₁₋₈ alkyl, and R^{7a}; or alternatively, R² and R⁷ can be combined with the carbon atom to which they are each attached to form a saturated or partially saturated 5- or 6-membered monocyclic ring fused to the phenyl ring, wherein the 5- or 6-membered monocyclic ring comprises 0, 1, 2, or 3 N atoms and 0 or 1 atom selected from O and S, and further wherein the 5- or 6-membered monocyclic ring is substituted with 0, 1, 2, or 3 groups selected from: halogen, C₁₋₆ alkyl, C₁₋₄ haloalkyl, -OR^{a}, -OC₁₋₄ haloalkyl, CN, -NR^{a}R^{a}, and oxo;
R⁶, R⁸, and R⁹ are each independently selected from hydrogen, halogen, cyano, C₁₋₈ alkyl, -O-C₁₋₈ alkyl, C₃₋₈ cycloalkyl, C₃₋₈ halocycloalkyl, and C₁₋₄ haloalkyl;
R¹⁰ is hydrogen, halogen, hydroxyl, cyano, C₁₋₈ alkyl, C₃₋₈ cycloalkyl, C₃₋₈ halocycloalkyl, C₁₋₄ haloalkyl, -O-R^{10a}, or -O-R^{10b};
R¹¹ is hydrogen, R^{11a}, or R^{11b};
R¹² is hydrogen, halogen, hydroxyl, cyano, R^{12a}, or R^{12b};
R¹³ is hydrogen, halogen, cyano, R^{13a}, or R^{13b};
R^{4a}, R^{7a}, R^{10a}, R^{11a}, R^{12a}, and R^{13a} are each independently selected from the group consisting of: a saturated, partially saturated, or unsaturated 3-, 4-, 5-, 6-, or 7-membered monocyclic ring or 4-, 5-, 6-, 7-, 8-, 9-, 10-, 11-, or 12-membered bicyclic ring comprising 0, 1, 2, or 3 N atoms and 0, 1, or 2 atoms selected from O and S, wherein the monocyclic or bicyclic ring is substituted with 0, 1, 2, or 3 groups selected from: halogen, cyano, amino, C₁₋₆ alkyl, C₁₋₆ haloalkyl, -OR^{a}, -OC₁₋₆ haloalkyl, -C(=O)R^{b}, -C(=O)OR^{a}, -C(=O)NR^{a}R^{a}, -C(=NR^{a})NR^{a}R^{a}, -OC(=O)R^{b}, - OC(=O)NR^{a}R^{a}, -OC₂₋₆ alkyl NR^{a}R^{a}, -OC₂₋₆ alkyl OR^{a}, -SR^{a}, -S(=O)R^{b}, -S(=O)₂R^{b}, -S(=O)₂NR^{a}R^{a}, -NR^{a}R^{a}, - N(R^{a})C(=O)R^{b}, -N(R^{a})C(=O)OR^{b}, -N(R^{a})C(=O)NR^{a}R^{a}, -N(R^{a})C(=NR^{a})NR^{a}R^{a}, -N(R^{a})S(=O)₂R^{b}, - N(R^{a})S(=O)₂NR^{a}R^{a}, -NR^{a}C₂₋₆ alkyl NR^{a}R^{a}, -NR^{a}C₂₋₆ alkyl OR^{a}, -C₁₋₆ alkyl NR^{a}R^{a}, -C₁₋₆ alkyl OR^{a}, -C₁₋₆ alkyl N(R^{a})C(=O)R^{b}, -C₁₋₆ alkyl OC(=O)R^{b}, -C₁₋₆ alkyl C(=O)NR^{a}R^{a}, -C₁₋₆ alkyl C(=O)OR^{a}, R¹⁴, and oxo;
R^{4b}, R^{10b}, R^{11b}, R^{12b}, and R^{13b} are each independently selected from the group consisting of: C₁₋₆ alkyl substituted with 0, 1, 2, 3, 4, or 5 groups selected from the group consisting of: halogen, cyano, C₁₋₆ alkyl, C₁₋₆ haloalkyl, -C₀₋₆NR^{a}R^{a}, -OR^{a}, -C(=O)OR^{a}, and a saturated, partially saturated, or unsaturated 3-, 4-, 5-, or 6-membered monocyclic ring;
R¹⁴ is independently selected from the group consisting of: a saturated, partially saturated, or unsaturated 3-, 4-, 5-, 6-, or 7-membered monocyclic ring or 4-, 5-, 6-, 7-, 8-, 9-, 10-, 11-, or 12-membered bicyclic ring comprising 0, 1, 2, or 3 N atoms and 0, 1, or 2 atoms selected from O and S, wherein the monocyclic or bicyclic ring is substituted with 0, 1, 2, or 3 groups selected from: halogen, cyano, amino, C₁₋₆ alkyl, C₁₋₆ haloalkyl, -OR^{a}, -OC₁₋₆ haloalkyl,-C(=O)R^{b}, -C(=O)OR^{a}, -C(=O)NR^{a}R^{a}, -C(=NR^{a})NR^{a}R^{a}, -OC(=O)R^{b}, -OC(=O)NR^{a}R^{a}, -OC₂₋₆ alkyl NR^{a}R^{a}, -OC₂₋₆ alkyl OR^{a}, -SR^{a}, -S(=O)R^{b}, -S(=O)₂R^{b}, -S(=O)₂NR^{a}R^{a}, -NR^{a}R^{a}, -N(R^{a})C(=O)R^{b}, -N(R^{a})C(=O)OR^{b},-N(R^{a})C(=O)NR^{a}R^{a}, -N(R^{a})C(=NR^{a})NR^{a}R^{a}, -N(R^{a})S(=O)₂R^{b}, -N(R^{a})S(=O)₂NR^{a}R^{a}, -NR^{a}C₂₋₆ alkyl NR^{a}R^{a}, -NR^{a}C₂₋₆ alkyl OR^{a}, -C₁₋₆ alkyl NR^{a}R^{a}, -C₁₋₆ alkyl OR^{a}, -C₁₋₆ alkyl N(R^{a})C(=O)R^{b}, -C₁₋₆ alkyl OC(=O)R^{b}, -C₁₋₆ alkyl C(=O)NR^{a}R^{a}, -C₁₋₆ alkyl C(=O)OR^{a}, and oxo;
R^{x} is selected from the group consisting of: hydrogen, 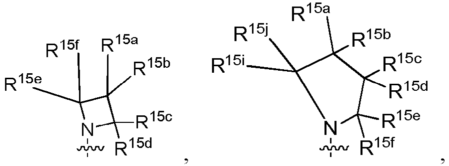 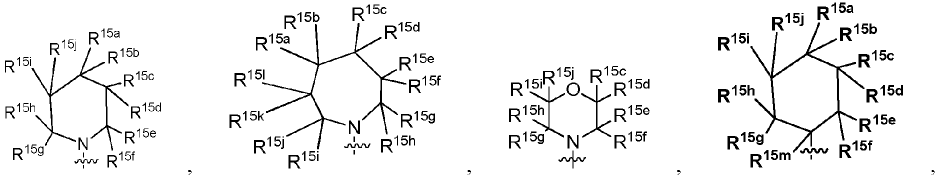 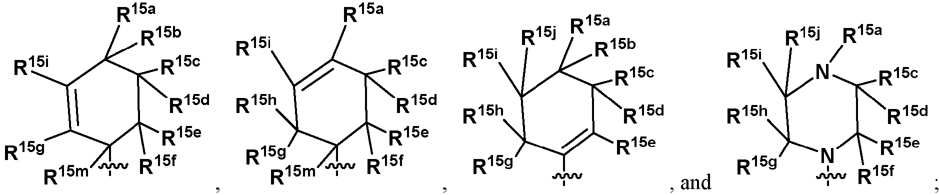 or
R^{x} is C₂₋₈ alkyl substituted with 0, 1, 2, 3, 4, or 5 groups selected from the group consisting of: halogen, cyano, amino, C₁₋₆ alkyl, C₁₋₆ haloalkyl, -OR^{a}, -NR^{a}R^{a}, -C(=O)OR^{a}, -OC₁₋₄ haloalkyl, and R¹⁵ⁿ; or
R^{x} is phenyl, or phenyl or an unsaturated 5-membered monocyclic ring substituted with 0, 1, 2, 3, 4, or 5 groups selected from the group consisting of: halogen, cyano, amino, C₁₋₆ alkyl, C₁₋₆ haloalkyl, -OR^{a}, -NR^{a}R^{a}, -C(=O)OR^{a}, -OC₁₋₄ haloalkyl, and R¹⁵ⁿ;
the 5-membered monocyclic ring comprises 0, 1, 2, or 3 N atoms and 0 or 1 atom selected from O and S;
R^{15a}, R^{15b}, R^{15c}, R^{15d}, R^{15e}, R^{15f}, R^{15g}, R^{15h}, R¹⁵ⁱ, R^{15j}, R^{15k}, R^{15l}, and R^{15m} are each independently hydrogen, halogen, R^{15o}, or R^{15p}; or
alternatively, each of the pairs of R^{15a} and R^{15b}, R^{15c} and R^{15d}, R^{15e} and R^{15f}, R^{15g} and R^{15h}, R¹⁵ⁱ and R^{15j}, and R^{15k} and R^{15l} can be independently combined with the carbon atom to which they are attached to form a saturated or partially saturated 3-, 4-, 5-, or 6-membered monocyclic ring spiro-attached to the R^{x} ring, wherein the 3-, 4-, 5-, or 6-membered monocyclic ring comprises 0, 1, 2, or 3 N atoms and 0, 1, or 2 atoms selected from O and S, and further wherein the 3-, 4-, 5-, or 6-membered monocyclic ring is substituted with 0, 1, 2, or 3 groups selected from: halogen, C₁₋₆ alkyl, C₁₋₄ haloalkyl, -OR^{a}, -OC₁₋₄ haloalkyl, CN, -NR^{a}R^{a}, and oxo; or
yet alternatively, each of the pairs of R^{15a} and R^{15b}, R^{15c} and R^{15d}, R^{15e} and R^{15f}, R^{15g} and R^{15h}, R¹⁵ⁱ and R^{15j}, and R^{15k} and R^{15l} may be independently combined to form a double bond;
R¹⁵ⁿ and R^{15o} may be independently selected from a saturated, partially saturated, or unsaturated 3-, 4-, 5-, 6-, or 7-membered monocyclic ring or 8-, 9-, 10-, 11-, or 12-membered bicyclic ring, wherein the monocyclic or bicyclic ring comprises 0, 1, 2, or 3 N atoms and 0, 1, or 2 atoms selected from O and S, and further wherein the monocyclic or bicyclic ring is substituted with 0, 1, 2, 3, or 4 groups selected from: halogen, C₁₋₆ alkyl, C₁₋₄ haloalkyl, -OR^{a}, -OC₁₋₄ haloalkyl, CN, -C(=O)OR^{a}, -C(=O)R^{b}, -C(=O)NR^{a}R^{a}, -C(=NR^{a})NR^{a}R^{a}, -OC(=O)R^{b}, -OC(=O)NR^{a}R^{a}, -OC₂₋₆ alkyl NR^{a}R^{a}, -NR^{a}R^{a}, -OC₂₋₆OR^{a}, -SR^{a}, -S(=O)R^{b}, -S(=O)₂R^{b}, -S(=O)₂NR^{a}R^{a}, -NR^{a}C(=O)R^{b}, -NR^{a}C(=O)OR^{b},-NR^{a}C(=O)NR^{a}R^{a}, -NR^{a}C(=NR^{a})NR^{a}R^{a}, -NR^{a}S(=O)₂NR^{b}, -NR^{a}S(=O)₂NR^{a}R^{a}, -R^{a}C₂₋₆ alkyl NR^{a}R^{a}, -NR^{a}C₂₋₆ alkyl OR^{a}, -C₁₋₆ alkyl NR^{a}R^{a}, -C₁₋₆ alkyl OR^{a}, -C₁₋₆ alkyl OC(=O)R^{b}, -C₁₋₆ alkyl NR^{a}C(=O)R^{b}, -C₁₋₆ alkyl NR^{a}C(=O)NR^{a}R^{a}, -C₁₋₆ alkyl-C(=O)OR^{a}, and oxo;
R^{15p} may be independently selected from C₁₋₈ alkyl substituted with 0, 1, 2, 3, 4, or 5 groups selected from halogen, C₁₋₄ haloalkyl, CN, -C(=O)OR^{a}, -OR^{a}, -OC₁₋₄ haloalkyl, and -NR^{a}R^{a};
R^{a} is independently selected from H and R^{b};
R^{b} is independently selected from C₁₋₆ alkyl, phenyl, and benzyl, wherein the C₁₋₆ alkyl is substituted with 0, 1, 2, or 3 substituents selected from: halogen, hydroxyl, -OC₁₋₆ alkyl, -NH₂, -NHC₁₋₆ alkyl, -OC(=O)C₁₋₆ alkyl, and -N(C₁₋₆ alkyl)C₁₋₆ alkyl; and the phenyl or benzyl is substituted with 0, 1, 2, or 3 substituents selected from: halogen, C₁₋₆ alkyl, C₁₋₄ haloalkyl, -OH, -OC₁₋₆ alkyl, -NH₂, -NHC₁₋₆ alkyl, -OC(=O)C₁₋₆ alkyl, and -N(C₁₋₆ alkyl)C₁₋₆ alkyl.

In one preferred embodiment of the present disclosure, the compound represented by formula V, or the tautomer, the stereoisomer, the hydrate, the solvate, the pharmaceutically acceptable salt, or the prodrug thereof has structural formula II-1: 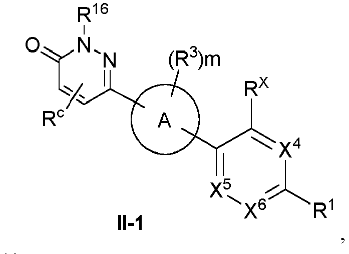 wherein X⁴, X⁵, X⁶, ring A, R¹, R³, R¹⁶, R^{c}, R^{x}, and m are as defined in the first aspect of the present disclosure.

In one preferred embodiment of the present disclosure, the compound represented by formula V, or the tautomer, the stereoisomer, the hydrate, the solvate, the pharmaceutically acceptable salt, or the prodrug thereof has structural formula I: 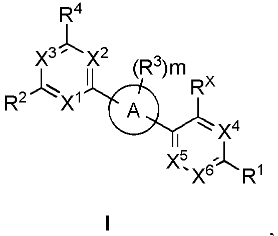 wherein X¹, X², X³, X⁴, X⁵, X⁶, ring A, R¹, R², R³, R⁴, R^{x}, and m are as defined in the first aspect of the present disclosure.

In one preferred embodiment of the present disclosure, ring A is 5- to 6-membered heteroaryl comprising 1-3 heteroatoms;
m is 0, 1, 2, 3, or 4;
R³ is selected from halogen, C₁-C₆ alkyl, and C₁-C₆ haloalkyl;
preferably, the heteroatom is selected from N, O, and S; when a plurality of heteroatoms are present, the heteroatoms are identical or different;
preferably, ring A is selected from pyrrole, pyrazole, imidazole, triazole, furan, oxazole, oxadiazole, thiophene, thiazole, thiadiazole, pyridine, pyrimidine, pyridazine, pyrazine, and triazine;
more preferably, ring A is selected from pyrazole, oxazole, oxadiazole, thiazole, thiadiazole, and triazole.

In one preferred embodiment of the present disclosure, R^{x} is selected from 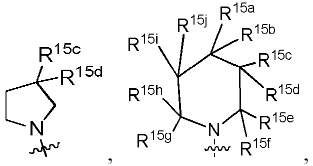 and 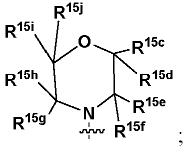 **the** R^{15a}, R^{15b}, R^{15c}, R^{15d}, R^{15e}, R^{15f}, R^{15g}, R^{15h}, R¹⁵ⁱ, and R^{15j} are each independently hydrogen, halogen, C₁₋₆ alkyl, or C₁₋₄ haloalkyl; or R^{15a} and R^{15b} are combined with the carbon atom to which they are attached to form a saturated 3-, 4-, or 5-membered monocyclic ring spiro-attached to the R^{x} ring, wherein the monocyclic ring comprises 0, 1, 2, or 3 N atoms and 0, 1, or 2 atoms selected from O and S; or yet alternatively, each of the pairs of R^{15a} and R^{15b}, R^{15c} and R^{15d}, R^{15e} and R^{15f}, R^{15g} and R^{15h}, R¹⁵ⁱ and R^{15j}, and R^{15k} and R^{15l} may be independently combined to form a double bond; the R^{15a}, R^{15b}, R^{15c}, R^{15d}, R^{15e}, R^{15f}, R^{15g}, R^{15h}, R¹⁵ⁱ, R^{15j}, R^{15k}, R^{15l}, and R^{15m} are each independently selected from hydrogen, halogen, and R^{15p}.

In one preferred embodiment of the present disclosure, R^{x} is selected from 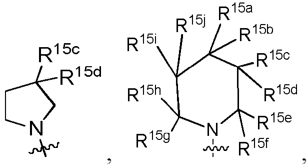 and 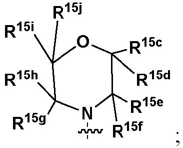 **the** R^{15a}, R^{15b}, R^{15c}, R^{15d}, R^{15e}, R^{15f}, R^{15g}, R^{15h}, R¹⁵ⁱ, and R^{15j} are each independently hydrogen, halogen, C₁₋₆ alkyl, or C₁₋₄ haloalkyl; and R^{15a} and R^{15b} are combined with the carbon atom to which they are attached to form a saturated 3-, 4-, or 5-membered monocyclic ring spiro-attached to the R^{x} ring, wherein the monocyclic ring comprises 0, 1, 2, or 3 N atoms and 0, 1, or 2 atoms selected from O and S.

In one preferred embodiment of the present disclosure, R^{15c}, R^{15d}, R¹⁵ⁱ, and R^{15j} are each independently hydrogen, methyl, or ethyl; and R^{15a} and R^{15b} are combined with the carbon atom to which they are attached to form a cyclopropyl, cyclobutyl, or cyclopentyl ring spiro-attached to the R^{x} ring.

In one preferred embodiment of the present disclosure, the 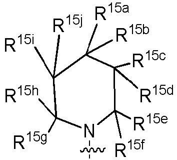 group is selected from 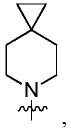 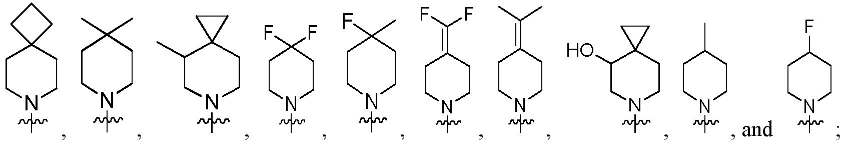 preferably, the 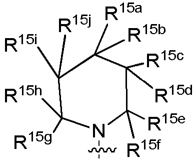 group is selected from 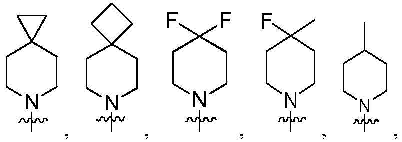 and

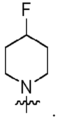

In one preferred embodiment of the present disclosure, the 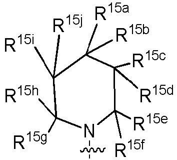 group is selected from 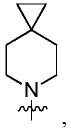 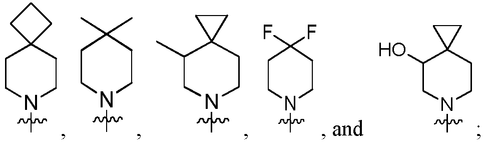 preferably, the 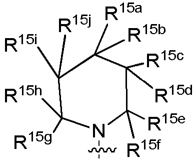 group is selected from

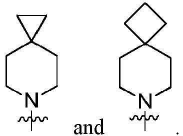

In one preferred embodiment of the present disclosure, the 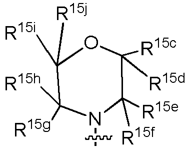 group is selected from

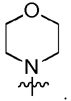

In one preferred embodiment of the present disclosure, the compound is selected from the following structure: 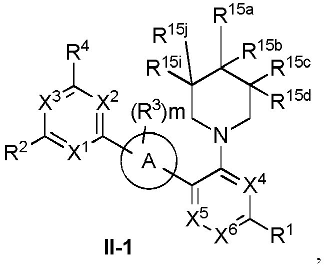
wherein X¹, X², X³, X⁴, X⁵, and X⁶ are as defined in the first aspect of the present disclosure;
R¹, R², R³, R⁴, and m are as defined in the first aspect of the present disclosure;
R^{15a}, R^{15b}, R^{15c}, R^{15d}, R¹⁵ⁱ, and R^{15j} are as defined in the first aspect of the present disclosure;
ring A is as defined in the first aspect of the present disclosure.

In one preferred embodiment of the present disclosure, the compound is selected from the following structure: 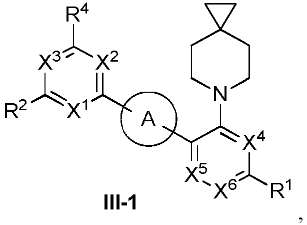
wherein X¹, X², X³, X⁴, X⁵, and X⁶ are as defined in the first aspect of the present disclosure;
R¹, R², and R⁴ are as defined in the first aspect of the present disclosure;
ring A is selected from pyrazole, oxazole, oxadiazole, thiazole, thiadiazole, and triazole.

In one preferred embodiment of the present disclosure, the compound is selected from the following structure: 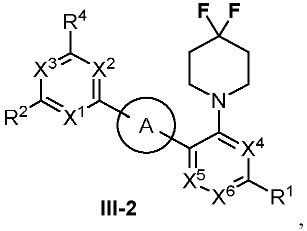
wherein X¹, X², X³, X⁴, X⁵, and X⁶ are as defined in the first aspect of the present disclosure;
R¹, R², and R⁴ are as defined in the first aspect of the present disclosure;
ring A is selected from pyrazole, oxazole, oxadiazole, thiazole, thiadiazole, and triazole.

In one preferred embodiment of the present disclosure, the compound is selected from the following structure: 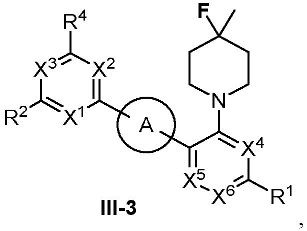
wherein X¹, X², X³, X⁴, X⁵, and X⁶ are as defined in the first aspect of the present disclosure;
R¹, R², and R⁴ are as defined in the first aspect of the present disclosure;
ring A is selected from pyrazole, oxazole, oxadiazole, thiazole, thiadiazole, and triazole.

In one preferred embodiment of the present disclosure, the compound is selected from the following structure: 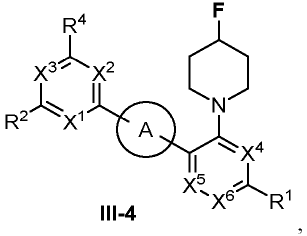
wherein X¹, X², X³, X⁴, X⁵, and X⁶ are as defined in the first aspect of the present disclosure;
R¹, R², and R⁴ are as defined in the first aspect of the present disclosure;
ring A is selected from pyrazole, oxazole, oxadiazole, thiazole, thiadiazole, and triazole.

In one preferred embodiment of the present disclosure, the compound is selected from the following structure: 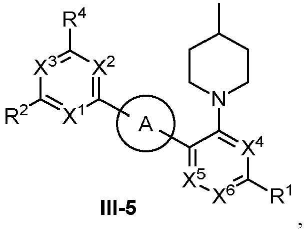
wherein X¹, X², X³, X⁴, X⁵, and X⁶ are as defined in the first aspect of the present disclosure;
R¹, R², and R⁴ are as defined in the first aspect of the present disclosure;
ring A is selected from pyrazole, oxazole, oxadiazole, thiazole, thiadiazole, and triazole.

In one preferred embodiment of the present disclosure, R¹ is -ZR¹²,
wherein Z is selected from -C₀₋₆ alkyl-NH-C₀₋₆ alkyl-, -C₀₋₆ alkyl-NHSO₂-C₀₋₆ alkyl-, -C₀₋₆ alkyl-SO₂NH-C₀₋₆ alkyl-, -C₀₋₆ alkyl-S(=O)(=NH)-C₀₋₆ alkyl-, and -C₀₋₆ alkyl-SO₂-C₀₋₆ alkyl-;
R¹² is halogen, hydroxyl, R^{12a}, or R^{12b},
wherein the R^{12a} is cyclopropyl or epoxybutane substituted with 0, 1, 2, or 3 groups selected from: fluoro, C₁₋₆ alkyl, C₁₋₆ haloalkyl, -OH, -OC₁₋₆ haloalkyl, and -C₁₋₆ alkyl-OH;
the R^{12b} is C₁₋₆ alkyl substituted with 0, 1, 2, 3, 4, or 5 groups selected from the group consisting of: hydroxyl, fluoro, and methyl;
preferably, Z is selected from -NH-C₀₋₆ alkyl-, -NHSO₂-C₀₋₆ alkyl-, -SO₂NH-C₀₋₆ alkyl-, -S(=O)(=NH)-C₀₋₆ alkyl-,-SO₂-C₀₋₆ alkyl-, and -CH₂-SO₂-C₀₋₆ alkyl-.

In one preferred embodiment of the present disclosure, the Z is a bond, -NH-, -NHSO₂-, -SO₂NH-, -S(=O)(=NH)-,-S-, -S(=O)-, -SO₂-, -CH₂-SO₂-, -O-, -P-, -P(=O)CH₃-, -P(=O)₂, -(C=O)-, -(C=O)NH-, or -NH(C=O)-.

In one preferred embodiment of the present disclosure, the Z is a bond, -NH-, -NHSO₂-, -SO₂NH-, -S(=O)(=NH)-,-S-, -S(=O)-, -SO₂-, -CH₂-SO₂-, -O-, -P-, -P(=O)CH₃-, -P(=O)₂, -(C=O)-, -(C=O)NH-, or -NH(C=O)-;
preferably, the Z is -NH-, -NHSO₂-, -SO₂NH-, -S(=O)(=NH)-, -SO₂-, or -CH₂-SO₂-.

In one preferred embodiment of the present disclosure, the R¹² is selected from R^{12a} and R^{12b};
R^{12a} is selected from: a saturated, partially saturated, or unsaturated 3-, 4-, 5-, 6-, or 7-membered monocyclic ring comprising 0, 1, 2, or 3 N atoms and 0, 1, or 2 atoms selected from O and S, wherein the monocyclic ring is substituted with 0, 1, 2, or 3 groups selected from: halogen, hydroxyl, C₁₋₆ alkyl, and C₁₋₆ haloalkyl;
R^{12b} is C₁₋₆ alkyl unsubstituted or substituted with a substituent selected from: halogen, hydroxyl, C₁₋₆ alkyl, and a saturated, partially saturated, or unsaturated 3-, 4-, 5-, or 6-membered monocyclic ring, wherein preferably, the 3-, 4-, 5-, or 6-membered monocyclic ring is cyclopropyl, cyclobutyl, cyclopentyl, or cyclohexyl;
preferably, R¹² is selected from: C₁₋₆ alkyl, C₃₋₆ cycloalkyl, C₁₋₆ alkyl-C₃₋₆ cycloalkyl, 4- to 6-membered heterocycloalkyl, and C₁₋₆ alkyl-4- to 6-membered heterocycloalkyl,
wherein the C₁₋₆ alkyl, C₃₋₆ cycloalkyl, or 4- to 6-membered heterocycloalkyl is optionally substituted with a substituent selected from: F, Cl, hydroxyl, methyl, ethyl, propyl, and butyl;
more preferably, the R¹² is selected from -CH₂CH₂OH, *tert*-butyl, 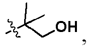 methyl, -CH₂F, 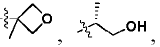 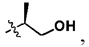 cyclopropyl, -CH₂-cyclopropyl, 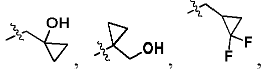 tetrahydrofuranyl, 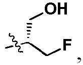 cyclopentyl, oxetanyl, azetidinyl, and 1,3,4-oxathiazinanyl.

In one preferred embodiment of the present disclosure, the compound is selected from the following structures: 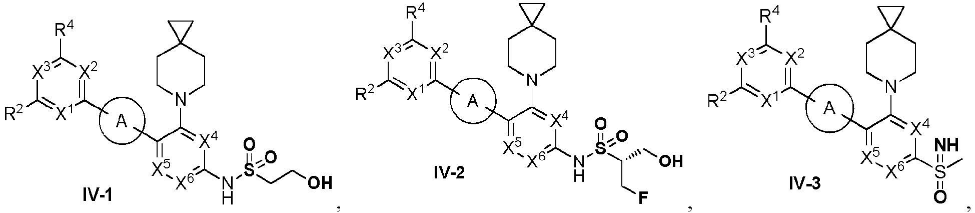 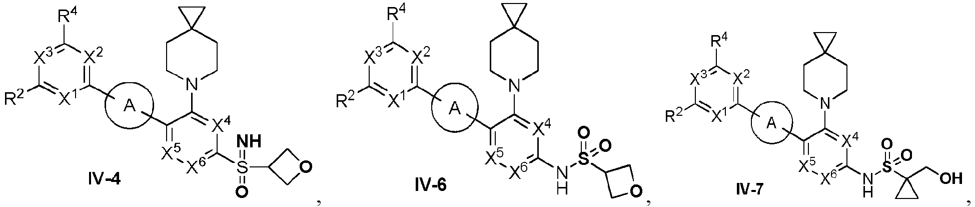 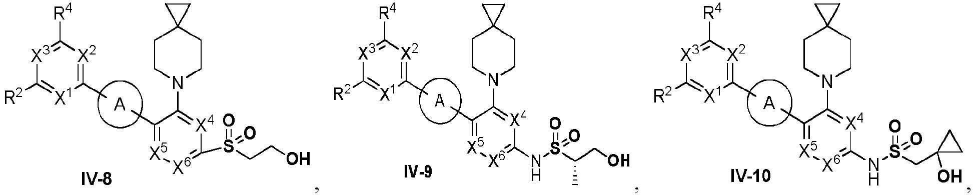 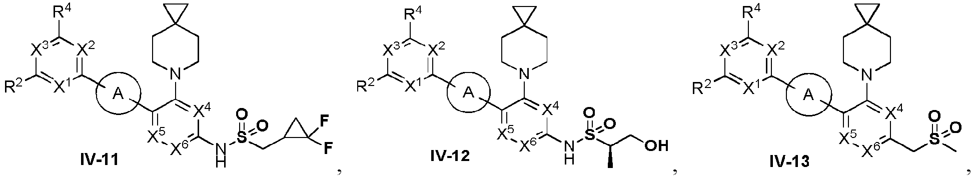 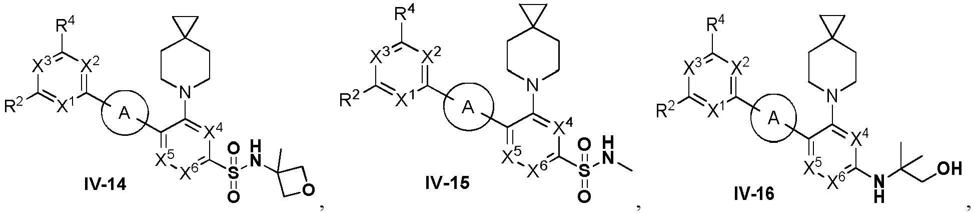 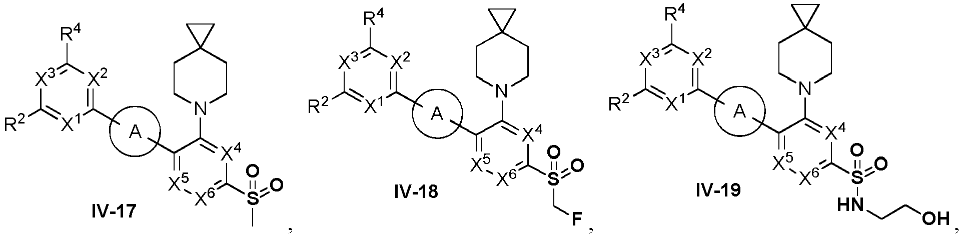 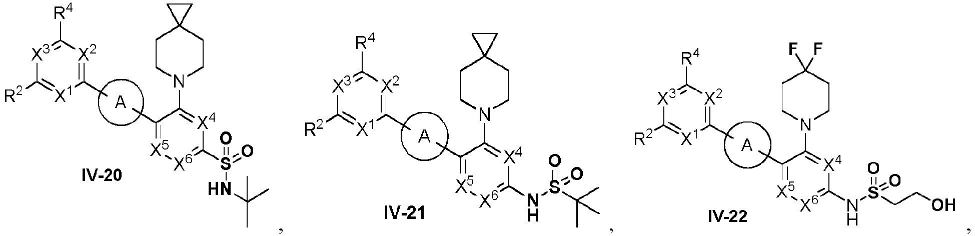 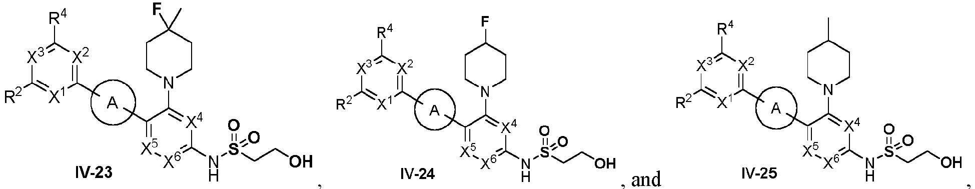
wherein X¹, X², X³, X⁴, X⁵, and X⁶ are as defined in the first aspect of the present disclosure;
R² and R⁴ are as defined in the first aspect of the present disclosure;
ring A is selected from pyrazole, oxazole, oxadiazole, thiazole, thiadiazole, and triazole.

In one preferred embodiment of the present disclosure, the compound is selected from the following structures: 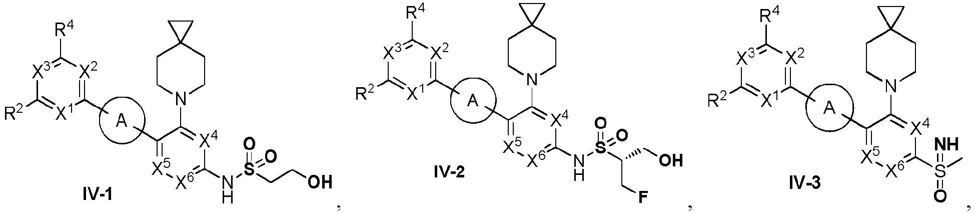 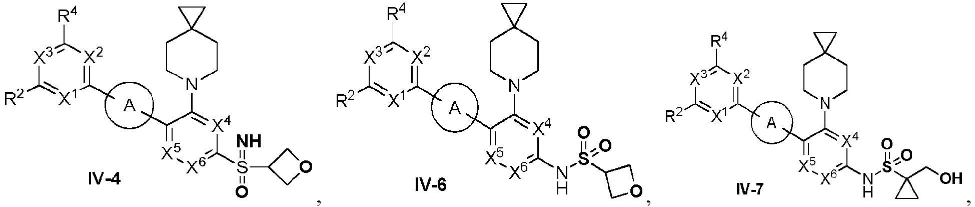 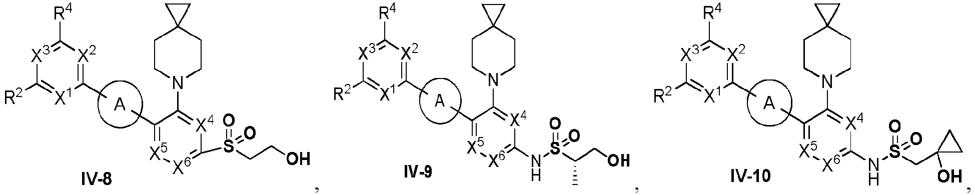 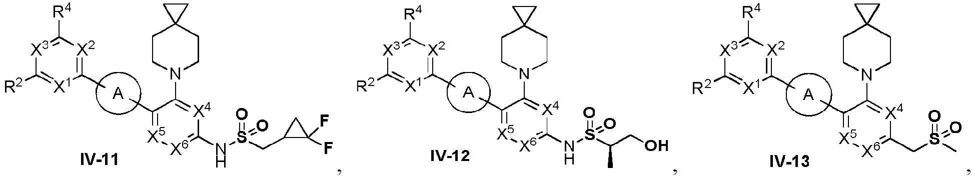 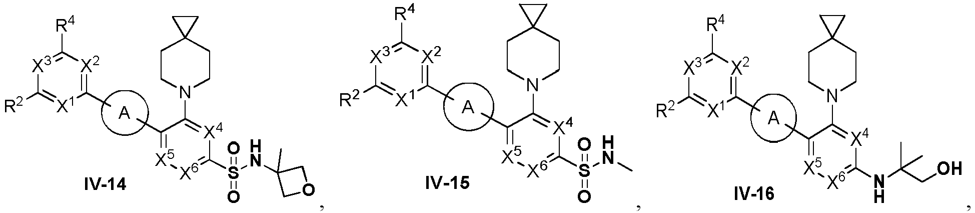 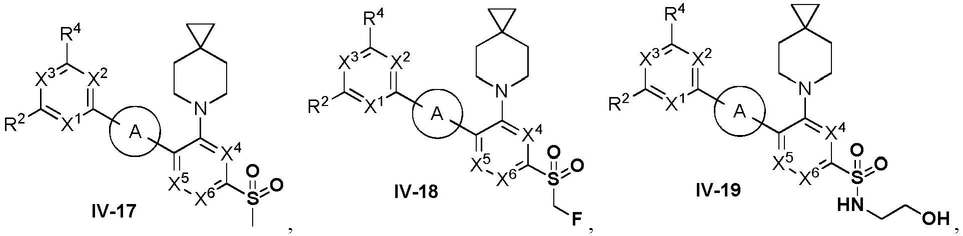 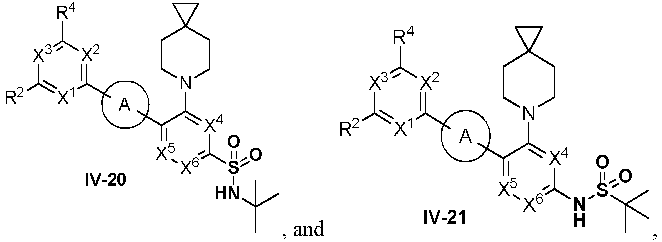
wherein X¹, X², X³, X⁴, X⁵, and X⁶ are as defined in the first aspect of the present disclosure;
R² and R⁴ are as defined in the first aspect of the present disclosure;
ring A is selected from pyrazole, oxazole, oxadiazole, thiazole, thiadiazole, and triazole.

In one preferred embodiment of the present disclosure, the R² is selected from F, Cl, Br, and a -Y-R¹³ group, wherein Y is a bond, -NH-, -NH-(CH₂)₀₋₄-, -O-(CH₂)₀₋₄-, or -SO₂NH-; and R¹³ is a saturated, partially saturated, or unsaturated 3-, 4-, 5-, 6-, or 7-membered monocyclic ring or 4-, 5-, 6-, 7-, 8-, 9-, 10-, 11-, or 12-membered bicyclic ring comprising 0, 1, 2, or 3 N atoms and 0 or 1 atom selected from O and S, wherein the monocyclic or bicyclic ring is substituted with 0, 1, 2, or 3 identical or different groups selected from: F, Cl, Br, C₁₋₆ alkyl, C₁₋₄ haloalkyl, -OH, - OC₁₋₄ haloalkyl, CN, R¹⁴, and oxo; or R¹³ is C₁₋₆ alkyl substituted with 0, 1, 2, 3, 4, or 5 identical or different substituents selected from F, Cl, Br, -OH, -OC₁₋₄ haloalkyl, and CN; R¹⁴ is as defined in the first aspect of the present disclosure;
preferably, Y is a bond, and R¹³ is a saturated 3-, 4-, 5-, 6-, or 7-membered monocyclic ring or 4-, 5-, 6-, 7-, 8-, 9-, 10-, 11-, or 12-membered bicyclic ring comprising 0 or 1 N atom and 0 or 1 O atom, wherein the monocyclic or bicyclic ring is substituted with 0, 1, 2, or 3 identical or different groups selected from: F, Cl, Br, -C₁₋₃ alkyl, C₁₋₃ haloalkyl, -OH, CN, and -C₁₋₃ alkyl-OH,
preferably selected from F, Cl, Br, methyl, -OH, -OCH₃, CN, -CH₂OH, and trifluoromethyl; or
Y is -NH-, -NH-(CH₂)₀₋₄-, -O-(CH₂)₀₋₄-, or -SO₂NH-; R¹³ is a saturated 3-, 4-, 5-, 6-, or 7-membered monocyclic ring comprising 0 or 1 N atom and 0 or 1 O atom, wherein the monocyclic ring is substituted with 0, 1, 2, or 3 identical or different groups selected from: fluoro, methyl, trifluoromethyl, -CN, -OH, and CH₂OH; or
R¹³ is C₁₋₆ alkyl substituted with 0, 1, 2, 3, 4, or 5 identical or different substituents, wherein the substituent is -OH, methyl, or F.

In one preferred embodiment of the present disclosure, R² is selected from the following structures:

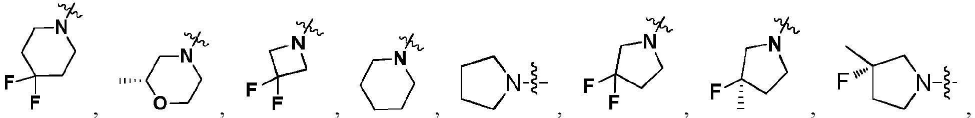

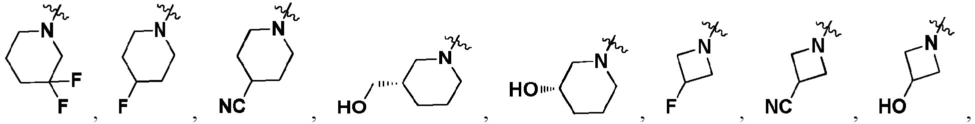

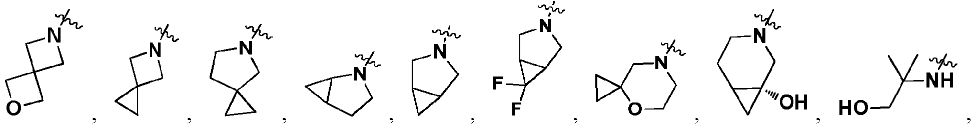

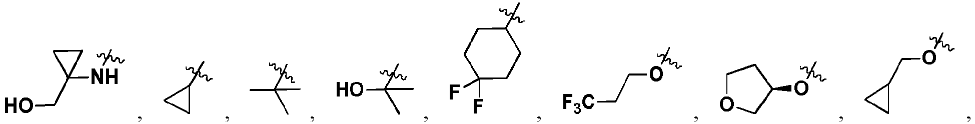

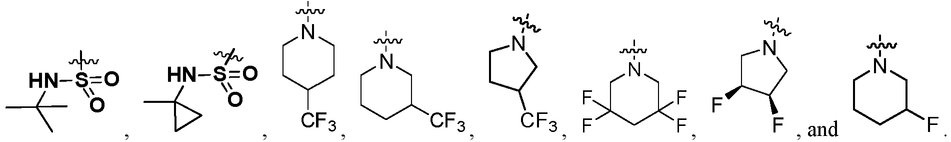

In one preferred embodiment of the present disclosure, R² is selected from the following structures:

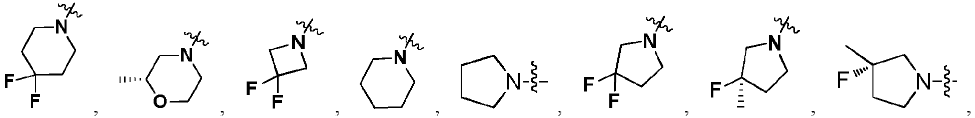

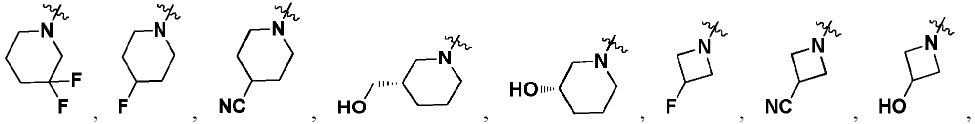

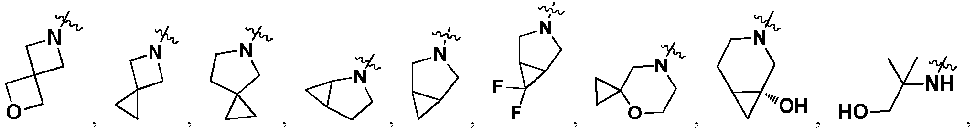

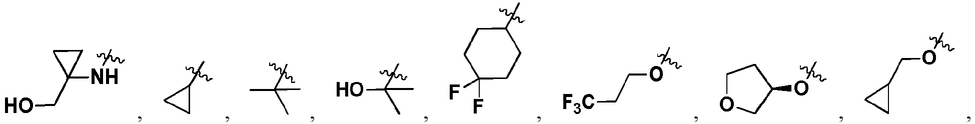

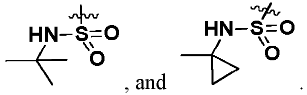

In one preferred embodiment of the present disclosure, the X¹ is N or -CR⁵;
X² is N or CR⁶;
X³ is N or CR⁷;
X⁴ is N or CR⁸;
X⁵ is N or CR⁹;
X⁶ is N or CR¹⁰;
X⁷ is N or CR⁴;
preferably, none, one, or two of the X¹, X², X³, and X⁷ are N;
preferably, none, one, or two of the X⁴, X⁵, and X⁶ are N.

In one preferred embodiment of the present disclosure, the X¹ is N or CR⁵;
X² is N or CR⁶;
X³ is N or CR⁷;
X⁴ is N or CR⁸;
X⁵ is N or CR⁹;
X⁶ is N or CR¹⁰;
preferably, none, one, or two of the X¹, X², and X³ are N;
preferably, none, one, or two of the X⁴, X⁵, and X⁶ are N.

In one preferred embodiment of the present disclosure, the X⁴ is N or CR⁸;
X⁵ is N or CR⁹;
X⁶ is N or CR¹⁰;
preferably, none, one, or two of the X⁴, X⁵, and X⁶ are N.

In one preferred embodiment of the present disclosure, the R¹⁶ is selected from a saturated, partially saturated, or unsaturated 3-, 4-, 5-, 6-, or 7-membered monocyclic ring or 4-, 5-, 6-, 7-, 8-, 9-, 10-, 11-, or 12-membered bicyclic ring comprising 0, 1, 2, or 3 N atoms and 0, 1, or 2 atoms selected from O and S, wherein the monocyclic or bicyclic ring is substituted with 0, 1, 2, or 3 groups selected from: halogen, cyano, amino, C₁₋₆ alkyl, C₁₋₆ haloalkyl, -OR^{a}, and -OC₁₋₆ haloalkyl;
preferably, the R¹⁶ is selected from a saturated, partially saturated, or unsaturated 3-, 4-, 5-, 6-, or 7-membered monocyclic ring or 4-, 5-, 6-, 7-, 8-, 9-, or 10-membered bicyclic ring comprising 0, 1, 2, or 3 N atoms and 0, 1, or 2 atoms selected from O and S, wherein the monocyclic or bicyclic ring is substituted with 0, 1, 2, or 3 groups selected from: halogen, cyano, amino, C₁₋₆ alkyl, C₁₋₆ haloalkyl, -OR^{a}, and -OC₁₋₆ haloalkyl;
preferably, the R¹⁶ is selected from cyclopropyl, cyclobutyl, cyclopentyl, and cyclohexyl, wherein the cyclopropyl, cyclobutyl, cyclopentyl, or cyclohexyl is substituted with 0, 1, 2, or 3 groups selected from: halogen, cyano, amino, hydroxyl, C₁₋₆ alkyl, C₁₋₆ haloalkyl, -OC₁₋₆ alkyl, and -OC₁₋₆ haloalkyl.

In one preferred embodiment of the present disclosure, ring B has a structure of 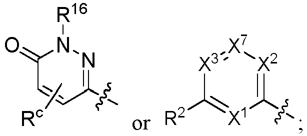 the 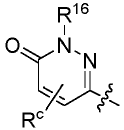 group has a structure of 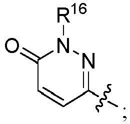 the R¹⁶ is selected from a saturated, partially saturated, or unsaturated 3-, 4-, 5-, 6-, or 7-membered monocyclic ring or 4-, 5-, 6-, 7-, 8-, 9-, 10-, 11-, or 12-membered bicyclic ring comprising 0, 1, 2, or 3 N atoms and 0, 1, or 2 atoms selected from O and S, wherein the monocyclic or bicyclic ring is substituted with 0, 1, 2, or 3 groups selected from: halogen, cyano, amino, C₁₋₆ alkyl, C₁₋₆ haloalkyl, -OR^{a}, and -OC₁₋₆ haloalkyl;
preferably, R¹⁶ is selected from a saturated 3-, 4-, 5-, 6-, or 7-membered monocyclic ring, wherein the monocyclic ring is substituted with 0, 1, 2, or 3 groups selected from: halogen, cyano, amino, hydroxyl, C₁₋₆ alkyl, C₁₋₆ haloalkyl, and -OC₁₋₆ haloalkyl;
preferably, the 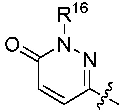 group has a structure of 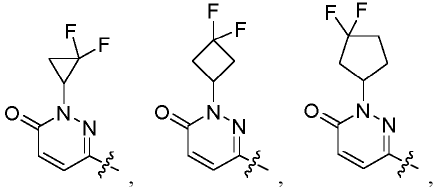 or 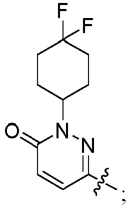 the 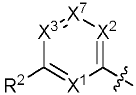 group has a structure of 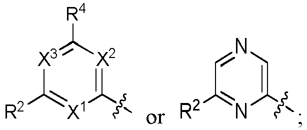
preferably, X¹ is N or CH, X² is N or CH, and X³ is N or CH;
preferably, none, one, or two of the X¹, X², X³, and X⁷ are N;
preferably, the 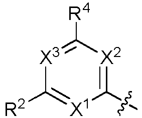 has a structure of

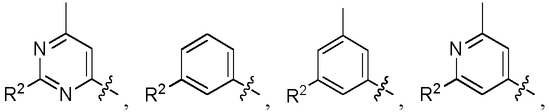

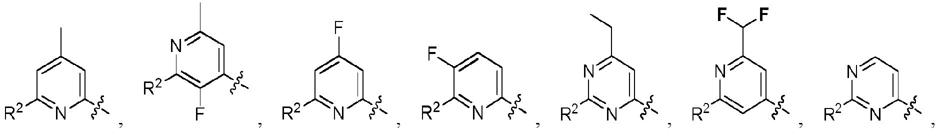

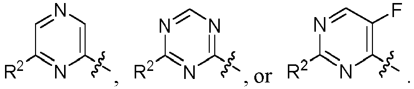

In one preferred embodiment of the present disclosure, the 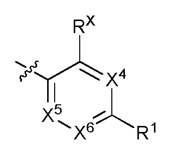 group is 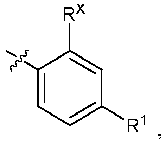 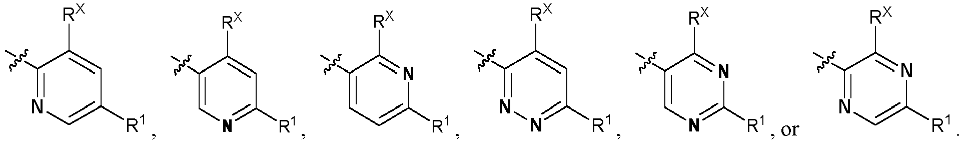
preferably, X¹ is N or CH, X² is N or CH, and X³ is N or CH;
preferably, none, one, or two of the X¹, X², X³, and X⁷ are N;
preferably, the 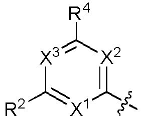 has a structure of

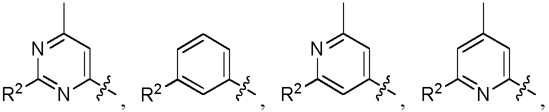

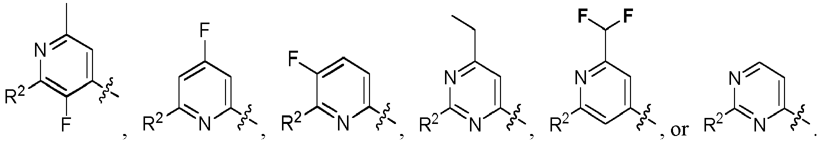

In one preferred embodiment of the present disclosure, the 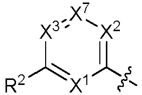 group has a structure of 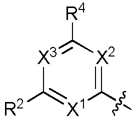 or 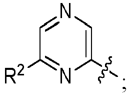 preferably, the 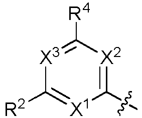 has a structure of

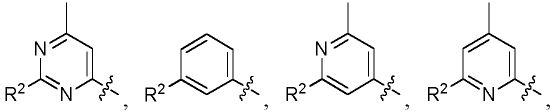

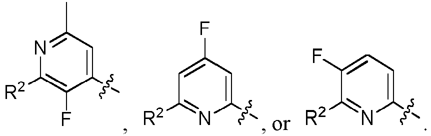

In one preferred embodiment of the present disclosure, R⁴ is selected from (a) hydrogen, F, Cl, Br, CN, -C₀₋₆ alkyl-SO₂N-(C₀₋₆ alkyl)₂, or -C₀₋₆ alkyl-(C=O)-N-(C₀₋₆ alkyl)₂; (b) C₁₋₆ alkyl or -OC₁₋₆ alkyl substituted with 0, 1, 2, or 3 OH groups; (c) 3- to 8-membered heterocycloalkyl comprising 0, 1, 2, or 3 N atoms and 0, 1, or 2 atoms selected from O and S; and (d) C₁₋₃ haloalkyl;
preferably, R⁴ is selected from hydrogen, methyl, ethyl, difluoromethyl, halogen, cyano, methoxy, cyclopropyl, cyclobutyl, -(C=O)NH₂, -CF₃, furanyl, pyridinyl, morpholinyl, -SO₂NHC(CH₃)₃, aziridinyl, and azetidinyl;
preferably, R⁴ is hydrogen, methyl, ethyl, fluoro, or difluoromethyl.

In one preferred embodiment of the present disclosure, R⁴ is selected from (a) hydrogen, F, Cl, Br, CN, -C₀₋₆ alkyl-SO₂N-(C₀₋₆ alkyl)₂, or -C₀₋₆ alkyl-(C=O)-N-(C₀₋₆ alkyl)₂; (b) C₁₋₆ alkyl or -OC₁₋₆ alkyl substituted with 0, 1, 2, or 3 OH groups; (c) 3- to 8-membered heterocycloalkyl comprising 0, 1, 2, or 3 N atoms and 0, 1, or 2 atoms selected from O and S; and (d) C₁₋₃ haloalkyl;
preferably, R⁴ is selected from hydrogen, methyl, halogen, cyano, methoxy, cyclopropyl, cyclobutyl, -(C=O)NH₂, - CF₃, furanyl, pyridinyl, morpholinyl, -SO₂NHC(CH₃)₃, aziridinyl, and azetidinyl.

In one preferred embodiment of the present disclosure, R⁵ is selected from hydrogen, halogen, methyl, halomethyl, methoxy, cyclopropyl, oxiranyl, and aziridinyl; preferably, R⁵ is selected from hydrogen and F.

In one preferred embodiment of the present disclosure, R⁶ is selected from hydrogen, halogen, C₁₋₃ alkyl, and C₁₋₄ haloalkyl; preferably, R⁶ is selected from hydrogen.

In one preferred embodiment of the present disclosure, R⁷ is selected from hydrogen, halogen, cyano, methoxy, cyclopropyl, C₁₋₃ alkyl, and C₁₋₄ haloalkyl.

In one preferred embodiment of the present disclosure, R⁸ is selected from hydrogen, halogen, cyclopropyl, - (C=O)CH₃, C₁₋₃ alkyl, and C₁₋₄ haloalkyl.

In one preferred embodiment of the present disclosure, R⁹ is selected from hydrogen, methyl, halogen, cyano, methoxy, and cyclopropyl; preferably, R⁹ is selected from hydrogen and F.

In one preferred embodiment of the present disclosure, R¹⁰ is selected from hydrogen, methyl, halogen, cyano, methoxy, and cyclopropyl; preferably, R¹⁰ is selected from hydrogen, F, and methyl.

In one preferred embodiment of the present disclosure, the compound comprises:

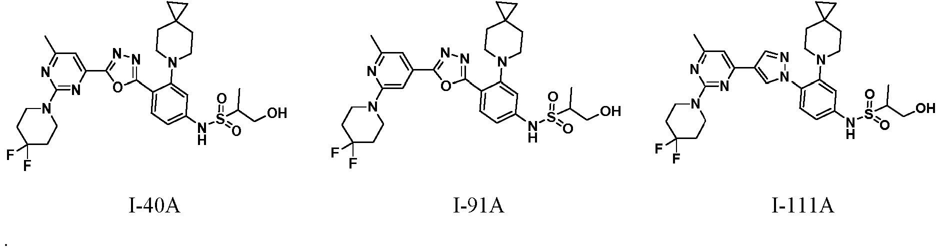

In one preferred embodiment of the present disclosure, the compound comprises:

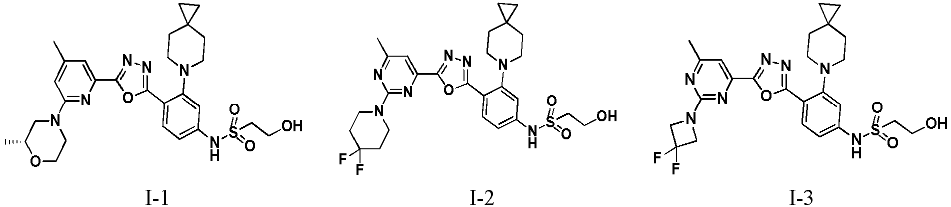

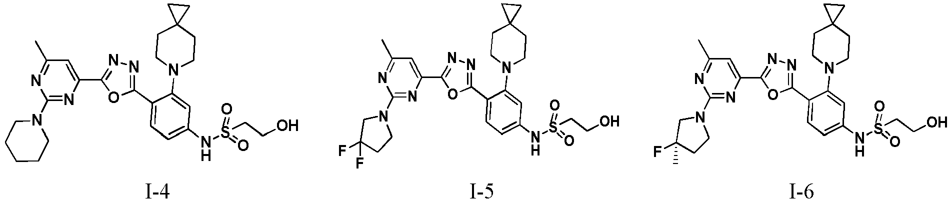

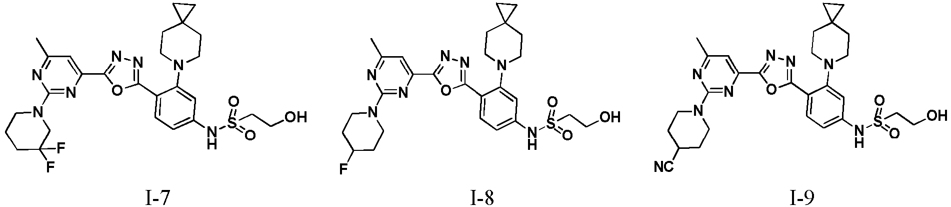

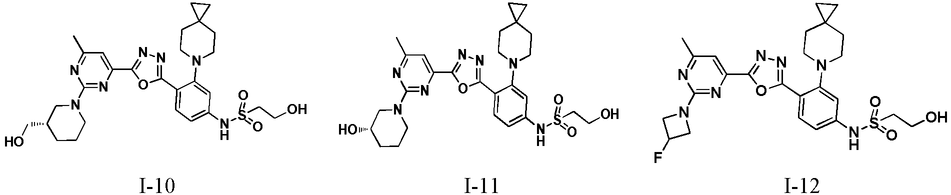

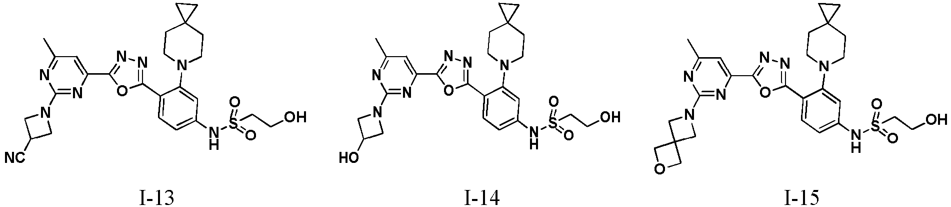

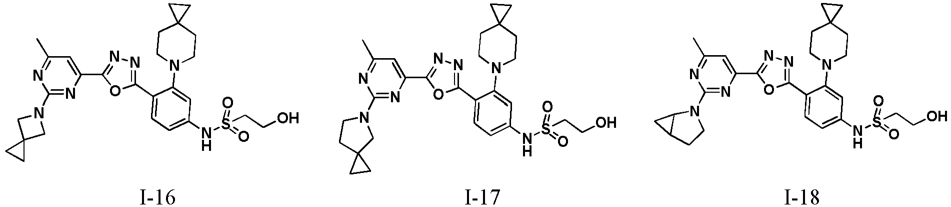

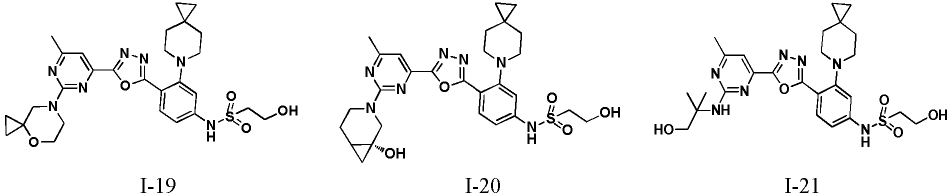

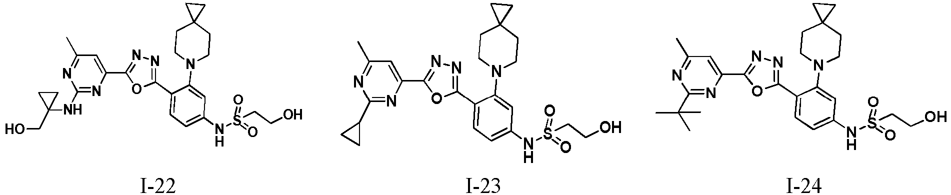

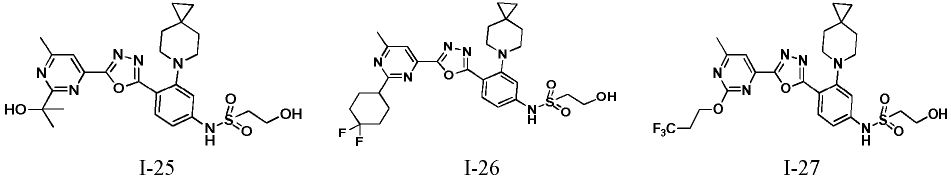

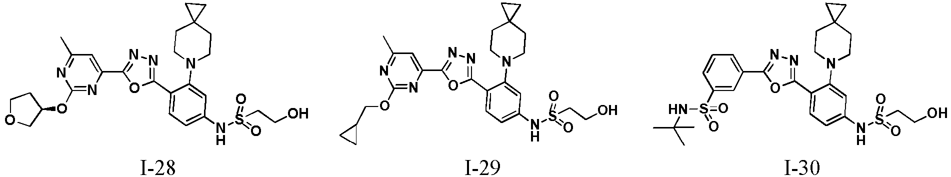

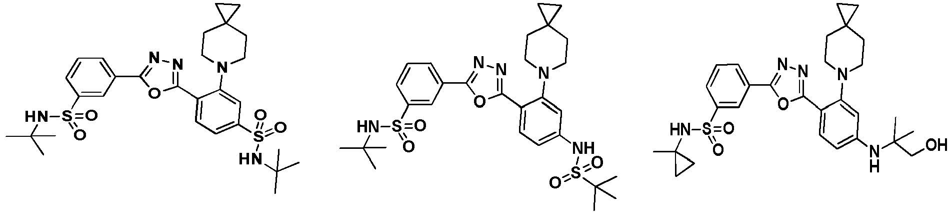

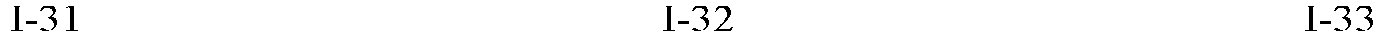

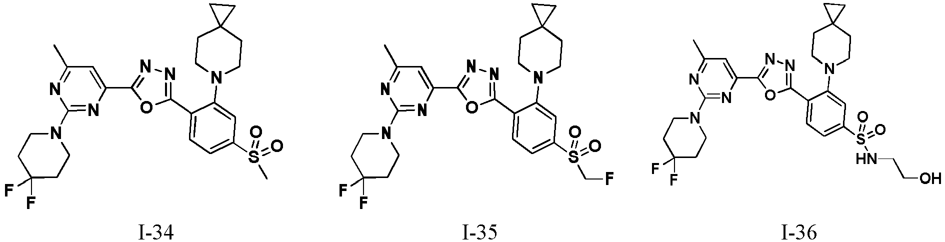

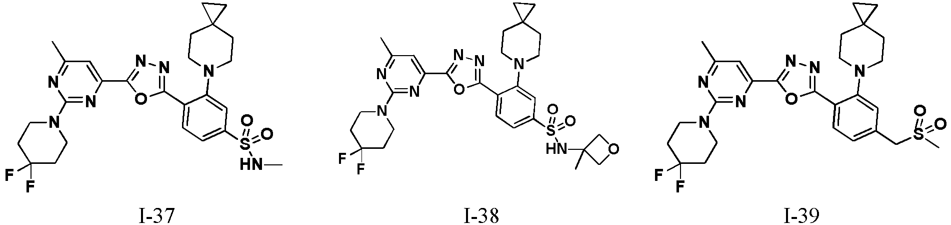

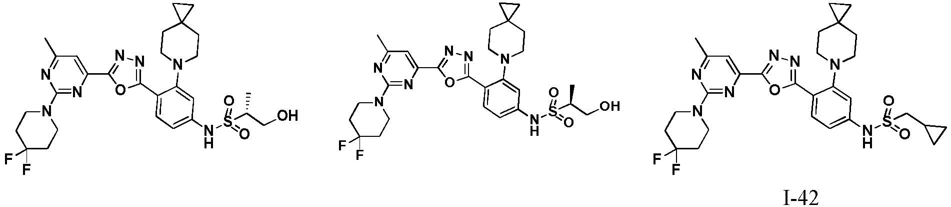

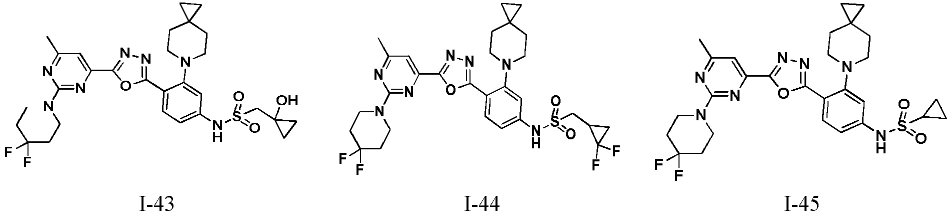

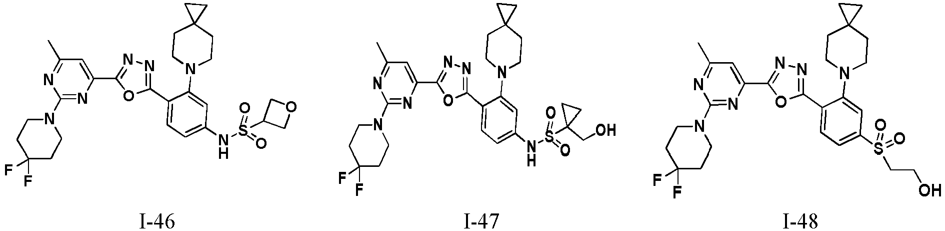

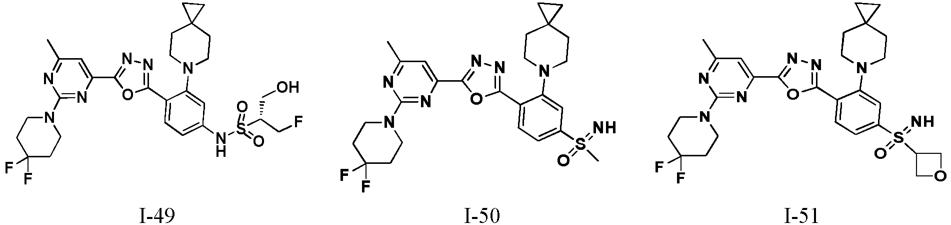

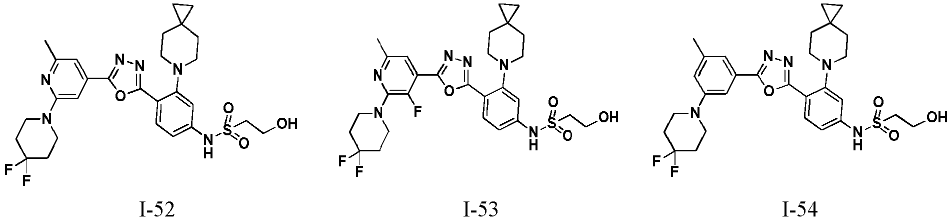

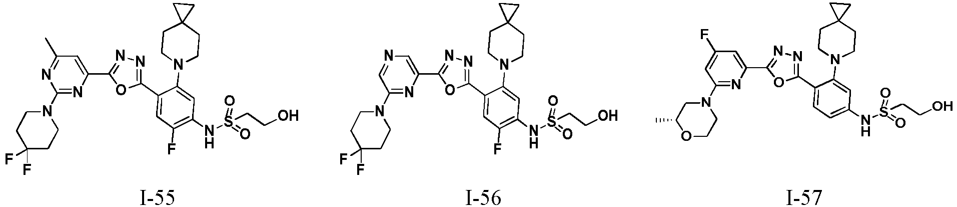

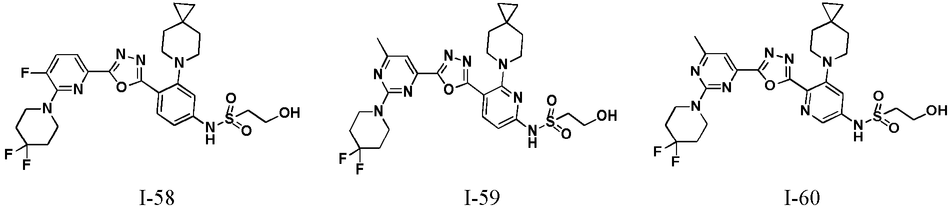

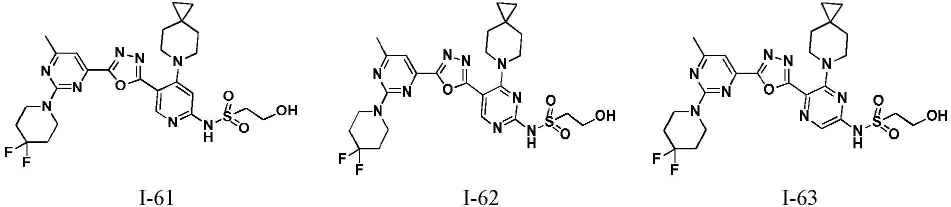

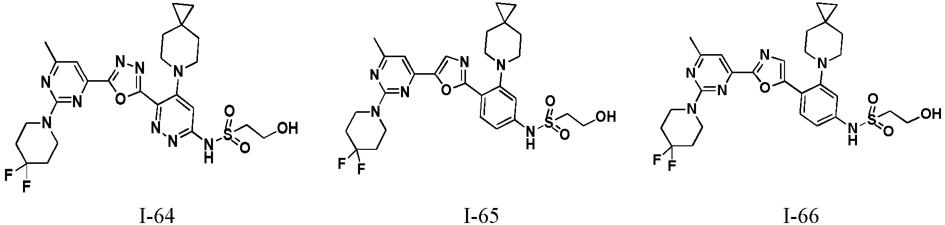

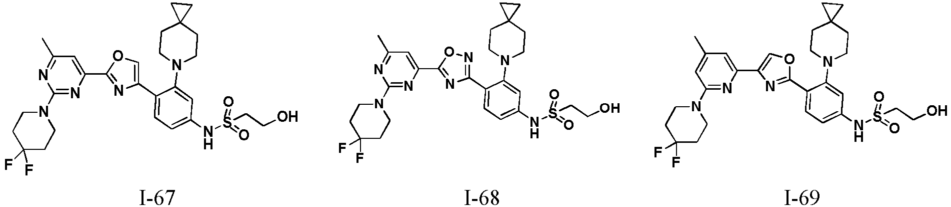

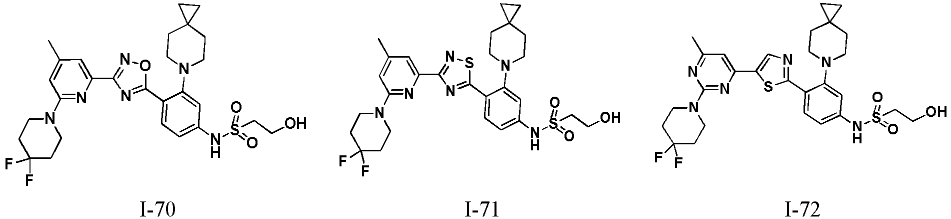

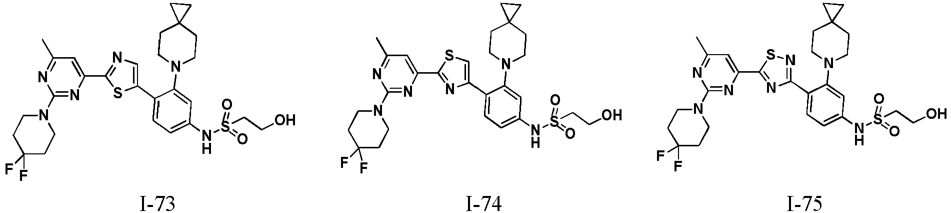

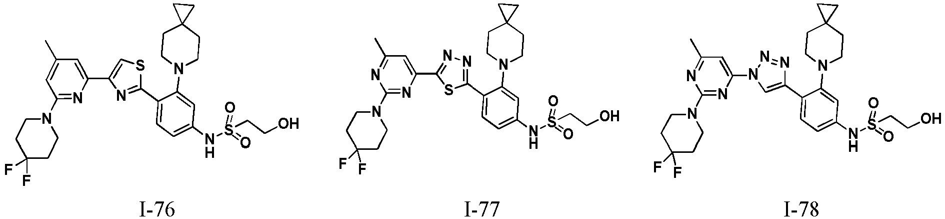

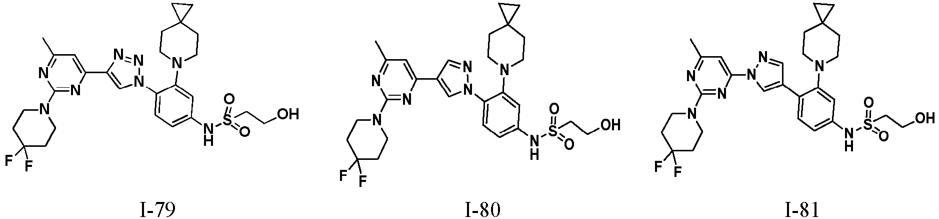

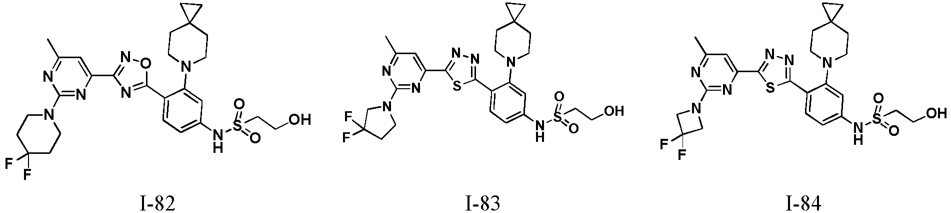

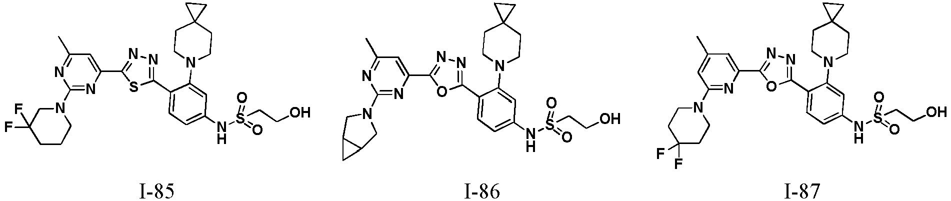

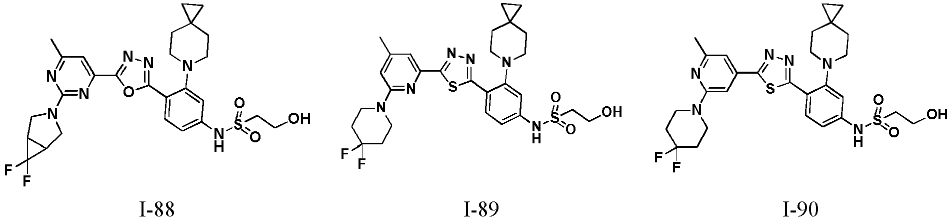

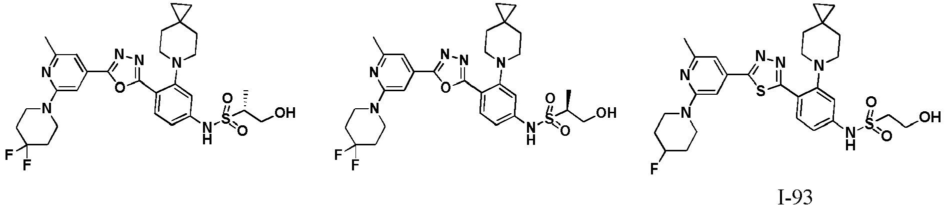

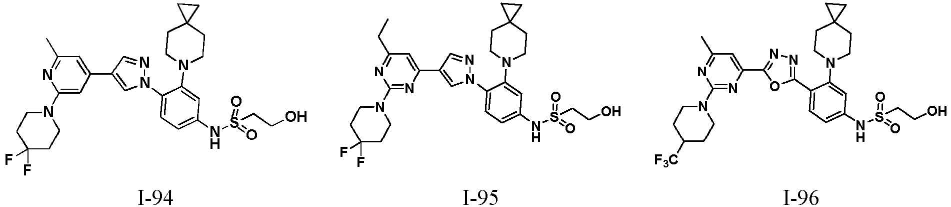

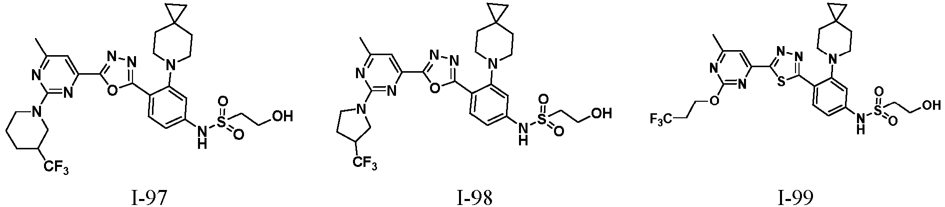

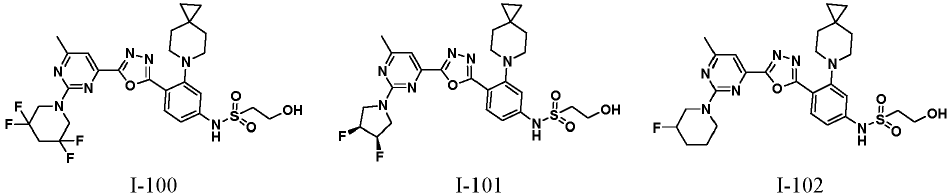

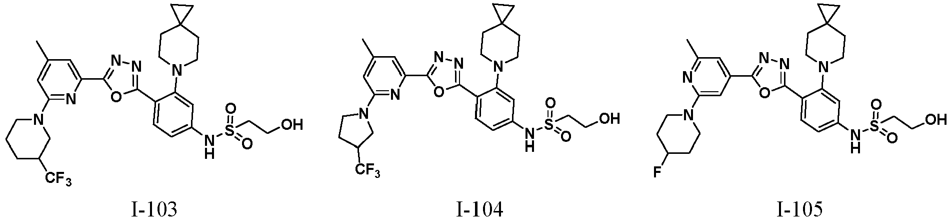

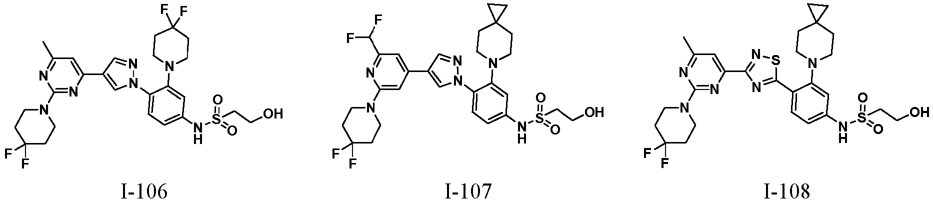

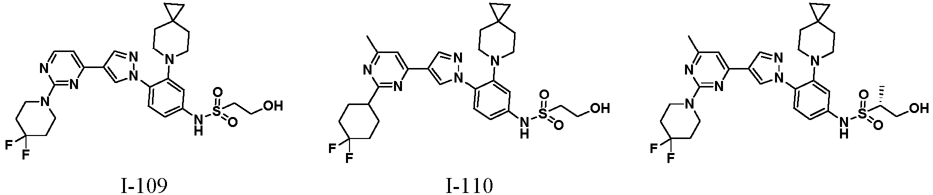

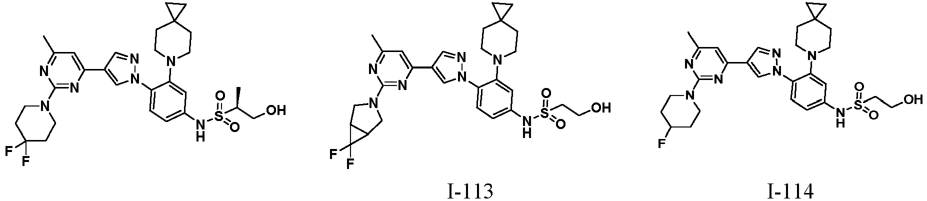

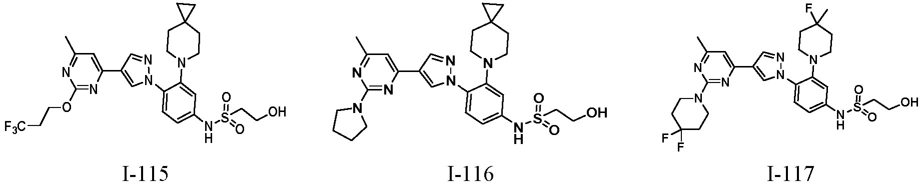

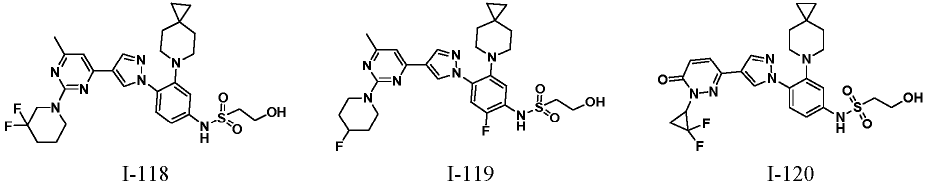

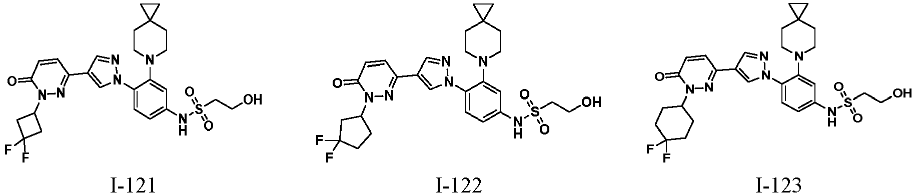

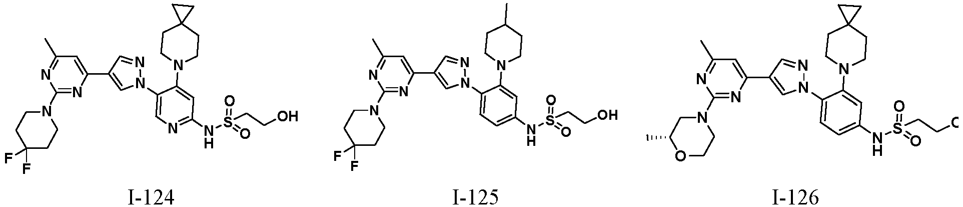

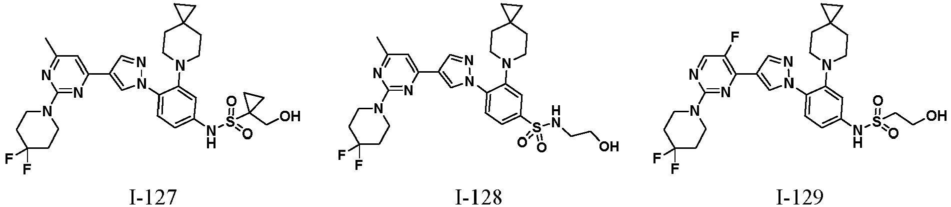

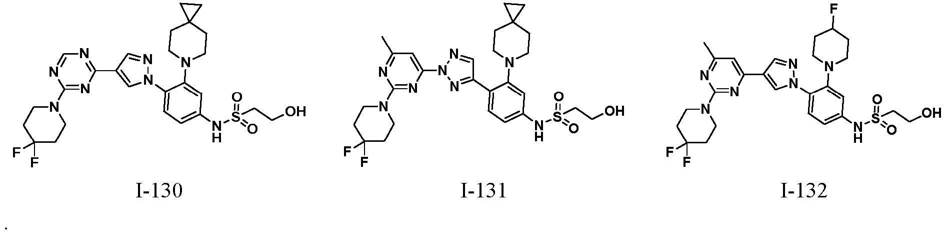

In a second aspect of the present disclosure, provided is a pharmaceutical composition comprising the compound represented by formula V, or the tautomer, the stereoisomer, the hydrate, the solvate, the pharmaceutically acceptable salt, or the prodrug thereof according to the first aspect, and a pharmaceutically acceptable carrier.

In a third aspect of the present disclosure, provided is use of the compound represented by formula V, or the tautomer, the stereoisomer, the hydrate, the solvate, the pharmaceutically acceptable salt, or the prodrug thereof according to the first aspect, or use of the pharmaceutical composition according to the second aspect, wherein the use comprises:
inhibiting KIF18A; and/or
preventing and/or treating a disease associated with KIF18A; and/or
manufacturing a medicament, a pharmaceutical composition, or a preparation for inhibiting KIF18A and/or for preventing and/or treating a disease associated with KIF18A.

Provided is the compound represented by formula V, or the tautomer, the stereoisomer, the hydrate, the solvate, the pharmaceutically acceptable salt, or the prodrug thereof according to the first aspect, or the pharmaceutical composition according to the second aspect, which is expected to be used for proliferative disorders of cancer, psoriasis, atopic dermatitis, autoimmune diseases, or inflammatory bowel diseases.

Cancers that should be mentioned include mesothelioma, neuroblastoma, rectal cancer, colon cancer, familial adenomatous polyposis and hereditary non-polyposis colorectal cancer, esophageal cancer, lip cancer, laryngeal cancer, hypopharyngeal cancer, tongue cancer, salivary gland cancer, gastric cancer, adenocarcinoma, medullary thyroid cancer, papillary thyroid cancer, renal cancer, renal parenchymal carcinoma, ovarian cancer, cervical cancer, corpus uteri cancer, endometrial cancer, choriocarcinoma, pancreatic cancer, prostate cancer, bladder cancer, testicular cancer, breast cancer, urinary cancer, melanoma, brain tumor, lymphoma, head and neck cancer, acute lymphoblastic leukemia, chronic lymphoblastic leukemia, acute myeloid leukemia, chronic myeloid leukemia, hepatocellular carcinoma, gallbladder cancer, bronchial cancer, small cell lung cancer, non-small cell lung cancer, multiple myeloma, basal sarcoma, teratoma, retinoblastoma, choroidal melanoma, seminoma, rhabdomyosarcoma, osteosarcoma, chondrosarcoma, myoma, liposarcoma, fibrosarcoma, Ewing's sarcoma, and plasmacytoma.

Autoimmune diseases that should be mentioned include rheumatoid arthritis, systemic lupus erythematosus, Sjogren's syndrome, sclerodenna, mixed connective tissue disease, dermatomyositis, polymyositis, Reiter's syndrome, autoimmune lymphoproliferative syndrome, multiple sclerosis, myasthenia gravis, and encephalomyelitis. Inflammatory bowel diseases that should be mentioned include ulcerative colitis or Crohn's disease.

Additional aspects and advantages of the present disclosure will be set forth in part in the following description and, in part, will be apparent from the following description, or may be learned by practice of the present disclosure.

### Beneficial Effects

After extensive and intensive studies, the inventors surprisingly developed a compound or a pharmaceutically acceptable salt thereof, a preparation method therefor, and use thereof.

The present disclosure provides a compound represented by formula V, or a tautomer, a stereoisomer, a hydrate, a solvate, a pharmaceutically acceptable salt, or a prodrug thereof. The compound represented by formula V exhibits a significant inhibitory effect on KIF18A, and can treat diseases or conditions mediated by KIF18A, showing excellent pharmacokinetic properties and relatively high safety and druggability.

The present disclosure provides a method for preparing the compound represented by formula V, or the tautomer, the stereoisomer, the hydrate, the solvate, the pharmaceutically acceptable salt, or the prodrug thereof, and intermediates therefor. The method is simple to operate, offers high yield and high purity, and can be used for industrial pharmaceutical production.

### Definitions and Description

Unless otherwise stated, the definitions of groups and terms described in the specification and claims of the present application, including definitions thereof as examples, exemplary definitions, preferred definitions, definitions documented in tables, definitions of specific compounds in the examples, and the like, may be arbitrarily combined and incorporated with each other. The definitions of groups and the structures of the compounds in such combinations and incorporations should fall within the scope of the specification of the present application.

Unless otherwise defined, all technical and scientific terms used herein have the same meaning as commonly understood by those skilled in the art to which the subject matter of the claims belongs. Unless otherwise stated, all patents, patent applications, and publications referred to herein are incorporated herein by reference in their entirety. If there are multiple definitions for terms herein, those in this section prevail.

It should be understood that the brief description described above and the detailed description below are exemplary and only for explanation, and do not limit the subject matter of the present disclosure in any way. In the present application, unless otherwise specified, the use of the singular also includes the plural. It must be noted that, unless otherwise specified herein, as used in the present specification and claims, the singular forms of a thing include plural forms thereof. It should also be noted that unless otherwise stated, the term "or" used means "and/or". In addition, the terms "comprise", "include" and "contain", and other forms thereof such as "comprises", "comprising", "includes", "including", "contains" and "containing" are not limiting.

Definitions of standardized chemical terms can be found in the literature of reference (including Carey and Sundberg "ADVANCED ORGANIC CHEMISTRY 4TH ED." Vols. A (2000) and B (2001), Plenum Press, New York). Unless otherwise stated, conventional methods within the skill of the art are employed, such as mass spectrometry, NMR, IR and UV/VIS spectroscopy, and pharmacological methods. Unless a specific definition is set forth, the terminology used herein in the pertinent description of analytical chemistry, organic synthetic chemistry, and pharmaceutical and medicinal chemistry is known in the art. Standard techniques can be used in chemical synthesis, chemical analysis, pharmaceutical preparation, preparations and delivery, and treatment of patients. For example, the reaction and purification can be carried out using the instructions of the manufacturer for use of the kit, or in a manner known in the art or as described herein. The techniques and methods described above can generally be implemented according to conventional methods well known in the art, as described in various general and relatively specific documents referred to and discussed in this specification. In the present specification, groups and substituents thereof can be selected by those skilled in the art to provide stable moieties and compounds.

When a substituent is described by a general formula written from left to right, the substituent also includes chemically equivalent substituents that are obtained when the structural formula is written from right to left. For example, CH₂O is equivalent to OCH₂. As used herein, 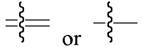 denotes the connection site of a group. As used herein, "R₁", "R1", and "R¹" carry the same meaning and are used interchangeably. For other symbols such as R₂, similar definitions are intended.

The section headings used herein are for organizational purposes only and are not to be construed as limiting the subject matter described. All documents, or portions of documents, cited in the present application, including, but not limited to, patents, patent applications, articles, books, operating manuals, and treatises, are incorporated herein by reference in their entirety.

In addition to the above description, when used in the specification and claims of the present application, the following terms have the meanings shown below, unless otherwise specified.

When a numerical range defined by "integer" is recited in the specification and claims of the present application, it shall be construed to include two endpoints of the range and every integer within the range. For example, "an integer of 1-6" shall be construed to include every integer of 0, 1, 2, 3, 4, 5, and 6.

In the present application, "saturated, partially saturated, or unsaturated" includes a substituent saturated with hydrogen, a substituent completely unsaturated with hydrogen, and a substituent partially saturated with hydrogen. In the present application, the term "halogen", alone or as part of another substituent, refers to fluorine, chlorine, bromine, or iodine.

As used herein, the term "cyano", alone or as part of another substituent, denotes -CN.

As used herein, the term "amino", alone or as part of another substituent, denotes -NH₂.

As used herein, the term "alkyl", alone or as part of another substituent, means a linear or branched hydrocarbon chain group consisting only of carbon and hydrogen atoms, having, for example, 1 to 6 carbon atoms, and connected to the rest of the molecule via a single bond. Examples of alkyl include, but are not limited to, methyl, ethyl, n-propyl, isopropyl, n-butyl, isobutyl, *tert-*butyl*,* pentyl, isopentyl, neopentyl, and hexyl. The alkyl may be unsubstituted or substituted with one or more suitable substituents. The alkyl may also be an isotopic isomer of a naturally abundant alkyl group enriched in carbon and/or hydrogen isotopes (i.e., deuterium or tritium).

The term "C_{α-β} alkyl" refers to an alkyl group comprising a minimum of α and a maximum of β carbon atoms in a branched relationship or a linear relationship or any combination thereof, wherein α and β denote integers; this term may also be expressed in the form of "C_{α}-C_{β} alkyl". The alkyl group described in this section may also comprise one or two double or triple bonds. The designation of C₀ alkyl denotes a direct bond. Exemplarily, C₀₋₆ alkyl includes a direct bond and C₁₋₆ alkyl (e.g., C₁, C₂, C₃, C₄, C₅, and C₆ alkyl). Examples of C₁₋₆ alkyl include, but are not limited to, the following:

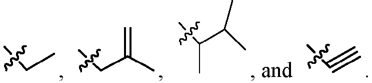

The term "benzo group", alone or in combination, refers to the divalent group C₄H₄=, wherein one representation is -CH=CH-CH=CH-, and the group, when ortho-attached to another ring, forms a benzene-like ring, e.g., tetrahydronaphthalene, indole, etc.

The term "C_{α-β} haloalkyl", alone or as part of another substituent, refers to the alkyl group as described above, wherein any number (at least one) of the hydrogen atoms attached to the alkyl chain are substituted with fluoro, chloro, bromo, or iodo.

The term "alkoxy", alone or as part of another substituent, refers to -O-R^{Q} group, wherein R^{Q} is "alkyl" as defined above.

The term "oxo", alone or as part of another substituent, means that two hydrogens on methylene are substituted with oxygen, i.e., methylene is substituted with carbonyl, denoted as =O.

The term "thio", alone or as part of another substituent, means that two hydrogens on methylene are substituted with sulfur, denoted as =S.

The term "cycloalkyl" or "carbocyclyl", alone or as part of another substituent, refers to a cyclic alkyl group. The term "m- to n-membered cycloalkyl" or "Cₘ-Cₙ cycloalkyl" should be understood to denote a saturated, unsaturated, or partially saturated carbocyclic ring having m to n atoms. For example, "3- to 15-membered cycloalkyl" or "C₃-C₁₅ cycloalkyl" refers to a cyclic alkyl group comprising 3 to 15, 3 to 9, 3 to 6, or 3 to 5 carbon atoms, which may contain 1 to 4 rings. "5- to 8-membered cycloalkyl" comprises 5-8 carbon atoms. The cycloalkyl includes a monocyclic ring, a bicyclic ring, a tricyclic ring, a spiro ring, or a bridged ring. Examples of unsubstituted cycloalkyl include, but are not limited to, cyclopropyl, cyclobutyl, cyclopentyl, cyclohexyl, and adamantyl, or bicyclic hydrocarbyl such as decahydronaphthalene ring. The cycloalkyl may be substituted with one or more substituents. In some embodiments, the cycloalkyl may be cycloalkyl fused to aryl or heteroaryl. The term "C₃-C₆ cycloalkyl" should be understood to denote a saturated monovalent monocyclic or bicyclic hydrocarbon ring having 3-6 carbon atoms, including fused or bridged polycyclic ring systems, e.g., cyclopropyl, cyclobutyl, cyclopentyl, or cyclohexyl.

The term "bicyclic ring" means a group having two linking rings. The bicyclic ring may be a carbocyclic ring (all the ring atoms are carbon atoms) or a heterocyclic ring (the ring atoms include, for example, 1, 2, or 3 heteroatoms such as N, O, or S, in addition to carbon atoms). Both rings may be aliphatic (e.g., decahydronaphthalene and norbornane), or aromatic (e.g., naphthalene), or a combination of aliphatic and aromatic (e.g., tetrahydronaphthalene). The bicyclic ring includes: (a) spiro compounds, wherein the two rings share only one single atom (a spiro atom, which is typically a quaternary carbon); examples of the spiro compounds include, but are not limited to: 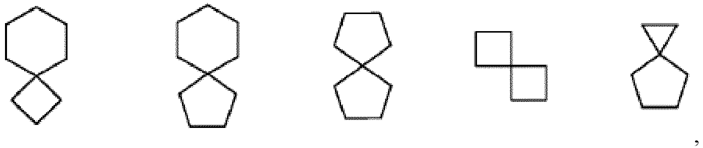 and also include spirocycloalkyl in which single spirocycloalkyl and heterocycloalkyl share a spiro atom, and the non-limiting examples include: 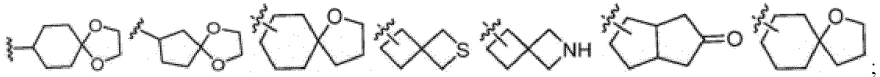 (b) fused bicyclic ring compounds, wherein the two rings share two adjacent atoms; in other words, the two rings share one covalent bond, i.e., the bridgehead atoms are directly connected (e.g., α-thujene and decahydronaphthalene); examples of the fused bicyclic rings include, but are not limited to: 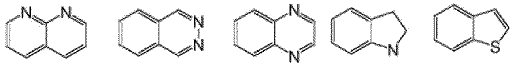 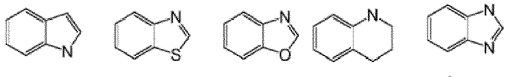 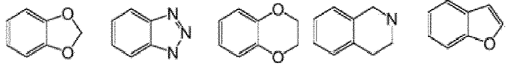 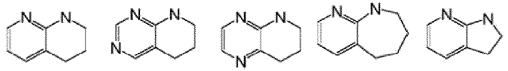 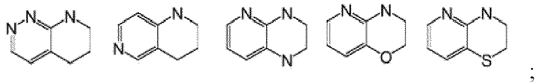 and
(c) bridged bicyclic ring compounds, wherein the two rings share three or more atoms, and the two bridgehead atoms are separated by a bridge comprising at least one atom; for example, norbornane, also known as bicyclo[2.2.1]heptane, may be considered as a pair of cyclopentane rings with each ring sharing three of the five carbon atoms; examples of the bridged bicyclic rings include, but are not limited to:

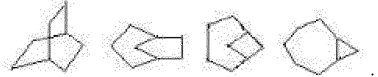

The term "heterocycloalkyl", alone or as part of another substituent, refers to cycloalkyl in which one or more (1 to 3 in some embodiments) carbon atoms are substituted with heteroatoms, wherein the heteroatom is, for example, but not limited to, N, O, S, or P. The term "3- to 8-membered heterocyclyl" or "3- to 8-membered heterocycloalkyl" should be understood to denote a monocyclic, bicyclic, or tricyclic ring having 3 to 8 atoms, wherein the heteroatom is preferably selected from N, O, and S. It should be understood that when the total number of S and O atoms in the heterocyclyl exceeds 1, these heteroatoms are not adjacent to each other. Examples of heterocycloalkyl include, but are not limited to: tetrahydroisoquinolyl, tetrahydroquinolyl, tetrahydropyranyl, tetrahydrofuranyl, and tetrahydrothiopyranyl.

The term "5- or 6-membered heteroaryl" should be understood as an aromatic ring group having 5 or 6 ring atoms and comprising 1-5 heteroatoms independently selected from N, O, and S, preferably 1-3 heteroatoms independently selected from N, O, and S. Examples of heteroaryl include, but are not limited to: thienyl, furanyl, pyrrolyl, oxazolyl, thiazolyl, imidazolyl, pyrazolyl, isoxazolyl, isothiazolyl, oxadiazolyl, triazolyl, thiadiazolyl, and the like; or pyridinyl, pyridazinyl, pyrimidinyl, pyrazinyl, triazinyl, and the like.

The NR^{a}R^{a} group, alone or as part of another substituent, may be present in the form of 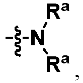 or may also include a group in which two R^{a} groups together form a ring optionally comprising N, O, or S atoms, and may also include groups such as:

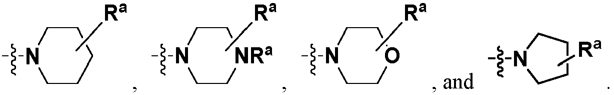

The N(C_{α-β} alkyl)C_{α-β} alkyl group (wherein α and β are as defined above), alone or as part of another substituent, includes a substituent in which two C_{α-β} alkyl groups together form a ring (optionally comprising N, O, or S atoms), and includes groups such as:

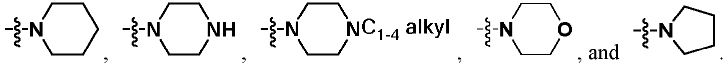

The term "inert solvent" includes, but is not limited to, toluene, benzene, water, methanol, ethanol, isopropanol, ethylene glycol, *N*-methylpyrrolidone, dimethylsulfoxide, tetrahydrofuran dichloromethane, trichloromethane, 1,2-dichloroethane, acetonitrile, *N,N-dimethylformamide, N,N-dimethylacetamide,* dioxane, or combinations thereof.

The compounds provided herein include intermediates that can be used for preparing the compounds provided herein, which comprise reactive functional groups (such as, but not limited to, carboxyl, hydroxyl, and amino moieties), and also include protected derivatives thereof. The term "protected derivatives" refers to the compounds in which one or more reactive sites are blocked by one or more protecting groups (also referred to as protective groups). Suitable carboxyl moiety protecting groups include benzyl, *tert-*butyl*,* and the like, as well as isotopes and the like. Suitable amino and amido protecting groups include acetyl, trifluoroacetyl, *tert*-butoxycarbonyl, benzyloxycarbonyl, and the like. Suitable hydroxyl protecting groups include benzyl and the like. Other suitable protecting groups are well known to those of ordinary skill in the art.

In the present application, "optional" or "optionally" means that the subsequently described event or circumstance may occur or may not occur, and the description includes instances where the event or circumstance occurs or does not occur. For example, "optionally substituted aryl" means that the aryl is substituted or unsubstituted, and the description includes both substituted aryl and unsubstituted aryl.

In the present application, the term "salt" or "pharmaceutically acceptable salt" includes pharmaceutically acceptable acid addition salts and pharmaceutically acceptable base addition salts. The term "pharmaceutically acceptable" refers to those compounds, materials, compositions, and/or dosage forms that are, within the scope of sound medical judgment, suitable for use in contact with the tissues of humans and animals without excessive toxicity, irritation, allergic response, or other problems or complications, and commensurate with a reasonable benefit/risk ratio.

The term "pharmaceutically acceptable acid addition salts" refers to salts that retain the bioavailability of the free base without other side effects and that are formed from an inorganic acid or an organic acid. The term "pharmaceutically acceptable base addition salts" refers to salts that retain the bioavailability of the free acid without other side effects and that are formed from an inorganic base or an organic base. In addition to pharmaceutically acceptable salts, the present disclosure also contemplates other salts. They may serve as intermediates in the purification of the compounds or in the preparation of other pharmaceutically acceptable salts, or may be used in the identification, characterization, or purification of the compounds disclosed herein.

The term "amine salt" refers to a product obtained by neutralizing a primary, secondary, or tertiary alkyl amine with an acid. The acid includes the inorganic acid or the organic acid as described in the present application.

The term "stereoisomer" refers to isomers resulting from different spatial arrangements of atoms in a molecule, including *cis-*trans isomers, enantiomers, diastereoisomers, and conformational isomers.

Based on the choice of starting materials and methods, the compound disclosed herein may be present in the form of one of the possible isomers or a mixture thereof, for example, as a pure optical isomer, or as an isomer mixture, such as a racemic mixture and a diastereoisomer mixture, depending on the number of asymmetric carbon atoms. When describing optically active compounds, the prefixes D and L or R and S are used to denote the absolute configuration of the molecule with respect to the chiral center(s) in the molecule. The prefixes D and L or (+) and (-) are the symbols used to designate the rotation of plane polarized light by a compound, where (-) or L indicates that the compound is levorotatory. Compounds with the prefix (+) or D are dextrorotatory.

When the bond to a chiral carbon in the formula of the present disclosure is depicted as a direct line, it should be understood that both the (R) and (S) configurations of the chiral carbon, as well as the resulting enantiomerically pure compounds and a mixture thereof, are encompassed within the scope of the general formula. The method of depicting the racemic or enantiomerically pure compounds herein is from Maehr, J. Chem. Ed. 1985, 62:114-120. The absolute configuration of a stereogenic center is represented by a wedged bond and a dashed bond.

The term "tautomer" refers to functional isomers resulting from the rapid movement of an atom in a molecule between two positions. The compound disclosed herein may exhibit the tautomerism. A tautomeric compound may be present in two or more interconvertible forms. Prototropic tautomers result from the migration of a covalently bonded hydrogen atom between two atoms. Tautomers are generally present in an equilibrium form. Efforts to separate a single tautomer usually lead to a mixture, the physicochemical properties of which are consistent with those of the mixture of the compound. The position of the equilibrium depends on the chemical properties of the molecule. For example, in many aliphatic aldehydes and ketones such as acetaldehyde, the keto form predominates; whereas in phenols, the enol form predominates. In the present disclosure, all tautomeric forms of the compound are included. In the present application, "pharmaceutical composition" refers to a preparation of the compound disclosed herein with a medium generally accepted in the art for delivery of biologically active compounds to a mammal (e.g., a human). The medium includes a pharmaceutically acceptable carrier. The purpose of the pharmaceutical composition is to facilitate administration to an organism, promote absorption of the active ingredient, and thereby exert biological activity.

In the present application, "pharmaceutically acceptable carrier" includes, but is not limited to, any adjuvant, carrier, excipient, glidant, sweetener, diluent, preservative, dye/colorant, flavoring agent, surfactant, wetting agent, dispersant, suspending agent, stabilizer, isotonic agent, solvent, or emulsifier that is approved by the relevant governmental regulatory agency for human or livestock use.

The term "solvate" means that the compound disclosed herein or a salt thereof comprises a stoichiometric or non-stoichiometric amount of a solvent bonded by non-covalent intermolecular forces, and when the solvent is water, the solvate is a hydrate.

The term "prodrug" can be converted to the compound disclosed herein with biological activity under physiological conditions or by solvolysis. The prodrug disclosed herein is prepared by modifying a functional group in the compound, wherein the modification may be removed by conventional procedures or be removed *in vivo* to give the parent compound. The prodrug includes a compound formed by linking a hydroxyl or amino group of the compound disclosed herein to any group. When the prodrug of the compound disclosed herein is administered to a mammalian individual, the prodrug is cleaved to form a free hydroxyl group or a free amino group.

The compound disclosed herein may contain an unnatural proportion of atomic isotope at one or more of the atoms that constitute the compound. For example, the compound may be labeled with a radioactive isotope, such as deuterium (²H), tritium (³H), iodine-125 (¹²⁵I), or C-14 (¹⁴C). All isotopic variations of the compound disclosed herein, whether radioactive or not, are encompassed within the scope of the present disclosure.

The term "auxiliary material" refers to a pharmaceutically acceptable inert ingredient. Examples of types of the term "excipient" include, without limitation, adhesives, disintegrants, lubricants, glidants, stabilizers, fillers, diluents, and the like. Excipients are capable of enhancing the handling characteristics of the pharmaceutical preparation, i.e., making the preparation more amenable to direct compression by increasing flowability and/or adhesiveness.

The term "treatment" and other similar synonyms as used herein include the following meanings:
(i) preventing the occurrence of a disease or condition in a mammal, particularly when the mammal is predisposed to the disease or condition but has not yet been diagnosed as having the disease or condition;
(ii) inhibiting the disease or condition, i.e., arresting its development;
(iii) alleviating the disease or condition, i.e., causing regression of the state of the disease or condition; or
(iv)reliving the symptoms caused by the disease or condition.

In the reaction of each step, the reaction temperature may be appropriately selected depending on the solvent, the starting material, the reagent, and the like, and the reaction time may also be appropriately selected depending on the reaction temperature, the solvent, the starting material, the reagent, and the like. After the reaction of each step is completed, the target compound may be separated and purified from the reaction system by a conventional method, such as filtration, extraction, recrystallization, washing, silica gel column chromatography, and the like. Under the condition of not affecting the next reaction, the target compound may directly enter the next reaction without separation and purification.

### DETAILED DESCRIPTION

The present disclosure will be further illustrated with reference to the following specific examples. It should be understood that the following descriptions are merely the most preferred embodiments of the present disclosure, and should not be construed as limiting the protection scope of the present disclosure. On the basis of a thorough understanding of the present disclosure, experimental procedures without specified conditions in the following examples are generally conducted according to conventional conditions or according to conditions recommended by the manufacturer. Those skilled in the art can make non-essential changes in the technical solutions of the present disclosure, and such changes should be regarded as included in the protection scope of the present disclosure. Unless otherwise stated, the starting materials and reagents used in the following examples are all commercially available products or can be prepared by using known methods.

### The present application has the following definitions:

### Symbols or units:

IC₅₀: the half maximal inhibitory concentration, referring to the concentration at which half of the maximal inhibitory effect is achieved
M: mol/L, for example, n-butyllithium (14.56 mL, 29.1 mmol, 2.5 M *n*-hexane solution) denotes a *n*-hexane solution of *n*-butyllithium with a molar concentration of 2.5 mol/L
N: equivalent concentration, for example, 2 N hydrochloric acid denotes a 2 mol/L hydrochloric acid solution
RT: retention time

### Reagents:

DMF: *N,N*-dimethylformamide
DMSO: dimethylsulfoxide
DIPEA: *N,N-diisopropylethylamine*
HATU: *O*-(7-azabenzotriazol-1-yl)-*N*,*N*,*N*',*N*'-tetramethyluronium hexafluorophosphate

### Test method:

LCMS: liquid chromatography-mass spectrometry

### Intermediate A1: Preparation of Intermediate A1

### 4-Iodo-2-(6-azaspiro[2.5]octan-6-yl)benzoic acid

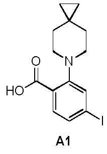

The synthetic route of **intermediate A1** was as follows:

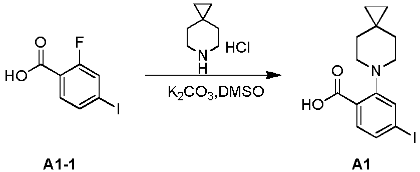

To a solution of 2-fluoro-4-iodobenzoic acid (Al-1) (8.00 g, 30.1 mmol) in dimethylsulfoxide (160 mL) were added 6-azaspiro[2.5]octane hydrochloride (5.77 g, 39.1 mmol) and potassium carbonate (12.5 g, 90.2 mmol) at room temperature, and the reactants were allowed to react under nitrogen atmosphere at 140 °C for 18 h. After the reaction was completed, water (800 mL) was added to the reaction liquid at room temperature for dilution, and then the mixture was extracted with ethyl acetate (200 mL × 3). The aqueous phase was adjusted to pH = 6 with diluted hydrochloric acid (2.00 M), and a solid was precipitated. The mixture was filtered, and the filter cake was dried to give the target compound 4-iodo-2-(6-azaspiro[2.5]octan-6-yl)benzoic acid (9.00 g, 25.2 mmol, yield: 83.8%).

¹H NMR (400 MHz, DMSO_d₆) δ 8.06 (s, 1H), 7.76-7.69 (m, 2H), 3.11-3.08 (t, 4H), 1.54 (s, 4H), 0.41 (s, 4H). LC-MS, M/Z (ESI): 358.0 [M+H]⁺.

### Intermediate A2: Preparation of Intermediate A2

### Synthesis of butyl 2-sulfamoylpropionate

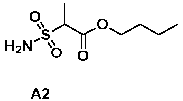

The synthetic route of intermediate A2 was as follows:

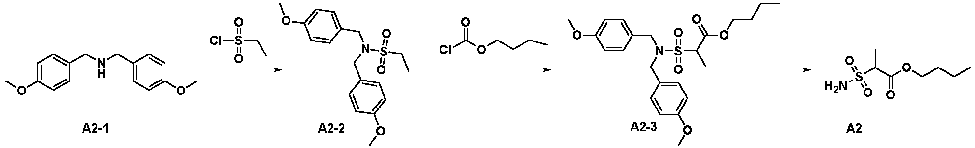

### Step 1: Synthesis of N,N-bis(4-methoxybenzyl)ethanesulfonamide

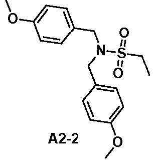

Bis(4-methoxybenzyl)amine (20 g, 78 mmol) was added to 300 mL of acetonitrile, and then potassium phosphate (66 g, 311 mmol) and ethanesulfonyl chloride (14.99 g, 117 mmol) were added. The mixture was allowed to react at room temperature overnight. LCMS showed that the starting materials were completely reacted. The reaction liquid was concentrated to dryness, and the residue was separated and purified by a silica gel column (PE:EA (V/V) = 3:1) to give *N,N*-bis(4-methoxybenzyl)ethanesulfonamide (20 g, yield: 73.6%).

LC-MS, M/Z (ESI): 350.1 [M+H]⁺.

### Step 2: Synthesis of butyl 2-(N,N-bis(4-methoxybenzyl)sulfamoyl)propionate

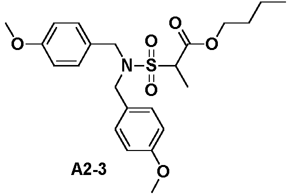

*N,N-*Bis(4-methoxybenzyl)ethanesulfonamide (5 g, 14.31 mmol) was added to 50 mL of tetrahydrofuran, and the mixture was cooled to -80 °C under nitrogen atmosphere. A solution of *n*-butyllithium in tetrahydrofuran (9.73 mL, 2.5 mol/L) was added, and after the addition was completed, the mixture was successively stirred for 30 min. Butyl chloroformate (3.32 g, 24.32 mmol) was added, and after the addition was completed, the mixture was allowed to react for 1 h. LCMS showed that the starting materials were completely reacted. The reaction liquid was poured into *50* mL of saturated ammonium chloride, and the mixture was extracted with ethyl acetate (100 mL × 2). The organic phase was concentrated to dryness, and the residue was separated and purified by a silica gel column (PE:EA (V/V) = 3:1) to give butyl 2-(*N,N*-bis(4-methoxybenzyl)sulfamoyl)propionate (4 g, yield: 62.2%).

LC-MS, M/Z (ESI): 450.2 [M+H]⁺.

### Step 3: Synthesis of butyl 2-sulfamoylpropionate

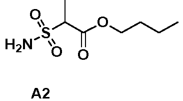

Butyl 2-(*N,N-*bis(4-methoxybenzyl)sulfamoyl)propionate (4 g, 8.89 mmol) was added to 20 mL of ethanol, and then 20 mL of trifluoroacetic acid was added. The mixture was allowed to react at room temperature for 6 h. LCMS showed that the starting materials were completely reacted. The reaction liquid was concentrated to dryness, 50 mL of saturated sodium carbonate was then added, and the mixture was extracted with ethyl acetate (100 mL × 2). The organic phase was concentrated to dryness, and the residue was separated and purified by a silica gel column (PE:EA (V/V) = 1:1) to give butyl 2-sulfamoylpropionate (1.86 g, yield: 100%).

### Intermediate A3: Preparation of Intermediate A3

### Ethyl 2-sulfamoylpropionate

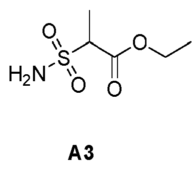

The synthetic route of intermediate **A3** was as follows:

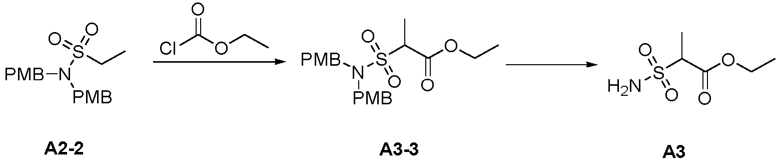

### Step 1: Synthesis of ethyl 2-(N,N-bis(4-methoxybenzyl)sulfamoyl)propionate

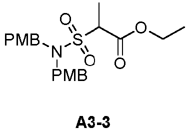

N,N-Bis(4-methoxybenzyl)ethanesulfonamide (10.0 g, 28.6 mmol) was dissolved in tetrahydrofuran (50 mL), and the solution was cooled to -78 °C. n-Butyllithium (2.00 M, 24.3 mL) was added dropwise under nitrogen atmosphere, and after the dropwise addition was completed, the mixture was allowed to react at -78 °C for 1 h. Ethyl chloroformate (5.28 g, 48.6 mmol) was then added dropwise, and the mixture was stirred at -78 °C for 1 h. After the reaction was completed, the reaction liquid was quenched with a saturated ammonium chloride solution (100 mL) and extracted with ethyl acetate (100 mL × 2). The organic phase was washed with saturated brine (100 mL), dried over anhydrous sodium sulfate, filtered, and concentrated to give a crude product. The crude product was separated and purified by a silica gel column (petroleum ether:ethyl acetate (V/V) = 10:1-0:1) to give ethyl 2-(*N,N*-bis(4-methoxybenzyl)sulfamoyl)propionate (5.00 g, yield: 41.5%).

LC-MS, M/Z (ESI): 420.1 [M-H]⁺.

### Step 2: Synthesis of ethyl 2-sulfamoylpropionate

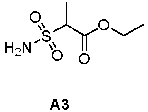

Ethyl 2-(N,N-bis(4-methoxybenzyl)sulfamoyl)propionate (1.00 g, 2.37 mmol) was dissolved in anisole (2 mL) and trifluoroacetic acid (10 mL), and the solution was allowed to react at -78 °C for 1 h. Ethyl chloroformate (5.28 g, 48.6 mmol) was then added dropwise, and the mixture was stirred at 25 °C for 2 h. After the reaction was completed, the reaction liquid was quenched with a saturated potassium carbonate solution (50 mL) and extracted with ethyl acetate (50 mL × 2). The organic phase was washed with saturated brine (50 mL), dried over anhydrous sodium sulfate, filtered, and concentrated to give ethyl 2-sulfamoylpropionate (400 mg, yield: 93.0%).

¹H NMR (400 MHz, DMSO_d₆) *δ* 7.14 (s, 2H), 4.15 (q, 2H), 3.94-3.99 (m, 1H), 1.45 (d, 3H), 1.22 (t, 3H).

### Example 1: Preparation of Target Compound I-1

### (R)-2-Hydroxy-N-(4-(5-(4-methyl-6-(2-methylmorpholino)pyridin-2-yl)-1,3,4-oxadiazol-2-yl)-3-(6-azaspiro[2.5]octan-6-yl)phenyl)ethane-1-sulfonamide

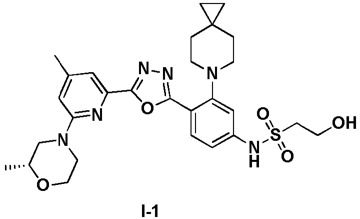

The synthetic route of the target compound I-1 was as follows:

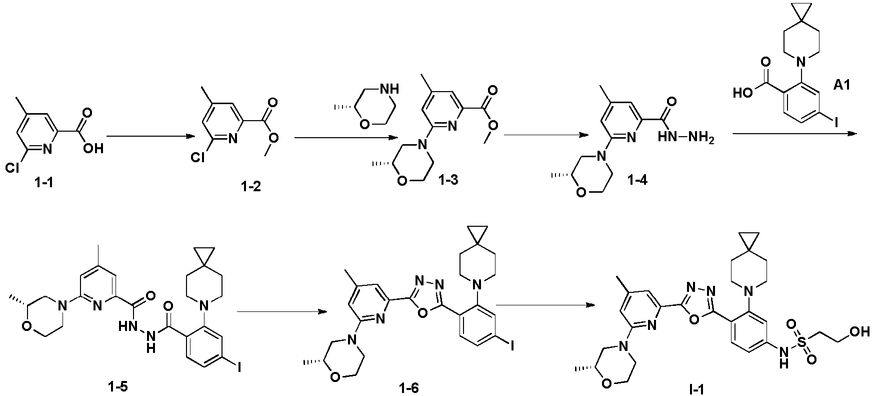

### Step 1: Synthesis of methyl 6-chloro-4-methylpicolinate

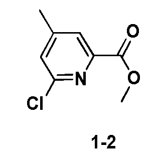

6-Chloro-4-methylpicolinic acid (2.2 g, 12.82 mmol) was added to methanol (10 mL), and then thionyl chloride (3.05 g, 25.6 mmol) was added. The mixture was allowed to react at room temperature for 1 h. LCMS showed that the starting materials were completely reacted. The reaction liquid was concentrated to dryness. Water (30 mL) was added for dilution, and the mixture was extracted with ethyl acetate (50 mL × 2). The organic phase was concentrated LC-MS, M/Z (ESI): 186.0 [M+H]⁺.

### Step 2: Synthesis of methyl (R)-4-methyl-6-(2-methylmorpholino)picolinate

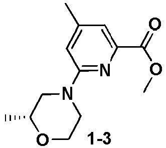

Methyl 6-chloro-4-methylpicolinate (2.38 g, 12.82 mmol) was added to 10 mL of N-methylpyrrolidone, and then (R)-2-methylmorpholine (2.65 g, 19.23 mmol) and *N,N-*diisopropylethylamine (6.63 g, 51.3 mmol) were added. The mixture was heated to 120 °C and allowed to react under microwave for 4 h. LCMS showed that the starting materials were completely reacted. Water (30 mL) was added for dilution, and the mixture was extracted with ethyl acetate (50 mL × 2). The organic phase was concentrated to dryness, and the residue was separated and purified by a silica gel column (PE:EA (V/V) = 2:1) to give methyl (*R*)-4-methyl-6-(2-methylmorpholino)picolinate (450 mg, yield: 14.04%).

LC-MS, M/Z (ESI): 251.1 [M+H]⁺.

### Step 3: Synthesis of (R)-4-methyl-6-(2-methylmorpholino)picolinohydrazide

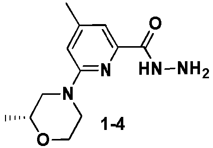

Methyl (*R*)-4-methyl-6-(2-methylmorpholino)picolinate (450 mg, 1.8 mmol) was added to 10 mL of ethanol, and then 2 mL of 80% hydrazine hydrate was added. The mixture was heated to 60 °C and allowed to react for 3 h. LCMS showed that the starting materials were completely reacted. The reaction liquid was concentrated to dryness, and the residue was lyophilized to give a crude product of (*R*)-4-methyl-6-(2-methylmorpholino)picolinohydrazide (450 mg, yield: 100%).

LC-MS, M/Z (ESI): 251.1 [M+H]⁺.

### Step 4: Synthesis of (R)-N-(4-iodo-2-(6-azaspiro[2.5]octan-6-yl)benzoyl)-4-methyl-6-(2-methylmorpholino) picolinohydrazide

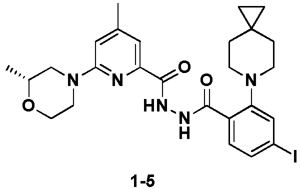

(*R*)-4-Methyl-6-(2-methylmorpholino)picolinohydrazide (0.45 g, 1.8 mmol) was added to 5 mL of pyridine, and then 4-iodo-2-(6-azaspiro[2.5]octan-6-yl)benzoic acid (**A1**) (0.64 g, 1.8 mmol) and *O*-(7-azabenzotriazol-1-yl)-*N,N,N',N'-*tetramethyluronium hexafluorophosphate (2.06 g, 5.38 mmol) were added. The mixture was heated to 60 °C and allowed to react for 8 h. LCMS showed that the starting materials were completely reacted. Water (5 mL) was added for dilution, and the mixture was extracted with ethyl acetate (10 mL × 2). The organic phase was concentrated to dryness, and the residue was separated and purified by a silica gel column (PE:EA = 3:1) to give (*R*)-*N*'-(4-iodo-2-(6-azaspiro[2.5]octan-6-yl)benzoyl)-4-methyl-6-(2-methylmorpholino)picolinohydrazide (300 mg, yield: 28.3%).

LC-MS, M/Z (ESI): 590.2 [M+H]⁺.

### Step 5: Synthesis of (R)-4-(6-(5-(4-iodo-2-(6-azaspiro[2.5]octan-6-yl)phenyl)-1,3,4-oxadiazol-2-yl)-4-methylpyridin-2-yl)methylmorpholine

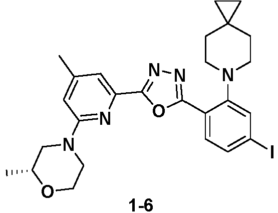

(*R*)-*N*'-(4-lodo-2-(6-azaspiro[2.5]octan-6-yl)benzoyl)-4-methyl-6-(2-methylmorpholino)picolinohydrazide (0.3 g, 0.509 mmol) was added to 5 mL of acetonitrile, and then p-methylbenzenesulfonyl chloride (0.39 g, 2.036 mmol) and N,N-diisopropylethylamine (0.526 g, 4.07 mmol) were added. The mixture was heated to 80 °C and allowed to react for 1 h. LCMS showed that the starting materials were completely reacted. Water (5 mL) was added for dilution, and the mixture was extracted with ethyl acetate (10 mL × 2). The organic phase was concentrated to dryness, and the residue was separated and purified by a silica gel column (PE:EA = 3:1) to give (*R*)-4-(6-(5-(4-iodo-2-(6-azaspiro[2.5]octan-6-yl)phenyl)-1,3,4-oxadiazol-2-yl)-4-methylpyridin-2-yl)methylmorpholine (120 mg, yield: 41.3%).

LC-MS, M/Z (ESI): 572.1 [M+H]⁺.

### Step 6: Synthesis of (R)-2-hydroxy-N-(4-(5-(4-methyl-6-(2-methylmorpholino)pyridin-2-yl)-1,3,4-oxadiazol-2-yl)-3-(6-azaspiro[2.5]octan-6-yl)phenyl)ethane-1-sulfonamide

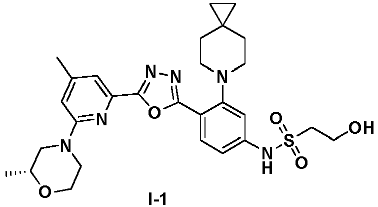

*(R)-4-(6-(5-(4-Iodo-2-(6-azaspiro[2.5]octan-6-yl)phenyl)-1,3,4-oxadiazol-2-yl)-4-methylpyridin-2-*yl)methylmorpholine (0.12 g, 0.289 mmol) was added to 5 mL of *N,N*-dimethylformamide, and then 2-hydroxyethane-1-sulfonamide (53 mg, 0.578 mmol), methylglycine (7.48 mg, 0.084 mmol), copper(I) iodide (8 mg, 0.042 mmol), and potassium carbonate (116 mg, 0.84 mmol) were added. The mixture was heated to 130 °C and allowed to react for 1 h. LCMS showed that the starting materials were completely reacted. Water (5 mL) was added for dilution, and the mixture was extracted with ethyl acetate (10 mL × 2). The organic phase was concentrated to dryness, and the residue was separated and purified by a silica gel column (PE:EA = 1:1) to give (*R*)-2-hydroxy-N-(4-(5-(4-methyl-6-(2-methylmorpholino)pyridin-2-yl)-1,3,4-oxadiazol-2-yl)-3-(6-azaspiro[2.5]octan-6-yl)phenyl)ethane-1-sulfonamide **(I-1)** (27 mg, yield: 22.61%).

LC-MS, M/Z (ESI): 569.3 [M+H]⁺.

¹H NMR (400 MHz, DMSO-d6) δ7.59 (d, 1H), 7.37 (s, 1H), 6.88 (s, 1H), 6.62 (s, 1H), 6.57-6.55 (dd, 1H), 4.25 (t, 2H), 3.92 (d, 1H), 3.68 (t, 2H), 3.63-3.57(m, 2H), 3.16 (s, 1H), 2.92 (t, 2H), 2.85 (q, 4H), 2.56-2.51 (m, 1H), 2.33 (s, 3H), 1.50 (s, 4H), 1.18 (d, 3H), 0.28 (s, 4H).

### Example 2: Preparation of Target Compound I-2

### N-(4-(5-(2-(4,4-Difluoropiperidin-1-yl)-6-methylpyrimidin-4-yl)-1,3,4-oxadiazol-2-yl)-3-(6-azaspiro[2.5]octan-6-yl)phenyl)-2-hydroxyethane-1-sulfonamide

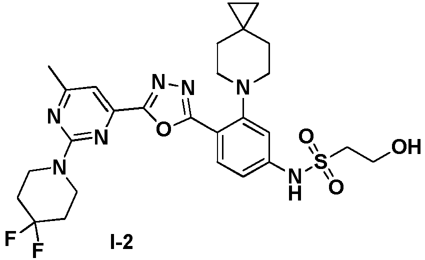

The synthetic route of the target compound **I-2** was as follows:

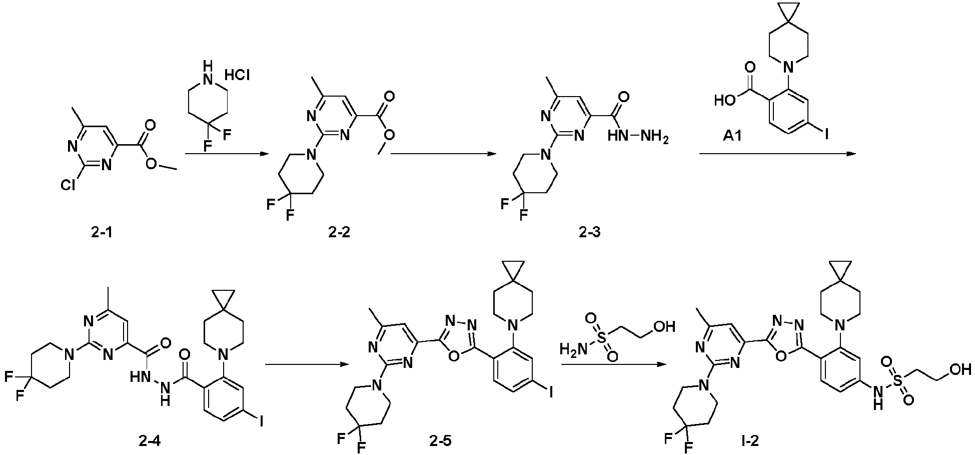

### Step 1: Synthesis of methyl 2-(4,4-difluoropiperidin-1-yl)-6-methylpyrimidine-4-carboxylate

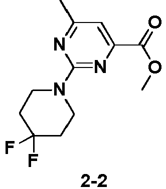

Methyl 2-chloro-6-methylpyrimidine-4-carboxylate (2 g, 10.72 mmol) was added to 20 mL of *N,N-*dimethylformamide, and then 4,4-difluoropiperidine hydrochloride (3.38 g, 21.44 mmol) and potassium carbonate (7.41 g, 53.6 mmol) were added. The mixture was heated to 120 °C and allowed to react for 2 h. LCMS showed that the starting materials were completely reacted. Water (30 mL) was added for dilution, and the mixture was extracted with ethyl acetate (50 mL × 2). The organic phase was concentrated to dryness, and the residue was separated and purified by a silica gel column (PE:EA = 5:1) to give methyl 2-(4,4-difluoropiperidin-1-yl)-6-methylpyrimidine-4-carboxylate (2 g, yield: 68.8%).

LC-MS, M/Z (ESI): 272.1 [M+H]⁺.

### Step 2: Synthesis of 2-(4,4-difluoropiperidin-1-yl)-6-methylpyrimidine-4-carbohydrazide

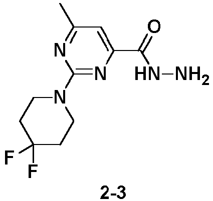

Methyl 2-(4,4-difluoropiperidin-1-yl)-6-methylpyrimidine-4-carboxylate (2 g, 7.34 mmol) was added to 20 mL of methanol, and then 4 mL of 80% hydrazine hydrate was added. The mixture was heated to 60 °C and allowed to react for 1 h. LCMS showed that the starting materials were completely reacted. The reaction liquid was concentrated to dryness, and the residue was lyophilized to give a crude product of 2-(4,4-difluoropiperidin-1-yl)-6-methylpyrimidine-4-carbohydrazide (2 g, yield: 100%).

LC-MS, M/Z (ESI): 272.1 [M+H]⁺.

### Step 3: Synthesis of 2-(4,4-difluoropiperidin-1-yl)-N'-(4-iodo-2-(6-azaspiro[2.5]octan-6-yl)benzoyl)-6-methylpyrimidine-4-carbohydrazide

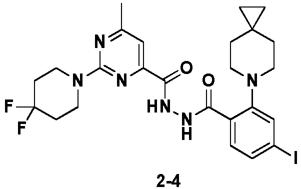

2-(4,4-Difluoropiperidin-1-yl)-6-methylpyrimidine-4-carbohydrazide (1.709 g, 6.3 mmol) was added to 15 mL of *N,N*-dimethylformamide, and then 4-iodo-2-(6-azaspiro[2.5]octan-6-yl)benzoic acid (1.5 g, 4.2 mmol), *N,N-*diisopropylethylamine (3.26 g, 25.2 mmol), and *O*-(7-azabenzotriazol-1-yl)-*N,N,N',N'*-tetramethyluronium hexafluorophosphate (4.79 g, 12.6 mmol) were added. The mixture was heated to 60 °C and allowed to react for 1 h. LCMS showed that the starting materials were completely reacted. Water (20 mL) was added for dilution, and the mixture was extracted with ethyl acetate (100 mL × 2). The organic phases were combined and concentrated to dryness, and the residue was separated and purified by a silica gel column (PE:EA = 3:1) to give 2-(4,4-difluoropiperidin-1-yl)-*N'*-(4-iodo-2-(6-azaspiro[2.5]octan-6-yl)benzoyl)-6-methylpyrimidine-4-carbohydrazide (1.5 g, yield: 58.5%).

LC-MS, M/Z (ESI): 611.1 [M+H]⁺.

### Step 4: Synthesis of 2-(2-(4,4-difluoropiperidin-1-yl)-6-methylpyrimidin-4-yl)-5-(4-iodo-2-(6-azaspiro[2.5]octan-6-yl)phenyl)-1,3,4-oxadiazole

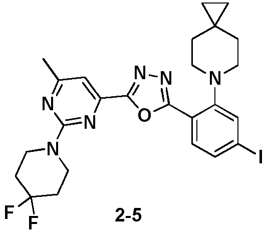

2-(4,4-Difluoropiperidin-1-yl)-*N'*-(4-iodo-2-(6-azaspiro[2.5]octan-6-yl)benzoyl)-6-methylpyrimidine-4-carbohydrazide (0.3 g, 0.491 mmol) was added to 5 mL of dichloromethane, and then p-methylbenzenesulfonyl chloride (0.281 g, 1.474 mmol) and triethylamine (0.298 g, 2.95 mmol) were added. The mixture was allowed to react at room temperature overnight. LCMS showed that the starting materials were completely reacted. Water (5 mL) was added for dilution, and the mixture was extracted with ethyl acetate (10 mL × 2). The organic phases were combined and concentrated to dryness, and the residue was separated and purified by a silica gel column (PE:EA = 3:1) to give 2-(2-(4,4-difluoropiperidin-1-yl)-6-methylpyrimidin-4-yl)-5-(4-iodo-2-(6-azaspiro[2.5]octan-6-yl)phenyl)-1,3,4-oxadiazole (120 mg, yield: 41.2%).

LC-MS, M/Z (ESI): 593.1 [M+H]⁺.

### Step 5: Synthesis of N-(4-(5-(2-(4,4-difluoropiperidin-1-yl)-6-methylpyrimidin-4-yl)-1,3,4-oxadiazol-2-yl)-3-(6-azaspiro[2.5]octan-6-yl)phenyl)-2-hydroxyethane-1-sulfonamide

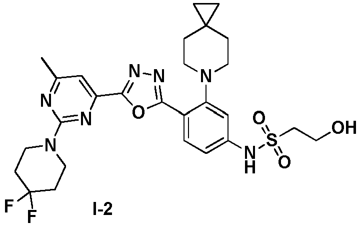

2-(2-(4,4-Difluoropiperidin-1-yl)-6-methylpyrimidin-4-yl)-5-(4-iodo-2-(6-azaspiro[2.5]octan-6-yl)phenyl)-1,3,4-oxadiazole (0.12 g, 0.203 mmol) was added to 5 mL of N,N-dimethylformamide, and then 2-hydroxyethane-1-sulfonamide (51 mg, 0.405 mmol), dimethylglycine (42 mg, 0.405 mmol), copper(I) iodide (39 mg, 0.203 mmol), and potassium carbonate (112 mg, 0.81 mmol) were added. The mixture was heated to 130 °C and allowed to react for 1 h. LCMS showed that the starting materials were completely reacted. Water (5 mL) was added for dilution, and the mixture was extracted with ethyl acetate (10 mL × 2). The organic phases were combined and concentrated to dryness, and the residue was separated and purified by a silica gel column (PE:EA = 1:1) to give N-(4-(5-(2-(4,4-difluoropiperidin-1-yl)-6-methylpyrimidin-4-yl)-1,3,4-oxadiazol-2-yl)-3-(6-azaspiro[2.5]octan-6-yl)phenyl)-2-hydroxyethane-1-sulfonamide (74 mg, yield: 62.0%).

LC-MS, M/Z (ESI): 590.2 [M+H]⁺.

¹H NMR (400 MHz, DMSO-d6) δ10.17 (s, 1H), 7.90 (d, 1H), 7.34 (s, 1H), 7.05 (d, 1H), 6.97-6.94 (dd, 1H), 4.93 (s, 1H),4.02 (t, 4H), 3.75 (t, 2H), 3.36 (t, 2H), 2.91 (t, 4H), 2.43 (s, 3H), 2.06-2.01 (m, 4H), 1.51 (s, 4H), 0.30 (s, 4H).

### Example 3: Preparation of Target Compound I-3

### N-(4-(5-(2-(3,3-Difluoroazetidin-1-yl)-6-methylpyrimidin-4-yl)-1,3,4-oxadiazol-2-yl)-3-(6-azaspiro[2.5]octan-6-yl)phenyl)-2-hydroxyethane-1-sulfonamide

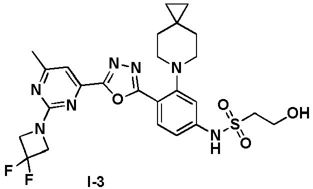

The synthetic route of the target compound **I-3** was as follows:

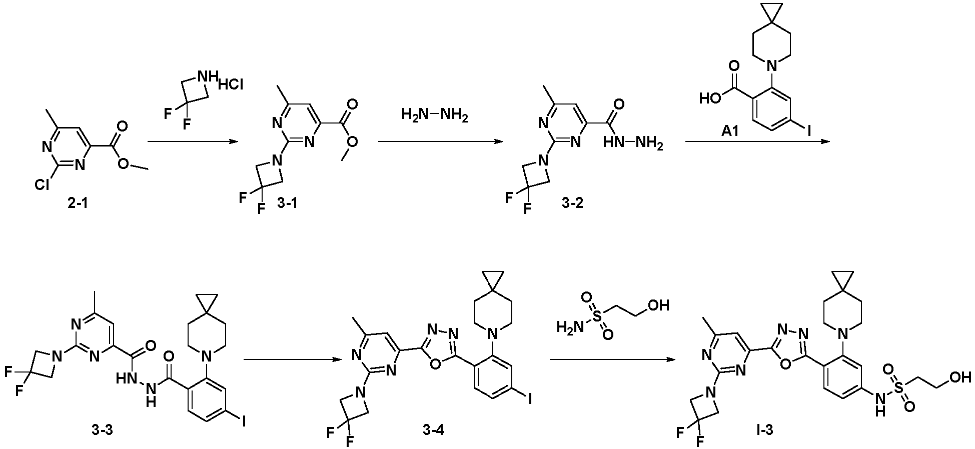

### Step 1: Synthesis of methyl 2-(3,3-difluoroazetidin-1-yl)-6-methylpyrimidine-4-carboxylate

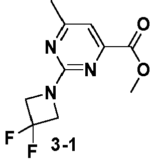

Methyl 2-chloro-6-methylpyrimidine-4-carboxylate (2 g, 10.72 mmol) was added to 20 mL of *N,N-*dimethylformamide, and then 3,3-difluoroazetidine hydrochloride (2.78 g, 21.44 mmol) and potassium carbonate (7.41 g, 53.6 mmol) were added. The mixture was heated to 100 °C and allowed to react for 2 h. LCMS showed that the starting materials were completely reacted. Water (30 mL) was added for dilution, and the mixture was extracted with ethyl acetate (50 mL × 2). The organic phase was concentrated to dryness, and the residue was separated and purified by a silica gel column (PE:EA = 5:1) to give methyl 2-(3,3-difluoroazetidin-1-yl)-6-methylpyrimidine-4-carboxylate (1.8 g, yield: 69%).

LC-MS, M/Z (ESI): 244.0 [M+H]⁺.

### Step 2: Synthesis of 2-(3,3-difluoroazetidin-1-yl)-6-methylpyrimidine-4-carbohydrazide

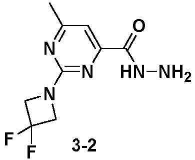

Methyl 2-(3,3-difluoroazetidin-1-yl)-6-methylpyrimidine-4-carboxylate (1.8 g, 7.4 mmol) was added to 20 mL of methanol, and then 4 mL of 80% hydrazine hydrate was added. The mixture was heated to 60 °C and allowed to react for 1 h. LCMS showed that the starting materials were completely reacted. The reaction liquid was concentrated to dryness, and the residue was lyophilized to give a crude product of 2-(3,3-difluoroazetidin-1-yl)-6-methylpyrimidine-4-carbohydrazide (1.8 g, yield: 100%).

LC-MS, M/Z (ESI): 244.0 [M+H]⁺.

### Step 3: Synthesis of 2-(3,3-difluoroazetidin-1-yl)-N'-(4-iodo-2-(6-azaspiro[2.5]octan-6-yl)benzoyl)-6-methylpyrimidine-4-carbohydrazide

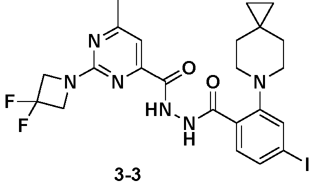

2-(3,3-Difluoroazetidin-1-yl)-6-methylpyrimidine-4-carbohydrazide (1.53 g, 6.3 mmol) was added to 15 mL of*N,N-*dimethylformamide, and then 4-iodo-2-(6-azaspiro[2.5]octan-6-yl)benzoic acid (1.5 g, 4.2 mmol), *N,N-*diisopropylethylamine (3.26 g, 25.2 mmol), and *O*-(7-azabenzotriazol-1-yl)-*N,N,N',N'*-tetramethyluronium hexafluorophosphate (4.79 g, 12.6 mmol) were added. The mixture was heated to 60 °C and allowed to react for 1 h. LCMS showed that the starting materials were completely reacted. Water (20 mL) was added for dilution, and the mixture was extracted with ethyl acetate (100 mL × 2). The organic phase was concentrated to dryness, and the residue was separated and purified by a silica gel column (PE:EA = 3:1) to give 2-(3,3-difluoroazetidin-1-yl)-*N'*-(4-iodo-2-(6-azaspiro[2.5]octan-6-yl)benzoyl)-6-methylpyrimidine-4-carbohydrazide (1.7 g, yield: 69.5%).

LC-MS, M/Z (ESI): 583.1 [M+H]⁺.

### Step 4: Synthesis of 2-(2-(3,3-difluoroazetidin-1-yl)-6-methylpyrimidin-4-yl)-5-(4-iodo-2-(6-azaspiro[2.5]octan-6-yl)phenyl)-1,3,4-oxadiazole

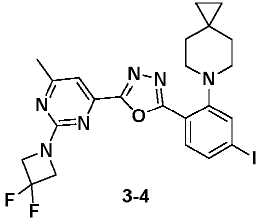

2-(3,3-Difluoroazetidin-1-yl)-*N'*-(4-iodo-2-(6-azaspiro[2.5]octan-6-yl)benzoyl)-6-methylpyrimidine-4-carbohydrazide (0.3 g, 0.515 mmol) was added to 5 mL of dichloromethane, and then p-methylbenzenesulfonyl chloride (0.295 g, 1.545 mmol) and triethylamine (0.313 g, 3.09 mmol) were added. The mixture was allowed to react at room temperature overnight. LCMS showed that the starting materials were completely reacted. Water (5 mL) was added for dilution, and the mixture was extracted with ethyl acetate (10 mL × 2). The organic phase was concentrated to dryness, and the residue was separated and purified by a silica gel column (PE:EA = 3:1) to give 2-(2-(3,3-difluoroazetidin-1-yl)-6-methylpyrimidin-4-yl)-5-(4-iodo-2-(6-azaspiro[2.5]octan-6-yl)phenyl)-1,3,4-oxadiazole (200 mg, yield: 68.8%).

LC-MS, M/Z (ESI): 565.0 [M+H]⁺.

### Step 5: Synthesis of N-(4-(5-(2-(3,3-difluoroazetidin-1-yl)-6-methylpyrimidin-4-yl)-1,3,4-oxadiazol-2-yl)-3-(6-azaspiro[2.5]octan-6-yl)phenyl)-2-hydroxyethane-1-sulfonamide

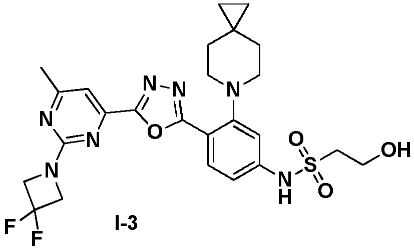

2-(2-(3,3-Difluoroazetidin-1-yl)-6-methylpyrimidin-4-yl)-5-(4-iodo-2-(6-azaspiro[2.5]octan-6-yl)phenyl)-1,3,4-oxadiazole (0.2 g, 0.354 mmol) was added to 5 mL of *N,N*-dimethylformamide, and then 2-hydroxyethane-1-sulfonamide (89 mg, 0.709 mmol), N,N-dimethylglycine (73 mg, 0.709 mmol), copper(I) iodide (67 mg, 0.354 mmol), and potassium carbonate (196 mg, 1.418 mmol) were added. The mixture was heated to 130 °C and allowed to react for 1 h. LCMS showed that the starting materials were completely reacted. Water (5 mL) was added for dilution, and the mixture was extracted with ethyl acetate (10 mL × 2). The organic phase was concentrated to dryness, and the residue was separated and purified by a silica gel column (PE:EA = 1:1) to give *N*-(4-(5-(2-(3,3-difluoroazetidin-1-yl)-6-methylpyrimidin-4-yl)-1,3,4-oxadiazol-2-yl)-3-(6-azaspiro[2.5]octan-6-yl)phenyl)-2-hydroxyethane-1-sulfonamide (90 mg, yield: 45.2%).

LC-MS, M/Z (ESI): 562.2 [M+H]⁺.

¹H NMR (400 MHz, DMSO-d6) δ7.93 (d, 1H), 7.50 (s, 1H), 7.02 (d, 1H), 6.93-6.91(dd, 1H), 4.56 (t, 4H), 3.75 (t, 2H), 3.16(t, 2H), 2.91 (t, 4H), 2.50 (d, 3H), 1.57 (s, 4H), 0.31 (s, 4H).

### Example 4: Preparation of Target Compound I-5

### N-(4-(5-(2-(3,3-Difluoropyrrolidin-1-yl)-6-methylpyrimidin-4-yl)-1,3,4-oxadiazol-2-yl)-3-(6-azaspiro[2.5]octan-6-yl)phenyl)-2-hydroxyethane-1-sulfonamide

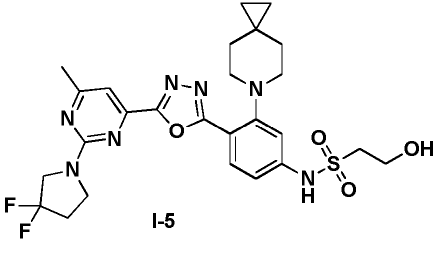

The synthetic route of the target compound **I-5** was as follows:

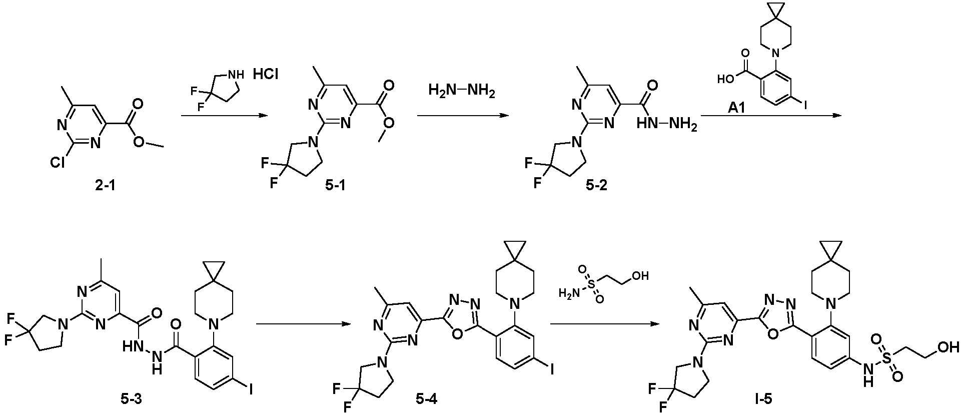

### Step 1: Synthesis of methyl 2-(3,3-difluoropyrrolidin-1-yl)-6-methylpyrimidine-4-carboxylate

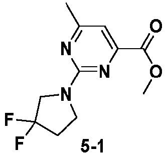

Methyl 2-chloro-6-methylpyrimidine-4-carboxylate (2 g, 10.72 mmol) was added to 20 mL of *N,N-*dimethylformamide, and then 3,3-difluoropyrrolidine hydrochloride (3.08 g, 21.44 mmol) and potassium carbonate (7.41 g, 53.6 mmol) were added. The mixture was heated to 100 °C and allowed to react for 2 h. LCMS showed that the starting materials were completely reacted. Water (30 mL) was added for dilution, and the mixture was extracted with ethyl acetate (50 mL × 2). The organic phase was concentrated to dryness, and the residue was separated and purified by a silica gel column (PE:EA = 5:1) to give methyl 2-(3,3-difluoropyrrolidin-1-yl)-6-methylpyrimidine-4-carboxylate (1.9 g, yield: 68.9%).

LC-MS, M/Z (ESI): 258.1 [M+H]⁺.

### Step 2: Synthesis of 2-(3,3-difluoropyrrolidin-1-yl)-6-methylpyrimidine-4-carbohydrazide

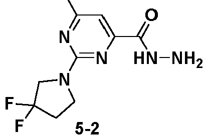

Methyl 2-(3,3-difluoropyrrolidin-1-yl)-6-methylpyrimidine-4-carboxylate (1.9 g, 7.39 mmol) was added to 20 mL of methanol, and then 4 mL of 80% hydrazine hydrate was added. The mixture was heated to 60 °C and allowed to react for 1 h. LCMS showed that the starting materials were completely reacted. The reaction liquid was concentrated to dryness, and the residue was lyophilized to give a crude product of 2-(3,3-difluoropyrrolidin-1-yl)-6-methylpyrimidine-4-carbohydrazide (1.9 g, yield: 100%).

LC-MS, M/Z (ESI): 258.1 [M+H]⁺.

### Step 3: Synthesis of 2-(3,3-difluoropyrrolidin-1-yl)-N'-(4-iodo-2-(6-azaspiro[2.5]octan-6-yl)benzoyl)-6-methylpyrimidine-4-carbohydrazide

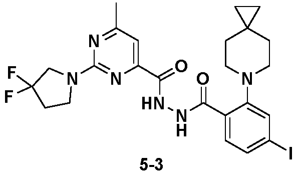

2-(3,3-Difluoropyrrolidin-1-yl)-6-methylpyrimidine-4-carbohydrazide (1.62 g, 6.3 mmol) was added to 15 mL of N,N-dimethylformamide, and then 4-iodo-2-(6-azaspiro[2.5]octan-6-yl)benzoic acid (1.5 g, 4.2 mmol), *N,N-*diisopropylethylamine (3.26 g, 25.2 mmol), and *O*-(7-azabenzotriazol-1-yl)-*N,N,N',N'*-tetramethyluronium hexafluorophosphate (4.79 g, 12.6 mmol) were added. The mixture was heated to 60 °C and allowed to react for 1 h. LCMS showed that the starting materials were completely reacted. Water (20 mL) was added for dilution, and the mixture was extracted with ethyl acetate (100 mL × 2). The organic phase was concentrated to dryness, and the residue was separated and purified by a silica gel column (PE:EA = 3:1) to give 2-(3,3-difluoropyrrolidin-1-yl)-N-(4-iodo-2-(6-azaspiro[2.5]octan-6-yl)benzoyl)-6-methylpyrimidine-4-carbohydrazide (1.6 g, yield: 63.9%).

LC-MS, M/Z (ESI): 597.1 [M+H]⁺.

### Step 4: Synthesis of 2-(2-(3,3-difluoropyrrolidin-1-yl)-6-methylpyrimidin-4-yl)-5-(4-iodo-2-(6-azaspiro[2.5]octan-6-yl)phenyl)-1,3,4-oxadiazole

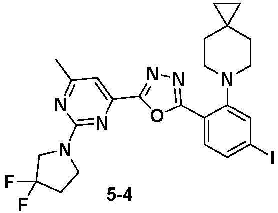

2-(3,3-Difluoropyrrolidin-1-yl)-*N*'-(4-iodo-2-(6-azaspiro[2.5]octan-6-yl)benzoyl)-6-methylpyrimidine-4-carbohydrazide (0.3 g, 0.503 mmol) was added to 5 mL of dichloromethane, and then p-methylbenzenesulfonyl chloride (0.288 g, 1.509 mmol) and triethylamine (0.305 g, 3.02 mmol) were added. The mixture was allowed to react at room temperature overnight. LCMS showed that the starting materials were completely reacted. Water (5 mL) was added for dilution, and the mixture was extracted with ethyl acetate (10 mL × 2). The organic phase was concentrated to dryness, and the residue was separated and purified by a silica gel column (PE:EA = 3:1) to give 2-(2-(3,3-difluoropyrrolidin-1-yl)-6-methylpyrimidin-4-yl)-5-(4-iodo-2-(6-azaspiro[2.5]octan-6-yl)phenyl)-1,3,4-oxadiazole (160 mg, yield: 55%).

LC-MS, M/Z (ESI): 579.1 [M+H]⁺.

### Step 5: Synthesis of N-(4-(5-(2-(3,3-difluoropyrrolidin-1-yl)-6-methylpyrimidin-4-yl)-1,3,4-oxadiazol-2-yl)-3-(6-azaspiro[2.5]octan-6-yl)phenyl)-2-hydroxyethane-1-sulfonamide

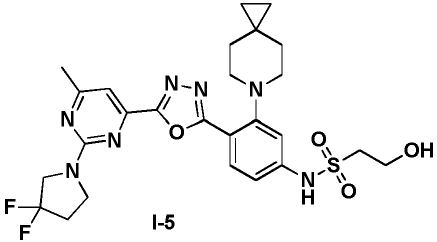

2-(2-(3,3-Difluoropyrrolidin-1-yl)-6-methylpyrimidin-4-yl)-5-(4-iodo-2-(6-azaspiro[2.5]octan-6-yl)phenyl)-1,3,4-oxadiazole (0.16 g, 0.277 mmol) was added to 5 mL of N,N-dimethylformamide, and then 2-hydroxyethane-1-sulfonamide (69 mg, 0.553 mmol), dimethylglycine (57 mg, 0.553 mmol), copper(I) iodide (53 mg, 0.277 mmol), and potassium carbonate (153 mg, 1.107 mmol) were added. The mixture was heated to 130 °C and allowed to react for 1 h. LCMS showed that the starting materials were completely reacted. Water (5 mL) was added for dilution, and the mixture was extracted with ethyl acetate (10 mL × 2). The organic phase was concentrated to dryness, and the residue was separated and purified by a silica gel column (PE:EA = 1:1) to give N-(4-(5-(2-(3,3-difluoropyrrolidin-1-yl)-6-methylpyrimidin-4-yl)-1,3,4-oxadiazol-2-yl)-3-(6-azaspiro[2.5]octan-6-yl)phenyl)-2-hydroxyethane-1-sulfonamide (80 mg, yield: 50.2%).

LC-MS, M/Z (ESI): 576.2 [M+H]⁺.

¹H NMR (400 MHz, DMSO-d6) δ7.86 (d, 1H), 7.38 (s, 1H), 6.88 (d, 1H), 6.81-6.79(dd, 1H), 4.01 (t, 2H), 3.82 (s, 2H), 3.73 (t, 2H), 3.19 (t, 2H), 2.89 (t, 4H), 2.60-2.54(m, 2H), 2.43(s, 3H), 1.58(s, 4H), 0.30 (s, 4H).

### Example 5: Preparation of Target Compound I-7

### N-(4-(5-(2-(3,3-Difluoropiperidin-1-yl)-6-methylpyrimidin-4-yl)-1,3,4-oxadiazol-2-yl)-3-(6-azaspiro[2.5]octan-6-yl)phenyl)-2-hydroxyethane-1-sulfonamide

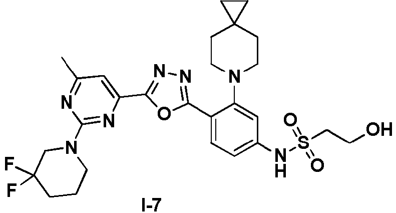

The synthetic route of the target compound **I-7** was as follows:

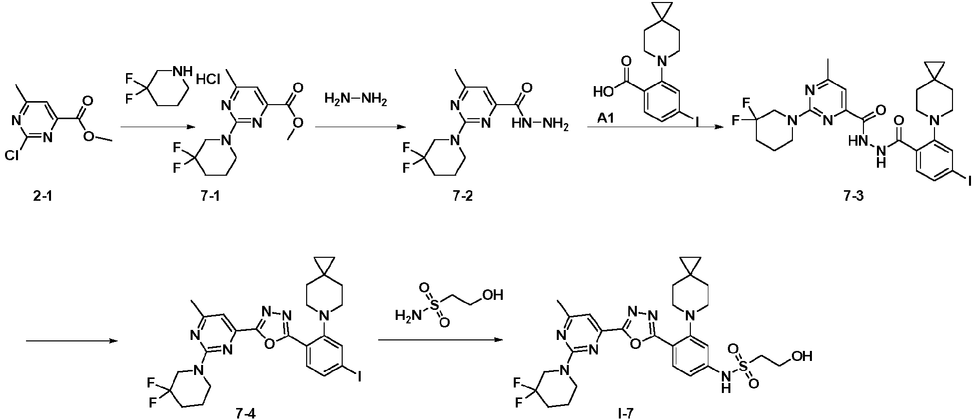

### Step 1: Synthesis of methyl 2-(3,3-difluoropiperidin-1-yl)-6-methylpyrimidine-4-carboxylate

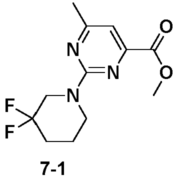

Methyl 2-chloro-6-methylpyrimidine-4-carboxylate (2 g, 10.72 mmol) was added to 20 mL of *N,N-*dimethylformamide, and then 3,3-difluoropiperidine hydrochloride (3.38 g, 21.44 mmol) and potassium carbonate (7.41 g, 53.6 mmol) were added. The mixture was heated to 100 °C and allowed to react for 2 h. LCMS showed that the starting materials were completely reacted. Water (30 mL) was added for dilution, and the mixture was extracted with ethyl acetate (50 mL × 2). The organic phase was concentrated to dryness, and the residue was separated and purified by a silica gel column (PE:EA = 5:1) to give methyl 2-(3,3-difluoropiperidin-1-yl)-6-methylpyrimidine-4-carboxylate (2 g, yield: 68.8%).

LC-MS, M/Z (ESI): 272.1 [M+H]⁺.

### Step 2: Synthesis of 2-(3,3-difluoropiperidin-1-yl)-6-methylpyrimidine-4-carbohydrazide

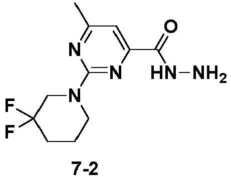

Methyl 2-(3,3-difluoropiperidin-1-yl)-6-methylpyrimidine-4-carboxylate (2 g, 7.4 mmol) was added to 20 mL of methanol, and then 4 mL of 80% hydrazine hydrate was added. The mixture was heated to 60 °C and allowed to react for 1 h. LCMS showed that the starting materials were completely reacted. The reaction liquid was concentrated to dryness, and the residue was lyophilized to give a crude product of 2-(3,3-difluoropiperidin-1-yl)-6-methylpyrimidine-4-carbohydrazide (2 g, yield: 100%).

LC-MS, M/Z (ESI): 272.1 [M+H]⁺.

### Step 3: Synthesis of 2-(3,3-difluoropiperidin-1-yl)-N'-(4-iodo-2-(6-azaspiro[2.5]octan-6-yl)benzoyl)-6-methylpyrimidine-4-carbohydrazide

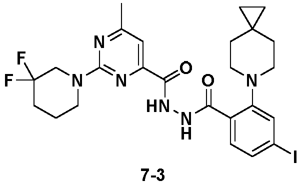

2-(3,3-Difluoropiperidin-1-yl)-6-methylpyrimidine-4-carbohydrazide (1.71 g, 6.3 mmol) was added to 15 mL *of N,N-*dimethylformamide, and then 4-iodo-2-(6-azaspiro[2.5]octan-6-yl)benzoic acid (1.5 g, 4.2 mmol), *N,N-*diisopropylethylamine (3.26 g, 25.2 mmol), and *O*-(7-azabenzotriazol-1-yl)-*N,N,N',N'*-tetramethyluronium hexafluorophosphate (4.79 g, 12.6 mmol) were added. The mixture was heated to 60 °C and allowed to react for 1 h. LCMS showed that the starting materials were completely reacted. Water (20 mL) was added for dilution, and the mixture was extracted with ethyl acetate (100 mL × 2). The organic phase was concentrated to dryness, and the residue was separated and purified by a silica gel column (PE:EA = 3:1) to give 2-(3,3-difluoropiperidin-1-yl)-*N*'-(4-iodo-2-(6-azaspiro[2.5]octan-6-yl)benzoyl)-6-methylpyrimidine-4-carbohydrazide (1.9 g, yield: 74.1%).

LC-MS, M/Z (ESI): 611.1 [M+H]⁺.

### Step 4: Synthesis of 2-(2-(3,3-difluoropiperidin-1-yl)-6-methylpyrimidin-4-yl)-5-(4-iodo-2-(6-azaspiro[2.5]octan-6-yl)phenyl)-1,3,4-oxadiazole

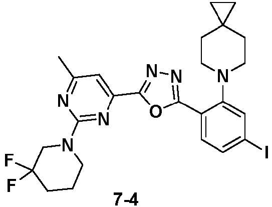

2-(3,3-Difluoropiperidin-l-yl)-*N'*-(4-iodo-2-(6-azaspiro[2.5]octan-6-yl)benzoyl)-6-methylpyrimidine-4-carbohydrazide (0.3 g, 0.491 mmol) was added to 5 mL of dichloromethane, and then p-methylbenzenesulfonyl chloride (0.281 g, 1.474 mmol) and triethylamine (0.298 g, 2.95 mmol) were added. The mixture was allowed to react at room temperature overnight. LCMS showed that the starting materials were completely reacted. Water (5 mL) was added for dilution, and the mixture was extracted with ethyl acetate (10 mL × 2). The organic phase was concentrated to dryness, and the residue was separated and purified by a silica gel column (PE:EA = 3:1) to give 2-(2-(3,3-difluoropiperidin-1-yl)-6-methylpyrimidin-4-yl)-5-(4-iodo-2-(6-azaspiro[2.5]octan-6-yl)phenyl)-1,3,4-oxadiazole (130 mg, yield: 44.7%).

LC-MS, M/Z (ESI): 593.1 [M+H]⁺.

### Step 5: Synthesis of N-(4-(5-(2-(3,3-difluoropiperidin-1-yl)-6-methylpyrimidin-4-yl)-1,3,4-oxadiazol-2-yl)-3-(6-azaspiro[2.5]octan-6-yl)phenyl)-2-hydroxyethane-1-sulfonamide

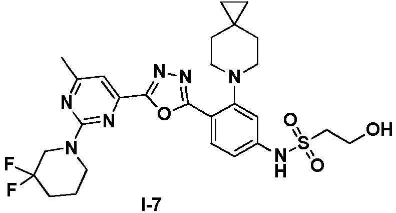

2-(2-(3,3-Difluoropiperidin-1-yl)-6-methylpyrimidin-4-yl)-5-(4-iodo-2-(6-azaspiro[2.5]octan-6-yl)phenyl)-1,3,4-oxadiazole (0.13 g, 0.219 mmol) was added to 5 mL of N,N-dimethylformamide, and then 2-hydroxyethane-1-sulfonamide (55 mg, 0.439 mmol), dimethylglycine (45 mg, 0.439 mmol), copper(I) iodide (42 mg, 0.219 mmol), and potassium carbonate (121 mg, 0.878 mmol) were added. The mixture was heated to 130 °C and allowed to react for 1 h. LCMS showed that the starting materials were completely reacted. Water (5 mL) was added for dilution, and the mixture was extracted with ethyl acetate (10 mL × 2). The organic phase was concentrated to dryness, and the residue was separated and purified by a silica gel column (PE:EA = 1:1) to give *N*-(4-(5-(2-(3,3-difluoropiperidin-1-yl)-6-methylpyrimidin-4-yl)-1,3,4-oxadiazol-2-yl)-3-(6-azaspiro[2.5]octan-6-yl)phenyl)-2-hydroxyethane-1-sulfonamide (30 mg, yield: 23.2%).

LC-MS, M/Z (ESI): 590.2 [M+H]⁺.

¹H NMR (400 MHz, DMSO-d6) δ7.72 (d, 1H), 7.32 (s, 1H), 6.76 (d, 1H), 6.70-6.67(dd, 1H), 4.20 (t, 2H), 3.93 (t, 2H), 3.70 (t, 2H), 3.06 (t, 2H), 2.87 (t, 4H), 2.42 (s, 3H), 2.14-2.11 (m, 2H), 2.09 (s, 2H), 1.52 (s, 4H), 0.28 (s, 4H).

### Example 6: Preparation of Target Compound I-17

### 2-Hydroxy-N-(4-(5-(6-methyl-2-(5-azaspiro[2.4]heptan-5-yl)pyrimidin-4-yl)-1,3,4-oxadiazol-2-yl)-3-(6-azaspiro[2.5]octan-6-yl)phenyl)ethane-1-sulfonamide

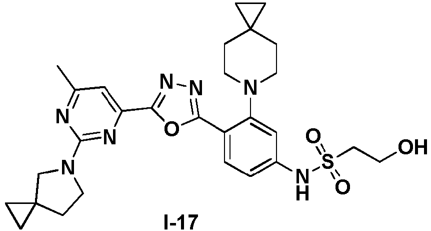

The synthetic route of the target compound **I-17** was as follows:

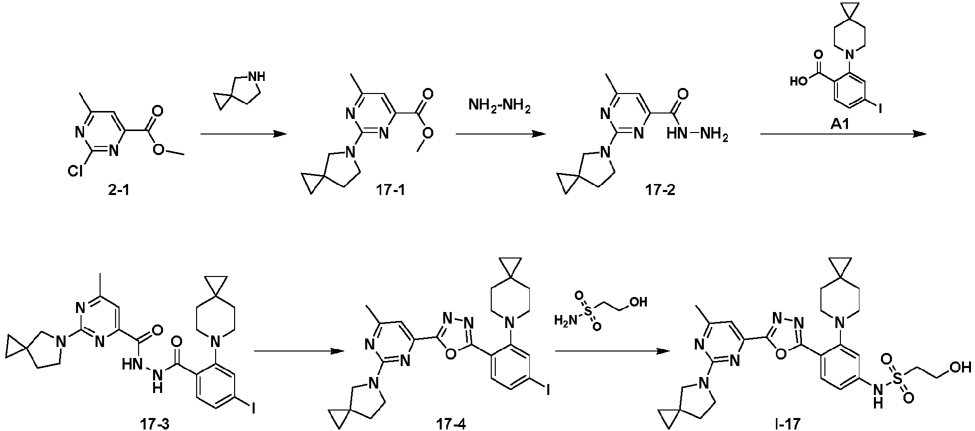

### Step 1: Synthesis of methyl 6-methyl-2-(5-azaspiro[2.4]heptan-5-yl)pyrimidine-4-carboxylate

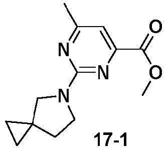

Methyl 2-chloro-6-methylpyrimidine-4-carboxylate (1 g, 5.4 mmol), 5-azaspiro[2.4]heptane (700 mg, 8.0 mmol), and N,N-diisopropylethylamine (1.4 g, 10.8 mol) were added to acetonitrile (15 mL), and the mixture was stirred at 60 °C for 14 h. Water (100 mL) was added for dilution, and the mixture was extracted with ethyl acetate (30 mL × 2). The organic phase was concentrated to dryness, and the residue was separated and purified by a silica gel column (petroleum ether:ethyl acetate = 50:1) to give methyl 6-methyl-2-(5-azaspiro[2.4]heptan-5-yl)pyrimidine-4-carboxylate (1.0 g, yield: 81%).

LC-MS, M/Z (ESI): 248.21 [M+H]⁺.

### Step 2: Synthesis of 6-methyl-2-(5-azaspiro[2.4]heptan-5-yl)pyrimidine-4-carbohydrazide

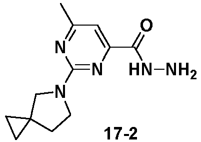

Methyl 6-methyl-2-(5-azaspiro[2.4]heptan-5-yl)pyrimidine-4-carboxylate (1.0 g, 4 mmol) and hydrazine hydrate (purity: 80%, 1.6 g, 40 mmol) were added to ethanol (10 mL), and the mixture was allowed to react at 60 °C for 2 h. Water (60 mL) was added for dilution, and the mixture was extracted with ethyl acetate (30 mL × 2). The organic phase was concentrated to dryness, and the residue was separated and purified by a silica gel column (petroleum ether:ethyl acetate = 10:1) to give 6-methyl-2-(5-azaspiro[2.4]heptan-5-yl)pyrimidine-4-carbohydrazide (500 mg, yield: 50%).

LC-MS, M/Z (ESI): 248.3 [M+H]⁺.

### Step 3: Synthesis of N'-(4-iodo-2-(6-azaspiro[2.5]octan-6-yl)benzoyl)-6-methyl-2-(5-azaspiro[2.4]heptan-3-yl)pyrimidine-4-carbohydrazide

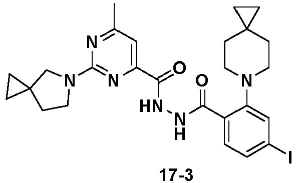

6-Methyl-2-(5-azaspiro[2.4]heptan-5-yl)pyrimidine-4-carbohydrazide (200 mg, 0.9 mmol), 4-iodo-2-(6-azaspiro[2.5]octan-6-yl)benzoic acid (0.2 g, 0.6 mmol), *O*-(7-azabenzotriazol-1-yl)-*N*,*N*,*N'*,*N'*-tetramethyluronium hexafluorophosphate (450 mg, 1.0 mmol), and N,N-diisopropylethylamine (232 mg, 1.8 mmol) were dissolved in N,N-dimethylformamide (3 mL), and the solution was stirred at room temperature for 2 h. Water (30 mL) was then added for dilution, and the mixture was extracted with dichloromethane (20 mL × 2). The organic phase was concentrated to dryness, and the residue was separated and purified by a silica gel column (petroleum ether:ethyl acetate = 10:1) to give *N'*-(4-iodo-2-(6-azaspiro[2.5]octan-6-yl)benzoyl)-6-methyl-2-(5-azaspiro[2.4]heptan-5-yl)pyrimidine-4-carbohydrazide (150 mg, yield: 50%).

LC-MS, M/Z (ESI): 587.2 [M+H]⁺.

### Step 4: Synthesis of 2-(4-iodo-2-(6-azaspiro[2.5]octan-6-yl)phenyl)-5-(6-methyl-2-(5-azaspiro[2.4]heptan-5-yl)pyrimidin-4-yl)-1,3,4-oxadiazole

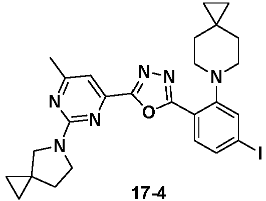

*N'*-(4-Iodo-2-(6-azaspiro[2.5]octan-6-yl)benzoyl)-6-methyl-2-(5-azaspiro[2.4]heptan-5-yl)pyrimidine-4-carbohydrazide (150 mg, 0.3 mmol),p-toluenesulfonyl chloride (175 mg, 0.9 mmol), and triethylamine (120 mg, 1.2 mmol) were added to 2 mL of dichloromethane, and the mixture was allowed to react at room temperature for 1 h. The reaction liquid was then directly concentrated to dryness, and the residue was separated and purified by a silica gel column (petroleum ether:ethyl acetate = 1:1) to give 2-(4-iodo-2-(6-azaspiro[2.5]octan-6-yl)phenyl)-5-(6-methyl-2-(5-azaspiro[2.4]heptan-5-yl)pyrimidin-4-yl)-1,3,4-oxadiazole (100 mg, yield: 60%).

LC-MS, M/Z (ESI): 569.2 [M+H]⁺.

### Step 5: Synthesis of 2-hydroxy-N-(4-(5-(6-methyl-2-(5-azaspiro[2.4]heptan-5-yl)pyrimidin-4-yl)-1,3,4-oxadiazol-2-yl)-3-(6-azaspiro[2.5]octan-6-yl)phenyl)ethane-1-sulfonamide

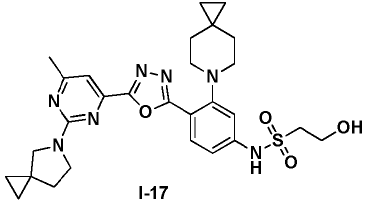

2-(4-Iodo-2-(6-azaspiro[2.5]octan-6-yl)phenyl)-5-(6-methyl-2-(5-azaspiro[2.4]heptan-5-yl)pyrimidin-4-yl)-1,3,4-oxadiazole (100 mg, 0.2 mmol) was added to 2 mL of *N,N*-dimethylformamide, and then 2-hydroxyethane-1-sulfonamide (100 mg, 0.8 mmol), *trans-N,N*-dimethylcyclohexane-1,2-diamine (15 mg, 0.04 mmol), copper(I) iodide (20 mg, 0.08 mmol), and potassium phosphate (170 mg, 0.8 mmol) were added. The mixture was heated to 120 °C and allowed to react for 1 h. Water (10 mL) was added for dilution, and the mixture was extracted with ethyl acetate (10 mL × 2). The organic phase was concentrated to dryness, and the residue was separated and purified by a silica gel column (ethyl acetate:methanol = 10:1) to give 2-hydroxy-N-(4-(5-(6-methyl-2-(5-azaspiro[2.4]heptan-5-yl)pyrimidin-4-yl)-1,3,4-oxadiazol-2-yl)-3-(6-azaspiro[2.5]octan-6-yl)phenyl)ethane-1-sulfonamide (40 mg, yield: 32%).

LC-MS, M/Z (ESI): 566.3 [M+H]⁺.

¹H NMR (400 MHz, DMSO-d6) δ10.16 (s, 1H), 7.92 (s, 1H), 7.25 (s, 1H), 7.04 (m, 1H), 6.96 (m, 1H), 4.93 (s, 1H), 3.77 (m, 4H), 3.54 (m, 2H), 3.37 (m, 2H), 2.90 (m, 4H), 2.39 (m, 3H), 1.92 (m, 2H), 1.53 (m, 4H), 0.63 (m, 4H), 0.28 (m, 4H).

### Example 7: Preparation of Target Compound I-52

### N-(4-(5-(2-(4,4-Difluoropiperidin-1-yl)-6-methylpyridin-4-yl)-1,3,4-oxadiazol-2-yl)-3-(6-azaspiro[2.5]octan-6-yl)phenyl)-2-hydroxyethane-1-sulfonamide

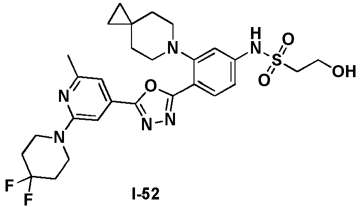

The synthetic route of the target compound **I-52** was as follows:

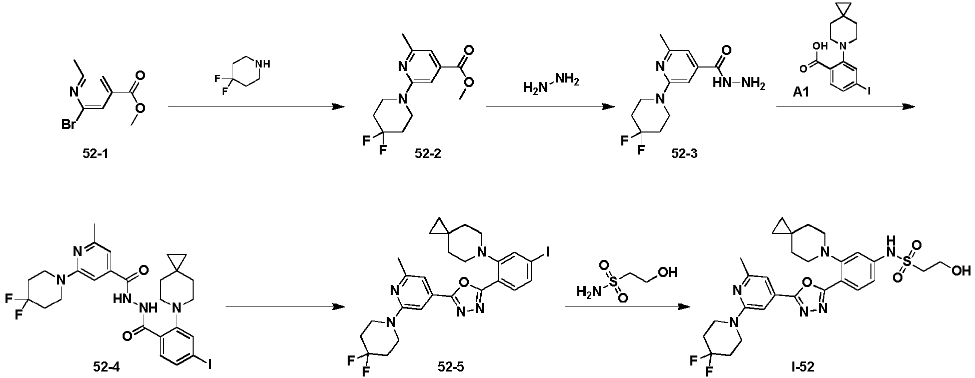

### Step 1: Synthesis of methyl 2-(4,4-Difluoropiperidin-1-yl)-6-methylisonicotinate

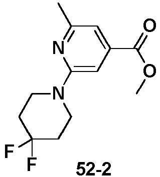

Methyl 2-bromo-6-methylisonicotinate (5 g, 21.7 mmol), 4,4-difluoropiperidine hydrochloride (5.1 g, 32.6 mmol), tris(dibenzylideneacetone)dipalladium (2.0 g, 2.17 mmol), potassium carbonate (9.0 g, 65 mmol), and dicyclohexyl(2,4,6-triisopropyl-[1,1-biphenyl]-2-yl)phosphine (2.07 g, 4.34 mmol) were added to *N,N-*dimethylformamide (50 mL), and the mixture was stirred at 120 °C for 2 h. Water (500 mL) was added for dilution, and the mixture was extracted with ethyl acetate (50 mL × 2). The organic phase was concentrated to dryness, and the residue was separated and purified by a silica gel column (petroleum ether:ethyl acetate = 50:1) to give methyl 2-(4,4-difluoropiperidin-1-yl)-6-methylisonicotinate (300 mg, yield: 5%).

LC-MS, M/Z (ESI): 271.16 [M+H]⁺.

### Step 2: Synthesis of 2-(4,4-difluoropiperidin-1-yl)-6-methylisonicotinohydrazide

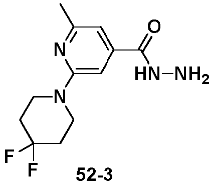

Methyl 2-(4,4-difluoropiperidin-1-yl)-6-methylisonicotinate (270 mg, 1 mmol) and hydrazine hydrate (purity: 80%, 160 mg, 4 mmol) were added to ethanol (3 mL), and the mixture was allowed to react at 60 °C for 2 h. Water (20 mL) was added for dilution, and the mixture was extracted with ethyl acetate (10 mL × 2). The organic phase was concentrated to dryness, and the residue was separated and purified by a silica gel column (petroleum ether:ethyl acetate = 10:1) to give 2-(4,4-difluoropiperidin-1-yl)-6-methylisonicotinohydrazide (250 mg, yield: 90%).

LC-MS, M/Z (ESI): 271.20 [M+H]⁺.

### Step 3: Synthesis of 2-(4,4-difluoropiperidin-1-yl)-N'-(4-iodo-2-(6-azaspiro[2.5]octan-6-yl)benzoyl)-6-methylisonicotinohydrazide

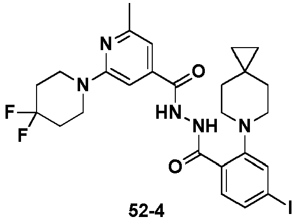

2-(4,4-Difluoropiperidin-1-yl)-6-methylisonicotinohydrazide (200 mg, 0.74 mmol), 4-iodo-2-(6-azaspiro[2.5]octan-6-yl)benzoic acid (0.23 g, 0.7 mmol), *O*-(7-azabenzotriazol-1-yl)-*N*,*N*,*N'*,*N'*-tetramethyluronium hexafluorophosphate (450 mg, 1.0 mmol), and N,N-diisopropylethylamine (232 mg, 1.8 mmol) were dissolved in N,N-dimethylformamide (3 mL), and the solution was stirred at room temperature for 2 h. Water (30 mL) was then added for dilution, and the mixture was extracted with dichloromethane (20 mL × 2). The organic phase was concentrated to dryness, and the residue was separated and purified by a silica gel column (petroleum ether:ethyl acetate = 10:1) to give 2-(4,4-difluoropiperidin-1-yl)-*N'*-(4-iodo-2-(6-azaspiro[2.5]octan-6-yl)benzoyl)-6-methylisonicotinohydrazide (100 mg, yield: 40%).

LC-MS, M/Z (ESI): 610.2 [M+H]⁺.

### Step 4: Synthesis of 2-(2-(4,4-difluoropiperidin-1-yl)-6-methylpyridin-4-yl)-5-(4-iodo-2-(6-azaspiro[2.5]octan-6-yl)phenyl)-1,3,4-oxadiazole

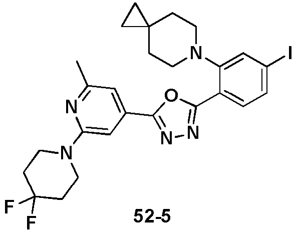

2-(4,4-Difluoropiperidin-1-yl)-*N*'-(4-iodo-2-(6-azaspiro[[2.5]octan-6-yl)benzoyl)-6-methylisonicotinohydrazide (100 mg, 0.3 mmol), p-toluenesulfonyl chloride (175 mg, 0.9 mmol), and triethylamine (120 mg, 1.2 mmol) were added to 2 mL of dichloromethane, and the mixture was allowed to react at room temperature for 1 h. The reaction liquid was then directly concentrated to dryness, and the residue was separated and purified by a silica gel column (petroleum ether:ethyl acetate = 1:1) to give 2-(2-(4,4-difluoropiperidin-1-yl)-6-methylpyridin-4-yl)-5-(4-iodo-2-(6-azaspiro[2.5]octan-6-yl)phenyl)-1,3,4-oxadiazole (60 mg, yield: 60%).

LC-MS, M/Z (ESI): 592.2 [M+H]⁺.

### Step 5: Synthesis of N-(4-(5-(2-(4,4-difluoropiperidin-1-yl)-6-methylpyridin-4-yl)-1,3,4-oxadiazol-2-yl)-3-(6-azaspiro[2.5]octan-6-yl)phenyl)-2-hydroxyethane-1-sulfonamide

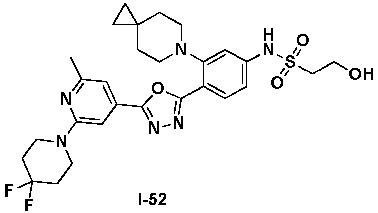

2-(2-(4,4-Difluoropiperidin-1-yl)-6-methylpyridin-4-yl)-5-(4-iodo-2-(6-azaspiro[2.5]octan-6-yl)phenyl)-1,3,4-oxadiazole (60 mg, 0.2 mmol) was added to 2 mL of N,N-dimethylformamide, and then 2-hydroxyethane-1-sulfonamide (100 mg, 0.8 mmol), *trans*-*N*,*N'*-dimethylcyclohexane-1,2-diamine (15 mg, 0.04 mmol), copper(I) iodide (20 mg, 0.08 mmol), and potassium phosphate (170 mg, 0.8 mmol) were added. The mixture was heated to 120 °C and allowed to react for 1 h. Water (10 mL) was added for dilution, and the mixture was extracted with ethyl acetate (10 mL × 2). The organic phase was concentrated to dryness, and the residue was separated and purified by a silica gel column (ethyl acetate:methanol = 10:1) to give N-(4-(5-(2-(4,4-difluoropiperidin-1-yl)-6-methylpyridin-4-yl)-1,3,4-oxadiazol-2-yl)-3-(6-azaspiro[2.5]octan-6-yl)phenyl)-2-hydroxyethane-1-sulfonamide (18 mg, yield: 12%).

LC-MS, M/Z (ESI): 589.2 [M+H]⁺.

¹H NMR (400 MHz, DMSO-d6) δ10.15 (s, 1H), 7.91 (d, 1H), 7.22 (m, 3H), 6.98 (d, 1H), 4.93 (m, 1H), 3.76 (m, 6H), 3.36 (m, 2H), 2.92 (m, 4H), 2.48 (m, 3H), 2.06 (m, 4H), 1.45 (m, 4H), 0.29 (m, 4H).

### Example 8: Preparation of Target Compound I-65

### N-(4-(5-(2-(4,4-Difluoropiperidin-1-yl)-6-methylpyrimidin-4-yl)oxazol-2-yl)-3-(6-azaspiro[2.5]octan-6-yl)phenyl)-2-hydroxyethane-1-sulfonamide

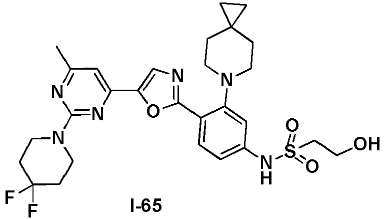

The synthetic route of the target compound **I-65** was as follows:

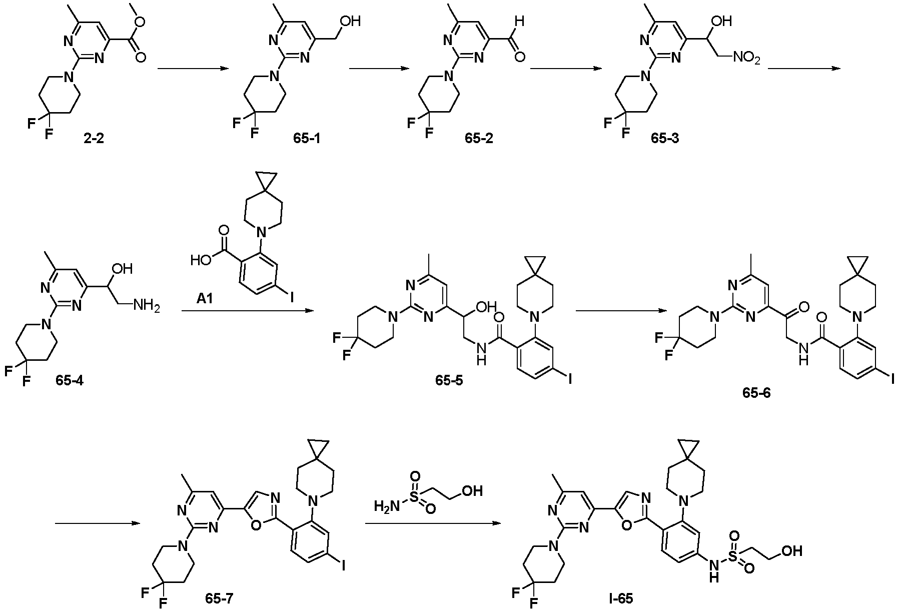

### Step 1: Synthesis of (2-(4,4-difluoropiperidin-1-yl)-6-methylpyrimidin-4-yl)methanol

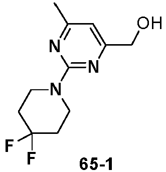

Methyl 2-(4,4-difluoropiperidin-1-yl)-6-methylpyrimidine-4-carboxylate (1 g, 3.7 mmol) was dissolved in methanol (20 mL), and sodium borohydride (422 mg, 11.1 mmol) was added. The mixture was allowed to react at room temperature overnight. Water (100 mL) was added to quench the reaction, and the mixture was extracted with ethyl acetate (100 mL × 3). The organic phases were combined and concentrated, and the residue was purified by a silica gel column (petroleum ether:ethyl acetate (V/V) = 5:1) to give (2-(4,4-difluoropiperidin-1-yl)-6-methylpyrimidin-4-yl)methanol (850 mg, yield: 94.8%).

LC-MS, M/Z (ESI): 244.2 [M+H]⁺.

### Step 2: Synthesis of 2-(4,4-difluoropiperidin-1-yl)-6-methylpyrimidine-4-carbaldehyde

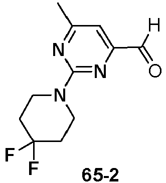

(2-(4,4-Difluoropiperidin-1-yl)-6-methylpyrimidin-4-yl)methanol (850 mg, 3.5 mmol) was dissolved in dry dichloromethane (10 mL), and Dess-Martin periodinane (2.22 g, 5.24 mmol) was added. The mixture was stirred and allowed to react at room temperature for 20 h. The mixture was filtered through celite, and the filter cake was washed with ethyl acetate. The filtrate was collected and concentrated, and the residue was separated by column chromatography (petroleum ether:ethyl acetate (V/V) = 10:1) to give 2-(4,4-difluoropiperidin-1-yl)-6-methylpyrimidine-4-carbaldehyde (800 mg, yield: 94.9%).

### Step 4: Synthesis of 1-(2-(4,4-difluoropiperidin-1-yl)-6-methylpyrimidin-4-yl)-2-nitroethan-1-ol

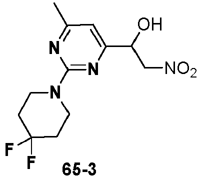

2-(4,4-Difluoropiperidin-1-yl)-6-methylpyrimidine-4-carbaldehyde (800 mg, 3.32 mmol) was dissolved in nitromethane (16 mL), and N,N-diisopropylethylamine (428 mg, 3.32 mmol) was added. The mixture was stirred and allowed to react at room temperature for 2 h. The reaction liquid was concentrated to remove the solvent, and the residue was separated by column chromatography (petroleum ether:ethyl acetate (V/V) = 5:1) to give 1-(2-(4,4-difluoropiperidin-1-yl)-6-methylpyrimidin-4-yl)-2-nitroethan-1-ol (900 mg, yield: 89.8%).

LC-MS, M/Z (ESI): 303.2 [M+H]⁺.

### Step 5: Synthesis of 2-amino-1-(2-(4,4-difluoropiperidin-1-yl)-6-methylpyrimidin-4-yl)ethan-1-ol

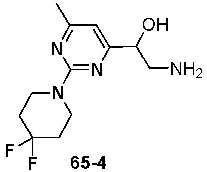

1-(2-(4,4-Difluoropiperidin-1-yl)-6-methylpyrimidin-4-yl)-2-nitroethan-1-ol (900 mg, 2.98 mmol) was dissolved in acetic acid (10 mL), and zinc powder (1.16 g, 17.8 mmol) was added. The mixture was heated to 40 °C and allowed to react for 4 h. The reaction liquid was concentrated to remove the solvent, and water (20 mL) was added to the residue for dilution. The mixture was adjusted to pH > 9 with a 10% aqueous sodium hydroxide solution, and then extracted with dichloromethane (30 mL × 3). The organic phase was collected and concentrated, and the residue was separated by column chromatography (dichloromethane:methanol (V/V) = 50:1, +1‰ aqueous ammonia) to give 2-amino-1-(2-(4,4-difluoropiperidin-1-yl)-6-methylpyrimidin-4-yl)ethan-1-ol (500 mg, yield: 61.7%).

LC-MS, M/Z (ESI): 273.2 [M+H]⁺.

### Step 6: Synthesis of N (2-(2-(4,4-difluoropiperidin-1-yl)-6-methylpyrimidin-4-yl)-2-hydroxyethyl)-4-iodo-2-(6-azaspiro[2.5]octan-6-yl)benzamide

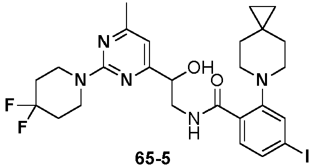

2-Amino-1-(2-(4,4-difluoropiperidin-1-yl)-6-methylpyrimidin-4-yl)ethan-1-ol (500 mg, 1.84 mmol) was dissolved in dry dichloromethane (10 mL), and 4-iodo-2-(6-azaspiro[2.5]octan-6-yl)benzoic acid (656 mg, 1.84 mmol), *N,N-*diisopropylethylamine (711 mg, 5.51 mmol), and *O*-(7-azabenzotriazol-1-yl)-*N,N,N',N'*-tetramethyluronium hexafluorophosphate (1.05 g, 2.76 mmol) were added sequentially. The mixture was stirred and allowed to react at room temperature for 20 h. Water (20 mL) was added to quench the reaction, followed by liquid separation, and the aqueous phase was extracted with dichloromethane (20 mL × 2). The organic phases were combined, dried over anhydrous sodium sulfate, and concentrated, and the residue was separated by column chromatography (petroleum ether:ethyl acetate (V/V) = 8:1) to give N-(2-(2-(4,4-difluoropiperidin-1-yl)-6-methylpyrimidin-4-yl)-2-hydroxyethyl)-4-iodo-2-(6-azaspiro[2.5]octan-6-yl)benzamide (220 mg, yield: 19.6%).

LC-MS, M/Z (ESI): 612.2 [M+H]⁺.

### Step 7: Synthesis of N-(2-(2-(4,4-difluoropiperidin-1-yl)-6-methylpyrimidin-4-yl)-2-oxoethyl)-4-iodo-2-(6-azaspiro[2.5]octan-6-yl)benzamide

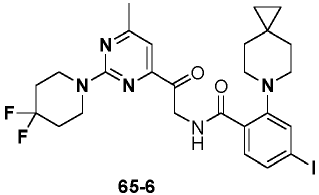

*N*-(2-(2-(4,4-Difluoropiperidin-1-yl)-6-methylpyrimidin-4-yl)-2-hydroxyethyl)-4-iodo-2-(6-azaspiro[2.5]octan-6-yl)benzamide (220 mg, 0.4 mmol) was dissolved in dry dichloromethane (5 mL), and Dess-Martin periodinane (254 mg, 0.6 mmol) was added. The mixture was stirred and allowed to react at room temperature for 20 h. The reaction liquid was filtered through celite, and the filter cake was washed with ethyl acetate. The filtrate was collected and concentrated to remove the solvent, and the residue was separated by column chromatography (petroleum ether:ethyl acetate (V/V) = 10:1) to give N-(2-(2-(4,4-difluoropiperidin-1-yl)-6-methylpyrimidin-4-yl)-2-oxoethyl)-4-iodo-2-(6-azaspiro[2.5]octan-6-yl)benzamide (200 mg, yield: 91.3%).

LC-MS, M/Z (ESI): 610.2 [M+H]⁺.

### Step 8: Synthesis of 5-(2-(4,4-difluoropiperidin-1-yl)-6-methylpyrimidin-4-yl)-2-(4-iodo-2-(6-azaspiro[2.5]octan-6-yl)phenyl)oxazole

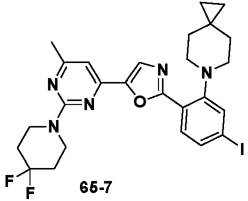

*N*-(2-(2-(4,4-Difluoropiperidin-1-yl)-6-methylpyrimidin-4-yl)-2-oxoethyl)-4-iodo-2-(6-azaspiro[2.5]octan-6-yl)benzamide (120 mg, 0.2 mmol) was dissolved in phosphorus oxychloride (3 mL), and the mixture was heated to 110 °C and allowed to react for 18 h. The reaction liquid was concentrated to remove the solvent, and a saturated sodium bicarbonate solution (50 mL) was added to the residue. The mixture was extracted with ethyl acetate (30 mL × 3). The organic phases were combined, dried over anhydrous sodium sulfate, and concentrated, and the residue was purified by a silica gel plate (petroleum ether:ethyl acetate (V/V) = 20:1) to give 5-(2-(4,4-difluoropiperidin-1-yl)-6-methylpyrimidin-4-yl)-2-(4-iodo-2-(6-azaspiro[2.5]octan-6-yl)phenyl)oxazole (80 mg, yield: 68.7%).

LC-MS, M/Z (ESI): 592.2 [M+H]⁺.

### Step 9: Synthesis of N (4-(5-(2-(4,4-difluoropiperidin-1-yl)-6-methylpyrimidin-4-yl)oxazol-2-yl)-3-(6-azaspiro[2.5]octan-6-yl)phenyl)-2-hydroxyethane-1-sulfonamide

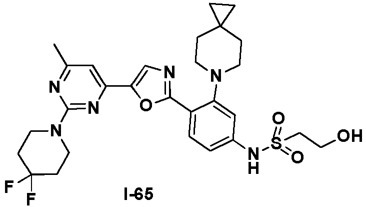

5-(2-(4,4-Difluoropiperidin-l-yl)-6-methylpyrimidin-4-yl)-2-(4-iodo-2-(6-azaspiro[2.5]octan-6-yl)phenyl)oxazole (80 mg, 0.14 mmol) was dissolved in dry *N,N*-dimethylformamide (3 mL), and 2-hydroxyethane-1-sulfonamide (37.5 mg, 0.3 mmol), copper(I) iodide (19 mg, 0.1 mmol), dimethylglycine (10.3 mg, 0.1 mmol), and potassium carbonate (41.5 mg, 0.3 mmol) were added sequentially. The mixture was purged three times with argon, and heated to 130 °C and allowed to react under argon atmosphere for 3 h. The reaction liquid was concentrated to remove the solvent, and the residue was separated by preparative chromatography (Welch, Ultimate C₁₈ column, 10 µm, 21.2 mm × 250 mm. Mobile phase A is a solution of 1‰ formic acid in purified water, and mobile phase B is an acetonitrile solution. Gradient conditions: 0-3 min, maintaining mobile phase A at 90%; 3-15 min, performing gradient elution from 90% to 5%; 15-20 min, maintaining at 5%.), and lyophilized to give N-(4-(5-(2-(4,4-difluoropiperidin-1-yl)-6-methylpyrimidin-4-yl)oxazol-2-yl)-3-(6-azaspiro[2.5]octan-6-yl)phenyl)-2-hydroxyethane-1-sulfonamide (24 mg, yield: 30.2%).

LC-MS, M/Z (ESI): 589.4 [M+H]⁺.

¹H NMR (400 MHz, dmso) δ 8.06 (s, 1H), 7.80 (d, 1H), 6.99 (s, 2H), 6.90 (d, 1H), 5.04 (s, 1H), 3.96 (s, 4H), 3.82 - 3.71 (m, 2H), 3.36 - 3.26 (m, 2H), 2.88 (s, 4H), 2.34 (s, 3H), 2.00 (m, 4H), 1.48 (s, 4H), 0.28 (s, 4H).

### Example 9: Preparation of Target Compound I-77

### N-(4-(5-(2-(4,4-Difluoropiperidin-1-yl)-6-methylpyrimidin-4-yl)-1,3,4-thiadiazol-2-yl)-3-(6-azaspiro[2.5]octan-6-yl)phenyl)-2-hydroxyethane-1-sulfonamide

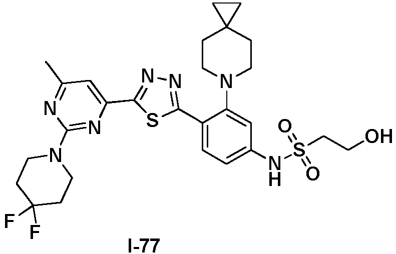

The synthetic route of the target compound **I-77** was as follows:

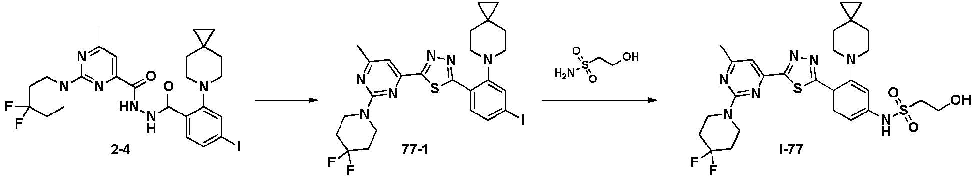

### Step 1: Synthesis of 2-(2-(4,4-difluoropiperidin-1-yl)-6-methylpyrimidin-4-yl)-5-(4-iodo-2-(6-azaspiro[2.5]octan-6-yl)phenyl)-1,3,4-thiadiazole

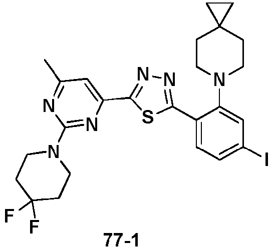

2-(4,4-Difluoropiperidin-l-yl)-*N'*-(4-iodo-2-(6-azaspiro[2.5]octan-6-yl)benzoyl)-6-methylpyrimidine-4-carbohydrazide (0.3 g, 0.491 mmol) was added to 5 mL of tetrahydrofuran, and then phosphorus pentasulfide (0.31 g, 2.457 mmol) was added. The mixture was heated to 100 °C and allowed to react overnight. The reaction liquid was concentrated to dryness, and the residue was separated and purified by a silica gel column (PE:EA = 3:1) to give 2-(2-(4,4-difluoropiperidin-1-yl)-6-methylpyrimidin-4-yl)-5-(4-iodo-2-(6-azaspiro[2.5]octan-6-yl)phenyl)-1,3,4-thiadiazole (130 mg, yield: 43.5%).

LC-MS, M/Z (ESI): 609.1 [M+H]⁺.

### Step 2: Synthesis of N (4-(5-(2-(4,4-difluoropiperidin-1-yl)-6-methylpyrimidin-4-yl)-1,3,4-thiadiazol-2-yl)-3-(6-azaspiro[2.5]octan-6-yl)phenyl)-2-hydroxyethane-1-sulfonamide

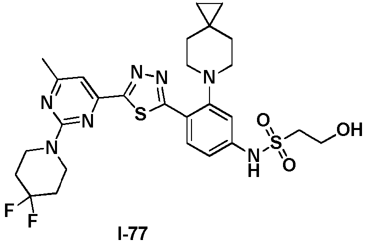

2-(2-(4,4-Difluoropiperidin-1-yl)-6-methylpyrimidin-4-yl)-5-(4-iodo-2-(6-azaspiro[2.5]octan-6-yl)phenyl)-1,3,4-thiadiazole (0.13 g, 0.214 mmol) was added to 5 mL of N,N-dimethylformamide, and then 2-hydroxyethane-1-sulfonamide (53 mg, 0.427 mmol), dimethylglycine (44 mg, 0.427 mmol), copper(I) iodide (41 mg, 0.214 mmol), and potassium carbonate (118 mg, 0.86 mmol) were added. The mixture was heated to 130 °C and allowed to react for 1 h. LCMS showed that the starting materials were completely reacted. Water (5 mL) was added for dilution, and the mixture was extracted with ethyl acetate (10 mL × 2). The organic phase was concentrated to dryness, and the residue was separated and purified by a silica gel column (PE:EA = 1:1) to give N-(4-(5-(2-(4,4-difluoropiperidin-1-yl)-6-methylpyrimidin-4-yl)-1,3,4-thiadiazol-2-yl)-3-(6-azaspiro[2.5]octan-6-yl)phenyl)-2-hydroxyethane-1-sulfonamide (56 mg, yield: 43.3%).

LC-MS, M/Z (ESI): 606.2 [M+H]⁺.

¹H NMR (400 MHz, DMSO-d6) δ8.23 (d, 1H), 7.33 (s, 1H), 7.25 (d, 1H), 7.10-7.07(dd, 1H), 3.96 (t, 4H), 3.75 (t, 2H), 3.28 (t, 2H), 2.86 (t, 4H), 2.43 (s, 3H), 2.05-2.00 (m, 4H), 1.62(s, 4H), 0.38 (s, 4H).

### Example 10: Preparation of Target Compound I-82

### N-(4-(3-(2-(4,4-Difluoropiperidin-1-yl)-6-methylpyrimidin-4-yl)-1,2,4-oxadiazol-5-yl)-3-(6-azaspiro[2.5]octan-6-yl)phenyl)-2-hydroxyethane-1-sulfonamide

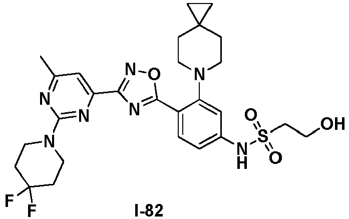

The synthetic route of the target compound **I-82** was as follows:

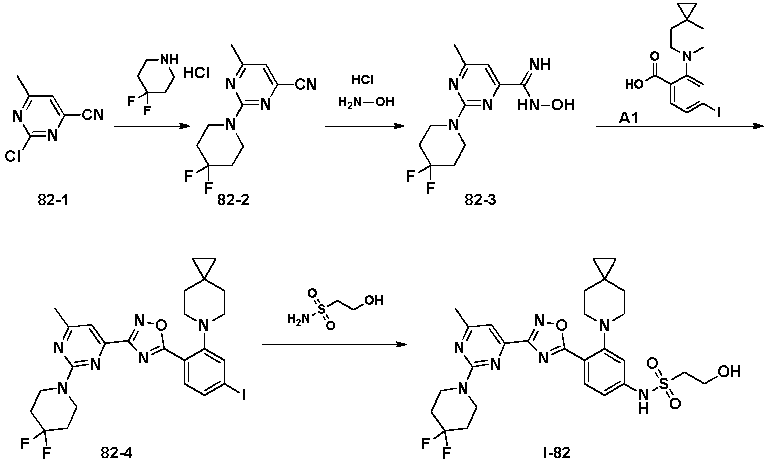

### Step 1: Synthesis of 2-(4,4-difluoropiperidin-1-yl)-6-methylpyrimidine-4-carbonitrile

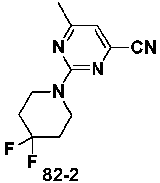

4,4-Difluoropiperidine hydrochloride (3.08 g, 19.54 mmol) was added to 20 mL of *N,N*-dimethylformamide, and then 2-chloro-6-methylpyrimidine-4-carbonitrile (2 g, 13.02 mmol) and potassium carbonate (7.2 g, 52.1 mmol) were added. The mixture was heated to 100 °C and allowed to react for 3 h. The reaction liquid was concentrated to dryness, and the residue was separated and purified by a silica gel column (PE:EA = 10:1) to give 2-(4,4-difluoropiperidin-1-yl)-6-methylpyrimidine-4-carbonitrile (3 g, yield: 97%).

LC-MS, M/Z (ESI): 239.1 [M+H]⁺.

### Step 2: Synthesis of 2-(4,4-difluoropiperidin-1-yl)-N-hydroxy-6-methylpyrimidine-4-carboximidamide

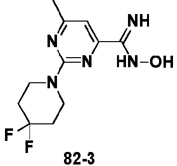

2-(4,4-Difluoropiperidin-1-yl)-6-methylpyrimidine-4-carbonitrile (1 g, 4.2 mmol) was added to 10 mL of ethanol, and then 0.6 mL of water, hydroxylamine hydrochloride (0.35 g, 5.04 mmol), and sodium bicarbonate (0.705 g, 8.4 mmol) were added. The mixture was heated to 85 °C and allowed to react for 4 h. LCMS showed that the starting materials were completely reacted. The reaction liquid was concentrated to dryness to give a crude product of 2-(4,4-difluoropiperidin-1-yl)-N-hydroxy-6-methylpyrimidine-4-carboximidamide (1 g, yield: 88%).

LC-MS, M/Z (ESI): 272.1 [M+H]⁺.

### Step 3: Synthesis of 3-(2-(4,4-difluoropiperidin-1-yl)-6-methylpyrimidin-4-yl)-5-(4-iodo-2-(6-azaspiro[2.5]octan-6-yl)phenyl)-1,2,4-oxadiazole

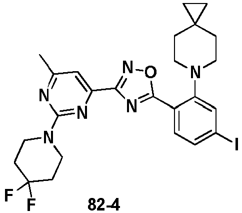

2-(4,4-Difluoropiperidin-1-yl)-*N*-hydroxy-6-methylpyrimidine-4-carboximidamide (0.3 g, 1.106 mmol) was added to 3 mL of N,N-dimethylformamide, and then 4-iodo-2-(6-azaspiro[2.5]octan-6-yl)benzoic acid (0.395 g, 1.106 mmol) and *N,N'*-carbonyldiimidazole (0.359 g, 2.212 mmol) were added. The mixture was heated to 120 °C and allowed to react for 4 h. The reaction liquid was concentrated to dryness, and the residue was separated and purified by a silica gel column (PE:EA = 10:1) to give 3-(2-(4,4-difluoropiperidin-1-yl)-6-methylpyrimidin-4-yl)-5-(4-iodo-2-(6-azaspiro[2.5]octan-6-yl)phenyl)-1,2,4-oxadiazole (0.1 g, yield: 15.3%).

LC-MS, M/Z (ESI): 593.1 [M+H]⁺.

### Step 4: Synthesis of N (4-(3-(2-(4,4-difluoropiperidin-1-yl)-6-methylpyrimidin-4-yl)-1,2,4-oxadiazol-5-yl)-3-(6-azaspiro[2.5]octan-6-yl)phenyl)-2-hydroxyethane-1-sulfonamide

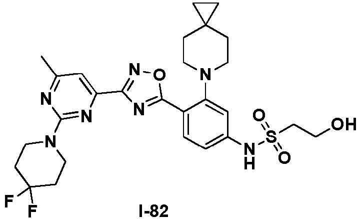

3-(2-(4,4-Difluoropiperidin-1-yl)-6-methylpyrimidin-4-yl)-5-(4-iodo-2-(6-azaspiro[2.5]octan-6-yl)phenyl)-1,2,4-oxadiazole (0.1 g, 0.169 mmol) was added to 3 mL of N,N-dimethylformamide, and then 2-hydroxyethane-1-sulfonamide (42 mg, 0.338 mmol), N,N-dimethylglycine (35 mg, 0.338 mmol), copper(I) iodide (32 mg, 0.169 mmol), and potassium carbonate (93 mg, 0.675 mmol) were added. The mixture was heated to 130 °C and allowed to react for 1 h. LCMS showed that the starting materials were completely reacted. Water (5 mL) was added for dilution, and the mixture was extracted with ethyl acetate (10 mL × 2). The organic phases were combined and concentrated to dryness, and the residue was separated and purified by a silica gel column (PE:EA = 1:1) to give *N-*(4-(3-(2-(4,4-difluoropiperidin-1-yl)-6-methylpyrimidin-4-yl)-1,2,4-oxadiazol-5-yl)-3-(6-azaspiro[2.5]octan-6-yl)phenyl)-2-hydroxyethane-1-sulfonamide (18 mg, yield: 18.08%).

LC-MS, M/Z (ESI): 590.2 [M+H]⁺.

¹H NMR (400 MHz, DMSO-d6) δ7.98 (d, 1H), 7.24 (s, 1H), 7.07 (d, 1H), 6.98-6.95 (dd, 1H), 3.98 (t, 4H), 3.76 (t, 2H), 3.37 (t, 2H), 2.96 (t, 4H), 2.43 (s, 3H), 2.04-2.00 (m, 4H), 1.54(s, 4H), 0.33 (s, 4H).

### Example 11: Preparation of Target Compound I-83

### N-(4-(5-(2-(3,3-Difluoropyrrolidin-1-yl)-6-methylpyrimidin-4-yl)-1,3,4-thiadiazol-2-yl)-3-(6-azaspiro[2.5]octan-6-yl)phenyl)-2-hydroxyethane-1-sulfonamide

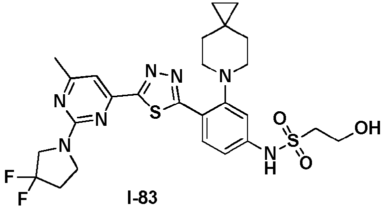

The synthetic route of the target compound **I-83** was as follows:

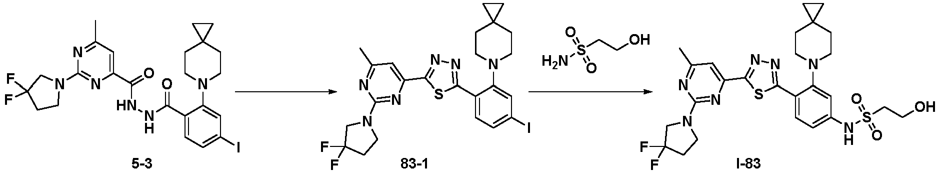

### Step 1: Synthesis of 2-(2-(3,3-difluoropyrrolidin-1-yl)-6-methylpyrimidin-4-yl)-5-(4-iodo-2-(6-azaspiro[2.5]octan-6-yl)phenyl)-1,3,4-thiadiazole

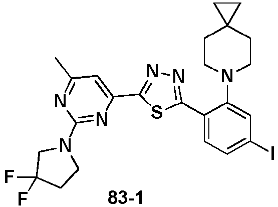

2-(3,3-Difluoropyrrolidin-1-yl)-*N'*-(4-iodo-2-(6-azaspiro[2.5]octan-6-yl)benzoyl)-6-methylpyrimidine-4-carbohydrazide (0.6 g, 1.006 mmol) was added to 20 mL of tetrahydrofuran, and then phosphorus pentasulfide (0.634 g, 5.03 mmol) was added. The mixture was heated to 100 °C and allowed to react overnight. The reaction liquid was concentrated to dryness, and the residue was separated and purified by a silica gel column (PE:EA = 3:1) to give 2-(2-(3,3-difluoropyrrolidin-1-yl)-6-methylpyrimidin-4-yl)-5-(4-iodo-2-(6-azaspiro[2.5]octan-6-yl)phenyl)-1,3,4-thiadiazole (120 mg, yield: 20.1%).

LC-MS, M/Z (ESI): 595.0 [M+1]⁺.

### Step 2: Synthesis of N-(4-(5-(2-(3,3-difluoropyrrolidin-1-yl)-6-methylpyrimidin-4-yl)-1,3,4-thiadiazol-2-yl)-3-(6-azaspiro[2.5]octan-6-yl)phenyl)-2-hydroxyethane-1-sulfonamide

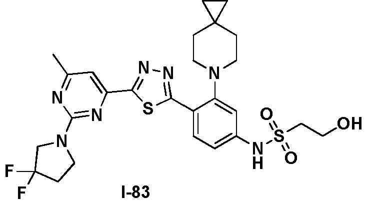

2-(2-(3,3-Difluoropyrrolidin-l-yl)-6-methylpyrimidin-4-yl)-5-(4-iodo-2-(6-azaspiro[2.5]octan-6-yl)phenyl)-1,3,4-thiadiazole (0.12 g, 0.202 mmol) was added to 3 mL of N,N-dimethylformamide, and then 2-hydroxyethane-1-sulfonamide (51 mg, 0.404 mmol), N,N-dimethylglycine (42 mg, 0.404 mmol), copper(I) iodide (38 mg, 0.202 mmol), and potassium carbonate (112 mg, 0.807 mmol) were added. The mixture was heated to 130 °C and allowed to react for 1 h. LCMS showed that the starting materials were completely reacted. Water (5 mL) was added for dilution, and the mixture was extracted with ethyl acetate (10 mL × 2). The organic phase was concentrated to dryness, and the residue was separated and purified by a silica gel column (PE:EA = 1:1) to give N-(4-(5-(2-(3,3-difluoropyrrolidin-1-yl)-6-methylpyrimidin-4-yl)-1,3,4-thiadiazol-2-yl)-3-(6-azaspiro [2.5] octan-6-yl)phenyl)-2-hydroxyethane-1-sulfonamide (30 mg, yield: 25.1%).

LC-MS, M/Z (ESI): 592.1 [M+1]⁺.

¹H NMR (400 MHz, DMSO-d6) δ8.00 (d, 1H), 7.34 (s, 1H), 6.90 (d, 1H), 6.72-6.07 (dd, 1H), 3.95 (t, 2H), 3.78 (t, 2H), 3.70 (t, 2H), 2.93 (t, 2H), 2.84 (t, 4H), 2.61-2.54 (m, 2H), 2.43 (s, 3H),1.73 (s, 4H), 0.38 (s, 4H).

### Example 12: Preparation of Target Compound I-84

### N-(4-(5-(2-(3,3-Difluoroazetidin-1-yl)-6-methylpyrimidin-4-yl)-1,3,4-thiadiazol-2-yl)-3-(6-azaspiro[2.5]octan-6-yl)phenyl)-2-hydroxyethane-1-sulfonamide

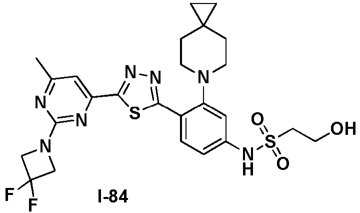

The synthetic route of the target compound 1-84 was as follows:

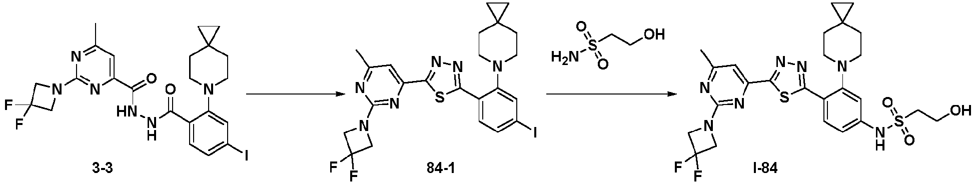

### Step 1: Synthesis of 2-(2-(3,3-difluoroazetidin-1-yl)-6-methylpyrimidin-4-yl)-5-(4-iodo-2-(6-azaspiro[2.5]octan-6-yl)phenyl)-1,3,4-thiadiazole

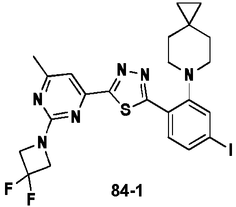

2-(3,3-Difluoroazetidin-1-yl)-*N'*-(4-iodo-2-(6-azaspiro[2.5]octan-6-yl)benzoyl)-6-methylpyrimidine-4-carbohydrazide (0.6 g, 1.03 mmol) was added to 20 mL of tetrahydrofuran, and then phosphorus pentasulfide (0.649 g, 5.15 mmol) was added. The mixture was heated to 100 °C and allowed to react overnight. The reaction liquid was concentrated to dryness, and the residue was separated and purified by a silica gel column (PE:EA = 3:1) to give 2-(2-(3,3-difluoroazetidin-1-yl)-6-methylpyrimidin-4-yl)-5-(4-iodo-2-(6-azaspiro[2.5]octan-6-yl)phenyl)-1,3,4-thiadiazole (110 mg, yield: 18.4%).

LC-MS, M/Z (ESI): 581.0 [M+H]⁺.

### Step 2: Synthesis of N (4-(5-(2-(3,3-difluoroazetidin-1-yl)-6-methylpyrimidin-4-yl)-1,3,4-thiadiazol-2-yl)-3-(6-azaspiro[2.5]octan-6-yl)phenyl)-2-hydroxyethane-1-sulfonamide

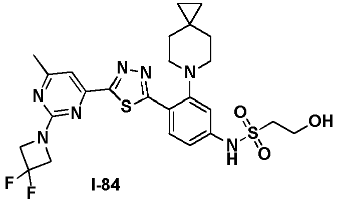

2-(2-(3,3-Difluoroazetidin-1-yl)-6-methylpyrimidin-4-yl)-5-(4-iodo-2-(6-azaspiro[2.5]octan-6-yl)phenyl)-1,3,4-thiadiazole (0.11 g, 0.19 mmol) was added to 3 mL of *N,N*-dimethylformamide, and then 2-hydroxyethane-1-sulfonamide (47 mg, 0.379 mmol), *N,N*-dimethylglycine (39 mg, 0.379 mmol), copper(I) iodide (36 mg, 0.19 mmol), and potassium carbonate (105 mg, 0.758 mmol) were added. The mixture was heated to 130 °C and allowed to react for 1 h. LCMS showed that the starting materials were completely reacted. Water (5 mL) was added for dilution, and the mixture was extracted with ethyl acetate (10 mL × 2). The organic phase was collected and concentrated to dryness, and the residue was separated and purified by a silica gel column (PE:EA = 1:1) to give *N*-(4-(5-(2-(3,3-difluoroazetidin-1-yl)-6-methylpyrimidin-4-yl)-1,3,4-thiadiazol-2-yl)-3-(6-azaspiro[2.5]octan-6-yl)phenyl)-2-hydroxyethane-1-sulfonamide (15 mg, yield: 13.7%).

LC-MS, M/Z (ESI): 578.1 [M+H]⁺.

¹H NMR (400 MHz, DMSO-d6) δ8.16 (d, 1H), 7.48 (s, 1H), 7.18 (d, 1H), 7.02-7.00 (dd, 1H), 4.50 (t, 4H), 3.75 (t, 2H), 3.22 (t, 2H), 2.85 (t, 4H), 2.49 (t, 3H),1.61 (s, 4H), 0.37 (s, 4H).

### Example 13: Preparation of Target Compound I-85

### N-(4-(5-(2-(3,3-Difluoropiperidin-1-yl)-6-methylpyrimidin-4-yl)-1,3,4-thiadiazol-2-yl)-3-(6-azaspiro[2.5]octan-6-yl)phenyl)-2-hydroxyethane-1-sulfonamide

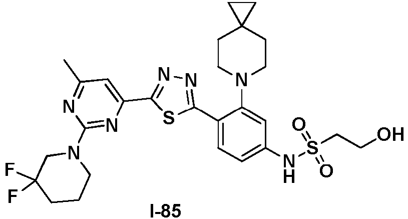

The synthetic route of the target compound **I-85** was as follows:

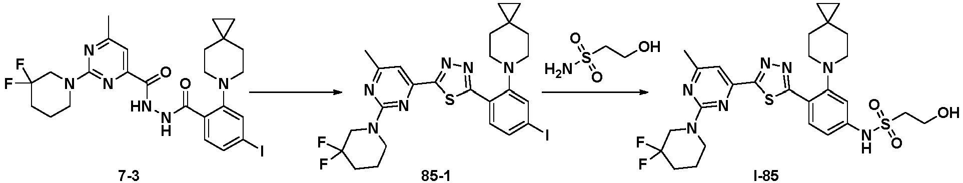

### Step 1: Synthesis of 2-(2-(3,3-difluoropiperidin-1-yl)-6-methylpyrimidin-4-yl)-5-(4-iodo-2-(6-azaspiro[2.5]octan-6-yl)phenyl)-1,3,4-thiadiazole

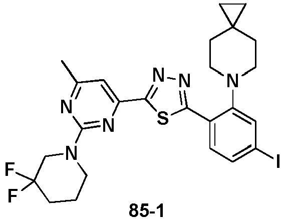

2-(3,3-Difluoropiperidin-l-yl)-*N'*-(4-iodo-2-(6-azaspiro[2.5]octan-6-yl)benzoyl)-6-methylpyrimidine-4-carbohydrazide (0.6 g, 0.983 mmol) was added to 20 mL of tetrahydrofuran, and then phosphorus pentasulfide (0.620 g, 4.91 mmol) was added. The mixture was heated to 100 °C and allowed to react overnight. The reaction liquid was concentrated to dryness, and the residue was separated and purified by a silica gel column (PE:EA = 3:1) to give 2-(2-(3,3-difluoropiperidin-1-yl)-6-methylpyrimidin-4-yl)-5-(4-iodo-2-(6-azaspiro[2.5]octan-6-yl)phenyl)-1,3,4-thiadiazole (150 mg, yield: 25.1%).

LC-MS, M/Z (ESI): 609.1 [M+H]⁺.

### Step 2: Synthesis of N (4-(5-(2-(3,3-difluoropiperidin-1-yl)-6-methylpyrimidin-4-yl)-1,3,4-thiadiazol-2-yl)-3-(6-azaspiro[2.5]octan-6-yl)phenyl)-2-hydroxyethane-1-sulfonamide

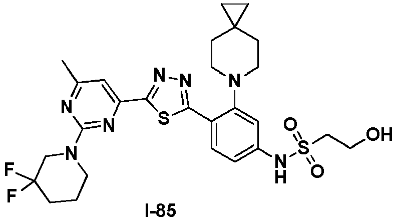

2-(2-(3,3-Difluoropiperidin-1-yl)-6-methylpyrimidin-4-yl)-5-(4-iodo-2-(6-azaspiro[2.5]octan-6-yl)phenyl)-1,3,4-thiadiazole (0.13 g, 0.247 mmol) was added to 3 mL of N,N-dimethylformamide, and then 2-hydroxyethane-1-sulfonamide (62 mg, 0.493 mmol), N,N-dimethylglycine (51 mg, 0.493 mmol), copper(I) iodide (47 mg, 0.247 mmol), and potassium carbonate (136 mg, 0.986 mmol) were added. The mixture was heated to 130 °C and allowed to react for 1 h. LCMS showed that the starting materials were completely reacted. Water (5 mL) was added for dilution, and the mixture was extracted with ethyl acetate (10 mL × 2). The organic phase was collected and concentrated to dryness, and the residue was separated and purified by a silica gel column (PE:EA = 1:1) to give *N-*(4-(5-(2-(3,3-difluoropiperidin-1-yl)-6-methylpyrimidin-4-yl)-1,3,4-thiadiazol-2-yl)-3-(6-azaspiro[2.5]octan-6-yl)phenyl)-2-hydroxyethane-1-sulfonamide (37 mg, yield: 24.8%).

LC-MS, M/Z (ESI): 606.2 [M+H]⁺.

¹H NMR (400 MHz, DMSO-d6) δ8.18 (d, 1H), 7.31 (s, 1H), 7.14 (d, 1H), 6.98-6.95 (dd, 1H), 4.17 (t, 2H), 3.88 (t, 2H), 3.73 (t, 2H), 3.17 (t, 2H), 2.86 (t, 4H), 2.42 (s, 3H), 2.14-2.11 (m, 2H), 1.75 (d, 2H), 1.64 (s, 4H), 0.36 (s, 4H).

### Example 14: Preparation of Target Compound I-86

### N-(4-(5-(2-(3-azabicyclo[3.1.0]hexan-3-yl)-6-methylpyrimidin-4-yl)-1,3,4-oxadiazol-2-yl)-3-(6-azaspiro[2.5]octan-6-yl)phenyl)-2-hydroxyethane-1-sulfonamide

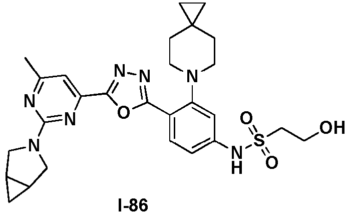

The synthetic route of the target compound **I-86** was as follows:

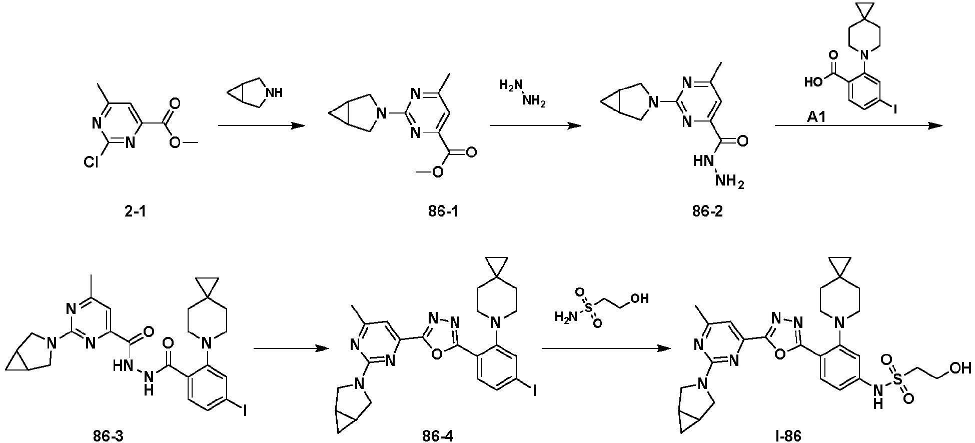

### Step 1: Synthesis of methyl 2-(3-azabicyclo[3.1.0]hexan-3-yl)-6-methylpyrimidine-4-carboxylate

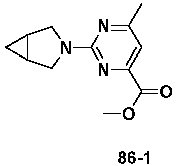

Methyl 2-chloro-6-methylpyrimidine-4-carboxylate (1 g, 5.4 mmol), 3-azabicyclo[3.1.0]hexane (670 mg, 8.1 mmol), and N,N-diisopropylethylamine (1.4 g, 10.8 mol) were added to acetonitrile (15 mL), and the mixture was stirred at 60 °C for 14 h. Water (100 mL) was added for dilution, and the mixture was extracted with ethyl acetate (30 mL × 2). The organic phase was collected and concentrated to dryness, and the residue was separated and purified by a silica gel column (petroleum ether:ethyl acetate = 50:1) to give methyl 2-(3-azabicyclo[3.1.0]hexan-3-yl)-6-methylpyrimidine-4-carboxylate (1.0 g, yield: 80%).

LC-MS, M/Z (ESI): 234.20 [M+H]⁺.

### Step 2: Synthesis of 2-(3-azabicyclo[3.1.0]hexan-3-yl)-6-methylpyrimidine-4-carbohydrazide

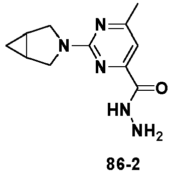

Methyl 2-(3-azabicyclo[3.1.0]hexan-3-yl)-6-methylpyrimidine-4-carboxylate (1.0 g, 4 mmol) and hydrazine hydrate (purity: 80%, 1.6 g, 40 mmol) were added to ethanol (10 mL), and the mixture was allowed to react at 60 °C for 2 h. Water (60 mL) was added for dilution, and the mixture was extracted with ethyl acetate (30 mL × 2). The organic phase was collected and concentrated to dryness, and the residue was separated and purified by a silica gel column (petroleum ether:ethyl acetate = 10:1) to give 2-(3-azabicyclo[3.1.0]hexan-3-yl)-6-methylpyrimidine-4-carbohydrazide (500 mg, yield: 50%).

LC-MS, M/Z (ESI): 234.28 [M+H]⁺.

### Step 3: Synthesis of 2-(3-azabicyclo[3.1.0]hexan-3-yl)-N'-(4-iodo-2-(6-azaspiro[2.5]octan-6-yl)benzoyl)-6-methylpyrimidine-4-carbohydrazide

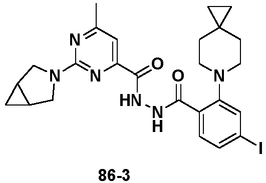

2-(3-Azabicyclo[3.1.0]hexan-3-yl)-6-methylpyrimidine-4-carbohydrazide (200 mg, 0.9 mmol), 4-iodo-2-(6-azaspiro[2.5]octan-6-yl)benzoic acid (0.2 g, 0.6 mmol), *O*-(7-azabenzotriazol-1-yl)-*N*,*N*,*N'*,*N'*-tetramethyluronium hexafluorophosphate (450 mg, 1.0 mmol), and N,N-diisopropylethylamine (232 mg, 1.8 mmol) were dissolved in N,N-dimethylformamide (3 mL), and the solution was stirred at room temperature for 2 h. Water (30 mL) was then added for dilution, and the mixture was extracted with dichloromethane (20 mL × 2). The organic phase was collected and concentrated to dryness, and the residue was separated and purified by a silica gel column (petroleum ether:ethyl acetate = 10:1) to give 2-(3-azabicyclo[3.1.0]hexan-3-yl)-*N'*-(4-iodo-2-(6-azaspiro[2.5]octan-6-yl)benzoyl)-6-methylpyrimidine-4-carbohydrazide (150 mg, yield: 52%).

LC-MS, M/Z (ESI): 573.20 [M+H]⁺.

### Step 4: Synthesis of 2-(2-(3-azabicyclo[3.1.0]hexan-3-yl)-6-methylpyrimidin-4-yl)-5-(4-iodo-2-(6-azaspiro[2.5] octan-6-yl)phenyl)-1,3,4-oxadiazole

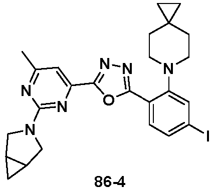

2-(3-Azabicyclo [3.1.0]hexan-3-yl)-N-(4-iodo-2-(6-azaspiro [2.5] octan-6-yl)benzoyl)-6-methylpyrimidine-4-carbohydrazide (150 mg, 0.3 mmol),p-toluenesulfonyl chloride (175 mg, 0.9 mmol), and triethylamine (120 mg, 1.2 mmol) were added to 2 mL of dichloromethane, and the mixture was allowed to react at room temperature for 1 h. The reaction liquid was then directly concentrated to dryness, and the residue was separated and purified by a silica gel column (petroleum ether:ethyl acetate = 1:1) to give 2-(2-(3-azabicyclo[3.1.0]hexan-3-yl)-6-methylpyrimidin-4-yl)-5-(4-iodo-2-(6-azaspiro[2.5]octan-6-yl)phenyl)-1,3,4-oxadiazole (100 mg, yield: 60%).

LC-MS, M/Z (ESI): 555.21 [M+H]⁺.

### Step 5: Synthesis of N-(4-(5-(2-(3-azabicyclo[3.1.0]hexan-3-yl)-6-methylpyrimidin-4-yl)-1,3,4-oxadiazol-2-yl)-3-(6-azaspiro[2.5]octan-6-yl)phenyl)-2-hydroxyethane-1-sulfonamide

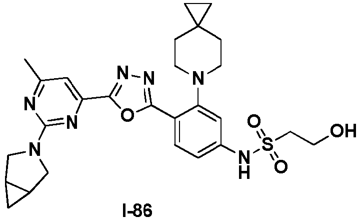

2-(2-(3-Azabicyclo[3.1.0]hexan-3-yl)-6-methylpyrimidin-4-yl)-5-(4-iodo-2-(6-azaspiro[2.5]octan-6-yl)phenyl)-1,3,4-oxadiazole (100 mg, 0.2 mmol) was added to 2 mL of *N*,*N*-dimethylformamide, and then 2-hydroxyethane-1-sulfonamide (100 mg, 0.8 mmol), *trans*-*N*,*N*-dimethylcyclohexane-1,2-diamine (15 mg, 0.04 mmol), copper(I) iodide (20 mg, 0.08 mmol), and potassium phosphate (170 mg, 0.8 mmol) were added. The mixture was heated to 120 °C and allowed to react for 1 h. Water (10 mL) was added for dilution, and the mixture was extracted with ethyl acetate (10 mL × 2). The organic phase was collected and concentrated to dryness, and the residue was separated and purified by a silica gel column (ethyl acetate:methanol = 10:1) to give *N*-(4-(5-(2-(3-azabicyclo[3.1.0]hexan-3-yl)-6-methylpyrimidin-4-yl)-1,3,4-oxadiazol-2-yl)-3-(6-azaspiro[2.5]octan-6-yl)phenyl)-2-hydroxyethane-1-sulfonamide (40 mg, yield: 32%).

LC-MS, M/Z (ESI): 552.2 [M+H]⁺.

¹H NMR (400 MHz, DMSO-d6) δ10.17 (s, 1H), 7.95 (m, 1H), 7.45 (s, 1H), 7.05 (m, 1H), 6.96 (m, 1H), 4.94 (s, 1H), 3.97 (m, 2H), 3.77 (m, 2H), 3.54 (m, 2H), 3.35 (m, 4H), 2.92 (m, 3H), 2.49 (m, 2H), 1.67 (m, 7H), 0.76 (m, 1H), 0.32 (m, 3H), 0.14 (m, 1H).

### Example 15: Preparation of Target Compound I-87

### N-(4-(5-(6-(4,4-Difluoropiperidin-1-yl)-4-methylpyridin-2-yl)-1,3,4-oxadiazol-2-yl)-3-(6-azaspiro[2.5]octan-6-yl)phenyl)-2-hydroxyethanesulfonamide

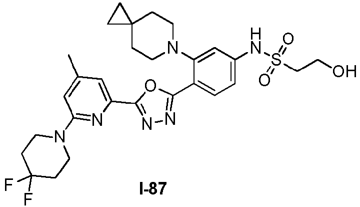

The synthetic route of the target compound **I-87** was as follows:

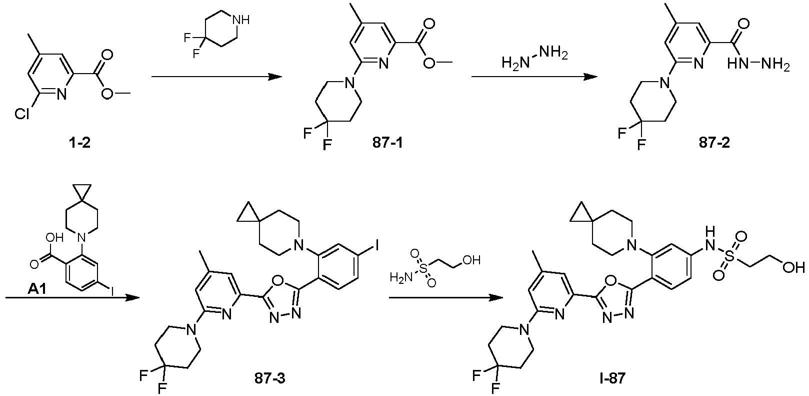

### Step 1: Synthesis of methyl 6-(4,4-difluoropiperidin-1-yl)-4-methylpicolinate

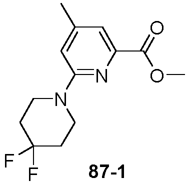

To a solution of methyl 6-chloro-4-methylpicolinate (2 g, 10.8 mmol) in N,N-dimethylformamide (20 mL) were added 4,4-difluoropiperidine (2.6 g, 21.6 mmol) and *N,N*-diisopropylethylamine (5.6 g, 43.2 mmol), and the mixture was allowed to react under microwave conditions at 120 °C for 2 h. The reaction liquid was poured into water (100 mL), and the mixture was extracted with ethyl acetate (50 mL × 3). The organic phase was washed with saturated brine (50 mL × 3), dried over anhydrous sodium sulfate, filtered, and concentrated to give a crude product. The crude product was purified by column chromatography (petroleum ether/ethyl acetate (V/V) = 1/0-1/1) to give methyl 6-(4,4-difluoropiperidin-1-yl)-4-methylpicolinate (350 mg, yield: 12.0%).

LC-MS, M/Z (ESI): 271.2 [M+H]⁺.

### Step 2: Synthesis of 6-(4,4-difluoropiperidin-1-yl)-4-methylpyridine carbohydrazide

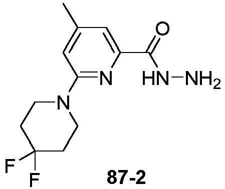

Methyl 6-(4,4-difluoropiperidin-1-yl)-4-methylpicolinate (350 mg, 1.3 mmol) was dissolved in methanol (3.5 mL), and then hydrazine hydrate (80%, 3.5 mL) was added slowly. The mixture was allowed to react at 80 °C for 5 h. The reaction liquid was poured slowly into water (100 mL), and the mixture was extracted with ethyl acetate (30 mL × 3). The organic phase was washed with saturated brine (20 mL × 3), dried over anhydrous sodium sulfate, filtered, and concentrated to give 6-(4,4-difluoropiperidin-1-yl)-4-methylpyridine carbohydrazide (230 mg, yield: 65.7%).

LC-MS, M/Z (ESI): 271.2 [M+H]⁺.

### Step 3: Synthesis of 2-(6-(4,4-difluoropiperidin-1-yl)-4-methylpyridin-2-yl)-5-(4-iodo-2-(6-azaspiro[2.5]octan-6-yl)phenyl)-1,3,4-oxadiazole

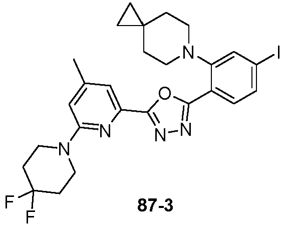

To 6-(4,4-difluoropiperidin-1-yl)-4-methylpyridine carbohydrazide (0.23 g, 0.9 mmol) and 4-iodo-2-(6-azaspiro[2.5]octan-6-yl)benzoic acid (0.32 g, 0.9 mmol) was added phosphorus oxychloride (2.3 mL) at 0 °C, and the mixture was stirred at 110 °C for 18 h. The reaction liquid was added dropwise and slowly to ice water (50 mL), and the mixture was extracted with ethyl acetate (20 mL × 3). The organic phase was washed with a saturated aqueous sodium bicarbonate solution (10 mL × 2), washed with saturated brine (10 mL × 2), dried over anhydrous sodium sulfate, filtered, and concentrated to give a crude product. The crude product was purified by column chromatography (petroleum ether/ethyl acetate (V/V) = 1/0-1/3) to give 2-(6-(4,4-difluoropiperidin-1-yl)-4-methylpyridin-2-yl)-5-(4-iodo-2-(6-azaspiro[2.5]octan-6-yl)phenyl)-1,3,4-oxadiazole (0.13 g, yield: 25.8%).

LC-MS, M/Z (ESI): 592.3 [M+H]⁺.

### Step 4: Synthesis of N-(4-(5-(6-(4,4-difluoropiperidin-1-yl)-4-methylpyridin-2-yl)-1,3,4-oxadiazol-2-yl)-3-(6-azaspiro[2.5]octan-6-yl)phenyl)-2-hydroxyethanesulfonamide

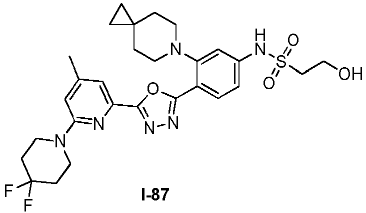

To a solution of 2-(6-(4,4-difluoropiperidin-1-yl)-4-methylpyridin-2-yl)-5-(4-iodo-2-(6-azaspiro[2.5]octan-6-yl)phenyl)-1,3,4-oxadiazole (130 mg, 0.2 mmol) and 2-hydroxyethylsulfonamide (38 mg, 0.3 mmol) in *N,N-*dimethylformamide (1 mL) were added *N,N*-dimethylglycine (4 mg, 0.04 mmol), copper(I) iodide (4 mg, 0.02 mmol), and potassium carbonate (83 mg, 0.6 mmol), and the mixture was allowed to react at 130 °C for 3 h. The reaction liquid was poured into water (20 mL), and the mixture was extracted with ethyl acetate (10 mL × 3). The organic phase was dried over anhydrous sodium sulfate, filtered, and concentrated to give a crude product. The crude product was purified by preparative reverse phase chromatography (column: Phenomenex Synergi C18 100 × 25 mm × 4 µm; solvents: A = water + 0.1 vol% formic acid (99%), B = acetonitrile; gradient: 5%-95%, 7 min) to give N-(4-(5-(6-(4,4-difluoropiperidin-1-yl)-4-methylpyridin-2-yl)-1,3,4-oxadiazol-2-yl)-3-(6-azaspiro[2.5]octan-6-yl)phenyl)-2-hydroxyethanesulfonamide (18.7 mg, yield: 14.5%).

LC-MS, M/Z (ESI): 589.1 [M+H]⁺.

¹H NMR (400 MHz, dmso) δ 7.84 (d, J = 8.6 Hz, 1H), 7.42 (s, 1H), 7.04 (s, 2H), 6.94 (dd, J = 8.6, 2.0 Hz, 1H), 3.87 - 3.80 (m, 4H), 3.76 (m, 2H), 3.36 (m, 2H), 2.91 (t, J = 5.0 Hz, 4H), 2.35 (s, 3H), 2.04 (ddd, J = 19.8, 13.8, 6.0 Hz, 4H), 1.50 (s, 4H), 0.29 (s, 4H).

### Example 16: Preparation of Target Compound I-88

### N-(4-(5-(2-(6,6-Difluoro-3-azabicyclo[3.1.0]hexan-3-yl)-6-methylpyrimidin-4-yl)-1,3,4-oxadiazol-2-yl)-3-(6-azaspiro[2.5]octan-6-yl)phenyl)-2-hydroxyethane-1-sulfonamide

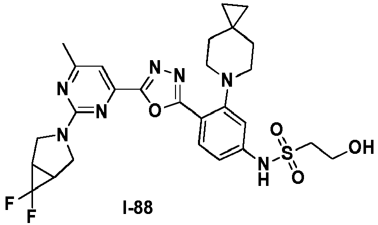

The synthetic route of the target compound **I-88** was as follows:

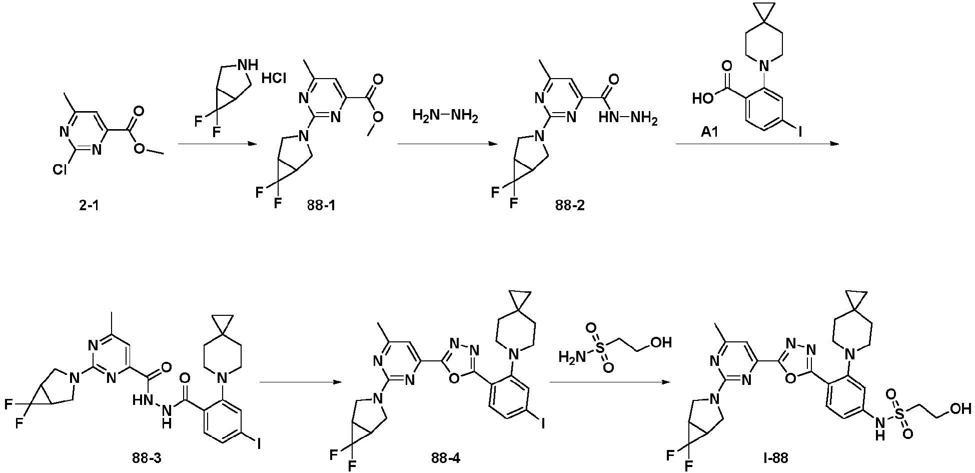

### Step 1: Synthesis of methyl 2-(6,6-difluoro-3-azabicyclo[3.1.0]hexan-3-yl)-6-methylpyrimidine-4-carboxylate

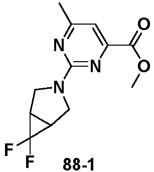

Methyl 2-chloro-6-methylpyrimidine-4-carboxylate (2 g, 10.72 mmol) was added to 20 mL of *N,N-*dimethylformamide, and then 6,6-difluoro-3-azabicyclo[3.1.0]hexane hydrochloride (3.33 g, 21.44 mmol) and potassium carbonate (7.41 g, 53.6 mmol) were added. The mixture was heated to 100 °C and allowed to react for 2 h. LCMS showed that the starting materials were completely reacted. Water (30 mL) was added for dilution, and the mixture was extracted with ethyl acetate (50 mL × 2). The organic phase was concentrated to dryness, and the residue was separated and purified by a silica gel column (PE:EA = 5:1) to give methyl 2-(6,6-difluoro-3-azabicyclo[3.1.0]hexan-3-yl)-6-methylpyrimidine-4-carboxylate (1.9 g, yield: 65.8%).

LC-MS, M/Z (ESI): 270.1 [M+H]⁺.

### Step 2: Synthesis of 2-(6,6-difluoro-3-azabicyclo[3.1.0]hexan-3-yl)-6-methylpyrimidine-4-carbohydrazide

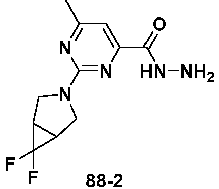

Methyl 2-(6,6-difluoro-3-azabicyclo[3.1.0]hexan-3-yl)-6-methylpyrimidine-4-carboxylate (1.9 g, 7.06 mmol) was added to 20 mL of methanol, and then 4 mL of 80% hydrazine hydrate was added. The mixture was heated to 60 °C and allowed to react for 1 h. LCMS showed that the starting materials were completely reacted. The reaction liquid was concentrated to dryness, and the residue was lyophilized to give a crude product of 2-(6,6-difluoro-3-azabicyclo[3.1.0]hexan-3-yl)-6-methylpyrimidine-4-carbohydrazide (1.9 g, yield: 100%).

LC-MS, M/Z (ESI): 270.1 [M+H]⁺.

### Step 3: Synthesis of 2-(6,6-difluoro-3-azabicyclo[3.1.0]hexan-3-yl)-N'-(4-iodo-2-(6-azaspiro[2.5]octan-6-yl)benzoyl)-6-methylpyrimidine-4-carbohydrazide

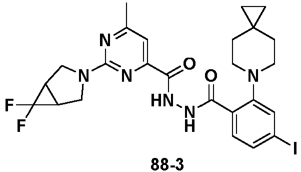

2-(6,6-Difluoro-3-azabicyclo[3.1.0]hexan-3-yl)-6-methylpyrimidine-4-carbohydrazide (1.696 g, 6.3 mmol) was added to 15 mL of *N,N*-dimethylformamide, and then 4-iodo-2-(6-azaspiro[2.5]octan-6-yl)benzoic acid (1.5 g, 4.2 mmol), N,N-diisopropylethylamine (3.26 g, 25.2 mmol), and *O*-(7-azabenzotriazol-1-yl)-*N,N,N',N'-*tetramethyluronium hexafluorophosphate (4.79 g, 12.6 mmol) were added. The mixture was heated to 60 °C and allowed to react for 1 h. LCMS showed that the starting materials were completely reacted. Water (20 mL) was added for dilution, and the mixture was extracted with ethyl acetate (100 mL × 2). The organic phase was collected and concentrated to dryness, and the residue was separated and purified by a silica gel column (PE:EA = 3:1) to give 2-(6,6-difluoro-3-azabicyclo[3.1.0]hexan-3-yl)-*N'*-(4-iodo-2-(6-azaspiro[2.5]octan-6-yl)benzoyl)-6-methylpyrimidine-4-carbohydrazide (1.6 g, yield: 62.6%).

LC-MS, M/Z (ESI): 609.1 [M+H]⁺.

### Step 4: Synthesis of 2-(2-(6,6-difluoro-3-azabicyclo[3.1.0]hexan-3-yl)-6-methylpyrimidin-4-yl)-5-(4-iodo-2-(6-azaspiro[2.5]octan-6-yl)phenyl)-1,3,4-oxadiazole

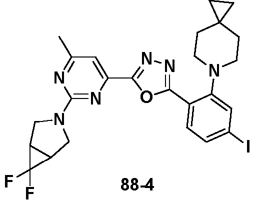

2-(6,6-Difluoro-3-azabicyclo[3.1.0]hexan-3-yl)-*N'*-(4-iodo-2-(6-azaspiro[2.5] octan-6-yl)benzoyl)-6-methylpyrimidine-4-carbohydrazide (0.3 g, 0.493 mmol) was added to 5 mL of dichloromethane, and then p-methylbenzenesulfonyl chloride (0.28 g, 1.479 mmol) and triethylamine (0.299 g, 2.96 mmol) were added. The mixture was allowed to react at room temperature overnight. LCMS showed that the starting materials were completely reacted. Water (5 mL) was added for dilution, and the mixture was extracted with ethyl acetate (10 mL × 2). The organic phase was concentrated to dryness, and the residue was separated and purified by a silica gel column (PE:EA = 3:1) to give 2-(2-(6,6-difluoro-3-azabicyclo[3.1.0]hexan-3-yl)-6-methylpyrimidin-4-yl)-5-(4-iodo-2-(6-azaspiro[2.5]octan-6-yl)phenyl)-1,3,4-oxadiazole (110 mg, yield: 37.8%).

LC-MS, M/Z (ESI): 591.1 [M+H]⁺.

### Step 5: Synthesis of N-(4-(5-(2-(6,6-difluoro-3-azabicyclo[3.1.0]hexan-3-yl)-6-methylpyrimidin-4-yl)-1,3,4-oxadiazol-2-yl)-3-(6-azaspiro[2.5]octan-6-yl)phenyl)-2-hydroxyethane-1-sulfonamide

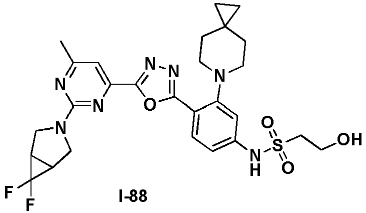

2-(2-(6,6-Difluoro-3-azabicyclo[3.1.0]hexan-3-yl)-6-methylpyrimidin-4-yl)-5-(4-iodo-2-(6-azaspiro[2.5]octan-6-yl)phenyl)-1,3,4-oxadiazole (0.11 g, 0.186 mmol) was added to 5 mL of *N*,*N*-dimethylformamide, and then 2-hydroxyethane-1-sulfonamide (47 mg, 0.373 mmol), *N*,*N*-dimethylglycine (38 mg, 0.373 mmol), copper(I) iodide (35 mg, 0.186 mmol), and potassium carbonate (103 mg, 0.745 mmol) were added. The mixture was heated to 130 °C and allowed to react for 1 h. LCMS showed that the starting materials were completely reacted. Water (5 mL) was added for dilution, and the mixture was extracted with ethyl acetate (10 mL × 2). The organic phases were combined and concentrated to dryness, and the residue was separated and purified by a silica gel column (PE:EA = 1:1) to give N-(4-(5-(2-(6,6-difluoro-3-azabicyclo[3.1.0]hexan-3-yl)-6-methylpyrimidin-4-yl)-1,3,4-oxadiazol-2-yl)-3-(6-azaspiro[2.5]octan-6-yl)phenyl)-2-hydroxyethane-1-sulfonamide (45 mg, yield: 41.1%).

LC-MS, M/Z (ESI): 588.2 [M+H]⁺.

¹H NMR (400 MHz, DMSO-d6) δ7.92 (d, 1H), 7.31 (s, 1H), 7.00 (d, 1H), 6.92-6.89(dd, 1H),4.13 (d, 1H), 3.98 (d, 1H), 3.82 (t, 2H), 3.75 (t, 2H), 3.29 (t, 2H), 2.90 (t, 4H), 2.67 (d, 2H), 2.40 (s, 3H), 1.57 (s, 4H), 0.30 (s, 4H).

### Example 17: Preparation of Target Compound I-8

### N-(4-(5-(2-(4-fluoropiperidin-1-yl)-6-methylpyrimidin-4-yl)-1,3,4-oxadiazol-2-yl)-3-(6-azaspiro[2.5]octan-6-yl)phenyl)-2-hydroxyethanesulfonamide

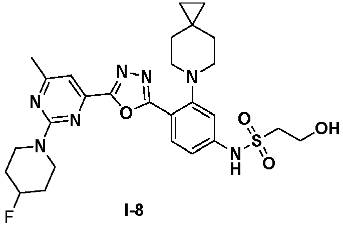

The synthetic route of the target compound **I-8** was as follows:

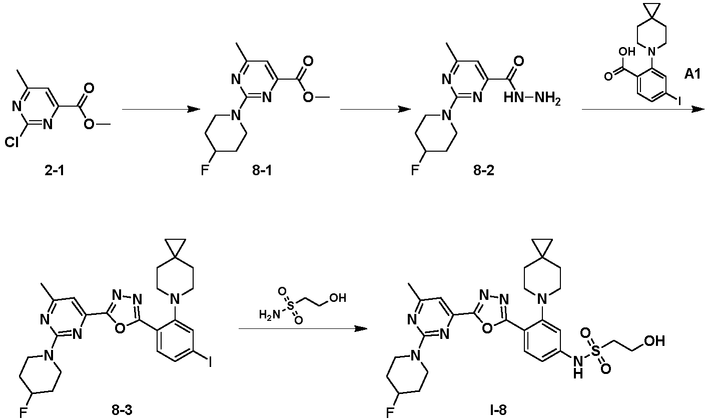

### Step 1: Synthesis of methyl 2-(4-fluoropiperidin-1-yl)-6-methylpyrimidine-4-carboxylate

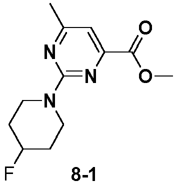

To a solution of methyl 2-chloro-6-methylpyrimidine-4-carboxylate (300 mg, 1.6 mmol) in N,N-dimethylformamide (3 mL) were added 4-fluoropiperidine (330 mg, 3.2 mmol) and potassium carbonate (885 mg, 6.4 mmol), and then the mixture was stirred at 100 °C for 2 h. Water (100 mL) was added to the reaction liquid, and the mixture was extracted with ethyl acetate (30 mL × 3). The organic phase was washed with saturated brine (20 mL × 2), dried over anhydrous sodium sulfate, filtered, and concentrated to give a crude product. The crude product was purified by column chromatography (petroleum ether/ethyl acetate (V/V) = 1/0-1/1) to give methyl 2-(4-fluoropiperidin-1-yl)-6-methylpyrimidine-4-carboxylate (200 mg, yield: 49.1%).

LC-MS, M/Z (ESI): 254.4 [M+H]⁺.

### Step 2: Synthesis of 2-(4-fluoropiperidin-1-yl)-6-methylpyrimidine-4-carbohydrazide

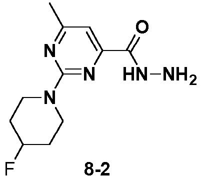

To a solution of methyl 2-(4-fluoropiperidin-1-yl)-6-methylpyrimidine-4-carboxylate (200 mg, 0.8 mmol) in methanol (2 mL) was added dropwise and slowly 80% hydrazine hydrate (2 mL), and then the mixture was stirred at 60 °C for 1 h. The reaction liquid was added dropwise and slowly to water (50 mL), and the mixture was extracted with ethyl acetate (20 mL × 3). The organic phase was washed with saturated brine (20 mL × 2), dried over anhydrous sodium sulfate, filtered, and concentrated to give 2-(4-fluoropiperidin-1-yl)-6-methylpyrimidine-4-carbohydrazide (150 mg, yield: 75%).

LC-MS, M/Z (ESI): 254.4 [M+H]⁺.

### Step 3: Synthesis of 2-(2-(4-fluoropiperidin-1-yl)-6-methylpyrimidin-4-yl)-5-(4-iodo-2-(6-azaspiro[2.5]octan-6-yl)phenyl)-1,3,4-oxadiazole

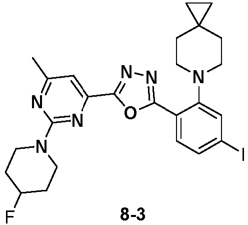

To 2-(4-fluoropiperidin-1-yl)-6-methylpyrimidine-4-carbohydrazide (150 mg, 0.6 mmol) and 4-iodo-2-(6-azaspiro[2.5]octan-6-yl)benzoic acid (214 mg, 0.6 mmol) was added dropwise and slowly phosphorus oxychloride (1.5 mL), and then the mixture was stirred at 110 °C for 18 h. The reaction liquid was added dropwise and slowly to ice water (100 mL), and the mixture was extracted with ethyl acetate (30 mL × 3). The organic phase was washed with saturated brine (30 mL × 2), dried over anhydrous sodium sulfate, filtered, and concentrated to give a crude product. The crude product was purified by column chromatography (petroleum ether/ethyl acetate (V/V) = 1/0-1/1) to give 2-(2-(4-fluoropiperidin-1-yl)-6-methylpyrimidin-4-yl)-5-(4-iodo-2-(6-azaspiro[2.5]octan-6-yl)phenyl)-1,3,4-oxadiazole (80 mg, yield: 23.5%).

LC-MS, M/Z (ESI): 575.4 [M+H]⁺.

### Step 4: Synthesis of N-(4-(5-(2-(4-fluoropiperidin-1-yl)-6-methylpyrimidin-4-yl)-1,3,4-oxadiazol-2-yl)-3-(6-azaspiro[2.5]octan-6-yl)phenyl)-2-hydroxyethanesulfonamide

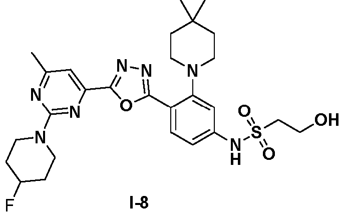

To a solution of 2-(2-(4-fluoropiperidin-1-yl)-6-methylpyrimidin-4-yl)-5-(4-iodo-2-(6-azaspiro[2.5]octan-6-yl)phenyl)-1,3,4-oxadiazole (80 mg, 0.14 mmol) in *N*,*N*-dimethylformamide (1 mL) were added 2-hydroxyethanesulfonamide (35 mg, 0.28 mmol), N,N-dimethylglycine (3 mg, 0.028 mmol), copper(I) iodide (3 mg, 0.014 mmol), and potassium carbonate (58 mg, 0.42 mmol), and the mixture was purged three times with nitrogen and then stirred at 120 °C for 2 h. The reaction liquid was poured into water (50 mL), and the mixture was extracted with ethyl acetate (20 mL × 3). The organic phase was washed with saturated brine (20 mL × 2), dried over anhydrous sodium sulfate, filtered, and concentrated to give a crude product. The crude product was purified by preparative reverse phase chromatography (column: Phenomenex Synergi C18 100 × 25 mm × 4 µm; solvents: A = water + 0.1 vol% formic acid (99%), B = acetonitrile; gradient: 5%-95%, 7 min) to give *N*-(4-(5-(2-(4-fluoropiperidin-1-yl)-6-methylpyrimidin-4-yl)-1,3,4-oxadiazol-2-yl)-3-(6-azaspiro [2.5] octan-6-yl)phenyl)-2-hydroxyethanesulfonamide (24.3 mg, yield: 30.5%).

¹H NMR (400 MHz, DMSO-d6) δ 7.91 (d, 1H), 7.29 (s, 1H), 7.06 (d, 1H), 6.96 (dd, 1H), 5.05-4.95 (m, 1H), 4.89 (dd, 1H), 4.03 (s, 2H), 3.93-3.82 (m, 2H), 3.76 (t, 2H), 3.36 (t, 3H), 2.91 (t, 4H), 2.42 (s, 2H), 2.03-1.87 (m, 2H), 1.81-1.68 (m, 2H), 1.52 (s, 4H), 0.30 (s, 4H).

LC-MS, M/Z (ESI): 572.3 [M+H]⁺.

### Example 18: Preparation of Target Compound I-27

### 2-Hydroxy-N-(4-(5-(6-methyl-2-(3,3,3-trifluoropropoxy)pyrimidin-4-yl)-1,3,4-oxadiazol-2-yl)-3-(6-azaspiro[2.5]octan-6-yl)phenyl)ethane-1-sulfonamide

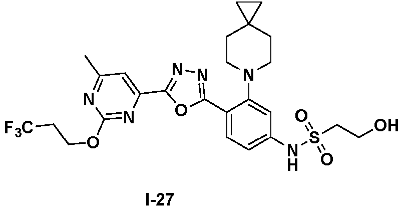

The synthetic route of the target compound **I-27** was as follows:

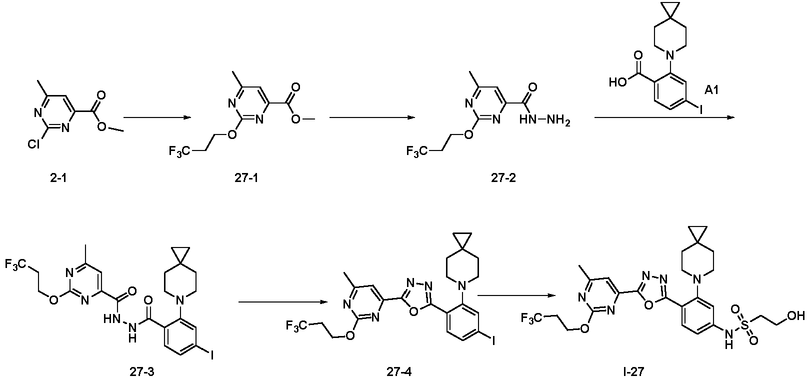

### Step 1: Synthesis of methyl 6-methyl-2-(3,3,3-trifluoropropoxy)pyrimidine-4-carboxylate

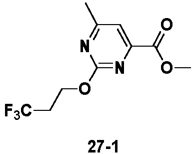

Methyl 2-chloro-6-methylpyrimidine-4-carboxylate (5 g, 26.8 mmol) was added to 40 mL of *N,N-*dimethylformamide, and then 3,3,3-trifluoropropanol (6.11 g, 53.6 mmol) and potassium carbonate (18.52 g, 134 mmol) were added. The mixture was heated to 100 °C and allowed to react for 2 h. LCMS showed that the starting materials were completely reacted. Water (60 mL) was added for dilution, and the mixture was extracted with ethyl acetate (100 mL × 2). The organic phase was concentrated to dryness, and the residue was separated and purified by a silica gel column (PE:EA (V/V) = 5:1) to give methyl 6-methyl-2-(3,3,3-trifluoropropoxy)pyrimidine-4-carboxylate (1.9 g, yield: 26.8%).

LC-MS, M/Z (ESI): 265.1 [M+H]⁺.

### Step 2: Synthesis of 6-methyl-2-(3,3,3-trifluoropropoxy)pyrimidine-4-carbohydrazide

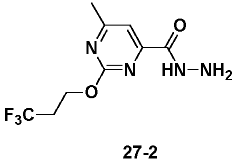

Methyl 6-methyl-2-(3,3,3-trifluoropropoxy)pyrimidine-4-carboxylate (1.9 g, 7.19 mmol) was added to 25 mL of methanol, and then 4 mL of 80% hydrazine hydrate was added. The mixture was heated to 60 °C and allowed to react for 1 h. LCMS showed that the starting materials were completely reacted. The reaction liquid was concentrated to dryness, and the residue was lyophilized to give a crude product of 6-methyl-2-(3,3,3-trifluoropropoxy)pyrimidine-4-carbohydrazide (1.9 g, yield: 100%).

LC-MS, M/Z (ESI): 265.1 [M+H]⁺.

### Step 3: Synthesis ofN'-(4-iodo-2-(6-azaspiro[2.5]octan-6-yl)benzoyl)-6-methyl-2-(3,3,3-trifluoropropoxy) pyrimidine-4-carbohydrazide

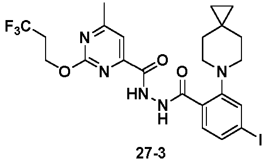

The crude product of 6-methyl-2-(3,3,3-trifluoropropoxy)pyrimidine-4-carbohydrazide (1.9 g, 7.19 mmol) was added to 25 mL of *N*,*N*-dimethylformamide, and then 4-iodo-2-(6-azaspiro[2.5]octan-6-yl)benzoic acid (1.7 g, 4.76 mmol), N,N-diisopropylethylamine (3.69 g, 28.6 mmol), and *O*-(7-azabenzotriazol-1-yl)-*N,N,N'*,*N'*-tetramethyluronium hexafluorophosphate (5.43 g, 14.28 mmol) were added. The mixture was heated to 60 °C and allowed to react for 1 h. LCMS showed that the starting materials were completely reacted. Water (20 mL) was added for dilution, and the mixture was extracted with ethyl acetate (100 mL × 2). The organic phase was concentrated to dryness, and the residue was separated and purified by a silica gel column (PE:EA (V/V) = 3:1) to give N-(4-iodo-2-(6-azaspiro[2.5]octan-6-yl)benzoyl)-6-methyl-2-(3,3,3-trifluoropropoxy)pyrimidine-4-carbohydrazide (0.9 g, yield: 31.3%).

LC-MS, M/Z (ESI): 604.1 [M+H]⁺.

### Step 4: Synthesis of 2-(4-iodo-2-(6-azaspiro[2.5]octan-6-yl)phenyl)-5-(6-methyl-2-(3,3,3-trifluoropropoxy) pyrimidin-4-yl)-1,3,4-oxadiazole

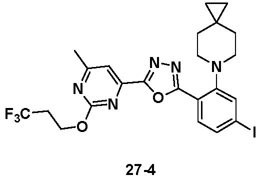

*N'*-(4-Iodo-2-(6-azaspiro[2.5]octan-6-yl)benzoyl)-6-methyl-2-(3,3,3-trifluoropropoxy)pyrimidine-4-carbohydrazide (0.3 g, 0.497 mmol) was added to 5 mL of dichloromethane, and then p-methylbenzenesulfonyl chloride (0.474 g, 2.486 mmol) and triethylamine (0.503 g, 4.97 mmol) were added. The mixture was allowed to react at room temperature overnight. LCMS showed that the starting materials were completely reacted. Water (5 mL) was added for dilution, and the mixture was extracted with ethyl acetate (10 mL × 2). The organic phase was concentrated to dryness, and the residue was separated and purified by a silica gel column (PE:EA (V/V) = 3:1) to give 2-(4-iodo-2-(6-azaspiro[2.5]octan-6-yl)phenyl)-5-(6-methyl-2-(3,3,3-trifluoropropoxy)pyrimidin-4-yl)-1,3,4-oxadiazole (0.2 g, yield: 68.7%).

LC-MS, M/Z (ESI): 586.1 [M+H]⁺.

### Step 5: Synthesis of 2-hydroxy-N-(4-(5-(6-methyl-2-(3,3,3-trifluoropropoxy)pyrimidin-4-yl)-1,3,4-oxadiazol-2-yl)-3-(6-azaspiro[2.5]octan-6-yl)phenyl)ethane-1-sulfonamide

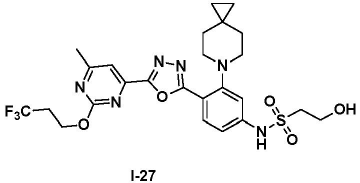

2-(4-Iodo-2-(6-azaspiro[2.5]octan-6-yl)phenyl)-5-(6-methyl-2-(3,3,3-trifluoropropoxy)pyrimidin-4-yl)-1,3,4-oxadiazole (0.2 g, 0.342 mmol) was added to 4 mL of N,N-dimethylformamide, and then 2-hydroxyethane-1-sulfonamide (86 mg, 0.683 mmol), N,N-dimethylglycine (70 mg, 0.683 mmol), copper(I) iodide (65 mg, 0.342 mmol), and potassium carbonate (189 mg, 1.367 mmol) were added. The mixture was heated to 130 °C and allowed to react for 1 h. LCMS showed that the starting materials were completely reacted. Water (5 mL) was added for dilution, and the mixture was extracted with ethyl acetate (10 mL × 2). The organic phase was concentrated to dryness, and the residue was separated and purified by a silica gel column (PE:EA = 1:1) to give 2-hydroxy-N-(4-(5-(6-methyl-2-(3,3,3-trifluoropropoxy)pyrimidin-4-yl)-1,3,4-oxadiazol-2-yl)-3-(6-azaspiro[2.5]octan-6-yl)phenyl)ethane-1-sulfonamide (50 mg, yield: 25.1%).

¹HNMR (400 MHz, DMSO-d6) δ7.90 (d, 1H), 7.82 (s, 1H), 7.06 (d, 1H), 6.94-6.97 (dd, 1H), 4.61 (t, 2H), 3.75 (t, 2H), 3.35 (t, 2H), 2.86-2.93 (m, 6H), 2.53 (s, 3H), 1.50 (s, 4H), 0.28 (s, 4H).

LC-MS, M/Z (ESI): 583.2 [M+H]⁺.

### Example 19: Preparation of Target Compound I-53

### N-(4-(5-(2-(4,4-Difluoropyridin-1-yl)-3-fluoro-6-methylpyridin-4-yl)-1,3,4-oxadiazol-2-yl)-3-(6-azaspiro[2.5]octan-6-yl)phenyl)-2-hydroxyethane-1-sulfonamide

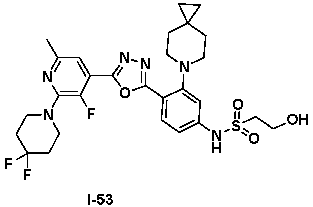

The synthetic route of the target compound **I-53** was as follows:

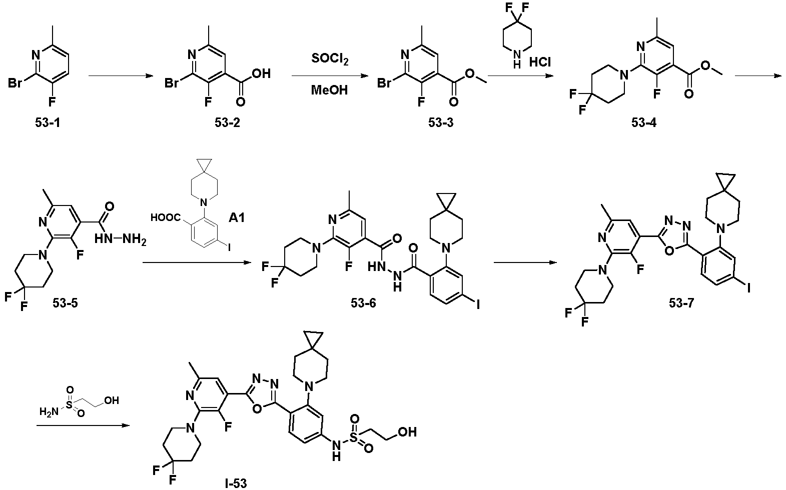

### Step 1: Synthesis of 2-bromo-3-fluoro-6-methylisonicotinic acid

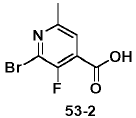

Diisopropylamine (3.99 g, 39.5 mmol) was dissolved in tetrahydrofuran (30 mL). The solution was cooled to -70 °C under nitrogen atmosphere, and then n-butyllithium (2.50 M, 15.8 mL) was added dropwise and slowly. After the dropwise addition was completed, the mixture was allowed to react at 25 °C for 30 min and then cooled to -70 °C. A solution of 2-bromo-3-fluoro-6-methylpyridine (5.00 g, 26.3 mmol) in tetrahydrofuran (30 mL) was then added dropwise and slowly to the above solution, and the mixture was allowed to react at -70 °C for 30 min. Dry carbon dioxide was then introduced, and the reaction system was allowed to react for 1 h. After the reaction was completed, the reaction liquid was quenched with water (20 mL), extracted with ethyl acetate (90 mL × 3), dried over anhydrous sodium sulfate, filtered, and concentrated to give a crude product. The crude product was then stirred with methyl *tert*-butyl ether (20 mL) at 25 °C for 30 min, and the mixture was filtered. The filter cake was collected and dried to give 2-bromo-3-fluoro-6-methylisonicotinic acid (5.60 g, yield: 90.9%).

LC-MS, M/Z (ESI): 234.1 [M+H]⁺.

### Step 2: Synthesis of methyl 2-bromo-3-fluoro-6-methylisonicotinate

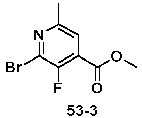

2-Bromo-3-fluoro-6-methylisonicotinic acid (4.00 g, 17.1 mmol) was dissolved in methanol (20.0 mL) and *N,N-*dimethylformamide (125 mg, 1.71 mmol), and then thionyl chloride (2.64 g, 22.2 mmol) was added dropwise. The mixture was stirred under nitrogen atmosphere at 60 °C for 5 h. The reaction liquid was concentrated to give methyl 2-bromo-3-fluoro-6-methylisonicotinate (3.50 g, yield: 82.5%), which was used directly in the next step.

LC-MS, M/Z (ESI): 250.0 [M+H]⁺.

### Step 3: Synthesis of methyl 2-(4,4-difluoropiperidin-1-yl)-3-fluoro-6-methylisonicotinate

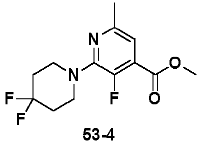

Methyl 2-bromo-3-fluoro-6-methylisonicotinate (3.50 g, 14.1 mmol), 4,4-difluoropiperidine hydrochloride (2.22 g, 14.1 mmol), tris(dibenzylideneacetone)dipalladium (1.94 g, 2.12 mmol), cesium carbonate (9.19 g, 28.2 mmol), and 4,5-bis(diphenylphosphino)-9,9-dimethylxanthene (1.63 g, 2.82 mmol) were dissolved in 1,4-dioxane (20 mL), and the mixture was stirred under nitrogen atmosphere at 100 °C for 10 h. Water (20 mL) was added at 25 °C to quench the reaction, and then the mixture was extracted with ethyl acetate (60 mL × 3). The organic phase was washed with saturated brine (30 mL × 3), dried over anhydrous sodium sulfate, and filtered. The reaction liquid was concentrated, and the crude product was purified by reverse phase Flash (0.1% aqueous ammonia) to give methyl 2-(4,4-difluoropiperidin-1-yl)-3-fluoro-6-methylisonicotinate (1.00 g, yield: 18.9%).

¹H NMR (400 MHz, CDCl₃) *δ* 6.91 (d, *J =* 4.0 Hz, 1H), 3.86 (s, 3H), 3.55-3.58 (m, 4H), 2.33 (s, 3H), 1.98 - 2.04 (m, 4H).

LC-MS, M/Z (ESI): 289.0 [M+H]⁺.

### Step 4: Synthesis of 2-(4,4-difluoropiperidin-1-yl)-3-fluoro-6-methylisonicotinohydrazide

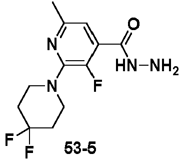

Methyl 2-(4,4-difluoropiperidin-1-yl)-3-fluoro-6-methylisonicotinate (0.4 g, 1.4 mmol) was added to 8 mL of ethanol, and then 80% hydrazine hydrate (0.9 mL, 14 mmol) was added, The mixture was heated to 60 °C and allowed to react for 3 h. Water (50 mL) was added for dilution, and the mixture was extracted with ethyl acetate (20 mL × 3). The organic phase was concentrated to dryness, and the residue was separated and purified by a silica gel column (PE:EA (V/V) = 1:1) to give 2-(4,4-difluoropiperidin-1-yl)-3-fluoro-6-methylisonicotinohydrazide (400 mg, yield: 99%).

LC-MS, M/Z (ESI): 289.2 [M+H]⁺.

### Step 5: Synthesis of 2-(4,4-difluoropiperidin-1-yl)-3-fluoro-N'-(4-iodo-2-(6-azaspiro[2.5]octan-6-yl)benzoyl)-6-methylisonicotinohydrazide

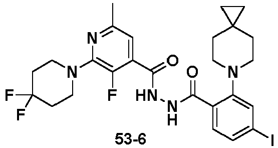

2-(4,4-Difluoropiperidin-1-yl)-3-fluoro-6-methylisonicotinohydrazide (375 mg, 1.3 mmol) and 4-iodo-2-(6-azaspiro[2.5]octan-6-yl)benzoic acid (450 mg, 1.3 mmol) were added to 9 mL of acetonitrile, and then 1-methyl-1H-imidazole (320 mg, 3.9 mmol) and N,N,N,N-tetramethylchloroformamidinium hexafluorophosphate (426 mg, 1.67 mmol) were added. The mixture was allowed to react at 20 °C for 3 h. Water (50 mL) was added for dilution, and the mixture was extracted with ethyl acetate (20 mL × 3). The organic phase was concentrated to dryness, and the residue was separated and purified by a silica gel column (PE:EA (V/V) = 3:1) to give 2-(4,4-difluoropiperidin-1-y1)-3-fluoro-*N*'-(4-iodo-2-(6-azaspiro[2.5]octan-6-yl)benzoyl)-6-methylisonicotinohydrazide (600 mg, yield: 75.9%).

LC-MS, M/Z (ESI): 628.3 [M+H]⁺.

### Step 6: Synthesis of 2-(2-(4,4-difluoropiperidin-1-yl)-3-fluoro-6-methylpyridin-4-yl)-5-(4-iodo-2-(6-azaspiro[2.5] octan-6-yl)phenyl)-1,3,4-oxadiazole

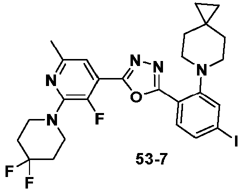

2-(4,4-Difluoropiperidin-1-y1)-3-fluoro-*N*'-(4-iodo-2-(6-azaspiro[2.5]octan-6-yl)benzoyl)-6-methylisonicotinohydrazide (300 mg, 0.5 mmol) was added to 6 mL of dichloromethane, and then p-methylbenzenesulfonyl chloride (286 mg, 1.5 mmol) and triethylamine (253 mg, 2.5 mmol) were added. The mixture was allowed to react at room temperature overnight. Water (30 mL) was added for dilution, and the mixture was extracted with ethyl acetate (10 mL × 2). The organic phase was concentrated to dryness, and the residue was separated and purified by a silica gel column (PE:EA (V/V) = 3:1) to give 2-(2-(4,4-difluoropiperidin-1-yl)-3-fluoro-6-methylpyridin-4-yl)-5-(4-iodo-2-(6-azaspiro[2.5]octan-6-yl)phenyl)-1,3,4-oxadiazole (280 mg, yield: 96%).

LC-MS, M/Z (ESI): 610.2 [M+H]⁺.

### Step 7: Synthesis of N-(4-(5-(2-(4,4-difluoropyridin-1-yl)-3-fluoro-6-methylpyridin-4-yl)-1,3,4-oxadiazol-2-yl)-3-(6-azaspiro[2.5]octan-6-yl)phenyl)-2-hydroxyethane-1-sulfonamide

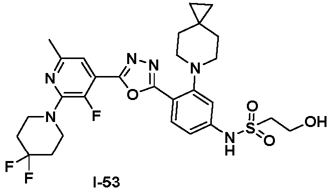

2-(2-(4,4-Difluoropiperidin-1-yl)-3-fluoro-6-methylpyridin-4-yl)-5-(4-iodo-2-(6-azaspiro[2.5]octan-6-yl)phenyl)-1,3,4-oxadiazole (250 mg, 0.5 mmol) was added to 5.6 mL of *N,N*-dimethylformamide, and then 2-hydroxyethane-1-sulfonamide (15 mg, 2.0 mmol), trans-*N*,*N'*-dimethylcyclohexane-1,2-diamine (14.2 mg, 0.1 mmol), copper(I) iodide (38 mg, 0.2 mmol), and potassium phosphate (424.5 mg, 2.0 mmol) were added. The mixture was heated to 120 °C and allowed to react for 3 h. Water (50 mL) was added for dilution, and the mixture was extracted with ethyl acetate (20 mL × 3). The organic phase was concentrated to dryness, and the residue was separated and purified by a reverse phase column (0.1% formic acid) to give *N*-(4-(5-(2-(4,4-difluoropyridin-1-yl)-3-fluoro-6-methylpyridin-4-yl)-1,3,4-oxadiazol-2-yl)-3-(6-azaspiro[2.5]octan-6-yl)phenyl)-2-hydroxyethane-1-sulfonamide (215 mg, yield: 77%).

¹H NMR (400 MHz, DMSO-d₆) δ 7.86 (d, 1H), 7.33 (d, 1H), 7.08 (d, 1H), 6.97 (dd, 1H), 3.75 (t, 2H), 3.61 (dd, 4H), 3.34 (t, 2H), 2.92 (t, 4H), 2.42 (s, 3H), 2.09 (tt, 4H), 1.44 (t, 4H), 0.29 (s, 4H).

LC-MS, M/Z (ESI): 607.6 [M+H]⁺.

### Example 20: Preparation of Target Compound I-80

### N-(4-(4-(2-(4,4-Difluoropiperidin-1-yl)-6-methylpyrimidin-4-yl)-1H-pyrazol-1-yl)-3-(6-azaspiro[2.5]octan-6-yl)phenyl)-2-hydroxyethane-1-sulfonamide

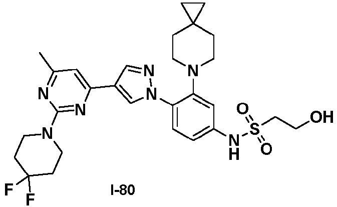

The synthetic route of the target compound **I-80** was as follows:

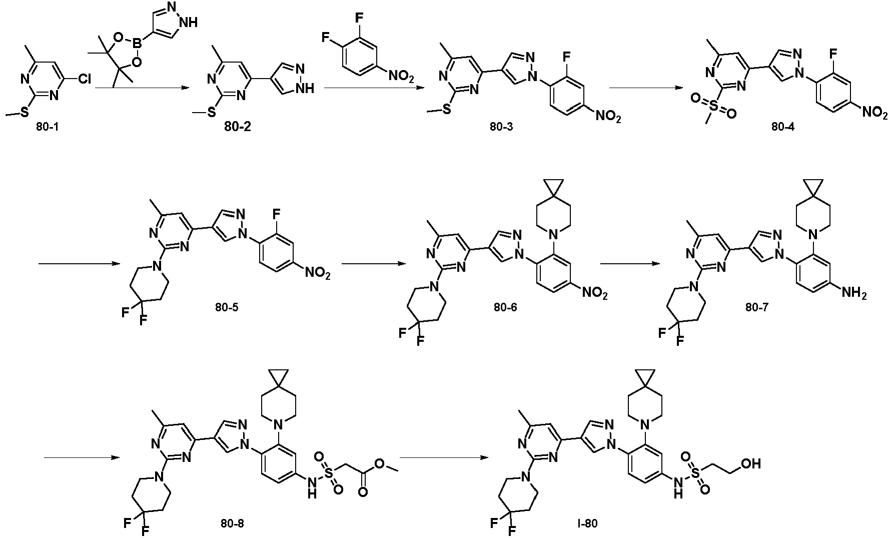

### Step 1: Synthesis of 4-methyl-2-(methylthio)-6-(1H-pyrazol-4-yl)pyrimidine

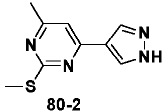

4-Chloro-6-methyl-2-(methylthio)pyrimidine (30 g, 172 mmol) was added to 300 mL of *N,N*-dimethylformamide, and then 4-(4,4,5,5-tetramethyl-1,3,2-dioxaborolan-2-yl)-1H-pyrazole (50 g, 258 mmol), potassium carbonate (47.5 g, 344 mmol), water (50 mL), and 1,1'-bis(diphenylphosphino)ferrocene dichloropalladium(II) (12.57 g, 17.18 mmol) were added. The mixture was heated to 105 °C and allowed to react for 16 h. LCMS showed that the starting materials were completely reacted. The reaction liquid was concentrated to dryness, and the residue was separated and purified by a silica gel column (PE:EA (V/V) = 1:1) to give 4-methyl-2-(methylthio)-6-(1H-pyrazol-4-yl)pyrimidine (20 g, yield: 56.4%).

LC-MS, M/Z (ESI): 207.2 [M+H]⁺.

### Step 2: Synthesis of 4-(1-(2-fluoro-4-nitrophenyl)-1H-pyrazol-4-yl)-6-methyl-2-(methylthio)pyrimidine

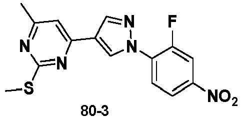

4-Methyl-2-(methylthio)-6-(1*H*-pyrazol-4-yl)pyrimidine (20 g, 97 mmol) was added to 20 mL of *N,N-*dimethylformamide, and then 1,2-difluoro-4-nitrobenzene (46.3 g, 291 mmol) and cesium carbonate (63.2 g, 194 mmol) were added. The mixture was allowed to react at room temperature for 16 h. LCMS showed that the starting materials were completely reacted. The reaction liquid was concentrated to dryness, and the residue was separated and purified by a silica gel column (volume ratio: PE:DCM:EA = 3:3:1) to give 4-(1-(2-fluoro-4-nitrophenyl)-1*H-*pyrazol-4-yl)-6-methyl-2-(methylthio)pyrimidine (23 g, yield: 68.7%).

LC-MS, M/Z (ESI): 346.1 [M+H]⁺.

### Step 3: Synthesis of 4-(1-(2-fluoro-4-nitrophenyl)-1H-pyrazol-4-yl)-6-methyl-2-(methylsulfonyl)pyrimidine

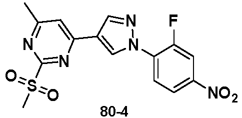

4-(1-(2-Fluoro-4-nitrophenyl)-1*H*-pyrazol-4-yl)-6-methyl-2-(methylthio)pyrimidine (23 g, 66.6 mmol) was added to 300 mL of dichloromethane, and then *m*-chloroperoxybenzoic acid (40.2 g, 233 mmol) was added. The mixture was allowed to react at room temperature for 1 h. LCMS showed that the starting materials were completely reacted. Saturated sodium sulfite (200 mL) was added, and the mixture was stirred for 30 min. Saturated sodium carbonate (100 mL) was added, and the mixture was stirred for 10 min and then extracted with dichloromethane (200 mL × 3), followed by liquid separation. The organic phase was concentrated to dryness, and the residue was separated and purified by a silica gel column (DCM:EA (V/V) = 3:1) to give 4-(1-(2-fluoro-4-nitrophenyl)-1*H*-pyrazol-4-yl)-6-methyl-2-(methylsulfonyl)pyrimidine (13 g, yield: 51.7%).

LC-MS, M/Z (ESI): 378.1 [M+H]⁺.

### Step 4: Synthesis of 2-(4,4-difluoropiperidin-1-yl)-4-(1-(2-fluoro-4-nitrophenyl)-1H-pyrazol-4-yl)-6-methylpyrimidine

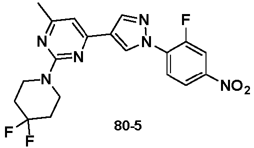

4-(1-(2-Fluoro-4-nitrophenyl)-1*H*-pyrazol-4-yl)-6-methyl-2-(methylsulfonyl)pyrimidine (6 g, 15.9 mmol) was added to 60 mL of *N*,*N*-dimethylformamide, and then 4,4-difluoropiperidine hydrochloride (3.76 g, 23.85 mmol) and potassium carbonate (8.79 g, 63.6 mmol) were added. The mixture was heated to 105 °C and allowed to react overnight. LCMS showed that the starting materials were completely reacted. The reaction liquid was concentrated to dryness, and the residue was separated and purified by a silica gel column (volume ratio: PE:DCM:EA = 3:3:1) to give 2-(4,4-difluoropiperidin-1-yl)-4-(1-(2-fluoro-4-nitrophenyl)-1*H*-pyrazol-4-yl)-6-methylpyrimidine (6 g, yield: 90%).

LC-MS, M/Z (ESI): 419.1 [M+H]⁺.

### Step 5: Synthesis of 6-(2-(4-(2-(4,4-difluoropiperidin-1-yl)-6-methylpyrimidin-4-yl)-1H-pyrazol-1-yl)-5-nitrophenyl)-6-azaspiro[2.5]octane

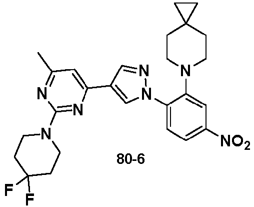

2-(4,4-Difluoropiperidin-1-yl)-4-(1-(2-fluoro-4-nitrophenyl)-1*H*-pyrazol-4-yl)-6-methylpyrimidine (3 g, 7.17 mmol) was added to 30 mL of dimethylsulfoxide, and then 6-azaspiro[2.5]octane hydrochloride (1.588 g, 10.76 mmol) and potassium carbonate (3.96 g, 28.7 mmol) were added. The mixture was heated to 140 °C and allowed to react for 8 h. LCMS showed that the starting materials were completely reacted. The reaction liquid was poured into 200 mL of water, and the mixture was filtered to give 6-(2-(4-(2-(4,4-difluoropiperidin-1-yl)-6-methylpyrimidin-4-yl)-1*H-*pyrazol-1-yl)-5-nitrophenyl)-6-azaspiro[2.5]octane (2.6 g, yield: 71.2%).

LC-MS, M/Z (ESI): 510.2 [M+H]⁺.

### Step 6: Synthesis of 4-(4-(2-(4,4-difluoropiperidin-1-yl)-6-methylpyrimidin-4-yl)-1H-pyrazol-1-yl)-3-(6-azaspiro [2.5]octan-6-yl)aniline

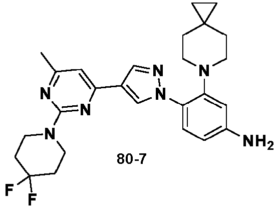

6-(2-(4-(2-(4,4-Difluoropiperidin-1-yl)-6-methylpyrimidin-4-yl)-1*H*-pyrazol-1-yl)-5-nitrophenyl)-6-azaspiro[2.5]octane (2.6 g, 5.1 mmol) was added to 60 mL of ethanol, and then iron powder (2.85 g, 51 mmol) and ammonium chloride (2.73 g, 51 mmol) were added. The mixture was heated to 90 °C and allowed to react for 8 h. LCMS showed that the starting materials were completely reacted. The mixture was filtered and washed with 60 mL of ethanol. The filtrate was concentrated to dryness, and the residue was separated and purified by a silica gel column (PE:EA (V/V) = 3:1) to give 4-(4-(2-(4,4-difluoropiperidin-1-yl)-6-methylpyrimidin-4-yl)-1*H*-pyrazol-1-yl)-3-(6-azaspiro[2.5]octan-6-yl)aniline (2.4 g, yield: 98%).

LC-MS, M/Z (ESI): 480.2 [M+H]⁺.

### Step 7: Synthesis of methyl 2-(N-(4-(4-(2-(4,4-difluoropiperidin-1-yl)-6-methylpyrimidin-4-yl)-1H-pyrazol-1-yl)-3-(6-azaspiro[2.5]octan-6-yl)phenyl)sulfamoyl)acetate

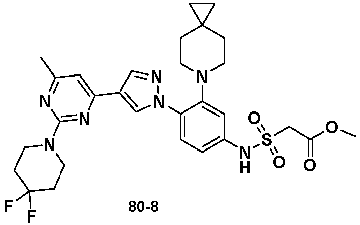

4-(4-(2-(4,4-Difluoropiperidin-1-yl)-6-methylpyrimidin-4-yl)-1*H*-pyrazol-1-yl)-3-(6-azaspiro[2.5]octan-6-yl)aniline (2.4 g, 5 mmol) was added to 30 mL of dichloromethane, and then triethylamine (1.94 g, 15.01 mmol) and methyl 2-(chlorosulfonyl)acetate (1.036 g, 5 mmol) were added. The mixture was allowed to react at room temperature for 1 h. LCMS showed that the starting materials were completely reacted. The reaction liquid was concentrated to dryness, and the residue was separated and purified by a silica gel column (PE:EA (V/V) = 5:1) to give methyl 2-(*N*-(4-(4-(2-(4,4-difluoropiperidin-1-yl)-6-methylpyrimidin-4-yl)-1*H*-pyrazol-1-yl)-3-(6-azaspiro [2.5]octan-6-yl)phenyl)sulfamoyl)acetate (1.9 g, yield: 61.7%).

LC-MS, M/Z (ESI): 616.2 [M+H]⁺.

### Step 8: Synthesis of N-(4-(4-(2-(4,4-difluoropiperidin-1-yl)-6-methylpyrimidin-4-yl)-1H-pyrazol-1-yl)-3-(6-azaspiro[2.5]octan-6-yl)phenyl)-2-hydroxyethane-1-sulfonamide

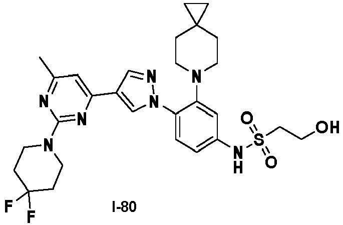

Methyl 2-(*N*-(4-(4-(2-(4,4-difluoropiperidin-1-yl)-6-methylpyrimidin-4-yl)-1*H*-pyrazol-1-yl)-3-(6-azaspiro[2.5] octan-6-yl)phenyl)sulfamoyl)acetate (1.90 g, 3.1 mmol) was dissolved in tetrahydrofuran (20 mL), and then lithium borohydride (2 M, 1.85 mL) was added dropwise under nitrogen atmosphere at 0 °C. The mixture was allowed to react at 0 °C for 1 h. After the reaction was completed, the reaction liquid was quenched with a saturated ammonium chloride solution (50 mL) and extracted with ethyl acetate (200 mL × 3). The organic phase was washed with saturated brine (100 mL), dried over anhydrous sodium sulfate, filtered, and concentrated to give a crude product, which was then separated and purified by reverse phase Flash (0.1% HCl) to give *N*-(4-(4-(2-(4,4-difluoropiperidin-1-yl)-6-methylpyrimidin-4-yl)-1*H*-pyrazol-1-yl)-3-(6-azaspiro[2.5]octan-6-yl)phenyl)-2-hydroxyethane-1-sulfonamide (1.31 g, 2.17 mmol, purity: 97.5%, yield: 71.0%).

¹H NMR (400 MHz, CDCl₃) δ 8.92 (s, 1H), 8.12 (s, 1H), 7.56 (d, *J =* 8.4 Hz, 1H), 7.10 (s, 1H), 6.91-6.94 (m, 1H), 6.77 (s, 1H), 6.65 (s, 1H), 4.13-4.16 (m, 2H), 4.05-4.08 (m, 4H), 3.50 (s, 1H), 3.31-3.34 (m, 2H), 2.80-2.83 (m, 4H), 2.40 (s, 3H), 1.98-2.07 (m, 4H), 1.41-1.45 (m, 4H), 0.30 (s, 4H).

LC-MS, M/Z (ESI): 588.3 [M+H]⁺.

### Example 21: Preparation of Target Compound I-81

### N-(4-(1-(2-(4,4-Difluoropiperidin-1-yl)-6-methylpyrimidin-4-yl)-1H-pyrazol-4-yl)-3-(6-azaspiro[2.5]octan-6-yl)phenyl)-2-hydroxyethane-1-sulfonamide

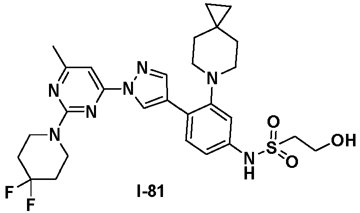

The synthetic route of the target compound **I-81** was as follows:

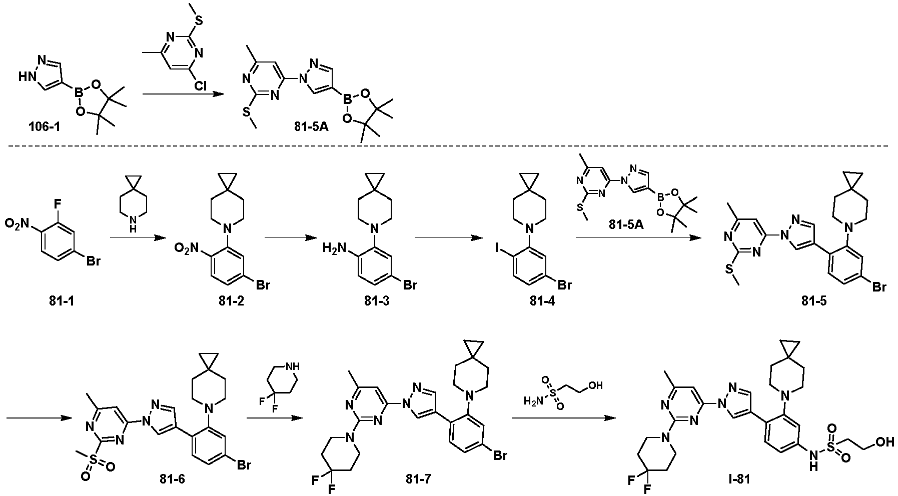

### Step 1: Synthesis of 4-methyl-2-methylthio-6-(4-(4,4,5,5-tetramethyl-1,3,2-dioxaborolan-2-yl)-1H-pyrazol-1-yl)pyrimidine

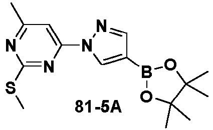

To a solution of 4-chloro-6-methyl-2-methylthiopyrimidine (10 g, 57.3 mmol) and 4-(4,4,5,5-tetramethyl-1,3,2-dioxaborolan-2-yl)-1*H*-pyrazole (12.2 g, 63 mmol) in N,N-dimethylformamide (100 mL) was added potassium carbonate (23.8 g, 172 mmol), and the mixture was stirred at 50 °C for 18 h. The reaction liquid was poured into water (500 mL), and the mixture was extracted with ethyl acetate (200 mL × 3). The organic phase was washed with saturated brine (200 mL × 3), dried over anhydrous sodium sulfate, filtered, and concentrated to give a crude product. The crude product was purified by column chromatography (petroleum ether/ethyl acetate (V/V) = 1/0-1/1) to give 4-methyl-2-methylthio-6-(4-(4,4,5,5-tetramethyl-1,3,2-dioxaborolan-2-yl)-1*H*-pyrazol-1-yl)pyrimidine (7 g, yield: 36.8%).

LC-MS, M/Z (ESI): 333.2 [M+H]⁺.

### Step 2: Synthesis of 6-(5-bromo-2-nitrophenyl)-6-azaspiro[2.5]octane

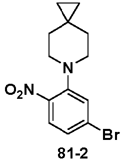

To a solution of 4-bromo-2-fluoro-1-nitrobenzene (5 g, 22.7 mmol) in N,N-dimethylformamide (50 mL) were added 6-azaspiro[2.5]octane (5 g, 45.4 mmol) and potassium carbonate (9.4 g, 68.1 mmol), and the mixture was allowed to react at 100 °C for 6 h. The reaction liquid was poured into water (200 mL), and the mixture was extracted with ethyl acetate (50 mL × 3). The organic phase was washed with saturated brine (30 mL × 5), dried over anhydrous sodium sulfate, filtered, and concentrated to give a crude product. The crude product was purified by column chromatography (petroleum ether/ethyl acetate (V/V) = 1/0-1/2) to give 6-(5-bromo-2-nitrophenyl)-6-azaspiro[2.5]octane (6 g, yield: 84.8%).

LC-MS, M/Z (ESI): 311.3 [M+H]⁺.

### Step 3: Synthesis of 4-bromo-2-(6-azaspiro[2.5]octan-6-yl)aniline

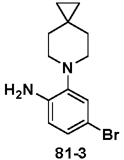

To a solution of 6-(5-bromo-2-nitrophenyl)-6-azaspiro[2.5]octane (6 g, 19.3 mmol) and iron powder (5.39 g, 96.5 mmol) in ethanol (60 mL) was added dropwise and slowly acetic acid (18 mL), and then the mixture was stirred at 80 °C for 2 h. The reaction liquid was adjusted to pH = 7-8 with sodium bicarbonate, then filtered, and concentrated to give a crude product. Methyl *tert*-butyl ether (20 mL) was added to the crude product, and the mixture was stirred for 0.5 h and filtered. The filter cake was dried to give 4-bromo-2-(6-azaspiro[2.5]octan-6-yl)aniline (5 g, yield: 92.2%).

LC-MS, M/Z (ESI): 281.3 [M+H]⁺.

### Step 4: Synthesis of 6-(5-bromo-2-iodophenyl)-6-azaspiro[2.5]octane

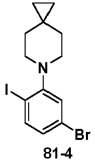

To a solution of 4-bromo-2-(6-azaspiro[2.5]octan-6-yl)aniline (5 g, 17.8 mmol) in acetonitrile (50 mL) was added copper(I) iodide (17 g, 89 mmol), and then isoamyl nitrite (6.3 g, 53.4 mmol) was added dropwise and slowly to the reaction solution at 0 °C. The mixture was stirred at 25 °C for 18 h. The reaction liquid was poured into water (200 mL), and the mixture was extracted with ethyl acetate (50 mL × 3). The organic phase was washed with saturated brine (50 mL × 3), dried over anhydrous sodium sulfate, filtered, and concentrated to give a crude product. The crude product was purified by column chromatography (petroleum ether/ethyl acetate (V/V) = 1/0-1/1) to give 6-(5-bromo-2-iodophenyl)-6-azaspiro[2.5]octane (2.7 g, yield: 38.7%).

LC-MS, M/Z (ESI): 394.4 [M+H]⁺.

### Step 5: Synthesis of 6-(5-bromo-2-(1-(6-methyl-2-methylthiopyrimidin-4-yl)-1H-pyrazol-4-yl)phenyl)-6-azaspiro[2.5]octane

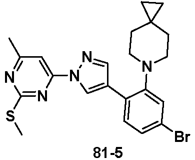

To a solution of 6-(5-bromo-2-iodophenyl)-6-azaspiro[2.5]octane (2 g, 5.1 mmol) and 4-methyl-2-methylthio-6-(4-(4,4,5,5-tetramethyl-1,3,2-dioxaborolan-2-yl)-1*H*-pyrazol-1-yl)pyrimidine (1.5 g, 4.6 mmol) in 1,4-dioxane (20 mL) and water (4 mL) were added [1,1'-bis(diphenylphosphino)ferrocene]dichloropalladium(II) (373 mg, 0.5 mmol) and potassium carbonate (2.1 g, 15.3 mmol), and the mixture was stirred at 80 °C for 18 h. The reaction liquid was poured into water (100 mL), and the mixture was extracted with ethyl acetate (30 mL × 3). The organic phase was washed with saturated brine (20 mL × 2), dried over anhydrous sodium sulfate, filtered, and concentrated to give a crude product. The crude product was purified by column chromatography (petroleum ether/ethyl acetate (V/V) = 1/0-1/1) to give 6-(5-bromo-2-(1-(6-methyl-2-methylthiopyrimidin-4-yl)-1*H*-pyrazol-4-yl)phenyl)-6-azaspiro[2.5]octane (1.6 g, yield: 66.7%).

LC-MS, M/Z (ESI): 470.3 [M+H]⁺.

### Step 6: Synthesis of 6-(5-bromo-2-(1-(6-methyl-2-(methylsulfonyl)pyrimidin-4-yl)-1H-pyrazol-4-yl)phenyl)-6-azaspiro[2.5]octane

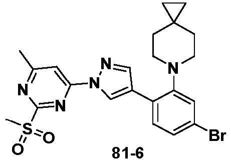

To a solution of 6-(5-bromo-2-(1-(6-methyl-2-methylthiopyrimidin-4-yl)-1*H*-pyrazol-4-yl)phenyl)-6-azaspiro[2.5]octane (0.8 g, 1.7 mmol) in dichloromethane (16 mL) was added m-chloroperoxybenzoic acid (1.0 g, 5.9 mmol) in batches at 0 °C, and then the mixture was stirred at 25 °C for 2 h. The reaction liquid was added slowly to a saturated sodium thiosulfate solution (100 mL), and the mixture was extracted with dichloromethane (20 mL × 3). The organic phase was washed with saturated brine (20 mL × 2), dried over anhydrous sodium sulfate, filtered, and concentrated to give a crude product. The crude product was purified by column chromatography (petroleum ether/ethyl acetate (V/V) = 1/0-1/1) to give 6-(5-bromo-2-(1-(6-methyl-2-(methylsulfonyl)pyrimidin-4-yl)-1H-pyrazol-4-yl)phenyl)-6-azaspiro[2.5]octane (250 mg, yield: 29.3%).

LC-MS, M/Z (ESI): 502.3 [M+H]⁺.

### Step 7: Synthesis of 6-(5-bromo-2-(1-(2-(4,4-difluoropiperidin-1-yl)-6-methylpyrimidin-4-yl)-1H-pyrazol-4-yl)phenyl)-6-azaspiro[2.5]octane

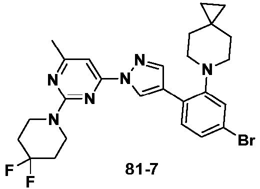

To a solution of 6-(5-bromo-2-(1-(6-methyl-2-(methylsulfonyl)pyrimidin-4-yl)-1H-pyrazol-4-yl)phenyl)-6-azaspiro[2.5]octane (0.25 g, 0.5 mmol) and 4,4-difluoropiperidine (0.16 g, 1.0 mmol) in dimethylsulfoxide (2.5 mL) was added potassium carbonate (0.2 g, 1.5 mmol), and then the mixture was stirred at 100 °C for 8 h. The reaction liquid was poured into water (50 mL), and the mixture was extracted with ethyl acetate (30 mL × 3). The organic phase was washed with saturated brine (10 mL × 2), dried over anhydrous sodium sulfate, filtered, and concentrated to give a crude product. The crude product was purified by column chromatography (petroleum ether/ethyl acetate (V/V) = 1/0-1/3) to give 6-(5-bromo-2-(1-(2-(4,4-difluoropiperidin-1-yl)-6-methylpyrimidin-4-yl)-1*H*-pyrazol-4-yl)phenyl)-6-azaspiro[2.5]octane (0.16 g, yield: 59.2%).

LC-MS, M/Z (ESI): 543.3 [M+H]⁺.

### Step 8: Synthesis of N-(4-(1-(2-(4,4-difluoropiperidin-1-yl)-6-methylpyrimidin-4-yl)-1H-pyrazol-4-yl)-3-(6-azaspiro[2.5]octan-6-yl)phenyl)-2-hydroxyethane-1-sulfonamide

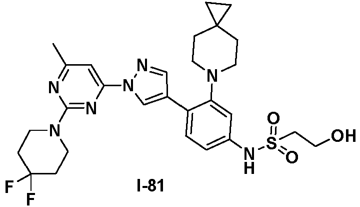

To a solution of 6-(5-bromo-2-(1-(2-(4,4-difluoropiperidin-1-yl)-6-methylpyrimidin-4-yl)-1*H*-pyrazol-4-yl)phenyl)-6-azaspiro[2.5]octane (0.16 g, 0.3 mmol) and 2-hydroxyethanesulfonamide (74 mg, 0.6 mmol) in *N,N-*dimethylformamide (1.6 mL) were added *N,N*-dimethylglycine (8.5 mg, 0.06 mmol), copper(I) iodide (5.7 mg, 0.03 mmol), and potassium carbonate (124 mg, 0.9 mmol), and then the mixture was stirred at 130 °C for 2 h. The reaction liquid was poured into water (20 mL), and the mixture was extracted with ethyl acetate (10 mL × 3). The organic phase was washed with saturated brine (10 mL × 5), dried over anhydrous sodium sulfate, filtered, and concentrated to give a crude product. The crude product was purified by preparative reverse phase chromatography (column: Phenomenex Synergi C18 100 × 25 mm × 4 µm; solvents: A = water + 0.1 vol% formic acid (99%), B = acetonitrile; gradient: 5%-95%, 7 min) to give *N*-(4-(1-(2-(4,4-difluoropiperidin-1-yl)-6-methylpyrimidin-4-yl)-1*H*-pyrazol-4-yl)-3-(6-azaspiro[2.5]octan-6-yl)phenyl)-2-hydroxyethane-1-sulfonamide (81.8 mg, purity: 97.35%, yield: 47%).

1H NMR (400 MHz, DMSO_d₆) δ 9.74 (s, 1H), 9.38 (s, 1H), 8.35 (s, 1H), 7.61 (d, J = 8.4 Hz, 1H), 7.15 - 7.02 (m, 2H), 6.96 (dd, J = 8.4, 2.2 Hz, 1H), 4.93 (s, 1H), 4.12 - 3.86 (m, 4H), 3.75 (t, J = 6.8 Hz, 2H), 3.26 (t, J = 6.8 Hz, 2H), 2.83 (s, 4H), 2.39 (s, 3H), 2.04 (dt, J = 19.4, 6.8 Hz, 4H), 1.51 (s, 4H), 0.34 (s, 4H).

LC-MS, M/Z (ESI): 588.5 [M+H]⁺.

### Example 22: Preparation of Target Compound I-90

### N-(4-(5-(2-(4,4-Difluoropiperidin-1-yl)-6-methylpyridin-4-yl)-1,3,4-thiadiazol-2-yl)-3-(6-azaspiro[2.5]octan-6-yl)phenyl)-2-hydroxyethane-1-sulfonamide

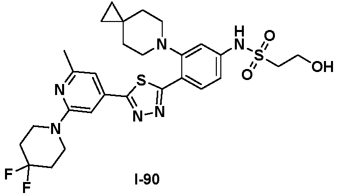

The synthetic route of the target compound **I-90** was as follows:

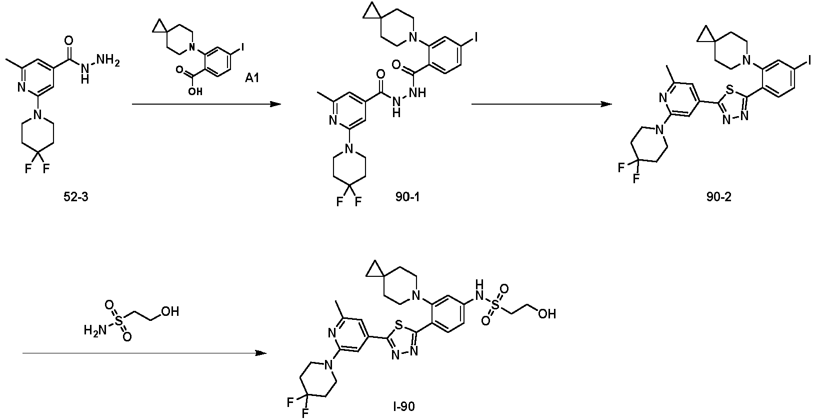

### Step 1: Synthesis of 2-(4,4-difluoropiperidin-1-yl)-N'-(4-iodo-2-(6-azaspiro[2.5]octan-6-yl)benzoyl)-6-methylisonicotinohydrazide

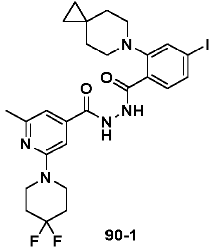

2-(4,4-Difluoropiperidin-1-yl)-6-methylisonicotinohydrazide (200 mg, 0.74 mmol), 4-iodo-2-(6-azaspiro[2.5]octan-6-yl)benzoic acid (0.23 g, 0.7 mmol), *O*-(7-azabenzotriazol-1-yl)-*N,N,N',N*'-tetramethyluronium hexafluorophosphate (450 mg, 1.0 mmol), and N,N-diisopropylethylamine (232 mg, 1.8 mmol) were dissolved in *N*,*N*-dimethylformamide (3 mL), and the solution was stirred at room temperature for 2 h. Water (30 mL) was then added for dilution, and the mixture was extracted with dichloromethane (20 mL × 2). The organic phase was concentrated to dryness, and the residue was separated and purified by a silica gel column (petroleum ether:ethyl acetate (V/V) = 10:1) to give 2-(4,4-difluoropiperidin-1-yl)-*N'*-(4-iodo-2-(6-azaspiro[2.5]octan-6-yl)benzoyl)-6-methylisonicotinohydrazide (250 mg, yield: 55%).

LC-MS, M/Z (ESI): 610.2 [M+H]⁺.

### Step 2: Synthesis of 2-(2-(4,4-difluoropiperidin-1-yl)-6-methylpyridin-4-yl)-5-(4-iodo-2-(6-azaspiro[2.5]octan-6-yl)phenyl)-1,3,4-thiadiazole

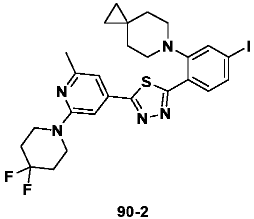

2-(4,4-Difluoropiperidin-1-yl)-*N'*-(4-iodo-2-(6-azaspiro[2.5]octan-6-yl)benzoyl)-6-methylisonicotinohydrazide (250 mg, 0.41 mmol) and Lawesson reagent (1.65 g, 4.1 mmol) were added to tetrahydrofuran, and the mixture was allowed to react at 80 °C for 3 h. The reaction liquid was then directly concentrated to dryness, and the residue was separated and purified by a silica gel column (petroleum ether:ethyl acetate (V/V) = 1:1) to give 2-(2-(4,4-difluoropiperidin-1-yl)-6-methylpyridin-4-yl)-5-(4-iodo-2-(6-azaspiro[2.5]octan-6-yl)phenyl)-1,3,4-thiadiazole (100 mg, yield: 40%).

LC-MS, M/Z (ESI): 608.2 [M+H]⁺.

### Step 3: Synthesis of N-(4-(5-(2-(4,4-difluoropiperidin-1-yl)-6-methylpyridin-4-yl)-1,3,4-thiadiazol-2-yl)-3-(6-azaspiro[2.5]octan-6-yl)phenyl)-2-hydroxyethane-1-sulfonamide

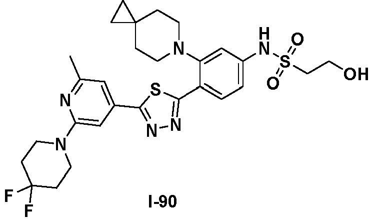

2-(2-(4,4-Difluoropiperidin-1-yl)-6-methylpyridin-4-yl)-5-(4-iodo-2-(6-azaspiro[2.5]octan-6-yl)phenyl)-1,3,4-thiadiazole (60 mg, 0.2 mmol) was added to 2 mL of N,N-dimethylformamide, and then 2-hydroxyethane-1-sulfonamide (100 mg, 0.8 mmol), *trans-N,N'*-dimethylcyclohexane-1,2-diamine (15 mg, 0.04 mmol), copper(I) iodide (20 mg, 0.08 mmol), and potassium phosphate (170 mg, 0.8 mmol) were added. The mixture was heated to 120 °C and allowed to react for 1 h. Water (10 mL) was added for dilution, and the mixture was extracted with ethyl acetate (10 mL × 2). The organic phase was concentrated to dryness, and the residue was separated and purified by a silica gel column (ethyl acetate:methanol (V/V) = 10:1) to give *N*-(4-(5-(2-(4,4-difluoropiperidin-1-yl)-6-methylpyridin-4-yl)-1,3,4-thiadiazol-2-yl)-3-(6-azaspiro[2.5]octan-6-yl)phenyl)-2-hydroxyethane-1-sulfonamide (30 mg, yield: 51%).

¹H NMR (400 MHz, DMSO-d6) δ10.15 (s, 1H), 8.20 (d, 1H), 7.32 (d, 1H), 7.25 (d, 1H), 7.16 (d, 1H), 7.05 (d, 1H), 4.95 (s, 1H), 3.78-3.76(m, 3H), 3.35 (m, 2H), 2.89 (m, 3H), 2.50(s, 3H), 2.06 (m, 4H), 1.60 (s, 2H), 1.22 (m, 4H), 0.83 (m, 1H) 0.29 (m, 4H).

LC-MS, M/Z (ESI): 605.2 [M+H]⁺.

### Example 23: Preparation of Target Compound I-96

### N (4-(5-(6-Methyl-2-(4-(trifluoromethyl)piperidin-1-yl)pyrimidin-4-yl)-1,3,4-oxadiazol-2-yl)-3-(6-azaspiro[2.5]octan-6-yl)phenyl)-2-hydroxyethane-1-sulfonamide

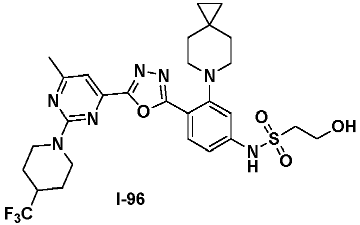

The synthetic route of the target compound **I-96** was as follows:

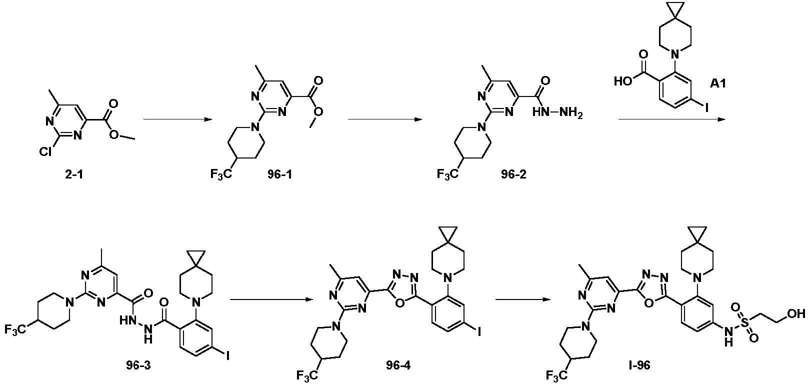

### Step 1: Synthesis of methyl 6-methyl-2-(4-(trifluoromethyl)piperidin-1-yl)pyrimidine-4-carboxylate

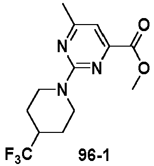

Methyl 2-chloro-6-methylpyrimidine-4-carboxylate (2 g, 10.7 mmol) was added to 20 mL of *N,N-*dimethylformamide, and then 4-(trifluoromethyl)piperidine hydrochloride (4.06 g, 21.4 mmol) and potassium carbonate (7.41 g, 53.6 mmol) were added. The mixture was heated to 100 °C and allowed to react for 2 h. LCMS showed that the starting materials were completely reacted. Water (30 mL) was added for dilution, and the mixture was extracted with ethyl acetate (50 mL × 2). The organic phase was concentrated to dryness, and the residue was separated and purified by a silica gel column (PE:EA (V/V) = 5:1) to give methyl 6-methyl-2-(4-(trifluoromethyl)piperidin-1-yl)pyrimidine-4-carboxylate (2.4 g, yield: 73.8%).

LC-MS, M/Z (ESI): 304.1 [M+H]⁺.

### Step 2: Synthesis of 6-methyl-2-(4-(trifluoromethyl)piperidin-1-yl)pyrimidine-4-carbohydrazide

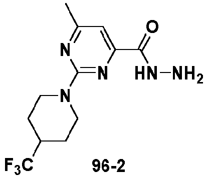

Methyl 6-methyl-2-(4-(trifluoromethyl)piperidin-1-yl)pyrimidine-4-carboxylate (2.4 g, 7.9 mmol) was added to 25 mL of methanol, and then 4 mL of 80% hydrazine hydrate was added. The mixture was heated to 60 °C and allowed to react for 1 h. LCMS showed that the starting materials were completely reacted. The reaction liquid was concentrated to dryness, and the residue was lyophilized to give a crude product of 6-methyl-2-(4-(trifluoromethyl)piperidin-1-yl)pyrimidine-4-carbohydrazide (2.4 g, yield: 100%).

LC-MS, M/Z (ESI): 304.1 [M+H]⁺.

### Step 3: Synthesis of N'-(4-iodo-2-(6-azaspiro[2.5]octan-6-yl)benzoyl)-6-methyl-2-(4-(trifluoromethyl)piperidin-1-yl)pyrimidine-4-carbohydrazide

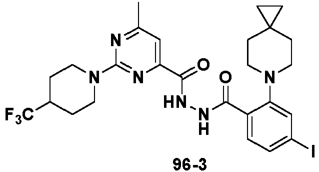

6-Methyl-2-(4-(trifluoromethyl)piperidin-1-yl)pyrimidine-4-carbohydrazide (2.4 g, 8.0 mmol) was added to 25 mL of *N*,*N*-dimethylformamide, and then 4-iodo-2-(6-azaspiro[2.5]octan-6-yl)benzoic acid (1.9 g, 5.3 mmol), *N,N-*diisopropylethylamine (4.12 g, 31.9 mmol), and *O*-(7-azabenzotriazol-1-yl)-*N,N,N,N*'-tetramethyluronium hexafluorophosphate (6.06 g, 16.0 mmol) were added. The mixture was heated to 60 °C and allowed to react for 1 h. LCMS showed that the starting materials were completely reacted. Water (20 mL) was added for dilution, and the mixture was extracted with ethyl acetate (100 mL × 2). The organic phase was concentrated to dryness, and the residue was separated and purified by a silica gel column (PE:EA (V/V) = 3:1) to give *N'*-(4-iodo-2-(6-azaspiro[2.5]octan-6-yl)benzoyl)-6-methyl-2-(4-(trifluoromethyl)piperidin-1-yl)pyrimidine-4-carbohydrazide (1.5 g, yield: 43.9%).

LC-MS, M/Z (ESI): 643.1 [M+H]⁺.

### Step 4: Synthesis of 2-(4-iodo-2-(6-azaspiro[2.5]octan-6-yl)phenyl)-5-(6-methyl-2-(4-(trifluoromethyl)piperidin-1-yl)pyrimidin-4-yl)-1,3,4-oxadiazole

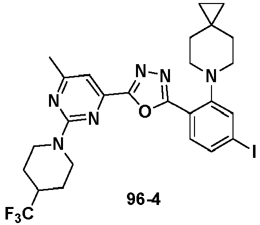

*N'*-(4-Iodo-2-(6-azaspiro [2.5] octan-6-yl)benzoyl)-6-methyl-2-(4-(trifluoromethyl)piperidin-1-yl)pyrimidine-4-carbohydrazide (0.3 g, 0.48 mmol) was added to 5 mL of dichloromethane, and then p-methylbenzenesulfonyl chloride (0.445 g, 2.34 mmol) and triethylamine (0.473 g, 4.67 mmol) were added. The mixture was allowed to react at room temperature overnight. LCMS showed that the starting materials were completely reacted. Water (5 mL) was added for dilution, and the mixture was extracted with ethyl acetate (10 mL × 2). The organic phase was concentrated to dryness, and the residue was separated and purified by a silica gel column (PE:EA (V/V) = 3:1) to give 2-(4-iodo-2-(6-azaspiro [2.5] octan-6-yl)phenyl)-5-(6-methyl-2-(4-(trifluoromethyl)piperidin-1-yl)pyrimidin-4-yl)-1,3,4-oxadiazole (0.2 g, yield: 68.6%).

LC-MS, M/Z (ESI): 625.1 [M+H]⁺.

### Step 5: Synthesis of N-(4-(5-(6-methyl-2-(4-(trifluoromethyl)piperidin-1-yl)pyrimidin-4-yl)-1,3,4-oxadiazol-2-yl)-3-(6-azaspiro[2.5]octan-6-yl)phenyl)-2-hydroxyethane-1-sulfonamide

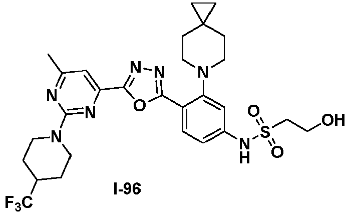

2-(4-Iodo-2-(6-azaspiro [2.5] octan-6-yl)phenyl)-5-(6-methyl-2-(4-(trifluoromethyl)piperidin-1-yl)pyrimidin-4-yl)-1,3,4-oxadiazole (0.2 g, 0.32 mmol) was added to 4 mL of *N,N*-dimethylformamide, and then 2-hydroxyethane-1-sulfonamide (80 mg, 0.64 mmol), N,N-dimethylglycine (66 mg, 0.64 mmol), copper(I) iodide (61 mg, 0.32 mmol), and potassium carbonate (177 mg, 1.13 mmol) were added. The mixture was heated to 130 °C and allowed to react for 1 h. LCMS showed that the starting materials were completely reacted. Water (5 mL) was added for dilution, and the mixture was extracted with ethyl acetate (10 mL × 3). The organic phase was concentrated to dryness, and the residue was separated and purified by a silica gel column (PE:EA (V/V) = 1:1) to give N-(4-(5-(6-methyl-2-(4-(trifluoromethyl)piperidin-1-yl)pyrimidin-4-yl)-1,3,4-oxadiazol-2-yl)-3-(6-azaspiro[2.5]octan-6-yl)phenyl)-2-hydroxyethane-1-sulfonamide (120 mg, yield: 60.3%).

¹HNMR (400 MHz, DMSO-d6) δ7.88 (d, 1H), 7.28 (s, 1H), 7.03 (d, 1H), 6.94-6.92 (dd, 1H), 4.92 (d, 2H), 3.74 (t, 2H), 3.34 (t, 2H), 2.98 (t, 2H), 2.89 (t, 4H), 2.70-2.67 (m, 1H), 2.40 (s, 3H), 1.90 (d, 2H), 1.50-1.36 (m, 6H), 0.27 (s, 4H).

LC-MS, M/Z (ESI): 622.2 [M+H]⁺.

### Example 24: Preparation of Target Compound I-97

### N (4-(5-(6-Methyl-2-(3-(trifluoromethyl)piperidin-1-yl)pyrimidin-4-yl)-1,3,4-oxadiazol-2-yl)-3-(6-azaspiro[2.5]octan-6-yl)phenyl)-2-hydroxyethane-1-sulfonamide

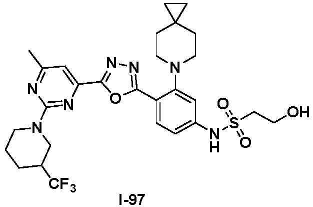

The synthetic route of the target compound **I-97** was as follows:

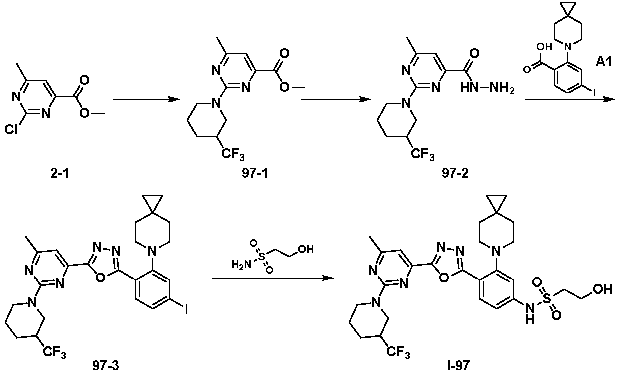

### Step 1: Synthesis of methyl 6-methyl-2-(3-(trifluoromethyl)piperidin-1-yl)pyrimidine-4-carboxylate

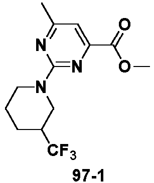

To a solution of methyl 2-chloro-6-methylpyrimidine-4-carboxylate (500 mg, 2.7 mmol) in *N,N*-dimethylformamide (5 mL) were added 3-(trifluoromethyl)piperidine (827 mg, 5.4 mmol) and potassium carbonate (1.87 g, 13.5 mmol), and then the mixture was stirred at 100 °C for 2 h. Water (100 mL) was added to the reaction liquid, and the mixture was extracted with ethyl acetate (30 mL × 3). The organic phase was washed with saturated brine (20 mL × 2), dried over anhydrous sodium sulfate, filtered, and concentrated to give a crude product. The crude product was purified by column chromatography (petroleum ether/ethyl acetate (V/V) = 1/0-1/1) to give methyl 6-methyl-2-(3-(trifluoromethyl)piperidin-1-yl)pyrimidine-4-carboxylate (700 mg, yield: 86%).

LC-MS, M/Z (ESI): 304.4 [M+H]⁺.

### Step 2: Synthesis of 6-methyl-2-(3-(trifluoromethyl)piperidin-1-yl)pyrimidine-4-carbohydrazide

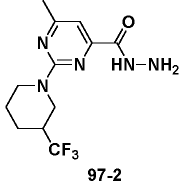

To a solution of methyl 6-methyl-2-(3-(trifluoromethyl)piperidin-1-yl)pyrimidine-4-carboxylate (700 mg, 2.3 mmol) in methanol (3.5 mL) was added dropwise and slowly 80% hydrazine hydrate (3.5 mL), and then the mixture was stirred at 60 °C for 1 h. The reaction liquid was added dropwise and slowly to water (100 mL), and the mixture was extracted with ethyl acetate (30 mL × 3). The organic phase was washed with saturated brine (20 mL × 2), dried over anhydrous sodium sulfate, filtered, and concentrated to give 6-methyl-2-(3-(trifluoromethyl)piperidin-1-yl)pyrimidine-4-carbohydrazide (680 mg, yield: 97%).

LC-MS, M/Z (ESI): 304.4 [M+H]⁺.

### Step 3: Synthesis of 2-(4-iodo-2-(6-azaspiro[2.5]octan-6-yl)phenyl)-5-(6-methyl-2-(3-(trifluoromethyl)piperidin-1-yl)pyrimidin-4-yl)-1,3,4-oxadiazole

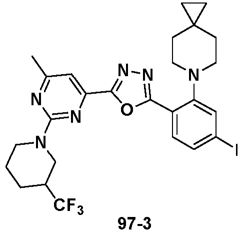

To 6-methyl-2-(3-(trifluoromethyl)piperidin-1-yl)pyrimidine-4-carbohydrazide (680 mg, 2.2 mmol) and 4-iodo-2-(6-azaspiro[2.5]octan-6-yl)benzoic acid (1.0 g, 2.9 mmol) was added dropwise and slowly phosphorus oxychloride (6.8 mL), and then the mixture was stirred at 110 °C for 18 h. The reaction liquid was added dropwise and slowly to ice water (100 mL), and the mixture was extracted with ethyl acetate (30 mL × 3). The organic phase was washed with saturated brine (30 mL × 2), dried over anhydrous sodium sulfate, filtered, and concentrated to give a crude product. The crude product was purified by column chromatography (petroleum ether/ethyl acetate (V/V) = 1/0-1/1) to give 2-(4-iodo-2-(6-azaspiro[2.5]octan-6-yl)phenyl)-5-(6-methyl-2-(3-(trifluoromethyl)piperidin-1-yl)pyrimidin-4-yl)-1,3,4-oxadiazole (700 mg, yield: 50%).

LC-MS, M/Z (ESI): 625.3 [M+H]⁺.

### Step 4: Synthesis of N-(4-(5-(6-methyl-2-(3-(trifluoromethyl)piperidin-1-yl)pyrimidin-4-yl)-1,3,4-oxadiazol-2-yl)-3-(6-azaspiro[2.5]octan-6-yl)phenyl)-2-hydroxyethane-1-sulfonamide

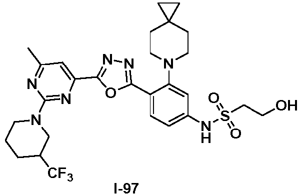

To a solution of 2-(4-iodo-2-(6-azaspiro[2.5]octan-6-yl)phenyl)-5-(6-methyl-2-(3-(trifluoromethyl)piperidin-1-yl)pyrimidin-4-yl)-1,3,4-oxadiazole (700 mg, 1.1 mmol) in N,N-dimethylformamide (7 mL) were added 2-hydroxyethanesulfonamide (275 mg, 2.2 mmol), *N,N-dimethylglycine* (23 mg, 0.22 mmol), copper(I) iodide (21 mg, 0.11 mmol), and potassium carbonate (456 mg, 3.3 mmol), and the mixture was purged three times with nitrogen and then stirred at 120 °C for 2 h. The reaction liquid was poured into water (50 mL), and the mixture was extracted with ethyl acetate (20 mL × 3). The organic phase was washed with saturated brine (20 mL × 2), dried over anhydrous sodium sulfate, filtered, and concentrated to give a crude product. The crude product was purified by preparative reverse phase chromatography (column: Phenomenex Synergi C18 100 × 25 mm × 4 µm; solvents: A = water + 0.1 vol% formic acid (99%), B = acetonitrile; gradient: 5%-95%, 7 min) to give N-(4-(5-(6-methyl-2-(3-(trifluoromethyl)piperidin-1-yl)pyrimidin-4-yl)-1,3,4-oxadiazol-2-yl)-3-(6-azaspiro[2.5]octan-6-yl)phenyl)-2-hydroxyethane-1-sulfonamide (402.8 mg, yield: 57.8%).

¹H NMR (400 MHz, DMSO-d6) δ 7.89 (d, 1H), 7.32 (s, 1H), 7.06 (d, 1H), 6.96 (dd, 1H), 4.91 (d, 1H), 4.75 (d, 1H), 3.76 (t, 2H), 3.36 (t, 2H), 3.05-2.91 (m, 7H), 2.54 (d, 1H), 2.43 (s, 3H), 2.02 (d, 1H), 1.82 (d, 1H), 1.68-1.45 (m, 6H), 0.30 (s, 4H).

LC-MS, M/Z (ESI): 622.4[M+H]⁺.

### Example 25: Preparation of Target Compound I-98

### N-(4-(5-(6-methyl-2-(3-(trifluoromethyl)pyrrolidin-1-yl)pyrimidin-4-yl)-1,3,4-oxadiazol-2-yl)-3-(6-azaspiro[2.5]octan-6-yl)phenyl)-2-hydroxyethane-sulfonamide

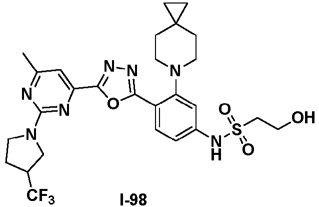

The synthetic route of the target compound **1-98** was as follows:

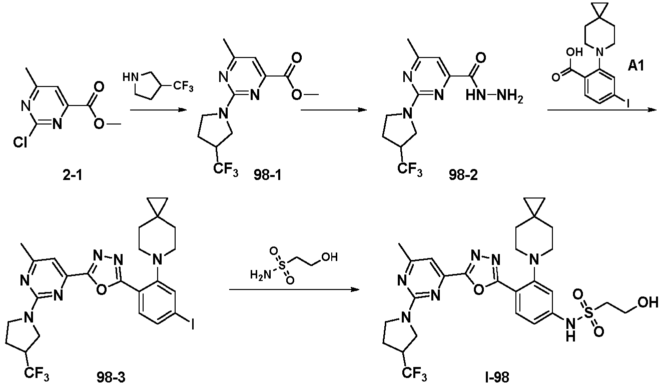

### Step 1: Synthesis of methyl 6-methyl-2-(3-(trifluoromethyl)pyrrolidin-1-yl)pyrimidine-4-carboxylate

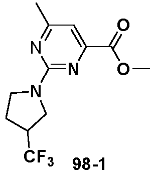

To a solution of methyl 2-chloro-6-methylpyrimidine-4-carboxylate (500 mg, 2.7 mmol) in *N,N*-dimethylformamide (5 mL) were added 3-(trifluoromethyl)pyrrolidine (751 mg, 5.4 mmol) and potassium carbonate (1.87 g, 13.5 mmol), and then the mixture was stirred at 100 °C for 2 h. Water (100 mL) was added to the reaction liquid, and the mixture was extracted with ethyl acetate (30 mL × 3). The organic phase was washed with saturated brine (20 mL × 2), dried over anhydrous sodium sulfate, filtered, and concentrated to give a crude product. The crude product was purified by column chromatography (petroleum ether/ethyl acetate (V/V) = 1/0-1/1) to give methyl 6-methyl-2-(3-(trifluoromethyl)pyrrolidin-1-yl)pyrimidine-4-carboxylate (500 mg, yield: 64.5%).

LC-MS, M/Z (ESI): 290.4 [M+H]⁺.

### Step 2: Synthesis of 6-methyl-2-(3-(trifluoromethyl)pyrrolidin-1-yl)pyrimidine-4-carbohydrazide

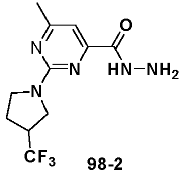

To a solution of methyl 6-methyl-2-(3-(trifluoromethyl)pyrrolidin-1-yl)pyrimidine-4-carboxylate (500 mg, 1.7 mmol) in methanol (2.5 mL) was added dropwise and slowly 80% hydrazine hydrate (2.5 mL), and then the mixture was stirred at 60 °C for 1 h. The reaction liquid was added dropwise and slowly to water (100 mL), and the mixture was extracted with ethyl acetate (30 mL × 3). The organic phase was washed with saturated brine (20 mL × 2), dried over anhydrous sodium sulfate, filtered, and concentrated to give 6-methyl-2-(3-(trifluoromethyl)pyrrolidin-1-yl)pyrimidine-4-carbohydrazide (450 mg, yield: 90%).

LC-MS, M/Z (ESI): 290.4 [M+H]⁺.

### Step 3: Synthesis of 2-(4-iodo-2-(6-azaspiro[2.5]octan-6-yl)phenyl)-5-(6-methyl-2-(3-(trifluoromethyl)pyrrolidin-1-yl)pyrimidin-4-yl)-1,3,4-oxadiazole

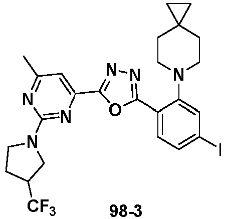

To 6-methyl-2-(3-(trifluoromethyl)pyrrolidin-1-yl)pyrimidine-4-carbohydrazide (450 mg, 1.6 mmol) and 4-iodo-2-(6-azaspiro[2.5]octan-6-yl)benzoic acid (750 mg, 2.1 mmol) was added dropwise and slowly phosphorus oxychloride (4.5 mL), and then the mixture was stirred at 110 °C for 18 h. The reaction liquid was added dropwise and slowly to ice water (100 mL), and the mixture was extracted with ethyl acetate (30 mL × 3). The organic phase was washed with saturated brine (30 mL × 2), dried over anhydrous sodium sulfate, filtered, and concentrated to give a crude product. The crude product was purified by column chromatography (petroleum ether/ethyl acetate (V/V) = 1/0-1/1) to give 2-(4-iodo-2-(6-azaspiro[2.5]octan-6-yl)phenyl)-5-(6-methyl-2-(3-(trifluoromethyl)pyrrolidin-1-yl)pyrimidin-4-yl)-1,3,4-oxadiazole (300 mg, yield: 31.6%).

LC-MS, M/Z (ESI): 611.3 [M+H]⁺.

### Step 4: Synthesis of N-(4-(5-(6-methyl-2-(3-(trifluoromethyl)pyrrolidin-1-yl)pyrimidin-4-yl)-1,3,4-oxadiazol-2-yl)-3-(6-azaspiro[2.5]octan-6-yl)phenyl)-2-hydroxyethane-1-sulfonamide

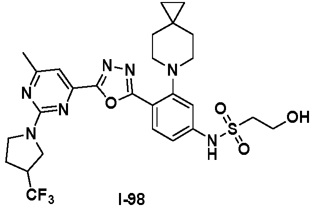

To a solution of 2-(4-iodo-2-(6-azaspiro[2.5]octan-6-yl)phenyl)-5-(6-methyl-2-(3-(trifluoromethyl)pyrrolidin-1-yl)pyrimidin-4-yl)-1,3,4-oxadiazole (300 mg, 0.5 mmol) in *N*,*N*-dimethylformamide (3 mL) were added 2-hydroxyethanesulfonamide (125 mg, 1 mmol), *N*,*N*-dimethylglycine (10 mg, 0.1 mmol), copper(I) iodide (10 mg, 0.05 mmol), and potassium carbonate (207 mg, 1.5 mmol), and the mixture was purged three times with nitrogen and then stirred at 120 °C for 2 h. The reaction liquid was poured into water (50 mL), and the mixture was extracted with ethyl acetate (20 mL × 3). The organic phase was washed with saturated brine (20 mL × 2), dried over anhydrous sodium sulfate, filtered, and concentrated to give a crude product. The crude product was purified by preparative reverse phase chromatography (column: Phenomenex Synergi C18 100 × 25 mm × 4 µm; solvents: A = water + 0.1 vol% formic acid (99%), B = acetonitrile; gradient: 5%-95%, 7 min) to give *N*-(4-(5-(6-methyl-2-(3-(trifluoromethyl)pyrrolidin-1-yl)pyrimidin-4-yl)-1,3,4-oxadiazol-2-yl)-3-(6-azaspiro[2.5]octan-6-yl)phenyl)-2-hydroxyethane-1-sulfonamide (71.1 mg, yield: 20.4%).

¹H NMR (400 MHz, dmso) δ 7.93 (d, 1H), 7.34 (s, 1H), 7.05 (d, 1H), 6.95 (dd, 1H), 3.91 (s, 1H), 3.83-3.60 (m, 5H), 3.46-3.33 (m, 3H), 2.92 (t, 4H), 2.43 (s, 3H), 2.32 (dd, 1H), 2.13 (dd, 1H), 1.56 (s, 4H), 0.30 (s, 4H).

LC-MS, M/Z (ESI): 608.4 [M+H]⁺.

### Example 26: Preparation of Target Compound I-99

### N-(4-(5-(6-Methyl-2-(3,3,3-trifluoropropoxy)pyrimidin-4-yl)-1,3,4-thiadiazol-2-yl)-3-(6-azaspiro[2.5]octan-6-yl)phenyl)-2-hydroxyethane-1-sulfonamide

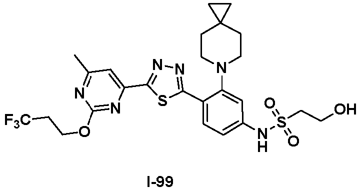

The synthetic route of the target compound 1-99 was as follows:

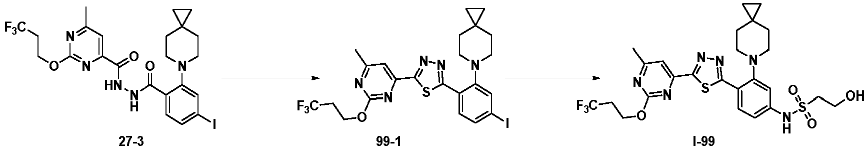

### Step 1: Synthesis of 2-(4-iodo-2-(6-azaspiro[2.5]octan-6-yl)phenyl)-5-(6-methyl-2-(3,3,3-trifluoropropoxy) pyrimidin-4-yl)-1,3,4-thiadiazole

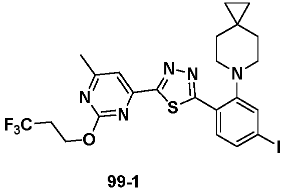

*N'*-(4-Iodo-2-(6-azaspiro[2.5]octan-6-yl)benzoyl)-6-methyl-2-(3,3,3-trifluoropropoxy)pyrimidine-4-carbohydrazide (0.4 g, 0.663 mmol) was added to 5 mL of tetrahydrofuran, and then phosphorus pentasulfide (0.737 g, 3.31 mmol) was added. The mixture was heated to 80 °C and allowed to react overnight. The reaction liquid was concentrated to dryness, and the residue was separated and purified by a silica gel column (PE:EA (V/V) = 3:1) to give 2-(4-iodo-2-(6-azaspiro[2.5]octan-6-yl)phenyl)-5-(6-methyl-2-(3,3,3-trifluoropropoxy)pyrimidin-4-yl)-1,3,4-thiadiazole (200 mg, yield: 50.1%).

LC-MS, M/Z (ESI): 602.06 [M+H]⁺.

### Step 2: Synthesis of N-(4-(5-(6-methyl-2-(3,3,3-trifluoropropoxy)pyrimidin-4-yl)-1,3,4-thiadiazol-2-yl)-3-(6-azaspiro[2.5]octan-6-yl)phenyl)-2-hydroxyethane-1-sulfonamide

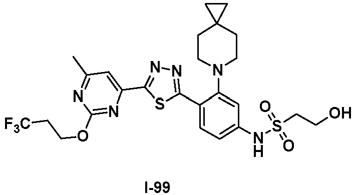

2-(4-Iodo-2-(6-azaspiro[2.5]octan-6-yl)phenyl)-5-(6-methyl-2-(3,3,3-trifluoropropoxy)pyrimidin-4-yl)-1,3,4-thiadiazole (200 mg, 0.33 mmol) was added to 5 mL of N,N-dimethylformamide, and then 2-hydroxyethane-1-sulfonamide (83 mg, 0.67 mmol), N,N-dimethylglycine (69 mg, 0.67 mmol), copper(I) iodide (63 mg, 0.33 mmol), and potassium carbonate (184 mg, 1.33 mmol) were added. The mixture was heated to 130 °C and allowed to react for 1 h. LCMS showed that the starting materials were completely reacted. Water (5 mL) was added for dilution, and the mixture was extracted with ethyl acetate (10 mL × 2). The organic phase was concentrated to dryness, and the residue was separated and purified by a silica gel column (PE:EA (V/V) = 1:1) to give *N*-(4-(5-(6-methyl-2-(3,3,3-trifluoropropoxy)pyrimidin-4-yl)-1,3,4-thiadiazol-2-yl)-3-(6-azaspiro[2.5]octan-6-yl)phenyl)-2-hydroxyethane-1-sulfonamide (125 mg, yield: 62.8%).

¹H NMR (400 MHz, DMSO-d6) δ8.23 (d, 1H), 7.81 (s, 1H), 7.25 (d, 1H), 7.07-7.10(dd, 1H), 4.60 (t, 2H),3.75 (t, 2H), 3.29 (t, 2H), 2.84-2.87 (m, 6H), 2.52 (s, 3H), 1.59(s, 4H), 0.36 (s, 4H).

LC-MS, M/Z (ESI): 599.16 [M+H]⁺.

### Example 27: Preparation of Target Compound I-100

### N-(4-(5-(6-Methyl-2-(3,3,5,5-tetrafluoropiperidin-1-yl)pyrimidin-4-yl)-1,3,4-oxadiazol-2-yl)-3-(6-azaspiro[2.5]octan-6-yl)phenyl)-2-hydroxyethane-1-sulfonamide

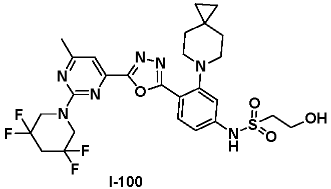

The synthetic route of the target compound **I-100** was as follows:

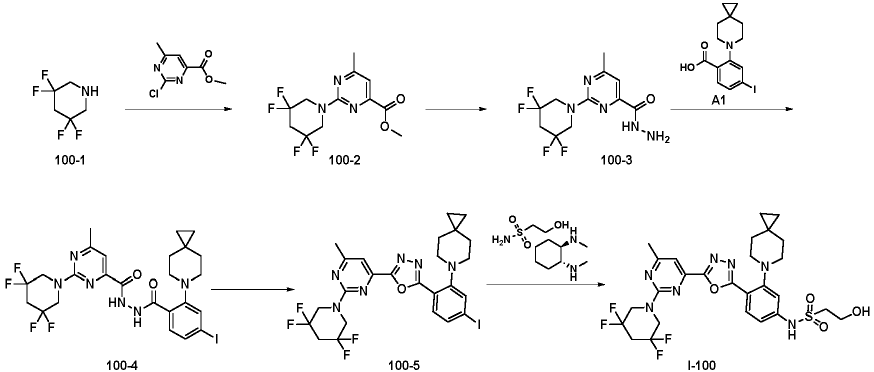

### Step 1: Synthesis of methyl 6-methyl-2-(3,3,5,5-tetrafluoropiperidin-1-yl)pyrimidine-4-carboxylate

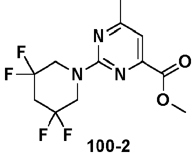

Methyl 2-chloro-6-methylpyrimidine-4-carboxylate (2 g, 10.7 mmol) was added to 20 mL of *N,N-*dimethylformamide, and then 3,3,5,5-tetrafluoropiperidine (2.3 g, 14.6 mmol) and potassium carbonate (7.41 g, 53.6 mmol) were added. The mixture was heated to 80 °C and allowed to react for 2 h. Water (30 mL) was added for dilution, and the mixture was extracted with ethyl acetate (50 mL × 2). The organic phase was concentrated to dryness, and the residue was separated and purified by a silica gel column (PE:EA (V/V) = 5:1) to give methyl 6-methyl-2-(3,3,5,5-tetrafluoropiperidin-1-yl)pyrimidine-4-carboxylate (200 mg, yield: 7%).

LC-MS, M/Z (ESI): 308.2 [M+H]⁺.

### Step 2: Synthesis of 6-methyl-2-(3,3,5,5-tetrafluoropiperidin-1-yl)pyrimidine-4-carbohydrazide

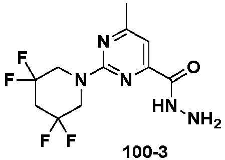

Methyl 6-methyl-2-(3,3,5,5-tetrafluoropiperidin-1-yl)pyrimidine-4-carboxylate (200 mg, 0.7 mmol) was added to 5 mL of ethanol, and then 2 mL of 80% hydrazine hydrate was added. The mixture was heated to 60 °C and allowed to react for 1 h, and then directly concentrated to dryness. The residue was lyophilized to give 6-methyl-2-(3,3,5,5-tetrafluoropiperidin-1-yl)pyrimidine-4-carbohydrazide (200 mg, yield: 100%).

LC-MS, M/Z (ESI): 308.2 [M+H]⁺.

### Step 3: Synthesis of N'-(4-iodo-2-(6-azaspiro[2.5]octan-6-yl)benzoyl)-6-methyl-2-(3,3,5,5-tetrafluoropiperidin-1-yl)pyrimidine-4-carbohydrazide

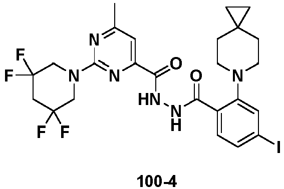

6-Methyl-2-(3,3,5,5-tetrafluoropiperidin-1-yl)pyrimidine-4-carbohydrazide (200 mg, 0.7 mmol) was added to 5 mL of *N,N*-dimethylformamide, and then 4-iodo-2-(6-azaspiro[2.5]octan-6-yl)benzoic acid (250 mg, 0.7 mmol), *N,N-*diisopropylethylamine (326 mg, 2.52 mmol), and *O*-(7-azabenzotriazol-1-yl)-*N,N,N,N*'-tetramethyluronium hexafluorophosphate (500 mg, 1.4 mmol) were added. The mixture was heated to 60 °C and allowed to react for 1 h. Water (20 mL) was added for dilution, and the mixture was extracted with ethyl acetate (100 mL × 2). The organic phase was concentrated to dryness, and the residue was separated and purified by a silica gel column (PE:EA (V/V) = 3:1) to give *N'*-(4-iodo-2-(6-azaspiro[2.5]octan-6-yl)benzoyl)-6-methyl-2-(3,3,5,5-tetrafluoropiperidin-1-yl)pyrimidine-4-carbohydrazide (120 mg, yield: 27%) as an off-white solid.

LC-MS, M/Z (ESI): 647.2 [M+H]⁺.

### Step 4: Synthesis of 2-(4-iodo-2-(6-azaspiro[2.5]octan-6-yl)phenyl)-5-(6-methyl-2-(3,3,5,5-tetrafluoropiperidin-1-yl)pyrimidin-4-yl)-1,3,4-oxadiazole

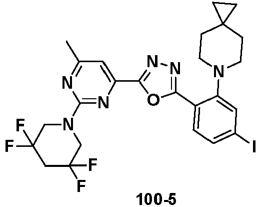

*N'*-(4-Iodo-2-(6-azaspiro[2.5]octan-6-yl)benzoyl)-6-methyl-2-(3,3,5,5-tetrafluoropiperidin-1-yl)pyrimidine-4-carbohydrazide (120 mg, 0.18 mmol) was added to 5 mL of dichloromethane, and then *p*-methylbenzenesulfonyl chloride (0.28 g, 1.5 mmol) and triethylamine (0.3 g, 2.96 mmol) were added. The mixture was allowed to react at room temperature overnight. Water (5 mL) was added for dilution, and the mixture was extracted with ethyl acetate (10 mL × 2). The organic phase was concentrated to dryness, and the residue was separated and purified by a silica gel column (PE:EA (V/V) = 3:1) to give 2-(4-iodo-2-(6-azaspiro[2.5]octan-6-yl)phenyl)-5-(6-methyl-2-(3,3,5,5-tetrafluoropiperidin-1-yl)pyrimidin-4-yl)-1,3,4-oxadiazole (45 mg, yield: 38%).

LC-MS, M/Z (ESI): 629.2 [M+H]⁺.

### Step 5: Synthesis of N-(4-(5-(6-methyl-2-(3,3,5,5-tetrafluoropiperidin-1-yl)pyrimidin-4-yl)-1,3,4-oxadiazol-2-yl)-3-(6-azaspiro[2.5]octan-6-yl)phenyl)-2-hydroxyethane-1-sulfonamide

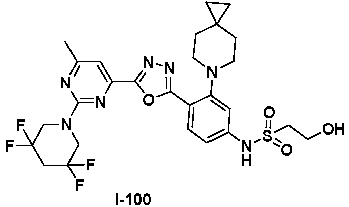

2-(4-Iodo-2-(6-azaspiro[2.5]octan-6-yl)phenyl)-5-(6-methyl-2-(3,3,5,5-tetrafluoropiperidin-1-yl)pyrimidin-4-yl)-1,3,4-oxadiazole (15 mg, 0.024 mmol) was added to 0.45 mL of *N*,*N*-dimethylformamide, and then 2-hydroxyethane-1-sulfonamide (15 mg, 0.12 mmol), trans-N,N-dimethylcyclohexane-1,2-diamine (4.3 mg, 0.03 mmol), copper(I) iodide (9 mg, 0.047 mmol), and potassium phosphate (50 mg, 0.24 mmol) were added. The mixture was heated to 120 °C and allowed to react for 3 h. Water (30 mL) was added for dilution, and the mixture was extracted with ethyl acetate (10 mL × 3). The organic phase was concentrated to dryness, and the residue was separated and purified by a reverse phase column (0.1% formic acid) to give *N*-(4-(5-(6-methyl-2-(3,3,5,5-tetrafluoropiperidin-1-yl)pyrimidin-4-yl)-1,3,4-oxadiazol-2-yl)-3-(6-azaspiro[2.5]octan-6-yl)phenyl)-2-hydroxyethane-1-sulfonamide (1 mg, yield: 6.7%).

¹H NMR (400 MHz, DMSO-d₆) δ 7.91 (d, 1H), 7.48 (s, 1H), 7.07 (d, 1H), 6.96 (dd, 1H), 4.42 (s, 4H), 3.76 (t, 2H), 2.95-2.92(m, 4H), 2.48(s, 3H), 1.99 (dt, 4H), 1.53 (s, 4H), 0.30 (s, 4H).

LC-MS, M/Z (ESI): 626.1 [M+H]⁺.

### Example 28: Preparation of Target Compound I-101

### N-(4-(5-(2-((3S,4R)-3,4-Difluoropyrrolidin-1-yl)-6-methylpyrimidin-4-yl)-1,3,4-oxadiazol-2-yl)-3-(6-azaspiro[2.5]octan-6-yl)phenyl)-2-hydroxyethane-1-sulfonamide

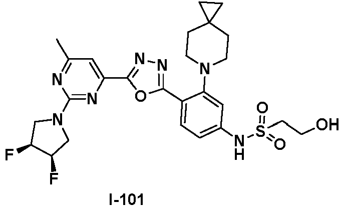

The synthetic route of the target compound **I-101** was as follows:

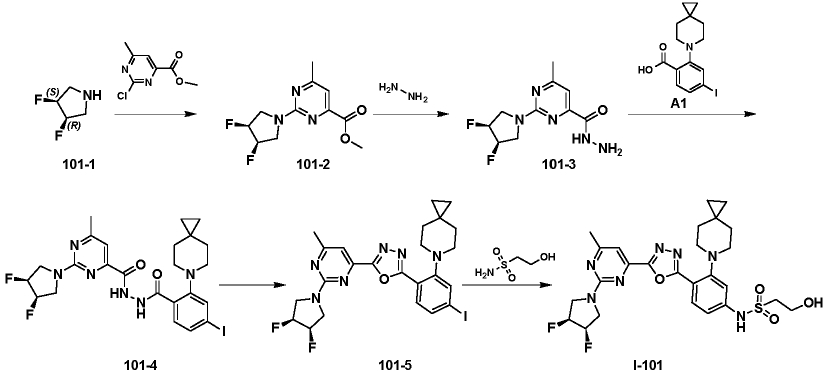

### Step 1: Synthesis of methyl 2-((3S,4R)-3,4-difluoropyrrolidin-1-yl)-6-methylpyrimidine-4-carboxylate

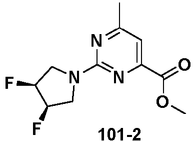

Methyl 2-chloro-6-methylpyrimidine-4-carboxylate (2 g, 10.7 mmol) was added to 20 mL of *N,N-*dimethylformamide, and then (3*S*,4*R*)-3,4-difluoropyrrolidine (2.28 g, 21.4 mmol) and potassium carbonate (7.41 g, 53.6 mmol) were added. The mixture was heated to 80 °C and allowed to react for 2 h. Water (30 mL) was added for dilution, and the mixture was extracted with ethyl acetate (50 mL × 2). The organic phase was concentrated to dryness, and the residue was separated and purified by a silica gel column (PE:EA (V/V) = 5:1) to give methyl 2-((3S,4R)-3,4-difluoropyrrolidin-1-yl)-6-methylpyrimidine-4-carboxylate (1.8 g, yield: 66%).

LC-MS, M/Z (ESI): 258.2 [M+H]⁺.

### Step 2: Synthesis of 2-((3S,4R)-3,4-difluoropyrrolidin-1-yl)-6-methylpyrimidine-4-carbohydrazide

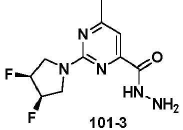

Methyl 2-((3S,4R)-3,4-difluoropyrrolidin-1-yl)-6-methylpyrimidine-4-carboxylate (1.8 g, 7.0 mmol) was added to 20 mL of ethanol, and then 4 mL of 80% hydrazine hydrate was added. The mixture was heated to 60 °C and allowed to react for 1 h. The reaction liquid was then directly concentrated to dryness, and the residue was lyophilized to give a crude product of 2-((3*S*,4*R*)-3,4-difluoropyrrolidin-1-yl)-6-methylpyrimidine-4-carbohydrazide (1.8 g, yield: 100%).

LC-MS, M/Z (ESI): 258.2 [M+H]⁺.

### Step 3: Synthesis of 2-((3S,4R)-3,4-difluoropyrrolidin-1-yl)-N'-(4-iodo-2-(6-azaspiro[2.5]octan-6-yl)benzoyl)-6-methylpyrimidine-4-carbohydrazide

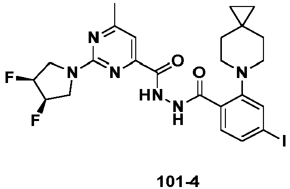

2-((3*S*,4*R*)-3,4-Difluoropyrrolidin-1-yl)-6-methylpyrimidine-4-carbohydrazide (1.7 g, 6.0 mmol) was added to 15 mL of *N*,*N*-dimethylformamide, and then 4-iodo-2-(6-azaspiro[2.5]octan-6-yl)benzoic acid (1.5 g, 4.2 mmol), *N,N-*diisopropylethylamine (3.26 g, 25.2 mmol), and *O*-(7-azabenzotriazol-1-yl)-*N,N,N,N*'-tetramethyluronium hexafluorophosphate (4.79 g, 12.6 mmol) were added. The mixture was heated to 60 °C and allowed to react for 1 h. Water (20 mL) was added for dilution, and the mixture was extracted with ethyl acetate (100 mL × 2). The organic phase was concentrated to dryness, and the residue was separated and purified by a silica gel column (PE:EA (V/V) = 3:1) to give 2-((3*S*,4*R*)-3,4-difluoropyrrolidin-1-yl)-N-(4-iodo-2-(6-azaspiro[2.5]octan-6-yl)benzoyl)-6-methylpyrimidine-4-carbohydrazide (1.2 g, yield: 55%).

LC-MS, M/Z (ESI): 597.2 [M+H]⁺.

### Step 4: Synthesis of 2-(2-((3S,4R)-3,4-difluoropyrrolidin-1-yl)-6-methylpyrimidin-4-yl)-5-(4-iodo-2-(6-azaspiro [2.5] octan-6-yl)phenyl)-1,3,4-oxadiazole

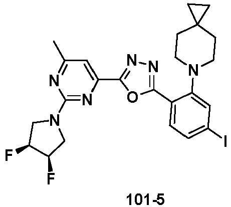

2-((3*S*,4*R*)-3,4-Difluoropyrrolidin-1-yl)-*N'*-(4-iodo-2-(6-azaspiro[2.5]octan-6-yl)benzoyl)-6-methylpyrimidine-4-carbohydrazide (0.3 g, 0.5 mmol) was added to 5 mL of dichloromethane, and thenp-methylbenzenesulfonyl chloride (0.28 g, 1.5 mmol) and triethylamine (0.3 g, 2.96 mmol) were added. The mixture was allowed to react at room temperature overnight. Water (5 mL) was added for dilution, and the mixture was extracted with ethyl acetate (10 mL × 2). The organic phase was concentrated to dryness, and the residue was separated and purified by a silica gel column (PE:EA (V/V) = 3: 1) to give 2-(2-((3S,4R)-3,4-difluoropyrrolidin-1-yl)-6-methylpyrimidin-4-yl)-5-(4-iodo-2-(6-azaspiro[2.5]octan-6-yl)phenyl)-1,3,4-oxadiazole (110 mg, yield: 38%).

LC-MS, M/Z (ESI): 579.2 [M+H]⁺.

### Step 5: Synthesis of N-(4-(5-(2-((3S,4R)-3,4-difluoropyrrolidin-1-yl)-6-methylpyrimidin-4-yl)-1,3,4-oxadiazol-2-yl)-3-(6-azaspiro [2.5] octan-6-yl)phenyl)-2-hydroxyethane-1-sulfonamide

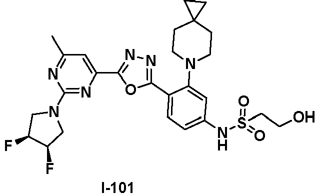

2-(2-((3*S*,4*R*)-3,4-Difluoropyrrolidin-1-yl)-6-methylpyrimidin-4-yl)-5-(4-iodo-2-(6-azaspiro[2.5]octan-6-yl)phenyl)-1,3,4-oxadiazole (0.11 g, 0.18 mmol) was added to 5 mL of N,N-dimethylformamide, and then 2-hydroxyethane-1-sulfonamide (47 mg, 0.37 mmol), *N*,*N*-dimethylglycine (38 mg, 0.37 mmol), copper(I) iodide (35 mg, 0.18 mmol), and potassium carbonate (103 mg, 0.75 mmol) were added. The mixture was heated to 130 °C and allowed to react for 1 h. Water (5 mL) was added for dilution, and the mixture was extracted with ethyl acetate (10 mL × 2). The organic phase was concentrated to dryness, and the residue was separated and purified by a silica gel column (PE:EA (V/V) = 1:1) to give N-(4-(5-(2-((3S,4R)-3,4-difluoropyrrolidin-1-yl)-6-methylpyrimidin-4-yl)-1,3,4-oxadiazol-2-yl)-3-(6-azaspiro[2.5]octan-6-yl)phenyl)-2-hydroxyethane-1-sulfonamide (50 mg, yield: 43%).

¹HNMR (400 MHz, DMSO-d₆) δ10.17 (s, 1H), 7.95 (d, 1H), 7.37 (s, 1H), 7.05 (d, 1H), 6.96-6.94 (dd, 1H), 5.53-5.37 (m, 2H), 4.01-3.73 (m, 7H), 3.49-3.33 (m, 2H), 2.91-2.90 (m, 4H), 2.49-2.43 (m, 3H), 1.58 (s, 4H), 0.30 (s, 4H). LC-MS, M/Z (ESI): 576.2 [M+H]⁺.

### Example 29: Preparation of Target Compound I-102

### N-(4-(5-(2-(3-Fluoropiperidin-1-yl)-6-methylpyrimidin-4-yl)-1,3,4-oxadiazol-2-yl)-3-(6-azaspiro[2.5]octan-6-yl)phenyl)-2-hydroxyethane-1-sulfonamide

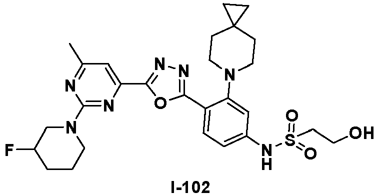

The synthetic route of the target compound **1-102** was as follows:

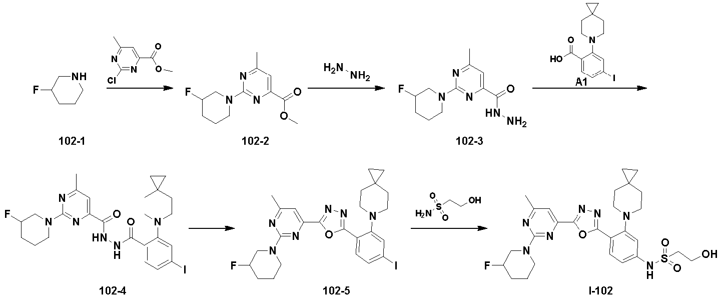

### Step 1: Synthesis of methyl 2-(3-fluoropiperidin-1-yl)-6-methylpyrimidine-4-carboxylate

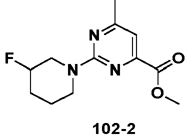

Methyl 2-chloro-6-methylpyrimidine-4-carboxylate (2 g, 10.7 mmol) was added to 20 mL of *N,N-*dimethylformamide, and then 3-fluoropiperidine (2.0 g, 19.4 mmol) and potassium carbonate (7.41 g, 53.6 mmol) were added. The mixture was heated to 80 °C and allowed to react for 2 h. Water (30 mL) was added for dilution, and the mixture was extracted with ethyl acetate (50 mL × 2). The organic phase was concentrated to dryness, and the residue was separated and purified by a silica gel column (PE:EA (V/V) = 5:1) to give methyl 2-(3-fluoropiperidin-1-yl)-6-methylpyrimidine-4-carboxylate (1.8 g, yield: 70%).

LC-MS, M/Z (ESI): 254.2 [M+H]⁺.

### Step 2: Synthesis of 2-(3-fluoropiperidin-1-yl)-6-methylpyrimidine-4-carbohydrazide

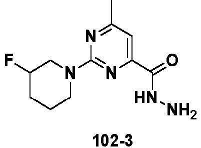

Methyl 2-(3-fluoropiperidin-1-yl)-6-methylpyrimidine-4-carboxylate (180 mg, 0.7 mmol) was added to 5 mL of ethanol, and then 2 mL of 80% hydrazine hydrate was added. The mixture was heated to 60 °C and allowed to react for 1 h. The reaction liquid was then directly concentrated to dryness, and the residue was lyophilized to give 2-(3-fluoropiperidin-1-yl)-6-methylpyrimidine-4-carbohydrazide (180 mg, yield: 100%).

LC-MS, M/Z (ESI): 254.2 [M+H]⁺.

### Step 3: Synthesis of 2-(3-fluoropiperidin-1-yl)-N'-(4-iodo-2-(6-azaspiro[2.5]octan-6-yl)benzoyl)-6-methylpyrimidine-4-carbohydrazide

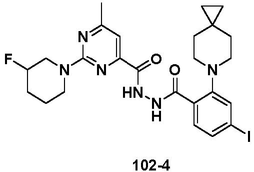

2-(3-Fluoropiperidin-1-yl)-6-methylpyrimidine-4-carbohydrazide (170 mg, 0.6 mmol) was added to 2 mL of *N,N-*dimethylformamide, and then 4-iodo-2-(6-azaspiro[2.5]octan-6-yl)benzoic acid (150 mg, 0.42 mmol), *N,N-*diisopropylethylamine (326 mg, 2.52 mmol), and *O*-(7-azabenzotriazol-1-yl)-*N,N,N,N*'-tetramethyluronium hexafluorophosphate (479 mg, 1.26 mmol) were added. The mixture was heated to 60 °C and allowed to react for 1 h. Water (20 mL) was added for dilution, and the mixture was extracted with ethyl acetate (100 mL × 2). The organic phase was concentrated to dryness, and the residue was separated and purified by a silica gel column (PE:EA (V/V) = 3:1) to give 2-(3-fluoropiperidin-1-yl)-*N'*-(4-iodo-2-(6-azaspiro[2.5]octan-6-yl)benzoyl)-6-methylpyrimidine-4-carbohydrazide (120 mg, yield: 50%).

LC-MS, M/Z (ESI): 593.2 [M+H]⁺.

### Step 4: Synthesis of 2-(2-(3-fluoropiperidin-1-yl)-6-methylpyrimidin-4-yl)-5-(4-iodo-2-(6-azaspiro[2.5]octan-6-yl)phenyl)-1,3,4-oxadiazole

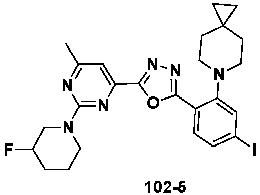

2-(3-Fluoropiperidin-1-yl)*-N*'-(4-iodo-2-(6-azaspiro[2.5]octan-6-yl)benzoyl)-6-methylpyrimidine-4-carbohydrazide (0.12 g, 0.22 mmol) was added to 5 mL of dichloromethane, and then *p*-methylbenzenesulfonyl chloride (0.28 g, 1.5 mmol) and triethylamine (0.3 g, 2.96 mmol) were added. The mixture was allowed to react at room temperature overnight. Water (5 mL) was added for dilution, and the mixture was extracted with ethyl acetate (10 mL × 2). The organic phase was concentrated to dryness, and the residue was separated and purified by a silica gel column (PE:EA (V/V) = 3:1) to give 2-(2-(3-fluoropiperidin-1-yl)-6-methylpyrimidin-4-yl)-5-(4-iodo-2-(6-azaspiro[2.5]octan-6-yl)phenyl)-1,3,4-oxadiazole (110 mg, yield: 80%).

LC-MS, M/Z (ESI): 575.2 [M+H]⁺.

### Step 5: Synthesis of N-(4-(5-(2-(3-fluoropiperidin-1-yl)-6-methylpyrimidin-4-yl)-1,3,4-oxadiazol-2-yl)-3-(6-azaspiro[2.5]octan-6-yl)phenyl)-2-hydroxyethane-1-sulfonamide

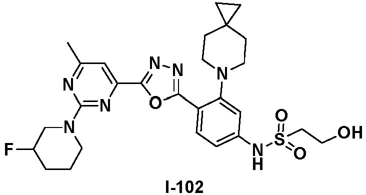

2-(2-(3-Fluoropiperidin-1-yl)-6-methylpyrimidin-4-yl)-5-(4-iodo-2-(6-azaspiro[2.5]octan-6-yl)phenyl)-1,3,4-oxadiazole (0.11 g, 0.17 mmol) was added to 5 mL of *N,N*-dimethylformamide, and then 2-hydroxyethane-1-sulfonamide (47 mg, 0.37 mmol), *N*,*N*-dimethylglycine (38 mg, 0.37 mmol), copper(I) iodide (35 mg, 0.18 mmol), and potassium carbonate (103 mg, 0.75 mmol) were added. The mixture was heated to 130 °C and allowed to react for 1 h. Water (5 mL) was added for dilution, and the mixture was extracted with ethyl acetate (10 mL × 2). The organic phase was concentrated to dryness, and the residue was separated and purified by a silica gel column (PE:EA (V/V) = 1:1) to give *N*-(4-(5-(2-(3-fluoropiperidin-1-yl)-6-methylpyrimidin-4-yl)-1,3,4-oxadiazol-2-yl)-3-(6-azaspiro[2.5]octan-6-yl)phenyl)-2-hydroxyethane-1-sulfonamide (50 mg, yield: 40%).

¹HNMR (400 MHz, DMSO-d₆) δ10.17 (s, 1H), 7.95 (d, 1H), 7.37 (s, 1H), 7.05 (d, 1H), 6.96-6.94 (dd, 1H), 5.87 (m, 1H), 4.35 (m, 1H), 4.20 (m, 1H), 3.78-3.75 (m, 4H), 3.38-3.36 (m, 2H), 2.93-2.91 (m, 4H), 2.42 (s, 3H), 1.95-1.88 (m, 4H), 1.78-1.53 (m, 5H), 0.30 (s, 4H).

LC-MS, M/Z (ESI): 572.2 [M+H]⁺.

### Example 30: Preparation of Target Compound I-103

### N-(4-(5-(4-Methyl-6-(3-(trifluoromethyl)piperidin-1-yl)pyridin-2-yl)-1,3,4-oxadiazol-2-yl)-3-(6-azaspiro[2.5]octan-6-yl)phenyl)-2-hydroxyethane-1-sulfonamide

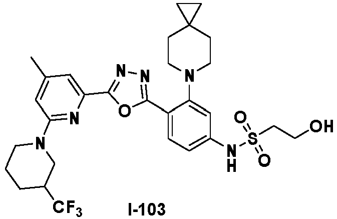

The synthetic route of the target compound **I-103** was as follows:

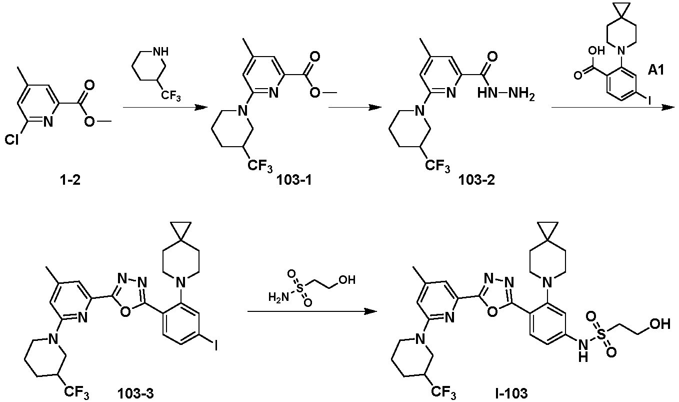

### Step 1: Synthesis of methyl 4-methyl-6-(3-(trifluoromethyl)piperidin-1-yl)picolinate

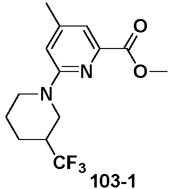

To a solution of methyl 6-chloro-4-methylpicolinate (1 g, 5.4 mmol) in 1,4-dioxane (10 mL) were added 3-(trifluoromethyl)piperidine (1.65 g, 10.8 mmol), cesium carbonate (8.8 g, 27 mmol), 4,5-bis-diphenylphosphino-9,9-dimethylxanthene (637 mg, 1.1 mmol), and tris(dibenzylideneacetone)dipalladium (288 mg, 0.5 mmol), and then the mixture was allowed to react at 90 °C for 18 h. The reaction liquid was poured into water (50 mL), and the mixture was extracted with ethyl acetate (20 mL × 3). The organic phase was washed with saturated brine (20 mL × 2), dried over anhydrous sodium sulfate, filtered, and concentrated to give a crude product. The crude product was purified by column chromatography (petroleum ether/ethyl acetate (V/V) = 1/0-1/1) to give methyl 4-methyl-6-(3-(trifluoromethyl)piperidin-1-yl)picolinate (130 mg, yield: 8%).

LC-MS, M/Z (ESI): 303.3 [M+H]⁺.

### Step 2: Synthesis of 4-methyl-6-(3-(trifluoromethyl)piperidin-1-yl)picolinohydrazide

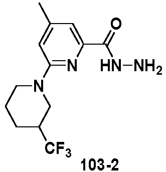

To a solution of methyl 4-methyl-6-(3-(trifluoromethyl)piperidin-1-yl)picolinate (130 mg, 0.4 mmol) in methanol (1.3 mL) was added dropwise and slowly 80% hydrazine hydrate (1.3 mL), and then the mixture was stirred at 60 °C for 3 h. The reaction liquid was poured into water (30 mL), and the mixture was extracted with ethyl acetate (10 mL × 3). The organic phase was washed with saturated brine (10 mL × 2), dried over anhydrous sodium sulfate, filtered, and concentrated to give 4-methyl-6-(3-(trifluoromethyl)piperidin-1-yl)picolinohydrazide (0.1 g, yield: 76.9%).

LC-MS, M/Z (ESI): 303.3 [M+H]⁺.

### Step 3: Synthesis of 2-(4-iodo-2-(6-azaspiro[2.5]octan-6-yl)phenyl)-5-(4-methyl-6-(3-(trifluoromethyl)piperidin-1-yl)pyridin-2-yl)-1,3,4-oxadiazole

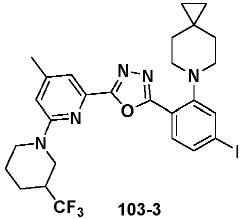

To 4-methyl-6-(3-(trifluoromethyl)piperidin-1-yl)picolinohydrazide (0.1 g, 0.3 mmol) and 4-iodo-2-(6-azaspiro[2.5]octan-6-yl)benzoic acid (0.11 g, 0.3 mmol) was added dropwise and slowly phosphorus oxychloride (1 mL), and then the mixture was stirred at 110 °C for 18 h. The reaction liquid was added dropwise and slowly to ice water (50 mL), and the mixture was extracted with ethyl acetate (30 mL × 3). The organic phase was washed with saturated brine (20 mL × 2), dried over anhydrous sodium sulfate, filtered, and concentrated to give a crude product. The crude product was purified by column chromatography (petroleum ether/ethyl acetate (V/V) = 1/0-1/1) to give 2-(4-iodo-2-(6-azaspiro[2.5]octan-6-yl)phenyl)-5-(4-methyl-6-(3-(trifluoromethyl)piperidin-1-yl)pyridin-2-yl)-1,3,4-oxadiazole (100 mg, yield: 48.5%).

LC-MS, M/Z (ESI): 624.3 [M+H]⁺.

### Step 4: Synthesis of N-(4-(5-(4-methyl-6-(3-(trifluoromethyl)piperidin-1-yl)pyridin-2-yl)-1,3,4-oxadiazol-2-yl)-3-(6-azaspiro[2.5]octan-6-yl)phenyl)-2-hydroxyethane-1-sulfonamide

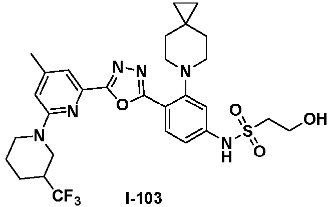

To a solution of 2-(4-iodo-2-(6-azaspiro[2.5]octan-6-yl)phenyl)-5-(4-methyl-6-(3-(trifluoromethyl)piperidin-1-yl)pyridin-2-yl)-1,3,4-oxadiazole (100 mg, 0.16 mmol) in N,N-dimethylformamide (1 mL) were added 2-hydroxyethanesulfonamide (40 mg, 0.32 mmol), *N*,*N*-dimethylglycine (3 mg, 0.032 mmol), copper(I) iodide (3 mg, 0.016 mmol), and potassium carbonate (66 mg, 0.48 mmol), and the mixture was purged three times with nitrogen and then stirred at 120 °C for 2 h. The reaction liquid was poured into water (30 mL), and the mixture was extracted with ethyl acetate (10 mL × 3). The organic phase was washed with saturated brine (10 mL × 2), dried over anhydrous sodium sulfate, filtered, and concentrated to give a crude product. The crude product was purified by preparative reverse phase chromatography (column: Phenomenex Synergi C18 100 × 25 mm × 4 µm; solvents: A = water + 0.1 vol% formic acid (99%), B = acetonitrile; gradient: 5%-95%, 7 min) to give *N*-(4-(5-(4-methyl-6-(3-(trifluoromethyl)piperidin-1-yl)pyridin-2-yl)-1,3,4-oxadiazol-2-yl)-3-(6-azaspiro[2.5]octan-6-yl)phenyl)-2-hydroxyethane-1-sulfonamide (18.3 mg, yield: 15%).

¹H NMR (400 MHz, DMSO-d₆) δ 7.87 (d, 1H), 7.37 (s, 1H), 7.05 (d, 1H), 6.94 (dd, 1H), 6.60 (s, 1H), 4.95 (s, 1H), 3.85 (dd, 1H), 3.76 (t, 2H), 3.69-3.50 (m, 3H), 3.46- 3.36 (m, 5H), 2.91 (t, 4H), 2.39-2.25 (m, 4H), 2.13 (dd, 1H), 1.53 (s, 4H), 0.28 (s, 4H).

LC-MS, M/Z (ESI): 621.4 [M+H]⁺.

### Example 31: Preparation of Target Compound 1-104

### N-(4-(5-(4-Methyl-6-(3-(trifluoromethyl)pyrrolidin-1-yl)pyridin-2-yl)-1,3,4-oxadiazol-2-yl)-3-(6-azaspiro[2.5]octan-6-yl)phenyl)-2-hydroxyethane-1-sulfonamide

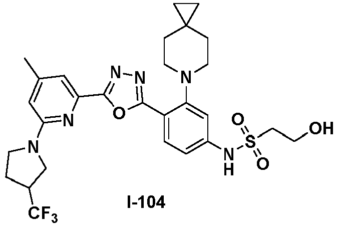

The synthetic route of the target compound **I-104** was as follows:

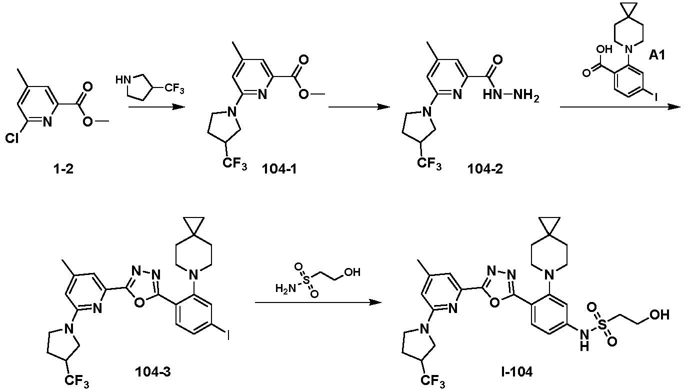

### Step 1: Synthesis of methyl 4-methyl-6-(3-(trifluoromethyl)pyrrolidin-1-yl)picolinate

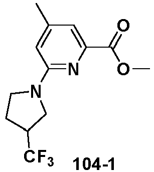

To a solution of methyl 6-chloro-4-methylpicolinate (1 g, 5.4 mmol) inN,N-dimethylformamide (10 mL) were added 3-(trifluoromethyl)pyrrolidine (1.5 g, 10.8 mmol) and potassium carbonate (3.7 g, 27 mmol), and then the mixture was allowed to react under microwave conditions at 110 °C for 2 h. The reaction liquid was poured into water (100 mL), and the mixture was extracted with ethyl acetate (30 mL × 3). The organic phase was washed with saturated brine (20 mL × 2), dried over anhydrous sodium sulfate, filtered, and concentrated to give a crude product. The crude product was purified by column chromatography (petroleum ether/ethyl acetate (V/V) = 1/0-1/1) to give methyl 4-methyl-6-(3-(trifluoromethyl)pyrrolidin-1-yl)picolinate (0.2 g, yield: 12.9%).

LC-MS, M/Z (ESI): 289.3 [M+H]⁺.

### Step 2: Synthesis of 4-methyl-6-(3-(trifluoromethyl)pyrrolidin-1-yl)picolinohydrazide

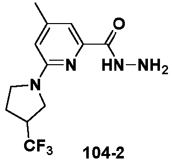

To a solution of methyl 4-methyl-6-(3-(trifluoromethyl)pyrrolidin-1-yl)picolinate (0.2 g, 0.7 mmol) in methanol (2 mL) was added dropwise and slowly 80% hydrazine hydrate (2 mL), and then the mixture was stirred at 60 °C for 3 h. The reaction liquid was poured into water (30 mL), and the mixture was extracted with ethyl acetate (10 mL × 3). The organic phase was washed with saturated brine (10 mL × 2), dried over anhydrous sodium sulfate, filtered, and concentrated to give 4-methyl-6-(3-(trifluoromethyl)pyrrolidin-1-yl)picolinohydrazide (0.2 g, yield: 100%).

LC-MS, M/Z (ESI): 289.3 [M+H]⁺.

### Step 3: Synthesis of 2-(4-iodo-2-(6-azaspiro[2.5]octan-6-yl)phenyl)-5-(4-methyl-6-(3-(trifluoromethyl)pyrrolidin-1-yl)pyridin-2-yl)-1,3,4-oxadiazole

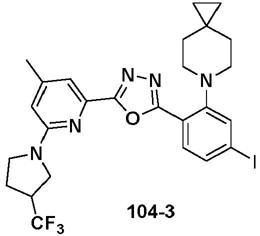

To 4-methyl-6-(3-(trifluoromethyl)pyrrolidin-1-yl)picolinohydrazide (0.2 g, 0.7 mmol) and 4-iodo-2-(6-azaspiro[2.5]octan-6-yl)benzoic acid (0.25 g, 0.7 mmol) was added dropwise and slowly phosphorus oxychloride (2 mL), and then the mixture was stirred at 110 °C for 18 h. The reaction liquid was added dropwise and slowly to ice water (50 mL), and the mixture was extracted with ethyl acetate (30 mL × 3). The organic phase was washed with saturated brine (20 mL × 2), dried over anhydrous sodium sulfate, filtered, and concentrated to give a crude product. The crude product was purified by column chromatography (petroleum ether/ethyl acetate (V/V) = 1/0-1/1) to give 2-(4-iodo-2-(6-azaspiro[2.5]octan-6-yl)phenyl)-5-(4-methyl-6-(3-(trifluoromethyl)pyrrolidin-1-yl)pyridin-2-yl)-1,3,4-oxadiazole (220 mg, yield: 52%).

LC-MS, M/Z (ESI): 610.3 [M+H]⁺.

### Step 4: N-(4-(5-(4-Methyl-6-(3-(trifluoromethyl)pyrrolidin-1-yl)pyridin-2-yl)-1,3,4-oxadiazol-2-yl)-3-(6-azaspiro[2.5]octan-6-yl)phenyl)-2-hydroxyethane-1-sulfonamide

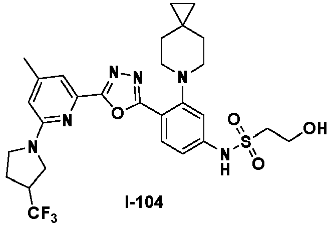

To a solution of 2-(4-iodo-2-(6-azaspiro[2.5]octan-6-yl)phenyl)-5-(4-methyl-6-(3-(trifluoromethyl)pyrrolidin-1-yl)pyridin-2-yl)-1,3,4-oxadiazole (220 mg, 0.36 mmol) in N,N-dimethylformamide (2.2 mL) were added 2-hydroxyethanesulfonamide (90 mg, 0.72 mmol), N,N-dimethylglycine (7 mg, 0.072 mmol), copper(I) iodide (7 mg, 0.036 mmol), and potassium carbonate (149 mg, 1.08 mmol), and the mixture was purged three times with nitrogen and then stirred at 120 °C for 2 h. The reaction liquid was poured into water (30 mL), and the mixture was extracted with ethyl acetate (10 mL × 3). The organic phase was washed with saturated brine (10 mL × 2), dried over anhydrous sodium sulfate, filtered, and concentrated to give a crude product. The crude product was purified by preparative reverse phase chromatography (column: Phenomenex Synergi C18 100 × 25 mm × 4 µm; solvents: A = water + 0.1 vol% formic acid (99%), B = acetonitrile; gradient: 5%-95%, 7 min) to give N-(4-(5-(4-methyl-6-(3-(trifluoromethyl)pyrrolidin-1-yl)pyridin-2-yl)-1,3,4-oxadiazol-2-yl)-3-(6-azaspiro[2.5]octan-6-yl)phenyl)-2-hydroxyethane-1-sulfonamide (95.1 mg, yield: 39%).

¹H NMR (400 MHz, DMSO-d₆) δ 7.83 (d, 1H), 7.39 (s, 1H), 7.06 (d, 1H), 6.98 (s, 1H), 6.95 (dd, 1H), 4.58 (d, 1H), 4.41 (d, 1H), 3.76 (t, 2H), 3.37 (d, 2H), 2.99-2.95 (m, 1H), 2.93-2.87 (m, 4H), 2.35 (s, 3H), 2.00 (s, 1H), 1.81 (d, 1H), 1.57 (dd, 2H), 1.49 (s, 4H), 0.29 (s, 4H).

LC-MS, M/Z (ESI): 607.4 [M+H]⁺.

### Example 32: Preparation of Target Compound I-105

### N-(4-(5-(2-(4-Fluoropiperidin-1-yl)-6-methylpyridin-4-yl)-1,3,4-oxadiazol-2-yl)-3-(6-azaspiro[2.5]octan-6-yl)phenyl)-2-hydroxyethane-sulfonamide

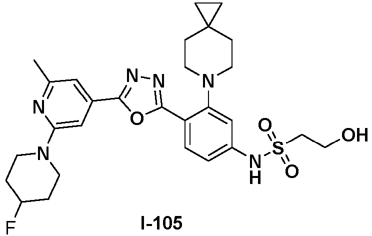

The synthetic route of the target compound **I-105** was as follows:

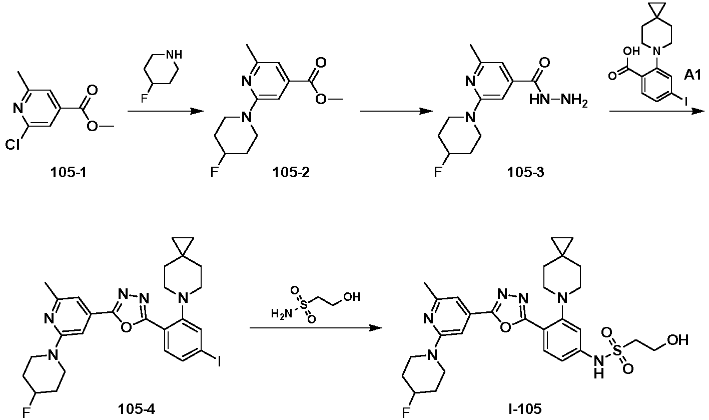

### Step 1: Synthesis of methyl 2-(4-fluoropiperidin-1-yl)-6-methylisonicotinate

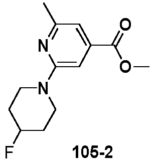

To a solution of methyl 2-chloro-6-methylisonicotinate (1 g, 5.4 mmol) in dimethylsulfoxide (10 mL) were added 4-fluoropiperidine (1.1 g, 10.8 mmol) and potassium carbonate (2.2 g, 16.2 mmol), and then the mixture was stirred under microwave conditions at 140 °C for 2 h. The reaction liquid was poured into water (10 mL), and the mixture was extracted with ethyl acetate (50 mL × 3). The organic phase was washed with saturated brine (20 mL × 3), dried over anhydrous sodium sulfate, filtered, and concentrated to give a crude product. The crude product was purified by column chromatography (petroleum ether/ethyl acetate (V/V) = 1/0-1/3) to give methyl 2-(4-fluoropiperidin-1-yl)-6-methylisonicotinate (100 mg, yield: 7.4%).

LC-MS, M/Z (ESI): 253.4 [M+H]⁺.

### Step 2: Synthesis of 2-(4-fluoropiperidin-1-yl)-6-methylisonicotinohydrazide

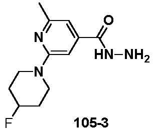

To a solution of methyl 2-(4-fluoropiperidin-1-yl)-6-methylisonicotinate (100 mg, 0.4 mmol) in methanol (0.5 mL) was added dropwise and slowly 80% hydrazine hydrate (0.5 mL), and then the mixture was stirred at 60 °C for 1 h. The reaction liquid was added dropwise and slowly to water (50 mL), and the mixture was extracted with ethyl acetate (20 mL × 3). The organic phase was washed with saturated brine (20 mL × 2), dried over anhydrous sodium sulfate, filtered, and concentrated to give 2-(4-fluoropiperidin-1-yl)-6-methylisonicotinohydrazide (80 mg, yield: 80%).

LC-MS, M/Z (ESI): 253.2 [M+H]⁺.

### Step 3: Synthesis of 2-(2-(4-fluoropiperidin-1-yl)-6-methylpyridin-4-yl)-5-(4-iodo-2-(6-azaspiro[2.5]octan-6-yl)phenyl)-1,3,4-oxadiazole

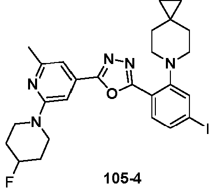

To 2-(4-fluoropiperidin-1-yl)-6-methylisonicotinohydrazide (80 mg, 0.3 mmol) and 4-iodo-2-(6-azaspiro[2.5]octan-6-yl)benzoic acid (143 mg, 0.4 mmol) was added dropwise and slowly phosphorus oxychloride (0.8 mL), and then the mixture was stirred at 110 °C for 18 h. The reaction liquid was added dropwise and slowly to ice water (50 mL), and the mixture was extracted with ethyl acetate (30 mL × 3). The organic phase was washed with saturated brine (20 mL × 2), dried over anhydrous sodium sulfate, filtered, and concentrated to give a crude product. The crude product was purified by column chromatography (petroleum ether/ethyl acetate (V/V) = 1/0-1/1) to give 2-(2-(4-fluoropiperidin-1-yl)-6-methylpyridin-4-yl)-5-(4-iodo-2-(6-azaspiro[2.5]octan-6-yl)phenyl)-1,3,4-oxadiazole (100 mg, yield: 55%).

LC-MS, M/Z (ESI): 574.3 [M+H]+.

### Step 4: Synthesis of N-(4-(5-(2-(4-fluoropiperidin-1-yl)-6-methylpyridin-4-yl)-1,3,4-oxadiazol-2-yl)-3-(6-azaspiro[2.5]octan-6-yl)phenyl)-2-hydroxyethane-sulfonamide

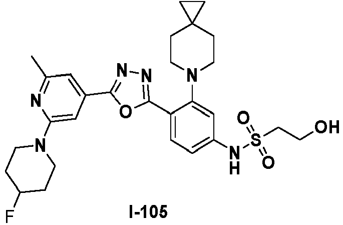

To a solution of 2-(2-(4-fluoropiperidin-1-yl)-6-methylpyridin-4-yl)-5-(4-iodo-2-(6-azaspiro[2.5]octan-6-yl)phenyl)-1,3,4-oxadiazole (80 mg, 0.14 mmol) in N,N-dimethylformamide (1 mL) were added 2-hydroxyethanesulfonamide (35 mg, 0.28 mmol), N,N-dimethylglycine (3 mg, 0.028 mmol), copper(I) iodide (3 mg, 0.014 mmol), and potassium carbonate (58 mg, 0.42 mmol), and the mixture was purged three times with nitrogen and then stirred at 120 °C for 2 h. The reaction liquid was poured into water (30 mL), and the mixture was extracted with ethyl acetate (10 mL × 3). The organic phase was washed with saturated brine (10 mL × 2), dried over anhydrous sodium sulfate, filtered, and concentrated to give a crude product. The crude product was purified by preparative reverse phase chromatography (column: Phenomenex Synergi C18 100 × 25 mm × 4 µm; solvents: A = water + 0.1 vol% formic acid (99%), B = acetonitrile; gradient: 5%-95%, 7 min) to give *N*-(4-(5-(2-(4-fluoropiperidin-1-yl)-6-methylpyridin-4-yl)-1,3,4-oxadiazol-2-yl)-3-(6-azaspiro [2.5] octan-6-yl)phenyl)-2-hydroxyethane-sulfonamide (14.8 mg, yield: 18.6%).

¹H NMR (400 MHz, DMSO-d₆) δ 7.91 (d, 1H), 7.15 (d, 2H), 7.09 (d, 1H), 6.98 (dd, 1H), 5.04-4.93 (m, 1H), 4.86 (dd, 1H), 3.92-3.71 (m, 4H), 3.65-3.52 (m, 2H), 3.37 (s, 2H), 3.03-2.84 (m, 4H), 2.42 (s, 3H), 2.03-1.87 (m, 2H), 1.80-1.67 (m, 2H), 1.48 (s, 4H), 0.31 (s, 4H).

LC-MS, M/Z (ESI): 571.3 [M+H]⁺.

### Example 33: Preparation of Target Compound I-106

### N-(3-(4,4-Difluoropiperidin-1-yl)-4-(4-(2-(4,4-difluoropiperidin-1-yl)-6-methylpyrimidin-4-yl)-1H-pyrazol-1-yl)phenyl)-2-hydroxyethane-1-sulfonamide

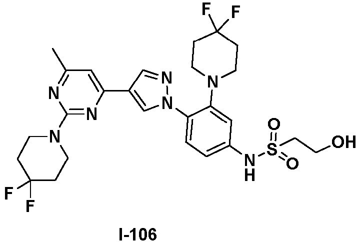

The synthetic route of the target compound **I-106** was as follows:

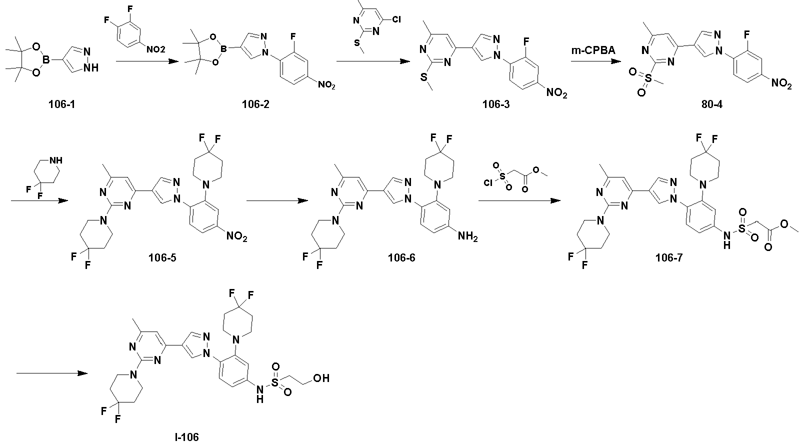

### Step 1: Synthesis of 1-(2-fluoro-4-nitrophenyl)-4-(4,4,5,5-tetramethyl-1,3,2-dioxaborolan-2-yl)-1H-pyrazole

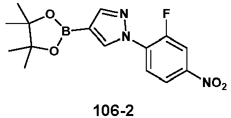

1,2-Difluoro-4-nitrobenzene (5 g, 31.4 mmol), 4-(4,4,5,5-tetramethyl-1,3,2-dioxaborolan-2-yl)-1H-pyrazole (6.7 g, 34.5 mmol), and cesium carbonate (12.3 g, 37.7 mmol) were added to *N*,*N*-dimethylformamide (50 mL), and the mixture was stirred at 60 °C for 2 h. Water (500 mL) was added for dilution, and the mixture was extracted with ethyl acetate (150 mL × 2) and washed with water (100 mL × 5). The organic phase was concentrated to give 1-(2-fluoro-4-nitrophenyl)-4-(4,4,5,5-tetramethyl-1,3,2-dioxaborolan-2-yl)-1*H*-pyrazole (8 g, yield: 76%).

LC-MS, M/Z (ESI): 334.2 [M+H]⁺.

### Step 2: Synthesis of 4-(1-(2-fluoro-4-nitrophenyl)-1H-pyrazol-4-yl)-6-methyl-2-(methylthio)pyrimidine

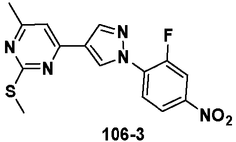

1-(2-Fluoro-4-nitrophenyl)-4-(4,4,5,5-tetramethyl-1,3,2-dioxaborolan-2-yl)-1*H*-pyrazole (5 g, 15 mmol), 4-chloro-6-methyl-2-(methylthio)pyrimidine (2 g, 11.5 mmol), [1,1'-bis(diphenylphosphino)ferrocene]dichloropalladium(II) (1.7 g, 2.3 mmol), and potassium carbonate (2.8 g, 20.7 mmol) were dissolved in *N*,*N*-dimethylformamide (50 mL) and water (15 mL), and the solution was allowed to react at 100 °C for 2 h, then extracted with ethyl acetate (50 mL × 2), and washed with water (100 mL × 5). The organic phase was concentrated to dryness, and the residue was separated and purified by a silica gel column (petroleum ether:ethyl acetate (V/V) = 1:1) to give 4-(1-(2-fluoro-4-nitrophenyl)-1*H*-pyrazol-4-yl)-6-methyl-2-(methylthio)pyrimidine (3.5 g, yield: 60%).

LC-MS, M/Z (ESI): 346.2 [M+H]⁺.

### Step 3: Synthesis of 4-(1-(2-fluoro-4-nitrophenyl)-1H-pyrazol-4-yl)-6-methyl-2-(methylsulfonyl)pyrimidine

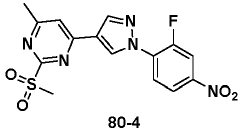

4-(1-(2-Fluoro-4-nitrophenyl)-1*H*-pyrazol-4-yl)-6-methyl-2-(methylthio)pyrimidine (3.5 g, 10 mmol) and *m-*chloroperoxybenzoic acid (10.5 g, 60 mmol) were dissolved in dichloromethane (70 mL), and the solution was stirred at room temperature for 2 h. Water (50 mL) was then added for dilution, and the mixture was extracted with dichloromethane (30 mL × 2) and then washed twice with a saturated sodium sulfite solution (100 mL) and a saturated sodium bicarbonate solution (100 mL). The organic phase was concentrated to dryness to give 4-(1-(2-fluoro-4-nitrophenyl)-1*H*-pyrazol-4-yl)-6-methyl-2-(methylsulfonyl)pyrimidine (3.2 g, yield: 85%).

LC-MS, M/Z (ESI): 378.2 [M+H]⁺.

### Step 4: Synthesis of 2-(4,4-difluoropiperidin-1-yl)-4-(1-(2-(4,4-difluoropiperidin-1-yl)-4-nitrophenyl)-1H-pyrazol-4-yl)-6-methylpyrimidine

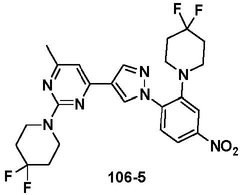

4-(1-(2-Fluoro-4-nitrophenyl)-1*H*-pyrazol-4-yl)-6-methyl-2-(methylsulfonyl)pyrimidine (3.2 g, 8.5 mmol), potassium carbonate (13 g, 94.5 mmol), and 4,4-difluoropiperidine (10.5 g, 85 mmol) were added to dimethylsulfoxide (60 mL), and the mixture was stirred at 140 °C for 8 h. Water (500 mL) was added for dilution, and the mixture was extracted with ethyl acetate (50 mL × 2) and washed with water (100 mL × 5). The organic phase was concentrated to dryness, and the residue was separated and purified by a silica gel column (petroleum ether:ethyl acetate (V/V) = 1:2) to give 2-(4,4-difluoropiperidin-1-yl)-4-(1-(2-(4,4-difluoropiperidin-1-yl)-4-nitrophenyl)-1H-pyrazol-4-yl)-6-methylpyrimidine (300 mg, yield: 8%).

LC-MS, M/Z (ESI): 520.2 [M+H]⁺.

### Step 5: Synthesis of 3-(4,4-difluoropiperidin-1-yl)-4-(4-(2-(4,4-difluoropiperidin-1-yl)-6-methylpyrimidin-4-yl)-1H-pyrazol-1-yl)aniline

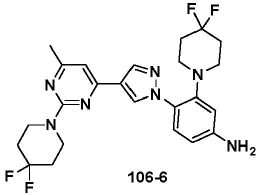

2-(4,4-Difluoropiperidin-1-yl)-4-(1-(2-(4,4-difluoropiperidin-1-yl)-4-nitrophenyl)-1*H*-pyrazol-4-yl)-6-methylpyrimidine (300 mg, 0.58 mmol), iron powder (100 mg, 1.8 mmol), and ammonium chloride (310 mg, 5.8 mmol) were added to 3 mL of ethanol and 1 mL of water, and the mixture was heated to 80 °C and allowed to react for 2 h. Water (20 mL) was added for dilution, and the mixture was extracted with ethyl acetate (20 mL × 2). The organic phase was concentrated to dryness, and the residue was separated and purified by a silica gel column (ethyl acetate:methanol (V/V) = 10:1) to give 3-(4,4-difluoropiperidin-1-yl)-4-(4-(2-(4,4-difluoropiperidin-1-yl)-6-methylpyrimidin-4-yl)-1*H*-pyrazol-1-yl)aniline (250 mg, yield: 85%).

LC-MS, M/Z (ESI): 490.2 [M+H]⁺.

### Step 6: Synthesis of methyl 2-(N-(3-(4,4-difluoropiperidin-1-yl)-4-(4-(2-(4,4-difluoropiperidin-1-yl)-6-methylpyrimidin-4-yl)-1H-pyrazol-1-yl)phenyl)sulfamoyl)acetate

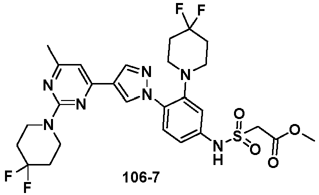

3-(4,4-Difluoropiperidin-1-yl)-4-(4-(2-(4,4-difluoropiperidin-1-yl)-6-methylpyrimidin-4-yl)-1*H*-pyrazol-1-yl)aniline (250 mg, 0.5 mmol), potassium phosphate (318 mg, 1.5 mmol), and methyl 2-(chlorosulfonyl)acetate (104 mg, 0.6 mmol) were added to 2 mL of acetonitrile, and the mixture was allowed to react at room temperature for 1 h. Water (10 mL) was added for dilution, and the mixture was extracted with ethyl acetate (10 mL × 2). The organic phase was concentrated to dryness, and the residue was separated and purified by a silica gel column (ethyl acetate:methanol = 10:1) to give methyl 2-(*N*-(3-(4,4-difluoropiperidin-1-yl)-4-(4-(2-(4,4-difluoropiperidin-1-yl)-6-methylpyrimidin-4-yl)-1*H*-pyrazol-1-yl)phenyl)sulfamoyl)acetate (120 mg, yield: 45%).

LC-MS, M/Z (ESI): 626.2 [M+H]⁺.

### Step 7: Synthesis of N-(3-(4,4-difluoropiperidin-1-yl)-4-(4-(2-(4,4-difluoropiperidin-1-yl)-6-methylpyrimidin-4-yl)-1H-pyrazol-1-yl)phenyl)-2-hydroxyethane-1-sulfonamide

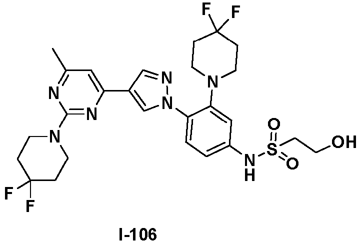

Methyl 2-(*N*-(3-(4,4-difluoropiperidin-1-yl)-4-(4-(2-(4,4-difluoropiperidin-1-yl)-6-methylpyrimidin-4-yl)-1*H-*pyrazol-1-yl)phenyl)sulfamoyl)acetate (25.0 mg, 40.0 µmol) was dissolved in tetrahydrofuran (5 mL), and then lithium borohydride (2 M, 24.0 µL) was added dropwise under nitrogen atmosphere at 0 °C. The mixture was allowed to react at 0 °C for 2 h. After the reaction was completed, the reaction liquid was quenched with a saturated ammonium chloride solution (25 mL) and extracted with ethyl acetate (20 mL × 3). The organic phase was washed with saturated brine (50 mL), dried over anhydrous sodium sulfate, filtered, and concentrated to give a crude product, which was then separated by reverse phase high performance liquid chromatography (separation method: column: Phenomenex luna C18 150 × 25 mm × 5 µm; solvents: A = water + 0.05% (by volume) trifluoroacetic acid (99.0%), B = acetonitrile; gradient: 38%-68%, 9 min), followed by lyophilization to give *N*-(3-(4,4-difluoropiperidin-1-yl)-4-(4-(2-(4,4-difluoropiperidin-1-yl)-6-methylpyrimidin-4-yl)-1*H*-pyrazol-1-yl)phenyl)-2-hydroxyethane-1-sulfonamide (14 mg, 67%).

¹H NMR (400 MHz, DMSO_d₆) *δ* 9.93 (s, 1H), 8.97 (s, 1H), 8.32 (s, 1H), 7.48 (d, 1H), 6.98-7.05 (m, 3H), 3.93-3.95 (m, 4H), 3.76 (t, 2H), 2.73-2.77 (m, 4H), 2.66 (t, 2H), 2.32 (s, 3H), 1.93-1.97 (m, 8H).

LC-MS, M/Z (ESI): 598.2 [M+H]⁺.

### Example 34: Preparation of Target Compound I-109

### N-(4-(4-(2-(4,4-Difluoropiperidin-1-yl)pyrimidin-4-yl)-1H-pyrazo1-1-yl)-3-(6-azaspiro[2.5]octan-6-yl)phenyl)-2-hydroxyethane-1-sulfonamide

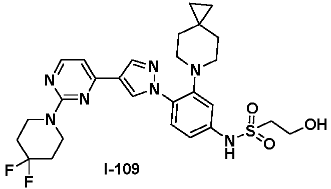

The synthetic route of the target compound **I-109** was as follows:

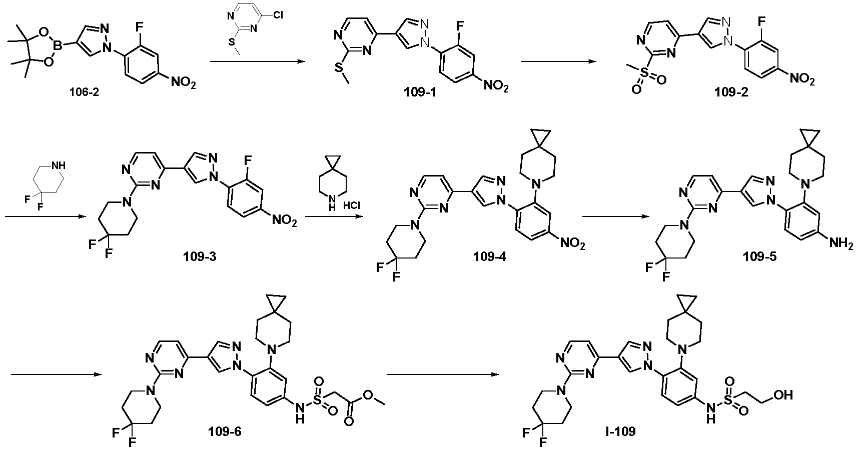

### Step 1: Synthesis of 4-(1-(2-fluoro-4-nitrophenyl)-1H-pyrazol-4-yl)-2-(methylthio)pyrimidine

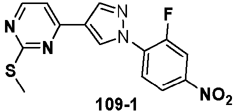

To a solution of 1-(2-fluoro-4-nitrophenyl)-4-(4,4,5,5-tetramethyl-1,3,2-dioxaborolan-2-yl)-1*H*-pyrazole (20.4 g, 61.24 mmol) in *N*,*N*-dimethylformamide (50 mL) were added 4-chloro-2-(methylthio)pyrimidine (7.45 g, 46.38 mmol), a 1,1'-bis(diphenylphosphino)ferrocene dichloropalladium(II) dichloromethane complex (4.25 g, 5.15 mmol), and water (10 mL), and the mixture was purged three times with nitrogen and then allowed to react under nitrogen atmosphere at 100 °C for 8 h. After the reaction was completed, the mixture was diluted with water (100 mL) and extracted three times with ethyl acetate (100 mL). The organic phases were combined, washed 5 times with saturated brine (100 mL), dried over anhydrous sodium sulfate, filtered, and concentrated to give a crude product. The crude product was subjected to column chromatography (PE/EA (V/V) = 3/1) to give 4-(1-(2-fluoro-4-nitrophenyl)-1H-pyrazol-4-yl)-2-(methylthio)pyrimidine (15.20 g, yield: 89%).

LC-MS, M/Z (ESI): 332.1 [M+H]⁺.

### Step 2: Synthesis of 4-(1-(2-fluoro-4-nitrophenyl)-1H-pyrazol-4-yl)-2-(methylsulfonyl)pyrimidine

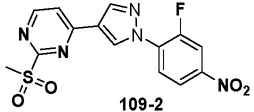

4-(1-(2-Fluoro-4-nitrophenyl)-1*H*-pyrazol-4-yl)-2-(methylthio)pyrimidine (2.00 g, 6.0 mmol) was dissolved in dichloromethane (20 mL), and then m-chloroperoxybenzoic acid (2.60 g, 15.09 mmol) was added slowly. The mixture was stirred at room temperature for 1 h. After the reaction was completed, the mixture was diluted with water (20 mL) and extracted three times with dichloromethane (20 mL). The organic phases were combined, washed sequentially with a saturated sodium sulfite solution (30 mL), a saturated sodium bicarbonate solution (30 mL) and saturated brine (30 mL), dried over anhydrous sodium sulfate, filtered, and concentrated to give a crude product. The crude product was subjected to column chromatography (PE/EA (V/V) = 1/1) to give 4-(1-(2-fluoro-4-nitrophenyl)-1*H*-pyrazol-4-yl)-2-(methylsulfonyl)pyrimidine (1.85 g, yield: 84%).

LC-MS, M/Z (ESI): 364.1 [M+H]⁺.

### Step 3: Synthesis of 2-(4,4-difluoropiperidin-1-yl)-4-(1-(2-fluoro-4-nitrophenyl)-1H-pyrazol-4-yl)pyrimidine

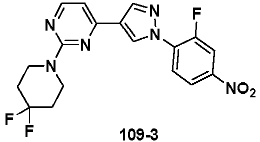

4-(1-(2-Fluoro-4-nitrophenyl)-1H-pyrazol-4-yl)-2-(methylsulfonyl)pyrimidine (1.80 g, 4.95 mmol) and potassium carbonate (2.05 g, 14.86 mmol) were dissolved in DMSO (20 mL), and then 4,4-difluoropiperidine (900 mg, 7.43 mmol) was added slowly. The mixture was stirred at 50 °C for 18 h. After the reaction was completed, the mixture was diluted with water (20 mL) and extracted three times with dichloromethane (20 mL). The organic phases were combined, washed 5 times with saturated brine (30 mL), dried over anhydrous sodium sulfate, filtered, and concentrated to give a crude product. The crude product was subjected to column chromatography (PE/EA (V/V) = 3/1) to give 2-(4,4-difluoropiperidin-1-yl)-4-(1-(2-fluoro-4-nitrophenyl)-1*H*-pyrazol-4-yl)pyrimidine (1.90 g, yield: 95%).

LC-MS, M/Z (ESI): 405.1 [M+H]⁺.

### Step 4: Synthesis of 6-(2-(4-(2-(4,4-difluoropiperidin-1-yl)pyrimidin-4-yl)-1H-pyrazol-1-yl)-5-nitrophenyl)-6-azaspiro[2.5]octane

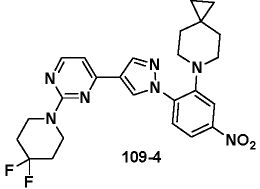

2-(4,4-Difluoropiperidin-1-yl)-4-(1-(2-fluoro-4-nitrophenyl)-1*H*-pyrazol-4-yl)pyrimidine (1.50 g, 3.7 mmol) and potassium carbonate (1.54 g, 11.1 mmol) were dissolved in DMSO (15 mL), and then 6-azaspiro[2.5]octane hydrochloride (822 mg, 5.56 mmol) was added slowly. The mixture was stirred at 140 °C for 8 h. After the reaction was completed, the mixture was diluted with water (20 mL) and extracted three times with dichloromethane (20 mL). The organic phases were combined, washed 5 times with saturated brine (30 mL), dried over anhydrous sodium sulfate, filtered, and concentrated to give a crude product. The crude product was subjected to column chromatography (PE/EA (V/V) = 3/1) to give 6-(2-(4-(2-(4,4-difluoropiperidin-1-yl)pyrimidin-4-yl)-1*H*-pyrazol-1-yl)-5-nitrophenyl)-6-azaspiro[2.5]octane (1.21 g, yield: 66%).

LC-MS, M/Z (ESI): 496.1 [M+H]⁺.

### Step 5: Synthesis of 4-(4-(2-(4,4-difluoropiperidin-1-yl)pyrimidin-4-yl)-1H-pyrazol-1-yl)-3-(6-azaspiro[2.5]octan-6-yl)aniline

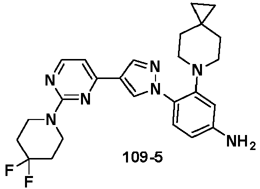

6-(2-(4-(2-(4,4-Difluoropiperidin-1-yl)pyrimidin-4-yl)-1*H-*pyrazol-1-yl)-5-nitrophenyl)-6-azaspiro[2.5]octane (1.20 g, 2.42 mmol) was dissolved in acetic acid (10 mL), and then zinc powder (475 mg, 7.26 mmol) was added. The mixture was stirred at 50 °C for 1 h. After the reaction was completed, the mixture was diluted with water (30 mL), adjusted to about pH 7 with a saturated sodium bicarbonate solution, and then extracted three times with dichloromethane (20 mL). The organic phases were combined, washed with saturated brine (30 mL), dried over anhydrous sodium sulfate, filtered, and concentrated to give a crude product. The crude product was subjected to column chromatography (PE/EA (V/V) = 2/1) to give 4-(4-(2-(4,4-difluoropiperidin-1-yl)pyrimidin-4-yl)-1*H-*pyrazol-1-yl)-3-(6-azaspiro[2.5]octan-6-yl)aniline (650 mg, yield: 58%).

LC-MS, M/Z (ESI): 466.1 [M+H]⁺.

### Step 6: Synthesis of methyl 2-(N-(4-(4-(2-(4,4-difluoropiperidin-1-yl)pyrimidin-4-yl)-1H-pyrazol-1-yl)-3-(6-azaspiro[2.5]octan-6-yl)phenyl)sulfamoyl)acetate

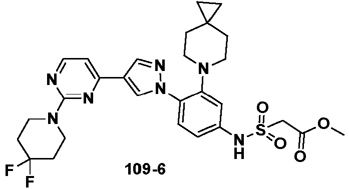

4-(4-(2-(4,4-Difluoropiperidin-1-yl)pyrimidin-4-yl)-1*H*-pyrazol-1-yl)-3-(6-azaspiro[2.5]octan-6-yl)aniline (600 mg, 1.29 mmol) was dissolved in acetonitrile (10 mL), and then potassium phosphate (621 mg, 3.87 mmol) and methyl 2-(chlorosulfonyl)acetate (334 mg, 1.93 mmol) were added. The mixture was stirred at room temperature for 1 h. After the reaction was completed, the mixture was diluted with water (10 mL) and extracted three times with dichloromethane (10 mL). The organic phases were combined, washed with saturated brine (20 mL), dried over anhydrous sodium sulfate, filtered, and concentrated to give a crude product. The crude product was subjected to column chromatography (DCM/MeOH (V/V) = 10/1) to give methyl 2-(N-(4-(4-(2-(4,4-difluoropiperidin-1-yl)pyrimidin-4-yl)-1*H*-pyrazol-1-yl)-3-(6-azaspiro[2.5]octan-6-yl)phenyl)sulfamoyl)acetate (420 mg, yield: 54%).

LC-MS, M/Z (ESI): 602.1 [M+H]⁺.

### Step 8: Synthesis of N-(4-(4-(2-(4,4-difluoropiperidin-1-yl)pyrimidin-4-yl)-1H-pyrazol-1-yl)-3-(6-azaspiro[2.5]octan-6-yl)phenyl)-2-hydroxyethane-1-sulfonamide

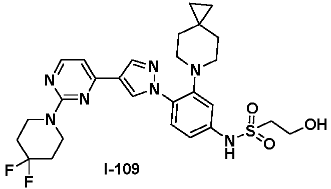

Methyl 2-(*N-*(4-(4-(2-(4,4-difluoropiperidin-1-yl)pyrimidin-4-yl)-1*H*-pyrazol-1-yl)-3-(6-azaspiro[2.5]octan-6-yl)phenyl)sulfamoyl)acetate (500 mg, 831 µmol) was dissolved in tetrahydrofuran (10 mL), and then lithium borohydride (2 M, 498 µL) was added dropwise under nitrogen atmosphere at 0 °C. The mixture was allowed to react at 25 °C for 1 h. After the reaction was completed, the reaction liquid was quenched with a saturated ammonium chloride solution (25 mL) and extracted with ethyl acetate (20 mL × 3). The organic phase was washed with saturated brine (50 mL), dried over anhydrous sodium sulfate, filtered, and concentrated to give a crude product, which was then separated by reverse phase high performance liquid chromatography (separation method: column: Phenomenex luna C18 150 × 25 mm × 5 µm; solvents: A = water + 0.05% (by volume) trifluoroacetic acid (99.0%), B = acetonitrile; gradient: 40%-70%, 15 min), followed by lyophilization to give *N*-(4-(4-(2-(4,4-difluoropiperidin-1-yl)pyrimidin-4-yl)-1*H*-pyrazol-1-yl)-3-(6-azaspiro[2.5]octan-6-yl)phenyl)-2-hydroxyethane-1-sulfonamide (129 mg, yield: 27.1%).

¹H NMR (400 MHz, DMSO_d₆) *δ* 9.89 (s, 1H), 9.10 (s, 1H), 8.38 (d, 1H), 8.34 (s, 1H), 7.49 (d, 1H), 7.06-7.08 (m, 2H), 6.97-6.99 (m, 1H), 3.96-3.99 (m, 4H), 3.77 (t, 2H), 3.30 (t, 2H), 2.69-2.71 (m, 4H), 1.98-2.07 (m, 4H), 1.30-1.40 (m, 4H), 0.26 (s, 4H).

LC-MS, M/Z (ESI): 574.4 [M+H]⁺.

### Example 35: Preparation of Target Compound I-110

### N-(4-(4-(2-(4,4-Difluorocyclohexyl)-6-methylpyrimidin-4-yl)-1H-pyrazol-1-yl)-3-(6-azaspiro[2.5]octan-6-yl)phenyl)-2-hydroxyethane-1-sulfonamide

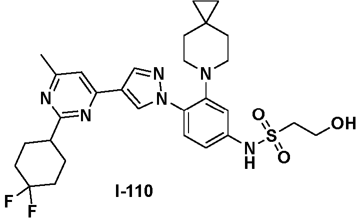

The synthetic route of the target compound **I-110** was as follows:

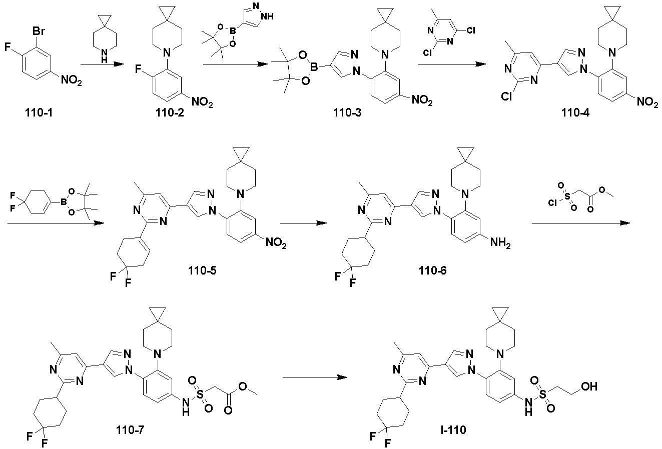

### Step 1: Synthesis of 6-(2-fluoro-5-nitrophenyl)-6-azaspiro[2.5]octane

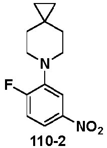

To a solution of 2-bromo-1-fluoro-4-nitrobenzene (5 g, 22.7 mmol) and 6-azaspiro[2.5]octane (5 g, 45.4 mmol) in 1,4-dioxane (50 mL) were added methanesulfonato(2-dicyclohexylphosphino-2',6'-diisopropoxy-1,1'-biphenyl)(2-amino-1,1'-biphenyl-2-yl)palladium(II) (1.9 g, 2.3 mmol), 2-dicyclohexylphosphino-2',6'-diisopropoxy-1,1'-biphenyl (2.1 g, 4.5 mmol), and cesium carbonate (22.2 g, 68.1 mmol), and the mixture was purged three times with nitrogen and then allowed to react under nitrogen atmosphere at 110 °C for 18 h. Water (200 mL) was added to the reaction liquid, and the mixture was extracted with ethyl acetate (100 mL × 3). The organic phase was washed with saturated brine (100 mL × 2), dried over anhydrous sodium sulfate, filtered, and concentrated to give a crude product. The crude product was purified by column chromatography (petroleum ether/ethyl acetate (V/V) = 1/0-1/1) to give 6-(2-fluoro-5-nitrophenyl)-6-azaspiro[2.5]octane (4.5 g, yield: 79.1%).

LC-MS, M/Z (ESI): 251.3 [M+H]⁺.

### Step 2: Synthesis of 6-(5-nitro-2-(4-(4,4,5,5-tetramethyl-1,3,2-dioxaborolan-2-yl)-1H-pyrazol-1-yl)phenyl)-6-azaspiro[2.5]octane

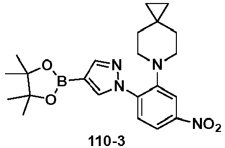

To a solution of 6-(2-fluoro-5-nitrophenyl)-6-azaspiro[2.5]octane (4.5 g, 18 mmol) in *N,N-dimethylformamide* (45 mL) were added 4-(4,4,5,5-tetramethyl-1,3,2-dioxaborolan-2-yl)-1*H-*pyrazole (4.2 g, 21.6 mmol) and potassium carbonate (7.5 g, 54 mmol), and then the mixture was stirred at 50 °C for 24 h. The reaction liquid was poured into water (200 mL), and the mixture was extracted with ethyl acetate (100 mL × 3). The organic phase was washed with saturated brine (50 mL × 3), dried over anhydrous sodium sulfate, filtered, and concentrated to give a crude product. The crude product was purified by column chromatography (petroleum ether/ethyl acetate (V/V) = 1/0-1/1) to give 6-(5-nitro-2-(4-(4,4,5,5-tetramethyl-1,3,2-dioxaborolan-2-yl)-1*H*-pyrazol-1-yl)phenyl)-6-azaspiro[2.5]octane (2.2 g, yield: 28.8%).

LC-MS, M/Z (ESI): 425.1 [M+H]⁺.

### Step 3: Synthesis of 6-(2-(4-(2-chloro-6-methylpyrimidin-4-yl)-1H-pyrazol-1-yl)-5-nitrophenyl)-6-azaspiro[2.5] octane

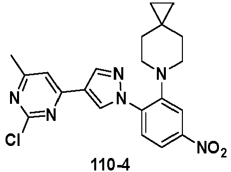

To a solution of 6-(5-nitro-2-(4-(4,4,5,5-tetramethyl-1,3,2-dioxaborolan-2-yl)-1*H*-pyrazol-1-yl)phenyl)-6-azaspiro[2.5]octane (2 g, 4.7 mmol) and 2,4-dichloro-6-methylpyrimidine (766 mg, 4.7 mmol) in toluene (20 mL) and water (4 mL) were added tetrakis(triphenylphosphine)palladium(0) (578 mg, 0.47 mmol) and potassium fluoride (819 mg, 14.1 mmol), and the mixture was stirred at 80 °C for 18 h. The reaction liquid was poured into water (100 mL), and the mixture was extracted with ethyl acetate (50 mL × 3). The organic phase was washed with saturated brine (50 mL × 2), dried over anhydrous sodium sulfate, filtered, and concentrated to give a crude product. The crude product was purified by column chromatography (petroleum ether/ethyl acetate (V/V) = 1/0-1/1) to give 6-(2-(4-(2-chloro-6-methylpyrimidin-4-yl)-1*H*-pyrazol-1-yl)-3-nitrophenyl)-6-azaspiro[2.5]octane (650 mg, yield: 43.3%).

LC-MS, M/Z (ESI): 425.2 [M+H]⁺.

### Step 4: Synthesis of 6-(2-(4-(2-(4,4-difluorocyclohex-1-en-1-yl)-6-methylpyrimidin-4-yl)-1H-pyrazol-1-yl)-5-nitrophenyl)-6-azaspiro[2.5]octane

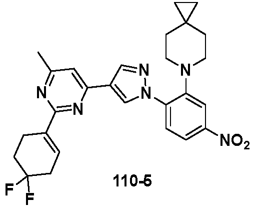

To a solution of 6-(2-(4-(2-chloro-6-methylpyrimidin-4-yl)-1*H*-pyrazol-1-yl)-5-nitrophenyl)-6-azaspiro[2.5]octane (0.6 g, 1.4 mmol) and 2-(4,4-difluorocyclohex-1-en-1-yl)-4,4,5,5-tetramethyl-1,3,2-dioxaborolane (1.0 mg, 4.2 mmol) in 1,4-dioxane (6 mL) and water (2 mL) were added [1,1'-bis(diphenylphosphino)ferrocene]dichloropalladium(II) (102 mg, 0.14 mmol) and potassium carbonate (774 mg, 5.6 mmol), and the mixture was purged three times with nitrogen and then allowed to react under nitrogen atmosphere at 100 °C for 18 h. The reaction liquid was poured into water (50 mL), and the mixture was extracted with ethyl acetate (20 mL × 3). The organic phase was washed with saturated brine (20 mL × 2), dried over anhydrous sodium sulfate, filtered, and concentrated to give a crude product. The crude product was purified by column chromatography (petroleum ether/ethyl acetate (V/V) = 1/0-1/1) to give 6-(2-(4-(2-(4,4-difluorocyclohex-1-en-1-yl)-6-methylpyrimidin-4-yl)-1*H*-pyrazol-1-yl)-5-nitrophenyl)-6-azaspiro[2.5]octane (100 mg, yield: 16.8%).

LC-MS, M/Z (ESI): 507.2 [M+H]⁺.

### Step 5: Synthesis of 4-(4-(2-(4,4-difluorocyclohexyl)-6-methylpyrimidin-4-yl)-1H-pyrazol-1-yl)-3-(6-azaspiro [2.5] octan-6-yl)aniline

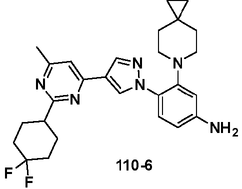

To a solution of 6-(2-(4-(2-(4,4-difluorocyclohex-1-en-1-yl)-6-methylpyrimidin-4-yl)-1*H*-pyrazol-1-yl)-5-nitrophenyl)-6-azaspiro[2.5]octane (0.1 g, 0.2 mmol) in methanol (5 mL) was added wet palladium on carbon (10%, 0.1 g), and the mixture was purged 3 times with hydrogen and then allowed to react under hydrogen atmosphere (15 psi) at room temperature for 18 h. The reaction liquid was filtered, and the filtrate was concentrated to give 4-(4-(2-(4,4-difluorocyclohexyl)-6-methylpyrimidin-4-yl)-1*H*-pyrazol-1-yl)-3-(6-azaspiro[2.5]octan-6-yl)aniline (80 mg, yield: 84.7%).

LC-MS, M/Z (ESI): 479.4 [M+H]⁺.

### Step 6: Synthesis of methyl 2-(N-(4-(4-(2-(4.4-difluorocyclohexyl)-6-methylpyrimidin-4-y1)-1H-pyrazol-1-y1)-3-(6-azaspiro[2.5]octan-6-yl)phenyl)sulfamoyl)acetate

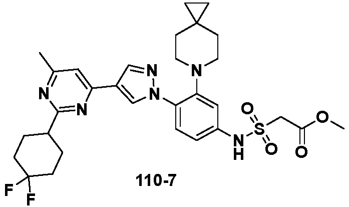

To a solution of 4-(4-(2-(4,4-difluorocyclohexyl)-6-methylpyrimidin-4-yl)-1*H*-pyrazol-1-yl)-3-(6-azaspiro[2.5]octan-6-yl)aniline (80 mg, 0.17 mmol) in acetonitrile (10 mL) was added potassium phosphate (108 mg, 0.51 mmol), and then methyl 2-(chlorosulfonyl)acetate (44 mg, 0.26 mmol) was added dropwise and slowly. The reaction liquid was allowed to react at 25 °C for 2 h. The reaction liquid was added dropwise and slowly to water (10 mL), and the mixture was extracted with ethyl acetate (5 mL × 3). The organic phase was washed with saturated brine (5 mL × 2), dried over anhydrous sodium sulfate, filtered, and concentrated to give a crude product. The crude product was purified by column chromatography (petroleum ether/ethyl acetate (V/V) = 1/0-1/1) to give methyl 2-(N (4-(4-(2-(4,4-difluorocyclohexyl)-6-methylpyrimidin-4-yl)-1*H*-pyrazol-1-yl)-3-(6-azaspiro[2.5]octan-6-yl)phenyl)sulfamoyl)acetate (50 mg, yield: 48.6%).

LC-MS, M/Z (ESI): 615.3 [M+H]⁺.

### Step 7: Synthesis of N-(4-(4-(2-(4.4-difluorocyclohexyl)-6-methylpyrimidin-4-y1)-1H-pyrazol-1-y1)-3-(6-azaspiro[2.5]octan-6-yl)phenyl)-2-hydroxyethane-1-sulfonamide

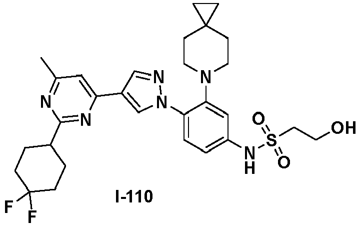

Methyl 2-(N (4-(4-(2-(4,4-difluorocyclohexyl)-6-methylpyrimidin-4-yl)-1*H*-pyrazol-1-yl)-3-(6-azaspiro[2.5]octan-6-yl)phenyl)sulfamoyl)acetate (50.0 mg, 81.3 µmol) was dissolved in tetrahydrofuran (5 mL), and then lithium borohydride (a 2 M solution in tetrahydrofuran, 48.8 µL, 97.6 µmol) was added dropwise under nitrogen atmosphere at 0 °C. The mixture was allowed to react at 0 °C for 2 h. After the reaction was completed, the reaction liquid was quenched with a saturated ammonium chloride solution (25 mL) and extracted with ethyl acetate (20 mL × 3). The organic phase was washed with saturated brine (50 mL), dried over anhydrous sodium sulfate, filtered, and concentrated to give a crude product, which was then separated by reverse phase high performance liquid chromatography (separation method: column: Phenomenex luna C18 150 × 25 mm × 5 µm; solvents: A = water + 0.05% (by volume) formic acid (99.0%), B = acetonitrile; gradient: 48%-78%, 10 min), followed by lyophilization to give N (4-(4-(2-(4,4-difluorocyclohexyl)-6-methylpyrimidin-4-yl)-1*H*-pyrazol-1-yl)-3-(6-azaspiro[2.5]octan-6-yl)phenyl)-2-hydroxyethane-1-sulfonamide (5.82 mg, 9.62 µmol, purity: 97.8%, yield: 11.8%).

¹H NMR (400 MHz, CDCl3) δ 9.04 (s, 1H), 8.16 (s, 1H), 7.59 (d, 1H), 7.16 (s, 1H), 7.10 (s, 1H), 6.95 (d, 1H), 6.60 (s, 1H), 4.15-4.17 (m, 2H), 3.33-3.35 (m, 2H), 2.93-2.95 (m, 1H), 2.81-2.84 (m, 4H), 2.54 (s, 3H), 2.12-2.24 (m, 6H), 1.85-1.88 (m, 2H), 1.41-1.43 (m, 4H), 0.30 (s, 4H).

LC-MS, M/Z (ESI): 587.3 [M+H]⁺.

### Example 36: Preparation of Target Compounds I-111 & I-112

### (R)-N-(4-(4-(2-(4,4-Difluoropiperidin-1-yl)-6-methylpyrimidin-4-yl)-1H-pyrazol-1-yl)-3-(6-azaspiro[2.5]octan-6-yl)phenyl)-1-hydroxypropane-2-sulfonamide

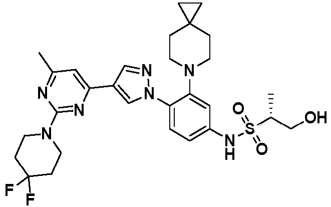

### (S)-N-(4-(4-(2-(4,4-Difluoropiperidin-1-yl)-6-methylpyrimidin-4-yl)-1H-pyrazol-1-yl)-3-(6-azaspiro[2.5]octan-6-yl)phenyl)-1-hydroxypropane-2-sulfonamide

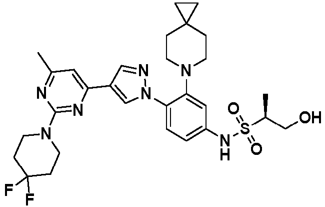

The synthetic route of the target compounds **I-111** *&* **I-112** was as follows:

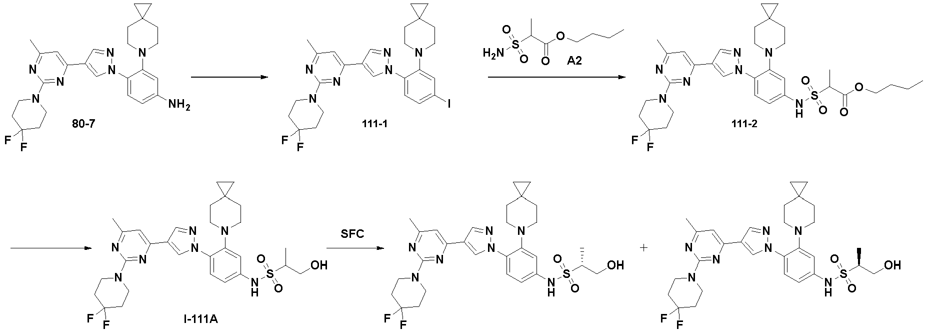

### Step 1: Synthesis of 6-(2-(4-(2-(4,4-difluoropiperidin-1-yl)-6-methylpyrimidin-4-yl)-1H-pyrazol-1-yl)-5-iodophenyl)-6-azaspiro[2.5]octane

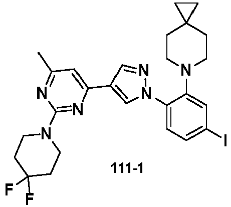

4-(4-(2-(4,4-Difluoropiperidin-1-yl)-6-methylpyrimidin-4-yl)-1*H*-pyrazol-1-yl)-3-(6-azaspiro[2.5]octan-6-yl)aniline (1 g, 2.085 mmol) was added to 10 mL of acetonitrile, and then isoamyl nitrite (0.733 g, 6.26 mmol) and copper(I) iodide (1.986 g, 10.43 mmol) were added. The mixture was allowed to react at room temperature overnight. LCMS showed that the starting materials were completely reacted. The reaction liquid was concentrated to dryness, and the residue was separated and purified by a silica gel column (volume ratio: PE:DCM:EA = 1:1:1) to give 6-(2-(4-(2-(4,4-difluoropiperidin-1-yl)-6-methylpyrimidin-4-yl)-1*H*-pyrazol-1-yl)-5-iodophenyl)-6-azaspiro[2.5]octane

(1 g, yield: 81%).

LC-MS, M/Z (ESI): 591.2 [M+H]⁺.

### Step 2: Synthesis of butyl 2-(N-(4-(4-(2-(4,4-difluoropiperidin-1-yl)-6-methylpyrimidin-4-yl)-1H-pyrazol-1-yl)-3-(6-azaspiro[2.5]octan-6-yl)phenyl)sulfatnoyl)propionate

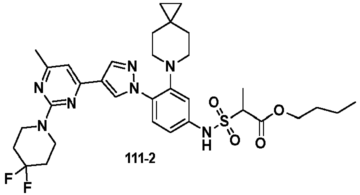

6-(2-(4-(2-(4,4-Difluoropiperidin-1-yl)-6-methylpyrimidin-4-yl)-1*H*-pyrazol-1-yl)-5-iodophenyl)-6-azaspiro[2.5]octane (1 g, 1.69 mmol) was added to 20 mL of N,N-dimethylformamide, and then butyl 2-sulfamoylpropionate (661 mg, 3.39 mmol), N,N-dimethylglycine (349 mg, 3.39 mmol), copper(I) iodide (323 mg, 3.39 mmol), and potassium carbonate (936 mg, 6.77 mmol) were added. The mixture was heated to 130 °C and allowed to react for 1 h. LCMS showed that the starting materials were completely reacted. Water (50 mL) was added for dilution, and the mixture was extracted with ethyl acetate (100 mL × 2). The organic phase was concentrated to dryness, and the residue was separated and purified by a silica gel column (PE:EA (V/V) = 1:1) to give a crude product of butyl 2-(*N*-(4-(4-(2-(4.4-difluoropiperidin-1-yl)-6-methylpyrimidin-4-yl)-1*H*-pyrazol-1-yl)-3-(6-azaspiro[2.5]octan-6-yl)phenyl)sulfamoyl)propionate (1.5 g).

LC-MS, M/Z (ESI): 672.3 [M+H]⁺.

### Step 3: Synthesis of (R)-N-(4-(4-(2-(4,4-difluoropiperidin-1-yl)-6-methylpyrimidin-4-yl)-1H-pyrazol-1-yl)-3-(6-azaspiro [2.5] octan-6-yl)phenyl)-1-hydroxypropane-2-sulfonamide

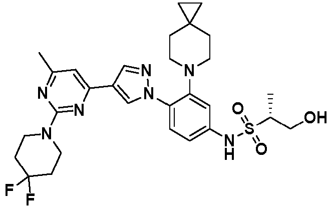

synthesis of (*S*)-*N*-(4-(4-(2-(4,4-difluoropiperidin-1-yl)-6-methylpyrimidin-4-yl)-1*H*-pyrazol-1-yl)-3-(6-azaspiro[2.5]octan-6-yl)phenyl)-1-hydroxypropane-2-sulfonamide

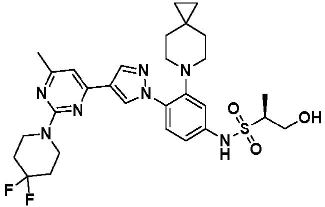

The crude product of butyl 2-(N (4-(4-(2-(4,4-difluoropiperidin-1-yl)-6-methylpyrimidin-4-yl)-1*H*-pyrazol-1-yl)-3-(6-azaspiro[2.5]octan-6-yl)phenyl)sulfamoyl)propionate (1.50 g, 2.23 mmol) was dissolved in tetrahydrofuran (50 mL), and then lithium borohydride (2 M, 1.34 mL) was added dropwise under nitrogen atmosphere at 0 °C. The mixture was allowed to react at 0 °C for 2 h. After the reaction was completed, the reaction liquid was quenched with a saturated ammonium chloride solution (50 mL) and extracted with ethyl acetate (50 mL × 3). The organic phase was washed with saturated brine (50 mL), dried over anhydrous sodium sulfate, filtered, and concentrated to give a crude product, which was then separated by reverse phase high performance liquid chromatography (separation method: column: Phenomenex luna C18 150 × 70 mm × 10 µm; solvents: A = water + 0.05% (by volume) formic acid (99.0%), B = acetonitrile; gradient: 40%-70%, 20 min), followed by lyophilization to give *N*-(4-(4-(2-(4,4-difluoropiperidin-1-yl)-6-methylpyrimidin-4-yl)-1*H*-pyrazol-1-yl)-3-(6-azaspiro[2.5]octan-6-yl)phenyl)-1-hydroxypropane-2-sulfonamide.

The compound was then subjected to resolution (resolution method: column: Chiralpak IG-3 50 × 4.6 mm I.D., 3 µm; solvents: A = carbon dioxide, B = 0.05% (by volume) diethylamine (99.0%) in ethanol; gradient: 5%-40%, flow rate = 3 mL/min, detector: PDA, column temperature = 35 °C, column pressure = 100 Bar).

Resolution and concentration of peak 2 gave the compound (*R*)-*N*-(4-(4-(2-(4,4-difluoropiperidin-1-yl)-6-methylpyrimidin-4-yl)-1*H*-pyrazol-1-yl)-3-(6-azaspiro[2.5]octan-6-yl)phenyl)-1-hydroxypropane-2-sulfonamide or (*S*)-*N*-(4-(4-(2-(4,4-difluoropiperidin-1-yl)-6-methylpyrimidin-4-yl)-1*H*-pyrazol-1-yl)-3-(6-azaspiro[2.5]octan-6-yl)phenyl)-1-hydroxypropane-2-sulfonamide (104 mg, SFC retention time: 2.145 min, yield: 7.71%).

LC-MS, M/Z (ESI): 602.3 [M+H]⁺.

¹H NMR (400 MHz, CDCl3) δ 8.92 (s, 1H), 8.12 (s, 1H), 7.55 (d, 1H), 7.10 (s, 1H), 6.92 (d, 1H), 6.65-6.67 (m, 2H), 3.95-4.08 (m, 6H), 3.39-3.42 (m, 1H), 2.80-2.82 (m, 4H), 2.51 (brs, 1H), 2.39 (s, 3H), 1.98-2.06 (m, 4H), 1.38-1.45 (m, 7H), 0.30 (s, 4H).

Resolution and concentration of peak 1 gave the compound (S)-N-(4-(4-(2-(4,4-difluoropiperidin-1-yl)-6-methylpyrimidin-4-yl)-1*H*-pyrazol-1-yl)-3-(6-azaspiro[2.5]octan-6-yl)phenyl)-1-hydroxypropane-2-sulfonamide or (*R*)-*N*-(4-(4-(2-(4,4-difluoropiperidin-1-yl)-6-methylpyrimidin-4-yl)-1*H*-pyrazol-1-yl)-3-(6-azaspiro[2.5]octan-6-yl)phenyl)-1-hydroxypropane-2-sulfonamide (73.0 mg, SFC retention time: 2.034 min, yield: 5.43%).

LC-MS, M/Z (ESI): 602.3 [M+H]⁺.

¹H NMR (400 MHz, CDCl3) δ 8.90 (s, 1H), 8.12 (s, 1H), 7.53 (d, 1H), 7.14 (s, 1H), 6.87-6.95 (m, 2H), 6.65 (s, 1H), 3.96-4.12 (m, 6H), 3.38-3.41 (m, 1H), 2.80-2.82 (m, 5H), 2.39 (s, 3H), 1.98-2.08 (m, 4H), 1.35-1.41 (m, 7H), 0.30 (s, 4H).

The SFC retention time for compound I-111 was 2.145 min, and the SFC retention time for compound I-112 was 2.034 min.

### Example 37: Preparation of Target Compound I-113

### N-(4-(4-(2-(6,6-Difluoro-3-azabicyclo[3.1.0]hexan-3-yl)-6-methylpyrimidin-4-yl)-1H-pyrazol-1-yl)-3-(6-azaspiro[2.5]octan-6-yl)phenyl)-2-hydroxyethane-1-sulfonamide

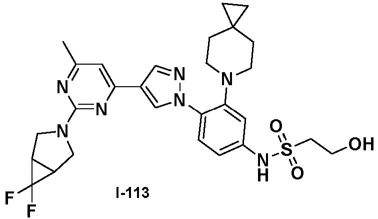

The synthetic route of the target compound **I-113** was as follows:

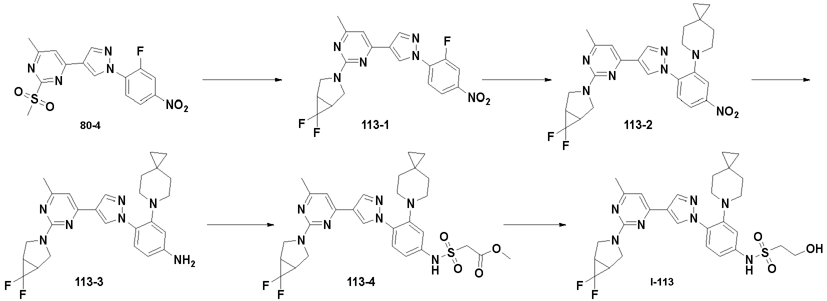

### Step 1: Synthesis of 6,6-difluoro-3-(4-(1-(2-fluoro-4-nitrophenyl)-1H-pyrazol-4-yl)-6-methylpyrimidin-2-yl)-3-azabicyclo[3.1.0]hexane

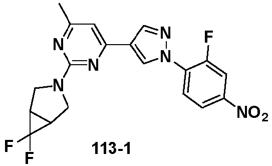

4-(1-(2-Fluoro-4-nitrophenyl)-1*H*-pyrazol-4-yl)-6-methyl-2-(methylsulfonyl)pyrimidine (1 g, 2.65 mmol) was added to 10 mL of dimethylsulfoxide, and then 6,6-difluoro-3-azabicyclo[3.1.0]hexane hydrochloride (0.825 g, 5.3 mmol) and potassium carbonate (1.831 g, 13.25 mmol) were added. The mixture was heated to 105 °C and allowed to react overnight. LCMS showed that the starting materials were completely reacted. The reaction liquid was concentrated to dryness, and the residue was separated and purified by a silica gel column (volume ratio: PE:DCM:EA = 3:3:1) to give 6,6-difluoro-3-(4-(1-(2-fluoro-4-nitrophenyl)-1*H*-pyrazol-4-yl)-6-methylpyrimidin-2-yl)-3-azabicyclo[3.1.0] hexane (0.9 g, yield: 82%).

LC-MS, M/Z (ESI): 417.12 [M+H]⁺.

### Step 2: Synthesis of 6-(2-(4-(2-(6,6-difluoro-3-azabicyclo[3.1.0]hexan-3-yl)-6-methylpyrimidin-4-yl)-1H-pyrazol-1-yl)-5-nitrophenyl)-6-azaspiro[2.5]octane

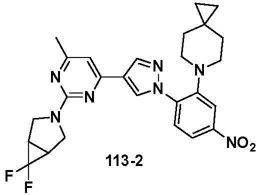

6,6-Difluoro-3-(4-(1-(2-fluoro-4-nitrophenyl)-1*H*-pyrazol-4-yl)-6-methylpyrimidin-2-yl)-3-azabicyclo[3.1.0]hexane (0.9 g, 2.162 mmol) was added to 3 mL of dimethylsulfoxide, and then 6-azaspiro[2.5]octane hydrochloride (0.798 g, 5.4 mmol) and potassium carbonate (2.241 g, 16.21 mmol) were added. The mixture was heated to 140 °C and allowed to react for 8 h. LCMS showed that the starting materials were completely reacted. The reaction liquid was poured into 50 mL of water, and the mixture was filtered to give 6-(2-(4-(2-(6,6-difluoro-3-azabicyclo[3.1.0]hexan-3-yl)-6-methylpyrimidin-4-yl)-1*H*-pyrazol-1-yl)-5-nitrophenyl)-6-azaspiro[2.5]octane (0.8 g, yield: 72.9%).

LC-MS, M/Z (ESI): 508.22 [M+H]⁺.

### Step 3: Synthesis of 4-(4-(2-(6,6-difluoro-3-azabicyclo[3.1.0]hexan-3-yl)-6-methylpyrimidin-4-yl)-1H-pyrazol-1-yl)-3-(6-azaspiro [2.5] octan-6-yl)aniline

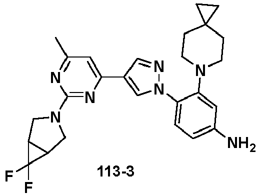

6-(2-(4-(2-(6,6-Difluoro-3-azabicyclo[3.1.0]hexan-3-yl)-6-methylpyrimidin-4-yl)-1H-pyrazol-1-yl)-5-nitrophenyl)-6-azaspiro[2.5]octane (0.8 g, 1.576 mmol) was added to 20 mL of ethanol, and then iron powder (1.761 g, 31.5 mmol) and ammonium chloride (0.843 g, 15.76 mmol) were added. The mixture was heated to 90 °C and allowed to react for 6 h. LCMS showed that the starting materials were completely reacted. The reaction liquid was concentrated to dryness, and the residue was separated and purified by a silica gel column (PE:EA (V/V) = 3:1) to give 4-(4-(2-(6,6-difluoro-3-azabicyclo [3.1.0]hexan-3-yl)-6-methylpyrimidin-4-yl)-1*H*-pyrazol-1-yl)-3-(6-azaspiro [2.5] octan-6-yl)aniline (0.6 g, yield: 80%).

LC-MS, M/Z (ESI): 478.25 [M+H]⁺.

### Step 4: Synthesis of methyl 2-(N-(4-(4-(2-(6,6-difluoro-3-azabicyclo[3.1.0]hexan-3-yl)-6-methylpyrimidin-4-yl)-1H-pyrazol-1-yl)-3-(6-azaspiro[2.5]octan-6-yl)phenyl)sulfamoyl)acetate

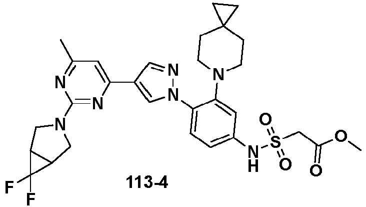

4-(4-(2-(6,6-Difluoro-3-azabicyclo[3.1.0]hexan-3-yl)-6-methylpyrimidin-4-yl)-1*H*-pyrazol-1-yl)-3-(6-azaspiro[2.5]octan-6-yl)aniline (0.6 g, 1.256 mmol) was added to 9 mL of acetonitrile, and then potassium carbonate (0.521 g, 3.77 mmol) and methyl 2-(chlorosulfonyl)acetate (0.282 g, 1.633 mmol) were added. The mixture was allowed to react at room temperature for 1 h. LCMS showed that the starting materials were completely reacted. The reaction liquid was concentrated to dryness, and the residue was separated and purified by a silica gel column (PE:EA (V/V) = 5:1) to give methyl 2-(*N*-(4-(4-(2-(6,6-difluoro-3-azabicyclo[3.1.0]hexan-3-yl)-6-methylpyrimidin-4-yl)-1*H*-pyrazol-1-yl)-3-(6-azaspiro[2.5]octan-6-yl)phenyl)sulfamoyl)acetate (0.6 g, yield: 78%).

LC-MS, M/Z (ESI): 614.24 [M+H]⁺.

### Step 5: Synthesis of N-(4-(4-(2-(6,6-difluoro-3-azabicyclo[3.1.0]hexan-3-yl)-6-methylpyrimidin-4-yl)-1H-pyrazol-1-yl)-3-(6-azaspiro[2.5]octan-6-yl)phenyl)-2-hydroxyethane-1-sulfonamide

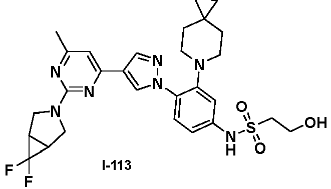

Methyl 2-(*N*-(4-(4-(2-(6,6-difluoro-3-azabicyclo[3.1.0]hexan-3-yl)-6-methylpyrimidin-4-yl)-1*H*-pyrazol-1-yl)-3-(6-azaspiro[2.5]octan-6-yl)phenyl)sulfamoyl)acetate (600 mg, 978 µmol) was dissolved in tetrahydrofuran (20 mL), and then lithium borohydride (2 M, 587 µL) was added dropwise under nitrogen atmosphere at 0 °C. The mixture was allowed to react at 25 °C for 6 h. After the reaction was completed, the reaction liquid was quenched with a saturated ammonium chloride solution (25 mL) and extracted with ethyl acetate (25 mL × 2). The organic phase was washed with saturated brine (50 mL), dried over anhydrous sodium sulfate, filtered, and concentrated to give a crude product, which was then separated by reverse phase high performance liquid chromatography (separation method: column: Phenomenex luna C18 150 × 25 mm × 5 µm; solvents: A = water + 0.05% (by volume) formic acid (99.0%), B = acetonitrile; gradient: 35%-65%, 11 min), followed by lyophilization to give *N*-(4-(4-(2-(6,6-difluoro-3-azabicyclo[3.1.0]hexan-3-yl)-6-methylpyrimidin-4-yl)-1*H*-pyrazol-1-yl)-3-(6-azaspiro[2.5]octan-6-yl)phenyl)-2-hydroxyethane-1-sulfonamide (109 mg, yield: 18.8%).

¹H NMR (400 MHz, DMSO_d₆) δ 9.08 (s, 1H), 8.26 (s, 1H), 7.49 (d, 1H), 7.09 (s, 1H), 6.91-6.98 (m, 2H), 3.97-4.03 (m, 2H), 3.75-3.78 (m, 4H), 3.27-3.29 (m, 2H), 2.66-2.71 (m, 6H), 2.31 (s, 3H), 1.38-1.45 (m, 4H), 0.27 (s, 4H). LC-MS, M/Z (ESI): 586.3 [M+H]⁺.

### Example 38: Preparation of Target Compound I-114

### N-(4-(4-(2-(4-Fluoropiperidin-1-y1)-6-methylpyrimidin-4-y1)-1H-pyrazol-1-yl)-3-(6-azaspiro[2.5]octan-6-yl)phenyl)-2-hydroxyethane-1-sulfonamide

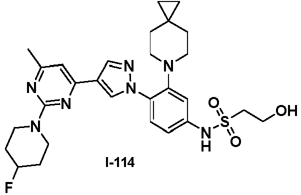

The synthetic route of the target compound **I-114** was as follows:

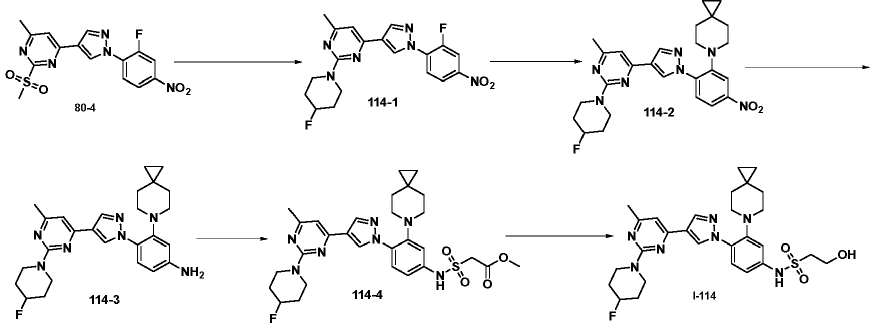

### Step 1: Synthesis of 4-(1-(2-fluoro-4-nitrophenyl)-1H-pyrazol-4-yl)-2-(4-fluoropiperidin-1-yl)-6-methylpyrimidine

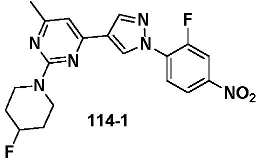

4-(1-(2-Fluoro-4-nitrophenyl)-1H-pyrazol-4-yl)-6-methyl-2-(methylsulfonyl)pyrimidine (1 g, 2.65 mmol) was added to 10 mL of dimethylsulfoxide, and then 4-fluoropiperidine hydrochloride (0.93 g, 6.33 mmol) and potassium carbonate (1.831 g, 13.3 mmol) were added. The mixture was heated to 105 °C and allowed to react overnight. LCMS showed that the starting materials were completely reacted. The reaction liquid was concentrated to dryness, and the residue was separated and purified by a silica gel column (volume ratio: PE:DCM:EA = 3:3:1) to give 4-(1-(2-fluoro-4-nitrophenyl)-1H-pyrazol-4-yl)-2-(4-fluoropiperidin-1-yl)-6-methylpyrimidine (0.9 g, yield: 85%).

LC-MS, M/Z (ESI): 401.2 [M+H]⁺.

### Step 2: Synthesis of 6-(2-(4-(2-(4-fluoropiperidin-1-yl)-6-methylpyrimidin-4-yl)-1H-pyrazol-1-yl)-5-nitrophenyl)-6-azaspiro[2.5]octane

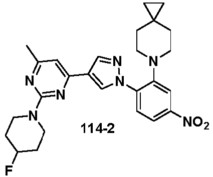

4-(1-(2-Fluoro-4-nitrophenyl)-1H-pyrazol-4-yl)-2-(4-fluoropiperidin-1-yl)-6-methylpyrimidine (0.9 g, 2.248 mmol) was added to 3 mL of dimethylsulfoxide, and then 6-azaspiro[2.5]octane hydrochloride (0.83 g, 5.62 mmol) and potassium carbonate (2.33 g, 16.86 mmol) were added. The mixture was heated to 140 °C and allowed to react for 8 h. LCMS showed that the starting materials were completely reacted. The reaction liquid was poured into 50 mL of water, and the mixture was filtered to give 6-(2-(4-(2-(4-fluoropiperidin-1-yl)-6-methylpyrimidin-4-yl)-1*H*-pyrazol-1-yl)-5-nitrophenyl)-6-azaspiro[2.5]octane (0.9 g, yield: 81%).

LC-MS, M/Z (ESI): 492.2 [M+H]⁺.

### Step 3: Synthesis of 4-(4-(2-(4-fluoropiperidin-1-yl)-6-methylpyrimidin-4-yl)-1H-pyrazol-1-yl)-3-(6-azaspiro [2.5] octan-6-yl)aniline

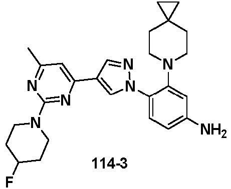

6-(2-(4-(2-(4-Fluoropiperidin-1-yl)-6-methylpyrimidin-4-yl)-1*H-*pyrazol-1-yl)-5-nitrophenyl)-6-azaspiro[2.5]octane (0.9 g, 1.831 mmol) was added to 20 mL of ethanol, and then iron powder (2.045 g, 36.6 mmol) and ammonium chloride (0.979 g, 18.31 mmol) were added. The mixture was heated to 90 °C and allowed to react for 6 h. LCMS showed that the starting materials were completely reacted. The reaction liquid was concentrated to dryness, and the residue was separated and purified by a silica gel column (PE:EA (V/V) = 3:1) to give 4-(4-(2-(4-fluoropiperidin-1-yl)-6-methylpyrimidin-4-yl)-1*H*-pyrazol-1-yl)-3-(6-azaspiro[2.5]octan-6-yl)aniline (0.7 g, yield: 83%).

LC-MS, M/Z (ESI): 462.3 [M+H]⁺.

### Step 4: Synthesis of methyl 2-(N-(4-(4-(2-(4-fluoropiperidin-1-yl)-6-methylpyrimidin-4-yl)-1H-pyrazol-1-yl)-3-(6-azaspiro[2.5]octan-6-yl)phenyl)sulfamoyl)acetate

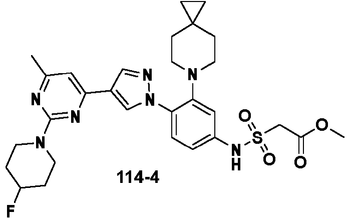

4-(4-(2-(4-Fluoropiperidin-1-yl)-6-methylpyrimidin-4-yl)-1*H*-pyrazol-1-yl)-3-(6-azaspiro[2.5]octan-6-yl)aniline (0.7 g, 1.517 mmol) was added to 9 mL of acetonitrile, and then potassium carbonate (0.629 g, 4.55 mmol) and methyl 2-(chlorosulfonyl)acetate (0.340 g, 1.971 mmol) were added. The mixture was allowed to react at room temperature for 1 h. LCMS showed that the starting materials were completely reacted. The reaction liquid was concentrated to dryness, and the residue was separated and purified by a silica gel column (PE:EA (V/V) = 5:1) to give methyl 2-(*N*-(4-(4-(2-(4-fluoropiperidin-1-yl)-6-methylpyrimidin-4-yl)-1H-pyrazol-1-yl)-3-(6-azaspiro[2.5] octan-6-yl)phenyl)sulfamoyl)acetate (0.76 g, yield: 84%).

LC-MS, M/Z (ESI): 598.3 [M+H]⁺.

### Step 5: Synthesis of N-(4-(4-(2-(4-fluoropiperidin-1-yl)-6-methylpyrimidin-4-yl)-1H-pyrazol-1-yl)-3-(6-azaspiro[2.5]octan-6-yl)phenyl)-2-hydroxyethane-1-sulfonamide

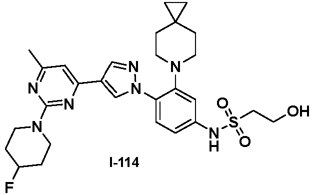

Methyl 2-(*N-*(4-(4-(2-(4-fluoropiperidin-1-yl)-6-methylpyrimidin-4-yl)-1*H*-pyrazol-1-yl)-3-(6-azaspiro[2.5]octan-6-yl)phenyl)sulfamoyl)acetate (760 mg, 1.24 mmol) was dissolved in tetrahydrofuran (10 mL), and then lithium borohydride (2 M, 743 µL) was added dropwise under nitrogen atmosphere at 0 °C. The mixture was allowed to react at 25 °C for 6 h. After the reaction was completed, the reaction liquid was quenched with a saturated ammonium chloride solution (25 mL) and extracted with ethyl acetate (25 mL × 2). The organic phase was washed with saturated brine (50 mL), dried over anhydrous sodium sulfate, filtered, and concentrated to give a crude product, which was then separated by reverse phase high performance liquid chromatography (separation method: column: UniSil 10-120 C18 150 × 25 mm × 5 µm; solvents: A = water + 0.05% (by volume) formic acid (99.0%), B = acetonitrile; gradient: 30%-60%, 27 min.), followed by lyophilization to give N-(4-(4-(2-(4-fluoropiperidin-1-yl)-6-methylpyrimidin-4-yl)-1H-pyrazol-1-yl)-3-(6-azaspiro[2.5]octan-6-y])phenyl)-2-hydroxyethane-1-sulfonamide (421 mg, 717 µmol, purity: 99.7%, yield: 57.9%).

¹H NMR (400 MHz, DMSO_d₆) *δ* 9.08 (s, 1H), 8.26 (s, 1H), 7.51 (d, *J =* 8.8 Hz, 1H), 7.06 (s, 1H), 6.94-6.97 (m, 1H), 6.90 (s, 1H), 4.85-5.00 (m, 1H), 4.02-4.05 (m, 2H), 3.74-3.77 (m, 4H), 3.27-3.33 (m, 2H), 2.68-2.73 (m, 5H), 2.31 (s, 3H), 1.74-1.95 (m, 2H), 1.68-1.70 (m, 2H), 1.36-1.44 (m, 4H), 0.26 (s, 4H).

LC-MS, M/Z (ESI): 570.3 [M+H]⁺.

### Example 38: Preparation of Target Compound I-121

### N-(4-(4-(1-(3,3-Difluorocyclobutyl)-6-oxo-1,6-dihydropyridazin-3-yl)-1H-pyrazol-1-yl)-3-(6-azaspiro[2.5]octan-6-yl)phenyl)-2-hydroxyethane-1-sulfonamide

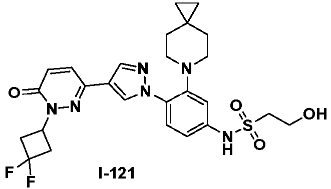

The synthetic route of the target compound **I-121** was as follows:

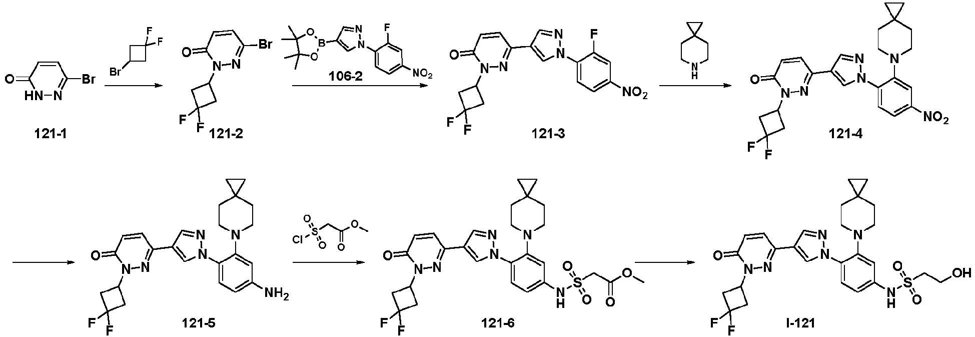

### Step 1: Synthesis of 6-bromo-2-(3,3-difluorocyclobutyl)pyridazin-3(2H)-one

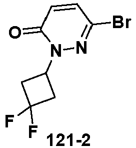

To a solution of 3-bromo-1,1-difluorocyclobutane (1 g, 5.8 mmol) in *N*,*N*-dimethylformamide (10 mL) were added 6-bromopyridazin-3(2*H*)-one (1 g, 5.8 mmol) and potassium carbonate (2.4 g, 17.4 mmol), and the mixture was stirred at 50 °C for 18 h. The reaction liquid was poured into water (100 mL), and the mixture was extracted with ethyl acetate (30 mL × 3). The organic phase was washed with saturated brine (20 mL × 2), dried over anhydrous sodium sulfate, filtered, and concentrated to give a crude product. The crude product was purified by column chromatography (petroleum ether/ethyl acetate (V/V) = 1/0-1/1) to give 6-bromo-2-(3,3-difluorocyclobutyl)pyridazin-3(2*H*)-one (200 mg, yield: 12.9%).

LC-MS, M/Z (ESI): 265.2 [M+H]⁺.

### Step 2: Synthesis of 2-(3,3-difluorocyclobutyl)-6-(1-(2-fluoro-4-nitrophenyl)-1H-pyrazol-4-yl)pyridazin-3(2H)-one

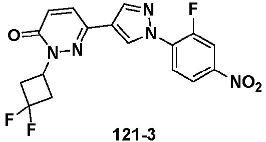

To a solution of 6-bromo-2-(3,3-difluorocyclobutyl)pyridazin-3(2*H*)-one (200 mg, 0.75 mmol) and 1-(2-fluoro-4-nitrophenyl)-4-(4,4,5,5-tetramethyl-1,3,2-dioxaborolan-2-yl)-1H-pyrazole (533 mg, 1.50 mmol) in *N,N-*dimethylformamide (2 mL) and water (0.4 mL) were added [1,1'-bis(diphenylphosphino)ferrocene]dichloropalladium(II) (54 mg, 0.075 mmol) and potassium carbonate (332 mg, 2.25 mmol), and the mixture was stirred at 90 °C for 18 h. The reaction liquid was added to water (20 mL), and the mixture was extracted with ethyl acetate (5 mL × 3). The organic phase was washed with saturated brine (5 mL × 2), dried over anhydrous sodium sulfate, filtered, and concentrated to give a crude product. The crude product was purified by column chromatography (petroleum ether/ethyl acetate (V/V) = 1/0-1/3) to give 2-(3,3-difluorocyclobutyl)-6-(1-(2-fluoro-4-nitrophenyl)-1*H*-pyrazol-4-yl)pyridazin-3(2*H*)-one (220 mg, yield: 74.5%).

LC-MS, M/Z (ESI): 392.2 [M+H]⁺.

### Step 3: Synthesis of 2-(3,3-difluorocyclobutyl)-6-(1-(4-nitro-2-(6-azaspiro[2.5]octan-6-yl)phenyl)-1H-pyrazol-4-yl)pyridazin-3(2H)-one

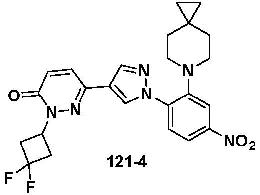

To a solution of 2-(3,3-difluorocyclobutyl)-6-(1-(2-fluoro-4-nitrophenyl)-1*H*-pyrazol-4-yl)pyridazin-3(2*H*)-one (200 mg, 0.5 mmol) in dimethylsulfoxide (2 mL) were added 6-azaspiro[2.5]octane (111 mg, 1 mmol) and potassium carbonate (207 mg, 1.5 mmol), and the mixture was allowed to react under microwave conditions at 140 °C for 8 h. The reaction liquid was poured into water (20 mL), and the mixture was extracted with ethyl acetate (5 mL × 3). The organic phase was washed with saturated brine (5 mL × 2), dried over anhydrous sodium sulfate, filtered, and concentrated to give a crude product. The crude product was purified by column chromatography (petroleum ether/ethyl acetate (V/V) = 1/0-1/1) to give 2-(3,3-difluorocyclobutyl)-6-(1-(4-nitro-2-(6-azaspiro[2.5]octan-6-yl)phenyl)-1*H*-pyrazol-4-yl)pyridazin-3(2*H*)-one (220 mg, yield: 89%).

LC-MS, M/Z (ESI): 483.3 [M+H]⁺.

### Step 4: Synthesis of 6-(1-(4-amino-2-(6-azaspiro[2.5]octan-6-yl)phenyl)-1H-pyrazol-4-yl)-2-(3,3-difluorocyclobutyl)pyridazin-3(2H)-one

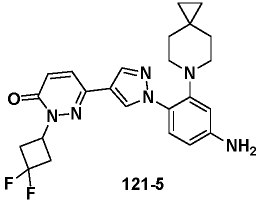

To a solution of 2-(3,3-difluorocyclobutyl)-6-(1-(4-nitro-2-(6-azaspiro[2.5]octan-6-yl)phenyl)-1*H*-pyrazol-4-yl)pyridazin-3(2H)-one (200 mg, 0.4 mmol) in ethanol (5 mL) were added zinc powder (131 mg, 2 mmol) and ammonium chloride (212 mg, 4 mmol), and the mixture was stirred at 85 °C for 2 h. Water (20 mL) and ethyl acetate (10 mL) were added to the reaction liquid, and the mixture was filtered. The filtrate was extracted with ethyl acetate (5 mL × 3). The organic phase was washed with saturated brine (10 mL × 2), dried over anhydrous sodium sulfate, filtered, and concentrated to give a crude product. The crude product was purified by column chromatography (petroleum ether/ethyl acetate (V/V) = 1/0-1/2) to give 6-(1-(4-amino-2-(6-azaspiro[2.5]octan-6-yl)phenyl)-1*H-*pyrazol-4-yl)-2-(3,3-difluorocyclobutyl)pyridazin-3(2*H*)-one (100 mg, yield: 53%).

LC-MS, M/Z (ESI): 453.3 [M+H]⁺.

### Step 5: Synthesis of methyl 2-(N (4-(4-(1-(3,3-difluorocyclobutyl)-6-oxo-1,6-dihydropyridazin-3-yl)-1H-pyrazol-1-yl)-3-(6-azaspiro[2.5]octan-6-yl)phenyl)sulfamoyl)acetate

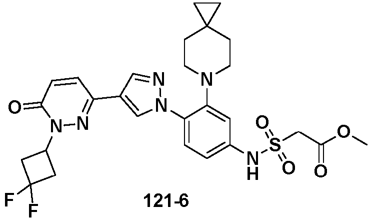

To a solution of 6-(1-(4-amino-2-(6-azaspiro[2.5]octan-6-yl)phenyl)-1*H*-pyrazol-4-yl)-2-(3,3-difluorocyclobutyl)pyridazin-3(2*H*)-one (100 mg, 0.22 mmol) in acetonitrile (1.5 mL) were added methyl 2-(chlorosulfonyl)acetate (57 mg, 0.33 mmol) and potassium phosphate (127 mg, 0.66 mmol), and then the mixture was stirred at 25 °C for 3 h. The reaction liquid was poured into water (20 mL), and the mixture was extracted with ethyl acetate (5 mL × 3). The organic phase was washed with saturated brine (5 mL × 2), dried over anhydrous sodium sulfate, filtered, and concentrated to give a crude product. The crude product was purified by column chromatography (petroleum ether/ethyl acetate (V/V) = 1/0-0/1) to give methyl 2-(*N-(*4-(4-(1-(3,3-difluorocyclobutyl)-6-oxo-1,6-dihydropyridazin-3-yl)-1*H*-pyrazol-1-yl)-3-(6-azaspiro[2.5]octan-6-yl)phenyl)sulfamoyl)acetate (80 mg, yield: 61.5%).

LC-MS, M/Z (ESI): 589.3 [M+H]⁺.

### Step 6: Synthesis of N (4-(4-(1-(3,3-difluorocyclobutyl)-6-oxo-1,6-dihydropyridazin-3-yl)-1H-pyrazol-1-yl)-3-(6-azaspiro[2.5]octan-6-yl)phenyl)-2-hydroxyethane-1-sulfonamide

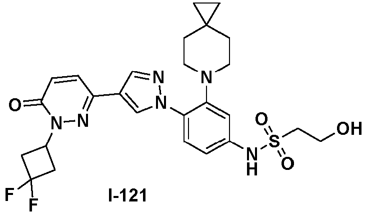

To a solution of methyl 2-(N-(4-(4-(1-(3,3-difluorocyclobutyl)-6-oxo-1,6-dihydropyridazin-3-yl)-1*H*-pyrazol-1-yl)-3-(6-azaspiro[2.5]octan-6-yl)phenyl)sulfamoyl)acetate (80 mg, 0.14 mmol) in tetrahydrofuran (1 mL) was added calcium chloride (151 mg, 1.4 mmol), and then sodium borohydride (51 mg, 1.4 mmol) was added slowly. The mixture was stirred at 25 °C for 2 h. The reaction liquid was added dropwise and slowly to a saturated aqueous ammonium chloride solution (10 mL), and ethyl acetate (5 mL) was added. The mixture was then filtered. The filtrate was extracted with ethyl acetate (5 mL × 3), and the organic phase was washed with saturated brine (5 mL × 2), dried over anhydrous sodium sulfate, filtered, and concentrated to give a crude product. The crude product was subjected to preparative reverse phase chromatography (column: Phenomenex Synergi C18 100 × 25 mm × 4 µm; solvents: A = water + 0.1 vol% aqueous ammonia (99%), B = acetonitrile; gradient: 5%-95%, 7 min) to give *N*-(4-(4-(1-(3,3-difluorocyclobutyl)-6-oxo-1,6-dihydropyridazin-3-yl)-1*H*-pyrazol-1-yl)-3-(6-azaspiro[2.5]octan-6-yl)phenyl)-2-hydroxyethane-1-sulfonamide (22.1 mg, purity: 96.28%, yield: 28.2%).

¹H NMR (400 MHz, DMSO_d₆) δ 9.88 (s, 1H), 8.90 (s, 1H), 8.23 (s, 1H), 7.93 (d, 1H), 7.46 (d, 1H), 7.06 (dd, 2H), 6.97 (dd, 1H), 5.30 (dd, 1H), 4.97 (s, 1H), 3.77 (t, 2H), 3.30 (t, 2H), 3.20-3.00 (m, 4H), 2.79-2.61 (m, 4H), 1.36 (s, 4H), 0.25 (s, 4H).

LC-MS, M/Z (ESI): 561.0 [M+H]⁺.

### Example 39: Preparation of Target Compounds I-40 & I-41

### (R)-N-(4-(5-(2-(4,4-Difluoropiperidin-1-yl)-6-methylpyrimidin-4-yl)-1,3,4-oxadiazol-2-yl)-3-(6-azaspiro [2.5] octan-6-yl)phenyl)-1-hydroxypropane-2-sulfonamide

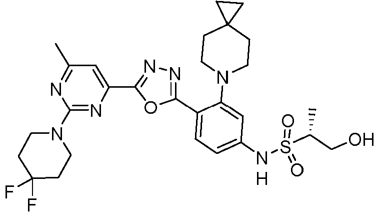

### (S)-N-(4-(5-(2-(4,4-Difluoropiperidin-1-yl)-6-methylpyrimidin-4-yl)-1,3,4-oxadiazol-2-yl)-3-(6-azaspiro[2.5]octan-6-yl)phenyl)-1-hydroxypropane-2-sulfonamide

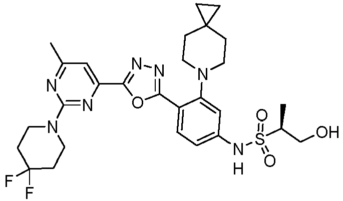

The synthetic route of the target compounds **I-40 & I-41** was as follows:

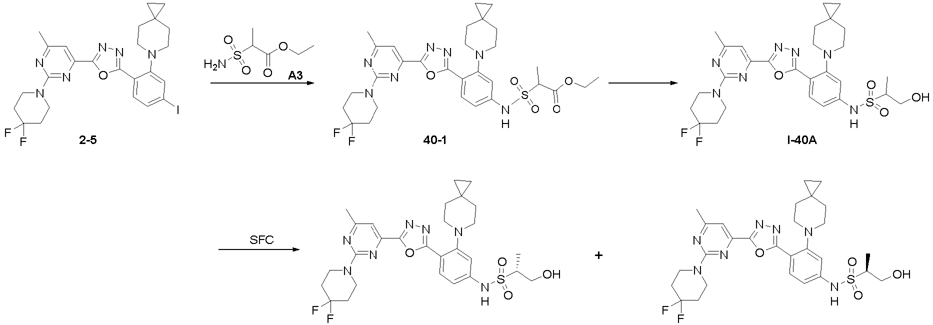

### Step 1: Synthesis of ethyl 2-(N-(4-(5-(2-(4,4-difluoropiperidin-1-yl)-6-methylpyrimidin-4-yl)-1,3,4-oxadiazol-2-yl)-3-(6-azaspiro[2.5]octan-6-yl)phenyl)sulfatnoyl)propionate

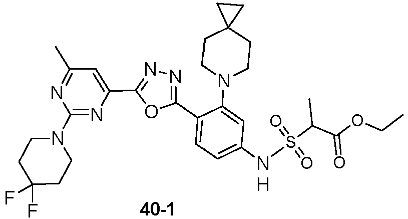

2-(2-(4,4-Difluoropiperidin-1-yl)-6-methylpyrimidin-4-yl)-5-(4-iodo-2-(6-azaspiro[2.5]octan-6-yl)phenyl)-1,3,4-oxadiazole (250 mg, 422 µmol), ethyl 2-sulfamoylpropionate (153 mg, 844 µmol), sarcosine (37.6 mg, 422 µmol), copper(I) iodide (80.4 mg, 422 µmol), and potassium carbonate (175 mg, 1.27 mmol) were dissolved in a *N,N-*dimethylformamide solution (10 mL), and the reactants were allowed to react under nitrogen atmosphere at 140 °C for 1 h. After the reaction was completed, the reaction liquid was quenched with water (25 mL) and extracted three times with ethyl acetate (50 mL). The organic phase was washed with saturated brine (50 mL), dried over anhydrous sodium sulfate, filtered, and concentrated to give a crude product, which was then separated by reverse phase high performance liquid chromatography (separation method: column: Waters Xbridge C18 150 × 25 mm × 5 µm; solvents: A = water + 0.05% (by volume) aqueous ammonia (30.0%), B = acetonitrile; gradient: 16%-46%, 8 min), followed by lyophilization to give ethyl 2-(*N*-(4-(5-(2-(4,4-difluoropiperidin-1-yl)-6-methylpyrimidin-4-yl)-1,3,4-oxadiazol-2-yl)-3-(6-azaspiro[2.5]octan-6-yl)phenyl)sulfamoyl)propionate (100 mg, yield: 36.7%).

LC-MS, M/Z (ESI): 646.5 [M+H]⁺.

### Step 2: Synthesis of (R)-N-(4-(5-(2-(4,4-difluoropiperidin-1-yl)-6-methylpyrimidin-4-yl)-1,3,4-oxadiazol-2-yl)-3-(6-azaspiro[2.5]octan-6-yl)phenyl)-1-hydroxypropane-2-sulfonamide

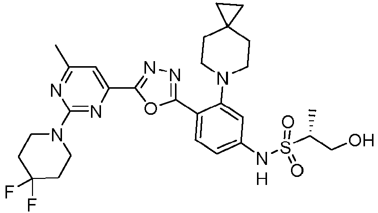

synthesis of (*S*)-*N*-(4-(5-(2-(4,4-difluoropiperidin-1-yl)-6-methylpyrimidin-4-yl)-1,3,4-oxadiazol-2-yl)-3-(6-azaspiro[2.5]octan-6-yl)phenyl)-1-hydroxypropane-2-sulfonamide

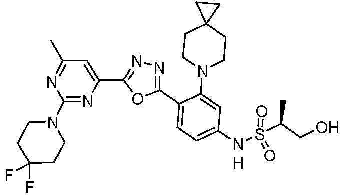

Ethyl 2-(N-(4-(5-(2-(4,4-difluoropiperidin-1-yl)-6-methylpyrimidin-4-yl)-1,3,4-oxadiazol-2-yl)-3-(6-azaspiro[2.5] octan-6-yl)phenyl)sulfamoyl)propionate (150 mg, 232 µmol) was dissolved in tetrahydrofuran (2 mL), and then lithium borohydride (2 M, 140 µL) was added dropwise under nitrogen atmosphere at 0 °C. The mixture was allowed to react at 0 °C for 2 h. After the reaction was completed, the reaction liquid was quenched with a saturated ammonium chloride solution (20 mL) and extracted with ethyl acetate (20 mL × 3). The organic phase was washed with saturated brine (20 mL), dried over anhydrous sodium sulfate, filtered, and concentrated to give a crude product, which was then separated by reverse phase high performance liquid chromatography (separation method: column: Phenomenex luna C18 150 × 70 mm × 10 µm; solvents: A = water + 0.05% (by volume) trifluoroacetic acid (99.0%), B = acetonitrile; gradient: 50%-80%, 10 min), followed by lyophilization to give *N*-(4-(5-(2-(4,4-difluoropiperidin-1-yl)-6-methylpyrimidin-4-yl)-1,3,4-oxadiazol-2-yl)-3-(6-azaspiro [2.5] octan-6-yl)phenyl)-1-hydroxypropane-2-sulfonamide.

The compound was then subjected to resolution (resolution method: column: Chiralpak IG-3 50 × 4.6 mm I.D., 3 µm; solvents: A = carbon dioxide, B = 0.05% (by volume) diethylamine in methanol; gradient: 40% B in A, flow rate = 3 mL/min, detector: PDA, column temperature = 35 °C, column pressure = 100 Bar).

Concentration of peak 1 gave the compound (R)-N-(4-(5-(2-(4,4-difluoropiperidin-1-yl)-6-methylpyrimidin-4-yl)-1,3,4-oxadiazol-2-yl)-3-(6-azaspiro[2.5]octan-6-yl)phenyl)-1-hydroxypropane-2-sulfonamide or (*S*)-*N*-(4-(5-(2-(4,4-difluoropiperidin-1-yl)-6-methylpyrimidin-4-yl)-1,3,4-oxadiazol-2-yl)-3-(6-azaspiro [2.5] octan-6-yl)phenyl)-1-hydroxypropane-2-sulfonamide (9.88 mg, SFC retention time: 0.932 min, yield: 9.88%).

LC-MS, M/Z (ESI): 604.5 [M+H]⁺.

¹H NMR (400 MHz, CDCl3) *δ* 7.91 (d, 1H), 7.20 (s, 1H), 7.04 (s, 1H), 6.84 (d, 1H), 4.03-4.06 (m, 4H), 3.93-3.95 (m, 2H), 3,36-3.38 (m, 1H), 2.95-2.97 (m, 4H), 2.40 (s, 3H), 1.94-2.03 (m, 4H), 1.50 (br. s, 4H), 1.32 (d, 3H), 0.27 (s, 4H).

Concentration of peak 2 gave the compound (S)-N-(4-(5-(2-(4,4-difluoropiperidin-1-yl)-6-methylpyrimidin-4-yl)-1,3,4-oxadiazol-2-yl)-3-(6-azaspiro[2.5]octan-6-yl)phenyl)-1-hydroxypropane-2-sulfonamide or *(R)-N-(4-(5-(2-*(4,4-difluoropiperidin-1-yl)-6-methylpyrimidin-4-yl)-1,3,4-oxadiazol-2-yl)-3-(6-azaspiro [2.5] octan-6-yl)phenyl)-1-hydroxypropane-2-sulfonamide (29.8 mg, SFC retention time: 1.313 min, yield: 29.8%).

LC-MS, M/Z (ESI): 604.5 [M+H]⁺.

¹H NMR (400 MHz, CD₃OD) *δ* 7.90 (d, 1H), 7.32 (s, 1H), 7.14 (s, 1H), 7.02 (d, 1H), 4.10-4.13 (m, 4H), 3.97-3.99 (m, 1H), 3.69-3.74 (m, 1H), 3.35-3.39 (m, 1H), 3.00-3.03 (m, 4H), 2.47 (s, 3H), 2.01-2.07 (m, 4H), 1.59 (br. s, 4H), 1.40 (d, 3H), 0.35 (s, 4H).

The SFC retention time for compound I-40 was 0.932 min, and the SFC retention time for compound I-41 was 1.313 min.

### Example 40: Preparation of Target Compound I-95

### N-(4-(4-(2-(4,4-Difluoropiperidin-1-yl)-6-ethylpyrimidin-4-yl)-1H-pyrazol-1-yl)-3-(6-azaspiro[2.5]octan-6-yl)phenyl)-2-hydroxyethane-1-sulfonamide

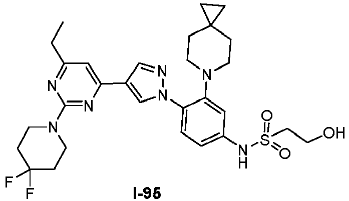

The synthetic route of the target compound **I-95** was as follows:

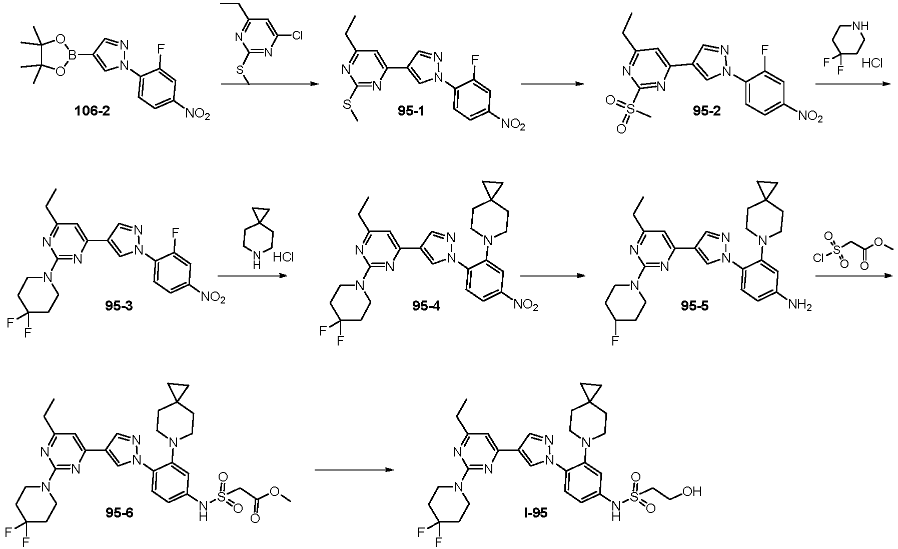

### Step 1: Synthesis of 4-ethyl-6-(1-(2-fluoro-4-nitrophenyl)-1H-pyrazol-4-yl)-2-(methylthio)pyrimidine

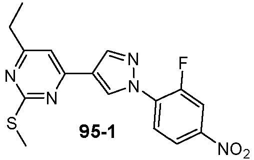

1-(2-Fluoro-4-nitrophenyl)-4-(4,4,5,5-tetramethyl-1,3,2-dioxaborolan-2-yl)-1H-pyrazole (10.0 g, 30.0 mmol), potassium carbonate (12.5 g, 90.0 mmol), [1,1-bis(diphenylphosphino)ferrocene]palladium chloride (2.20 g, 3.00 mmol), and 4-chloro-6-ethyl-2-(methylthio)pyrimidine (5.66 g, 30.0 mmol) were added to anhydrous 1,4-dioxane (100 mL) and water (100 mL), and the mixture was allowed to react under nitrogen atmosphere at 100 °C for 2 h. The reaction liquid was quenched with water (200 mL) and extracted with ethyl acetate (150 mL × 2). The organic phases were combined, washed with saturated brine (100 mL), dried over anhydrous sodium sulfate, filtered, and concentrated, and the residue was separated and purified by a silica gel column (petroleum ether:ethyl acetate (V/V) = 50:1-0:1) to give 4-ethyl-6-(1-(2-fluoro-4-nitrophenyl)-1H-pyrazol-4-yl)-2-(methylthio)pyrimidine (3.70 g, yield: 34.3%).

LC-MS, M/Z (ESI): 360.0 [M+H]⁺.

### Step 2: Synthesis of 4-ethyl-6-(1-(2-fluoro-4-nitrophenyl)-1H-pyrazol-4-yl)-2-(methylsulfonyl)pyrimidine

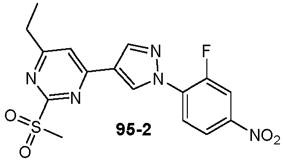

4-Ethyl-6-(1-(2-fluoro-4-nitrophenyl)-1*H*-pyrazol-4-yl)-2-(methylthio)pyrimidine (1.85 g, 5.15 mmol) was dissolved in dichloromethane (50 mL), and then the solution was added to m-chloroperoxybenzoic acid (4.18 g, 20.6 mmol, purity: 85%). The mixture was allowed to react under nitrogen atmosphere at 20 °C for 2 h. The reaction liquid was quenched with a saturated aqueous sodium sulfite solution (500 mL) and extracted with dichloromethane (100 mL × 2). The organic phases were combined, washed with saturated brine (100 mL), dried over anhydrous sodium sulfate, filtered, and concentrated, and the residue was separated and purified by a silica gel column (petroleum ether:ethyl acetate (V/V) = 50:1-0:1) to give 4-ethyl-6-(1-(2-fluoro-4-nitrophenyl)-1*H*-pyrazol-4-yl)-2-(methylsulfonyl)pyrimidine (2.10 g, yield: 52.1%).

LC-MS, M/Z (ESI): 392.1 [M+H]⁺.

### Step 3: Synthesis of 2-(4,4-difluoropiperidin-1-yl)-4-ethyl-6-(1-(2-fluoro-4-nitrophenyl)-1H-pyrazol-4-yl)pyrimidine

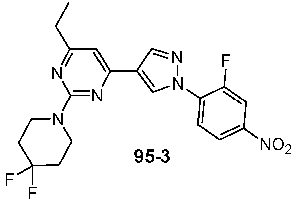

4-Ethyl-6-(1-(2-fluoro-4-nitrophenyl)-1*H*-pyrazol-4-yl)-2-(methylsulfonyl)pyrimidine (1.80 g, 4.60 mmol), potassium carbonate (1.91 g, 13.8 mmol), and 4,4-difluoropiperidine hydrochloride (797 mg, 5.06 mmol) were added to N-methylpyrrolidone (40 mL), and the mixture was allowed to react at 100 °C for 2 h. The reaction liquid was quenched with water (100 mL) and extracted with ethyl acetate (100 mL × 2). The organic phases were combined, washed with saturated brine (100 mL), dried over anhydrous sodium sulfate, filtered, and concentrated, and the residue was separated and purified by a silica gel column (petroleum ether:ethyl acetate (V/V) = 50:1-0:1) to give 2-(4,4-difluoropiperidin-1-yl)-4-ethyl-6-(1-(2-fluoro-4-nitrophenyl)-1*H*-pyrazol-4-yl)pyrimidine (400 mg, yield: 20.1%).

LC-MS, M/Z (ESI): 433.1 [M+H]⁺.

### Step 4: Synthesis of 6-(2-(4-(2-(4,4-difluoropiperidin-1-yl)-6-ethylpyrimidin-4-yl)-1H-pyrazol-1-yl)-5-nitrophenyl)-6-azaspiro[2.5]octane

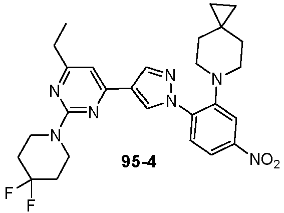

2-(4,4-Difluoropiperidin-1-yl)-4-ethyl-6-(1-(2-fluoro-4-nitrophenyl)-1*H*-pyrazol-4-yl)pyrimidine (350 mg, 809 umol), potassium carbonate (527 mg, 3.82 mmol), and 6-azaspiro[2.5]octane hydrochloride (143 mg, 971 µmol) were added to *N*,*N*-dimethylformamide (10 mL), and the mixture was allowed to react at 140 °C for 2 h. The reaction liquid was quenched with water (50 mL) and extracted with ethyl acetate (100 mL × 2). The organic phases were combined, washed with saturated brine (100 mL), dried over anhydrous sodium sulfate, filtered, and concentrated, and the residue was separated and purified by a silica gel column (petroleum ether:ethyl acetate (V/V) = 50:1-0:1) to give 6-(2-(4-(2-(4,4-difluoropiperidin-1-yl)-6-ethylpyrimidin-4-yl)-1*H*-pyrazol-1-yl)-5-nitrophenyl)-6-azaspiro[2.5] octane (300 mg, yield: 70.8%).

LC-MS, M/Z (ESI): 524.3 [M+H]⁺.

### Step 5: Synthesis of 4-(4-(2-(4,4-difluoropiperidin-1-yl)-6-ethylpyrimidin-4-yl)-1H-pyrazol-1-yl)-3-(6-azaspiro [2.5] octan-6-yl)aniline

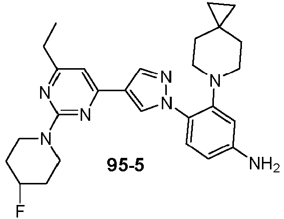

6-(2-(4-(2-(4,4-Difluoropiperidin-1-yl)-6-ethylpyrimidin-4-yl)-1*H*-pyrazol-1-yl)-5-nitrophenyl)-6-azaspiro[2.5]octane (200 mg, 382 µmol) was added to methanol (30.0 mL), and then platinum vanadium on carbon (100 mg, 38.2 µmol) was added. The reactants were allowed to react under hydrogen atmosphere (50 psi) at 25 °C for 6 h. After the reaction was completed, the mixture was filtered, and the filtrate was concentrated to give a crude product, which was then separated by reverse phase high performance liquid chromatography (separation method: column: Waters Xbridge 150 × 25 mm × 5 µm; solvents: A = water + 0.05% (by volume) formic acid (99.0%), B = acetonitrile; gradient: 65%-95%, 7 min), followed by lyophilization to give 4-(4-(2-(4,4-difluoropiperidin-1-yl)-6-ethylpyrimidin-4-yl)-1*H*-pyrazol-1-yl)-3-(6-azaspiro[2.5]octan-6-yl)aniline (150 mg, 304 µmol, yield: 79.6%).

LC-MS, M/Z (ESI): 494.3 [M+H]⁺.

### Step 6: Synthesis of methyl 2-(N-(4-(4-(2-(4,4-difluoropiperidin-1-yl)-6-ethylpyrimidin-4-yl)-1H-pyrazol-1-yl)-3-(6-azaspiro[2.5]octan-6-yl)phenyl)sulfamoyl)acetate

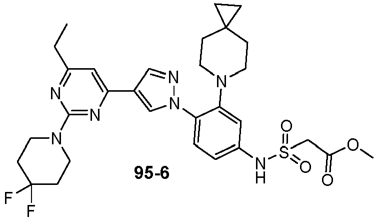

4-(4-(2-(4,4-Difluoropiperidin-1-yl)-6-ethylpyrimidin-4-yl)-1H-pyrazol-1-yl)-3-(6-azaspiro[2.5]octan-6-yl)aniline (146 mg, 296 µmol), potassium phosphate (188 mg, 887 µmol), and methyl 2-(chlorosulfonyl)acetate (51.0 mg, 296 µmol) were added to acetonitrile (20 mL), and the mixture was allowed to react at 20 °C for 2 h. The reaction liquid was quenched with water (20 mL) and extracted with ethyl acetate (25 mL × 2). The organic phases were combined, washed with saturated brine (20 mL), dried over anhydrous sodium sulfate, filtered, and concentrated to give methyl 2-(*N*-(4-(4-(2-(4,4-difluoropiperidin-1-yl)-6-ethylpyrimidin-4-yl)-1*H*-pyrazol-1-yl)-3-(6-azaspiro[2.5]octan-6-yl)phenyl)sulfamoyl)acetate (180 mg, yield: 96.6%).

LC-MS, M/Z (ESI): 630.3 [M+H]⁺.

### Step 7: Synthesis of N-(4-(4-(2-(4,4-difluoropiperidin-1-yl)-6-ethylpyrimidin-4-yl)-1H-pyrazol-1-yl)-3-(6-azaspiro[2.5]octan-6-yl)phenyl)-2-hydroxyethane-1-sulfonamide

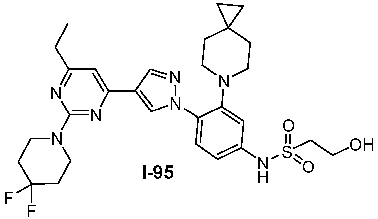

Methyl 2-(N (4-(4-(2-(4,4-difluoropiperidin-1-yl)-6-ethylpyrimidin-4-yl)-1*H*-pyrazol-1-yl)-3-(6-azaspiro[2.5]octan-6-yl)phenyl)sulfamoyl)acetate (180 mg, 286 µmol) was dissolved in tetrahydrofuran (20 mL), and then lithium borohydride (2 M, 171 µL) was added dropwise under nitrogen atmosphere at 0 °C. The mixture was allowed to react at 40 °C for 2 h. After the reaction was completed, the reaction liquid was quenched with a saturated ammonium chloride solution (50 mL) and extracted with ethyl acetate (30 mL × 3). The organic phase was washed with saturated brine (50 mL), dried over anhydrous sodium sulfate, filtered, and concentrated to give a crude product, which was then separated by reverse phase high performance liquid chromatography (separation method: column: Phenomenex luna C18 150 × 25 mm × 5 µm; solvents: A = water + 0.05% (by volume) formic acid (99.0%), B = acetonitrile; gradient: 65%-95%, 7 min), followed by lyophilization to give N-(4-(4-(2-(4,4-difluoropiperidin-1-yl)-6-ethylpyrimidin-4-yl)-1*H*-pyrazol-1-yl)-3-(6-azaspiro[2.5]octan-6-yl)phenyl)-2-hydroxyethane-1-sulfonamide (30.0 mg, yield: 17.3%).

¹H NMR (400 MHz, CDCl₃) *δ* 8.98 (s, 1H), 8.12 (s, 1H), 7.58 (d, 1H), 7.10 (s, 1H), 6.92-6.94 (m, 1H), 6.65 (s, 1H), 6.59 (s, 1H), 4.04-4.19 (m, 6H), 3.33 (t, 2H), 2.81-2.83 (m, 4H), 2.66 (q, 2H), 2.45 (s, 1H), 1.98-2.07 (m, 4H), 1.42-1.44 (m, 4H), 1.31 (t, 3H), 0.42 (s, 4H).

LC-MS, M/Z (ESI): 602.3 [M+H]⁺.

### Example 41: Preparation of Target Compound I-107

### N-(4-(4-(2-(Difluoromethyl)-6-(4,4-difluoropiperidin-1-yl)pyridin-4-yl)-1H-pyrazol-1-yl)-3-(6-azaspiro[2.5]octan-6-yl)phenyl)-2-hydroxyethane-1-sulfonamide

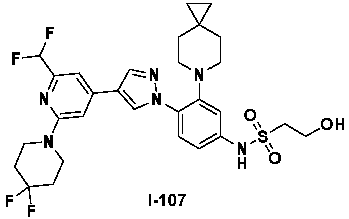

The synthetic route of the target compound **I-107** was as follows:

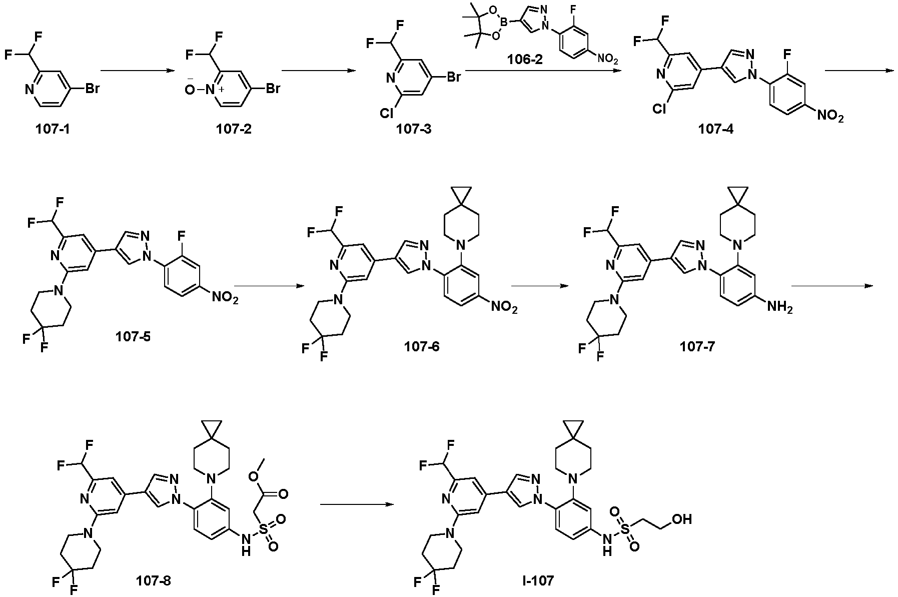

### Step 1: Synthesis of 4-bromo-2-(difluoromethyl)pyridine 1-oxide

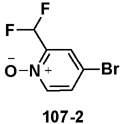

4-Bromo-2-(difluoromethyl)pyridine (5 g, 24 mmol) was added to 80 mL of dichloromethane, and the mixture was cooled to 0 °C. *m*-Chloroperoxybenzoic acid (12.5 g, 72 mmol) was then added, and the mixture was stirred at room temperature overnight. A saturated sodium bicarbonate solution (50 mL) and a sodium sulfite solution (100 mL) were added to quench the reaction, and then the mixture was extracted with dichloromethane (50 mL × 2). The organic phase was concentrated to dryness, and the residue was separated and purified by a silica gel column (PE:EA (V/V) = 1:1) to give 4-bromo-2-(difluoromethyl)pyridine 1-oxide (4.5 g, yield: 83%).

LC-MS, M/Z (ESI): 224.2 [M+H]⁺.

### Step 2: Synthesis of 4-bromo-2-chloro-6-(difluoromethyl)pyridine

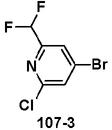

4-Bromo-2-(difluoromethyl)pyridine-1-oxide (7 g, 31.25 mmol) was added to phosphorus oxychloride (50 mL) at 0 °C, and the mixture was stirred at 90 °C for 5 h and then concentrated under vacuum. Water (200 mL) was added for dilution, and then the mixture was adjusted to neutral with a saturated aqueous sodium bicarbonate solution, extracted with ethyl acetate (50 mL × 3), washed with saturated brine (100 mL), filtered, dried over anhydrous sodium sulfate, concentrated by rotary evaporation, and subjected to column chromatography (PE/EA (V/V) = 10/1) to give 4-bromo-2-chloro-6-(difluoromethyl)pyridine (3 g, yield: 39.6%)

¹HNMR (400 MHz, Chloroform-d) δ 7.76-7.63 (m, 1H), 7.60-7.45 (m, 1H), 6.56 (td, 1H).

LC-MS, M/Z (ESI): 242.0 [M+H]⁺.

### Step 3: Synthesis of 2-chloro-6-(difluoromethyl)-4-(1-(2-fluoro-4-nitrophenyl)-1H-pyrazol-4-yl)pyridine

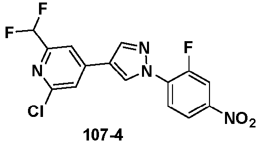

4-Bromo-2-chloro-6-(difluoromethyl)pyridine (1.2 g, 4.9 mmol), 1-(2-fluoro-4-nitrophenyl)-4-(4,4,5,5-tetramethyl-1,3,2-dioxaboron-2-yl)-1*H*-pyrazole (1.8 g, 5.4 mmol), and potassium carbonate (1.7 g, 12.3 mmol) were dissolved in 1,4-dioxane (24 mL) and water (4.8 mL), and then [1,1'-bis(diphenylphosphino)ferrocene]dichloropalladium(II) (366 mg, 0.5 mmol) was added. The mixture was purged three times with argon and then allowed to react at 90 °C for 16 h. After the reaction was completed, the mixture was diluted with water (100 mL) and extracted three times with ethyl acetate (50 mL). The organic phases were combined, washed with saturated brine (100 mL), dried over anhydrous sodium sulfate, filtered, and concentrated to give a crude product. The crude product was subjected to column chromatography (PE/EA (V/V) = 3/1) to give 2-chloro-6-(difluoromethyl)-4-(1-(2-fluoro-4-nitrophenyl)-1*H-*pyrazol-4-yl)pyridine (0.5 g, yield: 27.5%).

¹H NMR (400 MHz, Chloroform-d) δ 8.73 (d, 1H), 8.34-8.28 (m, 2H), 8.24-8.17 (m, 2H), 7.64 (dt, 1H), 7.53 (d, 1H), 6.64 (t, 1H).

LC-MS, M/Z (ESI): 368.9 [M+H]⁺.

### Step 4: Synthesis of 2-(difluoromethyl)-6-(4,4-difluoropiperidin-1-yl)-4-(1-(2-fluoro-4-nitrophenyl)-1H-pyrazol-4-yl)pyridine

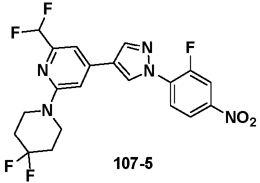

2-Chloro-6-(difluoromethyl)-4-(1-(2-fluoro-4-nitrophenyl)-1*H*-pyrazol-4-yl)pyridine, 4,4-difluoropiperidine hydrochloride (361.2 mg, 2.3 mmol), and 2,2'-bis(diphenylphosphino)-1,1'-binaphthyl (124.5 mg, 0.2 mmol) were dissolved in 1,4-dioxane (11 mL), and then cesium carbonate (1.46 g, 4.5 mmol) and palladium(II) acetate (44.9 mg, 0.2 mmol) were added. The mixture was purged three times with argon and then stirred at 100 °C for 16 h. After the reaction was completed, the mixture was diluted with water (100 mL) and extracted three times with ethyl acetate (50 mL). The organic phases were combined, washed with saturated brine (100 mL), dried over anhydrous sodium sulfate, filtered, and concentrated to give 2-(difluoromethyl)-6-(4,4-difluoropiperidin-1-yl)-4-(1-(2-fluoro-4-nitrophenyl)-1*H*-pyrazol-4-yl)pyridine (600 mg, yield: 88.8%).

LC-MS, M/Z (ESI): 453.9 [M+H]⁺.

### Step 5: Synthesis of 6-(2-(4-(2-(difluoromethyl)-6-(4,4-difluoropiperidin-1-yl)pyridin-4-yl)-1H-pyrazol-1-yl)-5-nitrophenyl)-6-azaspiro[2.5]octane

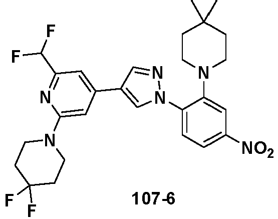

2-(Difluoromethyl)-6-(4,4-difluoropiperidin-1-yl)-4-(1-(2-fluoro-4-nitrophenyl)-1*H*-pyrazol-4-yl)pyridine (700 mg, 1.5 mmol), 6-azaspiro[2.5]octane hydrochloride (441.2 mg, 3.0 mmol), and cesium carbonate (1.47 g, 4.5 mmol) were dissolved in dimethylsulfoxide (14 mL), and the reaction liquid was allowed to react at 120 °C for 16 h. After the reaction was completed, the system was cooled to room temperature. Water (100 mL) was then added to the system, and the mixture was extracted with ethyl acetate (30 mL × 3). The organic phases were combined, washed with saturated brine (100 mL), dried over anhydrous sodium sulfate, filtered, and concentrated to give a crude product. The crude product was subjected to column chromatography (PE/EA (v/v) = 3/1) to give 6-(2-(4-(2-(difluoromethyl)-6-(4,4-difluoropiperidin-1-yl)pyridin-4-yl)-1*H*-pyrazol-1-yl)-5-nitrophenyl)-6-azaspiro[2.5]octane (500 mg, yield: 59.5%).

LC-MS, M/Z (ESI): 545.2 [M+H]⁺.

### Step 6: Synthesis of 4-(4-(2-(difluoromethyl)-6-(4,4-difluoropiperidin-1-yl)pyridin-4-yl)-1H-pyrazol-1-yl)-3-(6-azaspiro [2.5] octan-6-yl)aniline

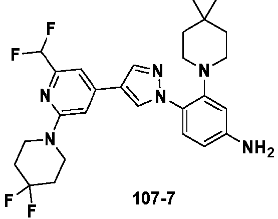

6-(2-(4-(2-(Difluoromethyl)-6-(4,4-difluoropiperidin-1-yl)pyridin-4-yl)-1*H*-pyrazol-1-yl)-5-nitrophenyl)-6-azaspiro[2.5]octane (450 mg, 0.8 mmol) was dissolved in methanol (10 mL), and the solution was purged three times with nitrogen. Wet palladium on carbon (300 mg, purity: 10%) was then added, and the mixture was allowed to react under hydrogen atmosphere (15 psi) at room temperature for 16 h. After the reaction was completed, the mixture was diluted with methanol (10 mL), filtered, and concentrated under reduced pressure to give the compound 4-(4-(2-(difluoromethyl)-6-(4,4-difluoropiperidin-1-yl)pyridin-4-yl)-1*H*-pyrazol-1-yl)-3-(6-azaspiro[2.5]octan-6-yl)aniline (300 mg, yield: 70.6%).

LC-MS, M/Z (ESI): 515.2 [M+H]⁺.

### Step 7: Synthesis of methyl 2-(N (4-(4-(2-(difluoromethyl)-6-(4,4-difluoropiperidin-1-yl)pyridin-4-yl)-1H-pyrazol-1-yl)-3-(6-azaspiro[2.5]octan-6-yl)phenyl)sulfamoyl)acetate

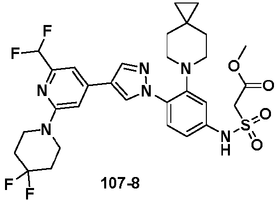

4-(4-(2-(Difluoromethyl)-6-(4,4-difluoropiperidin-1-yl)pyridin-4-yl)-1*H*-pyrazol-1-yl)-3-(6-azaspiro[2.5]octan-6-yl)aniline and potassium phosphate (424.5 mg, 2.0 mmol) were dissolved in acetonitrile (5 mL), and then methyl 2-(chlorosulfonyl)acetate (120.8 mg, 0.7 mmol) was added to the reaction liquid at 0 °C. The mixture was allowed to react at room temperature for 3 h. After the reaction was completed, the mixture was diluted with water (50 mL) and extracted with ethyl acetate (20 mL × 3). The organic phases were combined, washed with saturated brine (50 mL), dried over anhydrous sodium sulfate, filtered, and concentrated to give a crude product. The crude product was purified by a reverse phase column (0.1% FA) to give methyl 2-(N-(4-(4-(2-(difluoromethyl)-6-(4,4-difluoropiperidin-1-yl)pyridin-4-yl)-1*H*-pyrazol-1-yl)-3-(6-azaspiro[2.5]octan-6-yl)phenyl)sulfatnoyl)acetate (250 mg, yield: 79.4%).

LC-MS, M/Z (ESI): 651.2 [M+H]⁺.

### Step 8: Synthesis of N-(4-(4-(2-(difluoromethyl)-6-(4,4-difluoropiperidin-1-yl)pyridin-4-yl)-1H-pyrazol-1-yl)-3-(6-azaspiro[2.5]octan-6-yl)phenyl)-2-hydroxyethane-1-sulfonamide

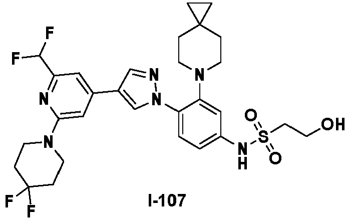

Methyl 2-(*N*-(4-(4-(2-(difluoromethyl)-6-(4,4-difluoropiperidin-1-yl)pyridin-4-yl)-1*H*-pyrazol-1-yl)-3-(6-azaspiro [2.5]octan-6-yl)phenyl)sulfamoyl)acetate (190 mg, 0.3 mmol) and calcium chloride (333 mg, 3 mmol) were dissolved in ethanol (5.7 mL) and tetrahydrofuran (1.9 mL), and then sodium borohydride (113.5 mg, 3 mmol) was added at 0 °C. The mixture was allowed to react at room temperature for 5 h. After the reaction was completed, the reaction liquid was quenched with a saturated aqueous ammonium chloride solution (30 mL) and water (30 mL) and extracted with ethyl acetate (30 mL × 3). The organic phases were combined, washed with saturated brine (60 mL), dried over anhydrous sodium sulfate, filtered, and concentrated to give a crude product, which was then purified by a reverse phase column (0.1% FA), followed by lyophilization to give *N*-(4-(4-(2-(difluoromethyl)-6-(4,4-difluoropiperidin-1-yl)pyridin-4-yl)-1*H*-pyrazol-1-yl)-3-(6-azaspiro[2.5]octan-6-yl)phenyl)-2-hydroxyethane-1-sulfonamide (104 mg, yield: 57.2%).

¹H NMR (400 MHz, DMSO-*d*₆) δ 8.90 (s, 1H), 8.23 (s, 1H), 7.42 (d, 1H), 7.38 (d, 1H), 7.06 (d, 1H), 7.03 (d, 1H), 6.95 (dd, 1H), 6.77 (t, 1H), 3.77 (t, 2H), 3.71-3.62 (m, 4H), 3.29 (d, 2H), 2.70 (t, 4H), 2.05 (td, 4H), 1.31 (t, 4H), 0.24 (s, 4H).

LC-MS, M/Z (ESI): 623.2 [M+H]⁺.

### Example 42: Preparation of Target Compound I-115

### N-(4-(4-(6-Methyl-2-(3,3,3-trifluoropropoxy)pyrimidin-4-yl)-1H-pyrazol-1-yl)-3-(6-azaspiro[2.5]octan-6-yl)phenyl)-2-hydroxyethane-1-sulfonamide

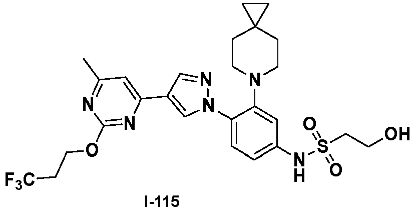

The synthetic route of the target compound **I-115** was as follows:

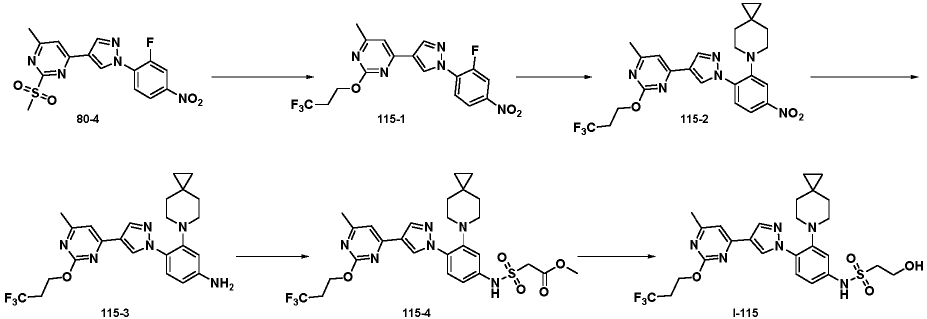

### Step 1: Synthesis of 4-(1-(2-fluoro-4-nitrophenyl)-1H-pyrazol-4-yl)-6-methyl-2-(3,3,3-trifluoropropoxy)pyrimidine

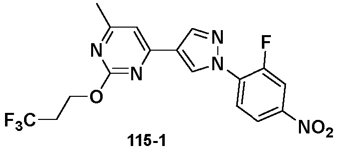

4-(1-(2-Fluoro-4-nitrophenyl)-1*H*-pyrazol-4-yl)-6-methyl-2-(methylsulfonyl)pyrimidine (1 g, 2.65 mmol) was added to 10 mL of *N*,*N*-dimethylformamide, and then 3,3,3-trifluoropropanol (0.61 g, 5.3 mmol) and potassium carbonate (1.831 g, 13.25 mmol) were added. The mixture was heated to 60 °C and allowed to react overnight. LCMS showed that the starting materials were completely reacted. The reaction liquid was concentrated to dryness, and the residue was separated and purified by a silica gel column (volume ratio: PE:DCM:EA = 3:3:1) to give 4-(1-(2-fluoro-4-nitrophenyl)-1*H*-pyrazol-4-yl)-6-methyl-2-(3,3,3-trifluoropropoxy)pyrimidine (0.9 g, yield: 83%).

LC-MS, M/Z (ESI): 412.1 [M+H]⁺.

### Step 2: Synthesis of 6-(2-(4-(6-methyl-2-(3,3,3-trifluoropropoxy)pyrimidin-4-yl)-1H-pyrazol-1-yl)-5-nitrophenyl)-6-azaspiro[2.5]octane

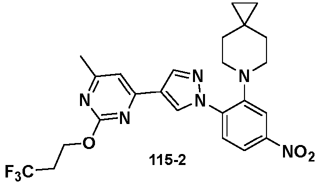

4-(1-(2-Fluoro-4-nitrophenyl)-1*H*-pyrazol-4-yl)-6-methyl-2-(3,3,3-trifluoropropoxy)pyrimidine (0.9 g, 2.188 mmol) was added to 3 mL of dimethylsulfoxide, and then 6-azaspiro[2.5]octane hydrochloride (0.808 g, 5.47 mmol) and potassium carbonate (1.51 g, 10.94 mmol) were added. The mixture was heated to 140 °C and allowed to react for 8 h. LCMS showed that the starting materials were completely reacted. The reaction liquid was poured into 50 mL of water, and the mixture was filtered and concentrated to give 6-(2-(4-(6-methyl-2-(3,3,3-trifluoropropoxy)pyrimidin-4-yl)-1*H*-pyrazol-1-yl)-5-nitrophenyl)-6-azaspiro[2.5]octane (0.9 g, yield: 82%).

LC-MS, M/Z (ESI): 503.2 [M+H]⁺.

### Step 3: Synthesis of 4-(4-(6-methyl-2-(3,3,3-trifluoropropoxy)pyrimidin-4-yl)-1H-pyrazol-1-yl)-3-(6-azaspiro [2.5]octan-6-yl)aniline

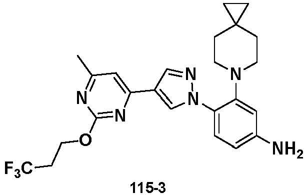

6-(2-(4-(6-Methyl-2-(3,3,3-trifluoropropoxy)pyrimidin-4-yl)-1*H*-pyrazol-1-yl)-5-nitrophenyl)-6-azaspiro[2.5]octane (0.9 g, 1.79 mmol) was added to 20 mL of ethanol, and then iron powder (1.761 g, 31.5 mmol) and ammonium chloride (0.843 g, 15.76 mmol) were added. The mixture was heated to 90 °C and allowed to react for 6 h. LCMS showed that the starting materials were completely reacted. The reaction liquid was filtered and then washed three times with 20 mL of ethanol. The filtrate was concentrated to dryness, and the residue was separated and purified by a silica gel column (volume ratio: PE:EA = 3:1) to give 4-(4-(6-methyl-2-(3,3,3-trifluoropropoxy)pyrimidin-4-yl)-1*H*-pyrazol-1-yl)-3-(6-azaspiro[2.5]octan-6-yl)aniline (0.7 g, yield: 83%).

LC-MS, M/Z (ESI): 473.2 [M+H]⁺.

### Step 4: Synthesis of methyl 2-(N-(4-(4-(6-methyl-2-(3,3,3-trifluoropropoxy)pyrimidin-4-yl)-1H-pyrazol-1-yl)-3-(6-azaspiro[2.5]octan-6-yl)phenyl)sulfamoyl)acetate

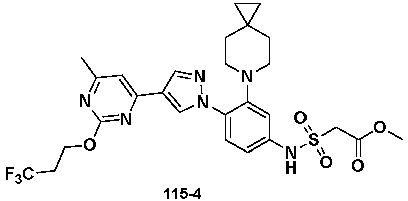

4-(4-(6-Methyl-2-(3,3,3-trifluoropropoxy)pyrimidin-4-yl)-1*H*-pyrazol-1-yl)-3-(6-azaspiro[2.5]octan-6-yl)aniline (0.7 g, 1.481 mmol) was added to 9 mL of acetonitrile, and then potassium phosphate (1.57 g, 7.41 mmol) and methyl 2-(chlorosulfonyl)acetate (0.384 g, 2.22 mmol) were added. The mixture was allowed to react at room temperature for 1 h. LCMS showed that the starting materials were completely reacted. The reaction liquid was concentrated to dryness, and the residue was separated and purified by a silica gel column (volume ratio: PE:EA = 5:1) to give methyl 2-(*N*-(4-(4-(6-methyl-2-(3,3,3-trifluoropropoxy)pyrimidin-4-yl)-1*H*-pyrazol-1-yl)-3-(6-azaspiro[2.5]octan-6-yl)phenyl)sulfamoyl)acetate (0.8 g, yield: 89%).

LC-MS, M/Z (ESI): 609.2 [M+H]⁺.

### Step 5: Synthesis of N-(4-(4-(6-methyl-2-(3,3,3-trifluoropropoxy)pyrimidin-4-yl)-1H-pyrazol-1-yl)-3-(6-azaspiro [2.5]octan-6-yl)phenyl)-2-hydroxyethane-1-sulfonamide

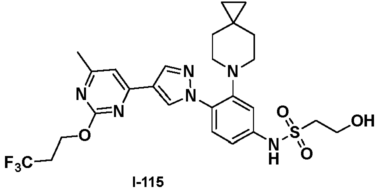

Methyl 2-(*N*-(4-(4-(6-methyl-2-(3,3,3-trifluoropropoxy)pyrimidin-4-yl)-1*H*-pyrazol-1-yl)-3-(6-azaspiro[2.5]octan-6-yl)phenyl)sulfamoyl)acetate (0.2 g, 0.329 mmol) was added to 5 mL of ethanol and 5 mL of tetrahydrofuran, and then sodium borohydride (0.124 g, 3.29 mmol) and calcium chloride (0.182 g, 1.643 mmol) were added. The mixture was allowed to react at room temperature for 1 h. LCMS showed that the starting materials were completely reacted. Saturated ammonium chloride (1 mL) was added to quench the reaction. The reaction liquid was then concentrated to dryness, and the residue was separated and purified by a silica gel column (PE:EA (V/V) = 1:1) to give N-(4-(4-(6-methyl-2-(3,3,3-trifluoropropoxy)pyrimidin-4-yl)-1*H*-pyrazol-1-yl)-3-(6-azaspiro[2.5]octan-6-yl)phenyl)-2-hydroxyethane-1-sulfonamide (103 mg, yield: 50.4%).

¹H NMR (400 MHz, CDCl₃) δ 8.97 (s, 1H), 8.33 (s, 1H), 7.42 (t, 2H), 7.04 (d, 1H), 6.93-6.96 (m, 1H), 4.55 (t, 2H), 3.75 (t, 2H), 3.31 (t, 2H), 2.79-2.84 (m, 2H), 2.67 (t, 4H), 2.34 (s, 3H), 1.29(s, 4H), 0.22 (s, 4H).

LC-MS, M/Z (ESI): 581.2 [M+H]⁺.

### Example 43: Preparation of Target Compound I-117

### N-(4-(4-(2-(4,4-Difluoropiperidin-1-yl)-6-methylpyrimidin-4-yl)-1H-pyrazol-1-yl)-3-(4-fluoro-4-methylpiperidin-1-yl)phenyl)-2-hydroxyethane-1-sulfonamide

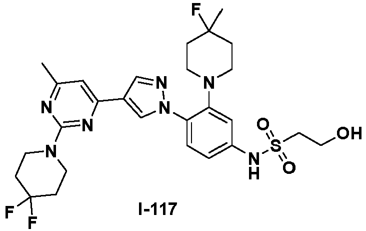

The synthetic route of the target compound **I-117** was as follows:

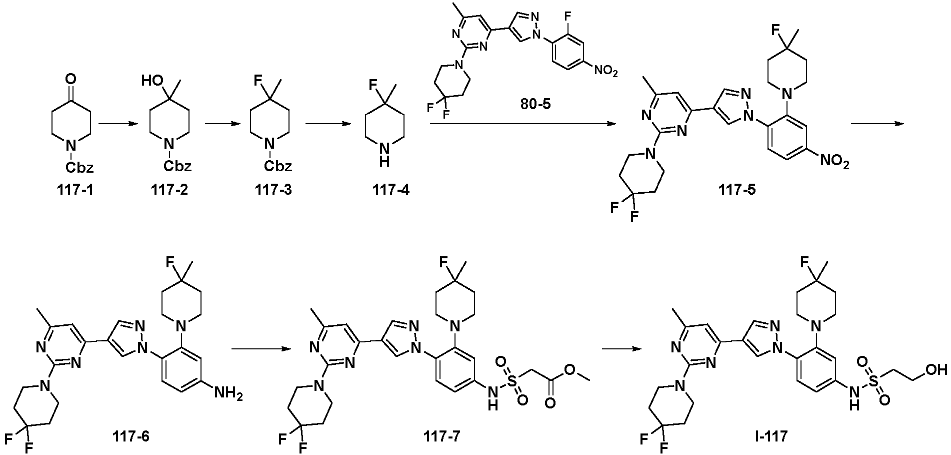

### Step 1: Synthesis of benzyl 4-hydroxy-4-methylpiperidine-1-carboxylate

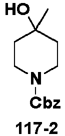

To a solution of benzyl 4-oxopiperidine-1-carboxylate (1 g, 4.3 mmol) in tetrahydrofuran (10 mL) was added dropwise methylmagnesium chloride (8.6 mL, 8.6 mmol, a 1 M solution in tetrahydrofuran) at -10 °C, and then the mixture was allowed to react at 0 °C for 1 h. The reaction liquid was added dropwise and slowly to a saturated aqueous ammonium chloride solution (20 mL), and then the mixture was extracted with ethyl acetate (10 mL × 3). The organic phase was washed with saturated brine (20 mL × 2), dried over anhydrous sodium sulfate, filtered, and concentrated to give a crude product. The crude product was purified by column chromatography (petroleum ether/ethyl acetate (V/V) = 1/0-1/2) to give benzyl 4-hydroxy-4-methylpiperidine-1-carboxylate (0.9 g, yield: 84.2%). LC-MS, M/Z (ESI): 250.2 [M+H]⁺.

### Step 2: Synthesis of benzyl 4-fluoro-4-methylpiperidine-1-carboxylate

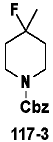

To a solution of benzyl 4-hydroxy-4-methylpiperidine-1-carboxylate (0.9 g, 3.6 mmol) in dichloromethane (9 mL) was added slowly diethylaminosulfur trifluoride (870 mg, 5.4 mmol) at 0 °C, and then the mixture was stirred at 25 °C for 8 h. The reaction liquid was added dropwise and slowly to water (20 mL), and the mixture was extracted with dichloromethane (5 mL × 3). The organic phase was dried over anhydrous sodium sulfate, filtered, and concentrated to give a crude product. The crude product was purified by column chromatography (petroleum ether/ethyl acetate (V/V) = 1/0-1/1) to give benzyl 4-fluoro-4-methylpiperidine-1-carboxylate (0.5 g, yield: 55%).

LC-MS, M/Z (ESI): 252.5 [M+H]⁺.

### Step 3: Synthesis of 4-fluoro-4-methylpiperidine

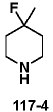

To a solution of benzyl 4-fluoro-4-methylpiperidine-1-carboxylate (0.5 g, 2.0 mmol) in methanol (5 mL) was added wet palladium on carbon (50 mg, content: 10%), and the mixture was purged 3 times with hydrogen and then stirred under hydrogen atmosphere (15 psi) at room temperature for 5 h. The reaction liquid was filtered and concentrated to give 4-fluoro-4-methylpiperidine (0.2 g, yield: 85.8%).

LC-MS, M/Z (ESI): 118.2 [M+H]⁺.

### Step 4: Synthesis of 2-(4,4-difluoropiperidin-1-yl)-4-(1-(2-(4-fluoro-4-methylpiperidin-1-yl)-4-nitrophenyl)-1H-pyrazol-4-yl)-6-methylpyrimidine

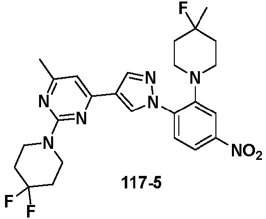

To a solution of 2-(4,4-difluoropiperidin-1-yl)-4-(1-(2-fluoro-4-nitrophenyl)-1*H*-pyrazol-4-yl)-6-methylpyrimidine (0.2 g, 0.5 mmol) in dimethylsulfoxide (2 mL) were added 4-fluoro-4-methylpiperidine (117 mg, 1.0 mmol) and potassium carbonate (207 mg, 1.5 mmol), and then the mixture was allowed to react under microwave conditions at 140 °C for 8 h. Water (20 mL) was added to the reaction liquid, and the mixture was extracted with ethyl acetate (10 mL × 3). The organic phase was washed with saturated brine (10 mL × 2), dried over anhydrous sodium sulfate, filtered, and concentrated to give a crude product. The crude product was purified by column chromatography (petroleum ether/ethyl acetate (V/V) = 1/0-1/3) to give 2-(4,4-difluoropiperidin-1-yl)-4-(1-(2-(4-fluoro-4-methylpiperidin-1-yl)-4-nitrophenyl)-1*H*-pyrazol-4-yl)-6-methylpyrimidine (0.2 g, yield: 80.5%).

LC-MS, M/Z (ESI): 520.2 [M+H]⁺.

### Step 5: Synthesis of 4-(4-(2-(4,4-difluoropiperidin-1-yl)-6-methylpyrimidin-4-yl)-1H-pyrazol-1-yl)-3-(4-fluoro-4-methylpiperidin-1-yl)aniline

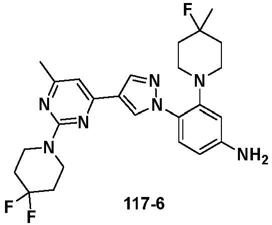

To a solution of 2-(4,4-difluoropiperidin-1-yl)-4-(1-(2-(4-fluoro-4-methylpiperidin-1-yl)-4-nitrophenyl)-1*H-*pyrazol-4-yl)-6-methylpyrimidine (0.2 g, 0.4 mmol) in ethanol (4 mL) were added zinc powder (131 mg, 2 mmol) and ammonium chloride (214 mg, 4 mmol), and then the mixture was stirred at 85 °C for 2 h. Water (50 mL) and ethyl acetate (10 mL) were added to the reaction liquid, and the mixture was filtered. The filtrate was extracted with ethyl acetate (10 mL × 3). The organic phase was washed with saturated brine (10 mL × 2), dried over anhydrous sodium sulfate, filtered, and concentrated to give a crude product. The crude product was purified by column chromatography (petroleum ether/ethyl acetate (V/V) = 1/0-0/1) to give 4-(4-(2-(4,4-difluoropiperidin-1-yl)-6-methylpyrimidin-4-yl)-1*H*-pyrazol-1-yl)-3-(4-fluoro-4-methylpiperidin-1-yl)aniline (130 mg, yield: 69%).

LC-MS, M/Z (ESI): 486.3 [M+H]⁺.

### Step 6: Synthesis of methyl 2-(N-(4-(4-(2-(4,4-difluoropiperidin-1-yl)-6-methylpyrimidin-4-yl)-1H-pyrazol-1-yl)-3-(4-fluoro-4-methylpiperidin-1-yl)phenyl)sulfamoyl)acetate

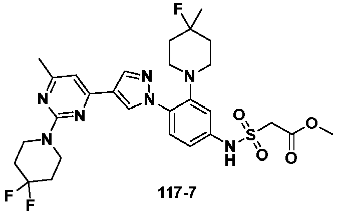

To a solution of 4-(4-(2-(4,4-difluoropiperidin-1-yl)-6-methylpyrimidin-4-yl)-1*H*-pyrazol-1-yl)-3-(4-fluoro-4-methylpiperidin-1-yl)aniline (130 mg, 0.3 mmol) in acetonitrile (2 mL) was added potassium phosphate (191 mg, 0.9 mmol), and then methyl 2-(chlorosulfonyl)acetate (69 mg, 0.4 mmol) was added dropwise and slowly. The mixture was stirred at 25 °C for 2 h. Water (10 mL) was added slowly to the reaction liquid, and the mixture was extracted with ethyl acetate (5 mL × 3). The organic phase was washed with saturated brine (5 mL × 3), dried over anhydrous sodium sulfate, filtered, and concentrated to give a crude product. The crude product was purified by column chromatography (petroleum ether/ethyl acetate (V/V) = 1/0-1/1) to give methyl 2-(*N*-(4-(4-(2-(4,4-difluoropiperidin-1-yl)-6-methylpyrimidin-4-yl)-1*H*-pyrazol-1-yl)-3-(4-fluoro-4-methylpiperidin-1-yl)phenyl)sulfatnoyl)acetate (100 mg, yield: 60%).

LC-MS, M/Z (ESI): 622.4 [M+H]⁺.

### Step 7: Synthesis of N-(4-(4-(2-(4,4-difluoropiperidin-1-yl)-6-methylpyrimidin-4-yl)-1H-pyrazol-1-yl)-3-(4-fluoro-4-methylpiperidin-1-yl)phenyl)-2-hydroxyethane-1-sulfonamide

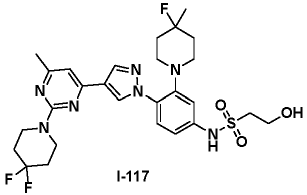

To a solution of methyl 2-(*N*-(4-(4-(2-(4,4-difluoropiperidin-1-yl)-6-methylpyrimidin-4-yl)-1*H*-pyrazol-1-yl)-3-(4-fluoro-4-methylpiperidin-1-yl)phenyl)sulfamoyl)acetate (100 mg, 0.2 mmol) in tetrahydrofuran (1 mL) were added calcium chloride (222 mg, 2 mmol) and sodium borohydride (76 mg, 2 mmol), and the mixture was stirred at 25 °C for 18 h. The reaction liquid was added dropwise and slowly to a saturated aqueous ammonium chloride solution (10 mL), and ethyl acetate (5 mL) was added. The mixture was then filtered. The filtrate was extracted with ethyl acetate (5 mL × 3), and the organic phase was washed with saturated brine (5 mL × 2), dried over anhydrous sodium sulfate, filtered, and concentrated to give a crude product. The crude product was subjected to preparative reverse phase chromatography (column: Phenomenex Synergi C18 100 × 25 mm × 4 µm; solvents: A = water + 0.1 vol% aqueous ammonia (99%), B = acetonitrile; gradient: 5%-95%, 7 min) to give N-(4-(4-(2-(4,4-difluoropiperidin-1-yl)-6-methylpyrimidin-4-yl)-1*H*-pyrazol-1-yl)-3-(4-fluoro-4-methylpiperidin-1-yl)phenyl)-2-hydroxyethane-1-sulfonamide (31.8 mg, yield: 33%).

¹H NMR (400 MHz, dmso) δ 8.90 (s, 1H), 8.31 (s, 1H), 7.45 (d, 1H), 7.05 (d, 1H), 7.01-6.93 (m, 2H), 4.02-3.88 (m, 4H), 3.77 (t, 2H), 3.30 (d, 2H), 2.70 (dt, 4H), 2.32 (s, 3H), 2.07-1.89 (m, 4H), 1.84-1.49 (m, 5H), 1.29 (d, 3H). LC-MS, M/Z (ESI): 594.1 [M+H]⁺.

### Example 44: Preparation of Target Compound I-118

### N-(4-(4-(2-(3,3-Difluoropiperidin-1-yl)-6-methylpyrimidin-4-yl)-1H-pyrazol-1-yl)-3-(6-azaspiro[2.5]octan-6-yl)phenyl)-2-hydroxyethane-1-sulfonamide

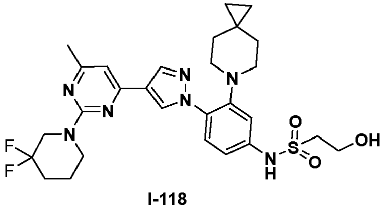

The synthetic route of the target compound **I-118** was as follows:

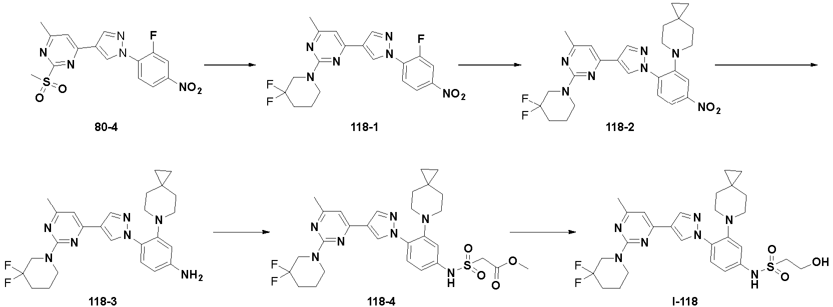

### Step 1: Synthesis of 2-(3,3-difluoropiperidin-1-yl)-4-(1-(2-fluoro-4-nitrophenyl)-1H-pyrazol-4-yl)-6-methylpyrimidine

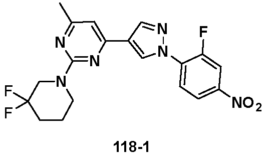

4-(1-(2-Fluoro-4-nitrophenyl)-1H-pyrazol-4-yl)-6-methyl-2-(methylsulfonyl)pyrimidine (1.80 g, 4.95 mmol) and potassium carbonate (2.05 g, 14.86 mmol) were dissolved in DMSO (20 mL), and then 3,3-difluoropiperidine (900 mg, 7.43 mmol) was added slowly. The mixture was stirred at 50 °C for 18 h. After the reaction was completed, the mixture was diluted with water (20 mL) and extracted three times with dichloromethane (20 mL). The organic phases were combined, washed with saturated brine (30 mL), dried over anhydrous sodium sulfate, filtered, and concentrated to give a crude product. The crude product was subjected to column chromatography (PE/EA (v/v) = 3/1) to give 2-(3,3-difluoropiperidin-1-yl)-4-(1-(2-fluoro-4-nitrophenyl)-1H-pyrazol-4-yl)-6-methylpyrimidine (1.90 g, yield: 95%).

LC-MS, M/Z (ESI): 419.1 [M+H]⁺.

### Step 2: Synthesis of 6-(2-(4-(2-(3,3-difluoropiperidin-1-yl)-6-methylpyrimidin-4-yl)-1H-pyrazol-1-yl)-5-nitrophenyl)-6-azaspiro[2.5]octane

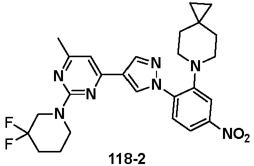

2-(3,3-Difluoropiperidin-1-yl)-4-(1-(2-fluoro-4-nitrophenyl)-1*H*-pyrazol-4-yl)-6-methylpyrimidine (1.50 g, 3.71 mmol) and potassium carbonate (1.54 g, 11.13 mmol) were dissolved in DMSO (15 mL), and then 6-azaspiro[2.5]octane hydrochloride (822 mg, 5.56 mmol) was added slowly. The mixture was stirred at 140 °C for 8 h. After the reaction was completed, the mixture was diluted with water (20 mL) and extracted three times with dichloromethane (20 mL). The organic phases were combined, washed with saturated brine (30 mL), dried over anhydrous sodium sulfate, filtered, and concentrated to give a crude product. The crude product was subjected to column chromatography (PE/EA (v/v) = 3/1) to give 6-(2-(4-(2-(3,3-difluoropiperidin-1-yl)-6-methylpyrimidin-4-yl)-1*H*-pyrazol-1-yl)-5-nitrophenyl)-6-azaspiro[2.5]octane (1.21 g, yield: 66%).

LC-MS, M/Z (ESI): 510.2 [M+H]⁺.

### Step 3: Synthesis of 4-(4-(2-(3,3-difluoropiperidin-1-yl)-6-methylpyrimidin-4-yl)-1H-pyrazol-1-yl)-3-(6-azaspiro [2.5] octan-6-yl)aniline

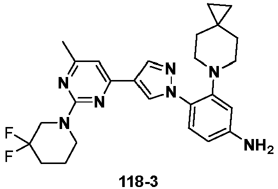

6-(2-(4-(2-(3,3-Difluoropiperidin-1-yl)-6-methylpyrimidin-4-yl)-1*H*-pyrazol-1-yl)-5-nitrophenyl)-6-azaspiro[2.5]octane (1.20 g, 2.42 mmol) was dissolved in acetic acid (10 mL), and then zinc powder (475 mg, 7.26 mmol) was added. The mixture was stirred at 50 °C for 1 h. After the reaction was completed, the mixture was diluted with water (30 mL), adjusted to about pH 7 with a saturated sodium bicarbonate solution, and then extracted three times with dichloromethane (20 mL). The organic phases were combined, washed with saturated brine (30 mL), dried over anhydrous sodium sulfate, filtered, and concentrated to give a crude product. The crude product was subjected to column chromatography (PE/EA (v/v) = 2/1) to give 4-(4-(2-(3,3-difluoropiperidin-1-yl)-6-methylpyrimidin-4-yl)-1*H*-pyrazol-1-yl)-3-(6-azaspiro[2.5]octan-6-yl)aniline (650 mg, yield: 58%).

LC-MS, M/Z (ESI): 480.2 [M+H]⁺.

### Step 4: Synthesis of methyl 2-(N (4-(4-(2-(3,3-difluoropiperidin-1-yl)-6-methylpyrimidin-4-yl)-1H-pyrazol-1-yl)-3-(6-azaspiro[2.5]octan-6-yl)phenyl)sulfamoyl)acetate

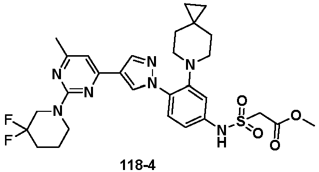

4-(4-(2-(3,3-Difluoropiperidin-1-yl)-6-methylpyrimidin-4-yl)-1*H*-pyrazol-1-yl)-3-(6-azaspiro[2.5]octan-6-yl)aniline (600 mg, 1.3 mmol) was dissolved in acetonitrile (10 mL), and then potassium phosphate (621 mg, 3.87 mmol) and methyl 2-(chlorosulfonyl)acetate (334 mg, 1.93 mmol) were added. The mixture was stirred at room temperature for 1 h. After the reaction was completed, the mixture was diluted with water (10 mL) and extracted three times with dichloromethane (10 mL). The organic phases were combined, washed with saturated brine (20 mL), dried over anhydrous sodium sulfate, filtered, and concentrated to give a crude product. The crude product was subjected to column chromatography (DCM/MeOH (v/v) = 10/1) to give methyl 2-(*N*-(4-(4-(2-(3,3-difluoropiperidin-1-yl)-6-methylpyrimidin-4-yl)-1*H*-pyrazol-1-yl)-3-(6-azaspiro [2.5] octan-6-yl)phenyl)sulfamoyl)acetate (420 mg, yield: 54%).

LC-MS, M/Z (ESI): 616.2 [M+H]⁺.

### Step 5: Synthesis of N-(4-(4-(2-(3,3-difluoropiperidin-1-yl)-6-methylpyrimidin-4-yl)-1H-pyrazol-1-yl)-3-(6-azaspiro[2.5]octan-6-yl)phenyl)-2-hydroxyethane-1-sulfonamide

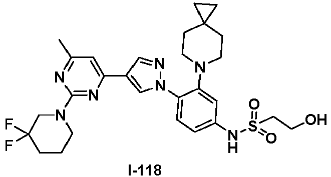

Methyl 2-(N (4-(4-(2-(3,3-difluoropiperidin-1-yl)-6-methylpyrimidin-4-yl)-1*H*-pyrazol-1-yl)-3-(6-azaspiro[2.5] octan-6-yl)phenyl)sulfamoyl)acetate (50 mg, 81 µmol) was dissolved in tetrahydrofuran (20 mL), and then sodium borohydride (15 mg, 45 µmol) was added under nitrogen atmosphere at 0 °C. Calcium chloride (90 mg, 810 µmol) was then added, and the mixture was allowed to react at 25 °C overnight. After the reaction was completed, the reaction liquid was quenched with a saturated ammonium chloride solution (25 mL) and extracted with ethyl acetate (30 mL × 3). The organic phase was washed with saturated brine (50 mL), dried over anhydrous sodium sulfate, filtered, and concentrated to give a crude product, which was then separated by reverse phase high performance liquid chromatography (separation method: column: Phenomenex luna C18 150 × 25 mm × 5 µm; solvents: A = water + 0.05% (by volume) formic acid (99.0%), B = acetonitrile; gradient: 25%-55%, 7 min), followed by lyophilization to give *N*-(4-(4-(2-(3,3-difluoropiperidin-1-yl)-6-methylpyrimidin-4-yl)-1*H*-pyrazol-1-yl)-3-(6-azaspiro[2.5]octan-6-yl)phenyl)-2-hydroxyethane-1-sulfonamide (3 mg, yield: 6.3%).

¹H NMR (400 MHz, DMSO) *δ* 9.01 (s, 1H), 8.26 (s, 1H), 7.46 (d, 1H), 7.03 (s, 1H), 6.93 (d, 2H), 4.14 (t, 2H), 3.86 (t, 2H), 3.74 (t, 2H), 3.25 (m, 4H), 2.68-2.65 (m, 4H), 2.30(s, 3H), 1.78-1.72 (m, 2H), 1.34-1.42 (m, 4H), 0.23 (s, 4H).

LC-MS, M/Z (ESI): 588.2 [M+H]⁺.

### Example 45: Preparation of Target Compound I-79

### N-(4-(4-(2-(4,4-Difluoropiperidin-1-yl)-6-methylpyrimidin-4-yl)-1H-1,2,3-triazol-1-yl)-3-(6-azaspiro[2.5]octan-6-yl)phenyl)-2-hydroxyethane-1-sulfonamide

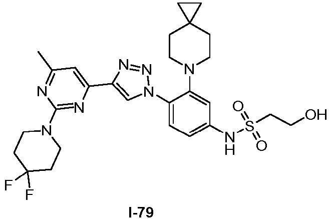

The synthetic route of the target compound **I-79** was as follows:

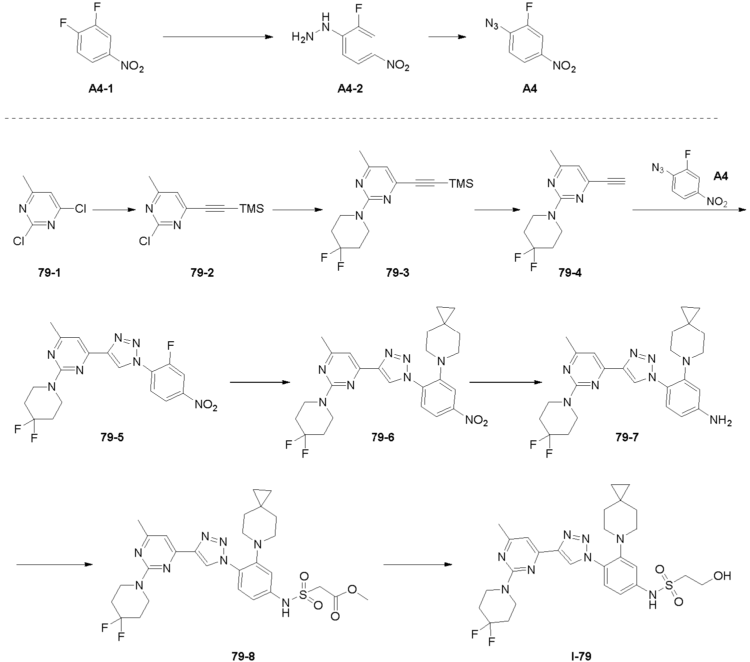

### Step 1: Synthesis of (2-fluoro-4-nitrophenyl)hydrazine

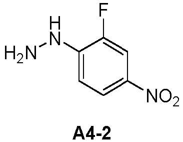

To a solution of 1,2-difluoro-4-nitrobenzene (5.00 g, 31.4 mmol) in ethanol (50.0 mL) was added hydrazine hydrate (4.00 g, 67.9 mmol, 3.88 mL, purity: 85.0%), and the reactants were allowed to react at 90 °C for 8 h. The reaction liquid was concentrated to remove most of the ethanol and then added to methyl *tert-butyl* ether (30.0 mL). The mixture was cooled to 0 °C, and a solid was precipitated. The mixture was then filtered, and the filter cake was dried to give (2-fluoro-4-nitrophenyl)hydrazine (3.60 g, yield: 66.9%).

¹H NMR (400 MHz, CDCl₃) *δ* 8.01-8.10 (m, 1H), 7.89 (dd, 1H), 7.21-7.27 (m, 1H), 6.01 (br s, 1H,) 3.75 (br s, 2H) Step 2: Synthesis of 1-azido-2-fluoro-4-nitrobenzene

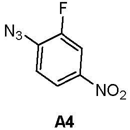

(2-Fluoro-4-nitrophenyl)hydrazine (3.00 g, 17.5 mmol) was dissolved in a hydrochloric acid solution (20 mL), and the resulting solution was stirred at 0 °C for 0.5 h. Methyl *tert-butyl* ether (15 mL) and a solution of sodium nitrite (1.57 g, 22.8 mmol) in water (10 mL) were then added slowly, and the reaction liquid was stirred and allowed to react at 0 °C for 3 h. The reaction liquid was poured into water (30 mL), and the mixture was extracted with ethyl acetate (50 mL × 3). The organic phase was collected, washed with a saturated aqueous sodium bicarbonate solution (50.0 mL), dried over anhydrous sodium sulfate, filtered, and concentrated, and the residue was separated and purified by a silica gel column (petroleum ether:methyl *tert-butyl* ether (V/V) = 1:0-1:1) to give 1-azido-2-fluoro-4-nitrobenzene (2.50 g, yield: 78.3%).

¹H NMR (400 MHz, CDCl3) *δ* 7.98-8.13 (m, 2H), 7.20 (t, 1H).

### Step 3: Synthesis of 2-chloro-4-methyl-6-((trimethylsilyl)ethynyl)pyrimidine

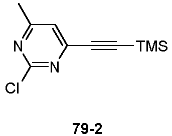

To a solution of 2,4-dichloro-6-methylpyrimidine (25.0 g, 153 mmol) and ethynyltrimethylsilane (15.1 g, 153 mmol) in tetrahydrofuran (250 mL) were added bis(triphenylphosphine)palladium(II) dichloride (5.38 g, 7.67 mmol), copper(I) iodide (2.92 g, 15.3 mmol), and triethylamine (46.6 g, 460 mmol) under nitrogen atmosphere, and the reactants were allowed to react at 50 °C for 2 h. The reaction liquid was poured into water (200 mL), and the mixture was extracted with dichloromethane (200 mL). The organic phase was collected, washed with brine (200 mL), dried over anhydrous sodium sulfate, filtered, and concentrated to give 2-chloro-4-methyl-6-((trimethylsilyl)ethynyl) pyrimidine (35.0 g, yield: 50.8%).

LC-MS, M/Z (ESI): 225.2 [M+H]⁺.

### Step 4: Synthesis of 2-(4,4-difluoropiperidin-1-yl)-4-methyl-6-((trimethylsilyl)ethynyl)pyrimidine

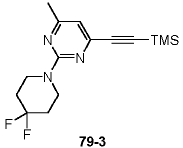

To a solution of 2-chloro-4-methyl-6-((trimethylsilyl)ethynyl)pyrimidine (10.0 g, 44.5 mmol) in *N,N-*dimethylformamide (50 mL) were added diisopropylethylamine (17.3 g, 133 mmol) and 4,4-difluoropiperidine hydrochloride (7.71 g, 48.9 mmol), and the reactants were allowed to react at 50 °C for 6 h. The reaction liquid was poured into water (100 mL), and the mixture was extracted with ethyl acetate (50 mL × 2). The organic phase was collected, dried over anhydrous sodium sulfate, filtered, and concentrated, and the crude product was separated and purified by a silica gel column (petroleum ether:ethyl acetate (V/V) = 20:1-0:1) to give 2-(4,4-difluoropiperidin-1-yl)-4-methyl-6-((trimethylsilyl)ethynyl)pyrimidine (10.9 g, yield: 78.9%).

LC-MS, M/Z (ESI): 310.3 [M+H]⁺.

### Step 5: Synthesis of 2-(4,4-difluoropiperidin-1-yl)-4-ethynyl-6-methylpyrimidine

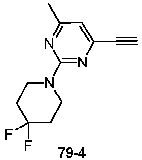

To a solution of 2-(4,4-difluoropiperidin-1-yl)-4-methyl-6-((trimethylsilyl)ethynyl)pyrimidine (5.50 g, 17.8 mmol) in acetonitrile (50 mL) was added potassium carbonate (7.37 g, 53.3 mmol), and the reactants were allowed to react at 50 °C for 8 h. The reaction liquid was filtered, and the filter cake was washed with ethyl acetate (30 × 3 mL). The filtrate was concentrated to give 2-(4,4-difluoropiperidin-1-yl)-4-ethynyl-6-methylpyrimidine (4.00 g, yield: 94.8%). LC-MS, M/Z (ESI): 238.0 [M+H]⁺.

### Step 6: Synthesis of 2-(4,4-difluoropiperidin-1-yl)-4-(1-(2-fluoro-4-nitrophenyl)-1H-1,2,3-triazol-4-yl)-6-methylpyrimidine

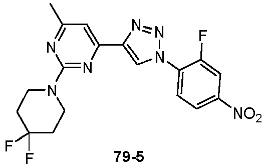

To a solution of 2-(4,4-difluoropiperidin-1-yl)-4-ethynyl-6-methylpyrimidine (2.10 g, 8.85 mmol) and 1-azido-2-fluoro-4-nitrobenzene (1.97 g, 10.8 mmol) in *tert*-butanol (30 mL) and water (15 mL) were added copper sulfate pentahydrate (663 mg, 2.66 mmol) and sodium ascorbate (877 mg, 4.43 mmol) under nitrogen atmosphere, and the reactants were allowed to react at 30 °C for 8 h. The reaction liquid was poured into water (80 mL), and the mixture was extracted with ethyl acetate (80 × 3 mL). The organic phase was collected, dried over anhydrous sodium sulfate, filtered, and concentrated to give a crude product. The crude product was slurried with methyl *tert-butyl* ether (30 mL) for 12 h to give 2-(4,4-difluoropiperidin-1-yl)-4-(1-(2-fluoro-4-nitrophenyl)-1*H*-1,2,3-triazol-4-yl)-6-methylpyrimidine (3.50 g, yield: 94.3%).

LC-MS, M/Z (ESI): 420.1 [M+H]⁺.

### Step 7: Synthesis of 6-(2-(4-(2-(4,4-difluoropiperidin-1-yl)-6-methylpyrimidin-4-yl)-1H-1,2,3-triazol-1-yl)-5-nitrophenyl)-6-azaspiro[2.5]octane

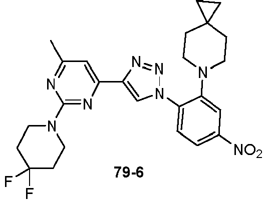

Three parallel batches were set up for reaction. To a solution of 2-(4,4-difluoropiperidin-1-yl)-4-(1-(2-fluoro-4-nitrophenyl)-1*H*-1,2,3-triazol-4-yl)-6-methylpyrimidine (500 mg, 1.19 mmol) in *N*-methylpyrrolidone (20.0 mL) were added potassium carbonate (494 mg, 3.58 mmol) and 6-azaspiro[2.5]octane hydrochloride (200 mg, 1.35 mmol), and then the mixture was stirred and allowed to react at 140 °C for 1 h. The reaction liquid was poured into water (50 mL), and the mixture was extracted with ethyl acetate (50 mL × 3). The organic phase was collected, washed with water (50 mL × 3), dried over anhydrous sodium sulfate, filtered, and concentrated, and the residue was separated and purified by a silica gel column (petroleum ether:ethyl acetate (V/V) = 20:1-5:1) to give a crude product, which was then slurried with methyl *tert-butyl* ether (10 mL) to give 6-(2-(4-(2-(4,4-difluoropiperidin-1-yl)-6-methylpyrimidin-4-yl)-1*H*-1,2,3-triazol-1-yl)-5-nitrophenyl)-6-azaspiro[2.5]octane (500 mg, yield: 27.4%).

LC-MS, M/Z (ESI): 511.2 [M+H]⁺.

### Step 8: Synthesis of 4-(4-(2-(4,4-difluoropiperidin-1-yl)-6-methylpyrimidin-4-yl)-1H-1,2,3-triazol-1-yl)-3-(6-azaspiro[2.5]octan-6-yl)aniline

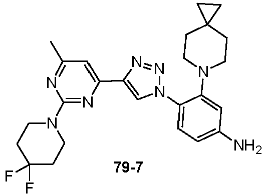

To a solution of 6-(2-(4-(2-(4,4-difluoropiperidin-1-yl)-6-methylpyrimidin-4-yl)-1*H*-1,2,3-triazol-1-yl)-5-nitrophenyl)-6-azaspiro[2.5]octane (400 mg, 783 µmol) in methanol (20 mL) and tetrahydrofuran (20 mL) was added platinum vanadium on carbon (100 mg, 383 µmol), and the mixture was purged three times with hydrogen and then stirred under hydrogen atmosphere (50 psi) at 50 °C for 12 h. The reaction liquid was filtered, and the filter cake was washed with dichloromethane (20 mL). The filtrate was concentrated to give 4-(4-(2-(4,4-difluoropiperidin-1-yl)-6-methylpyrimidin-4-yl)-1*H*-1,2,3-triazol-1-yl)-3-(6-azaspiro[2.5]octan-6-yl)aniline (380 mg, crude product). LC-MS, M/Z (ESI): 481.4 [M+H]⁺.

### Step 9: Synthesis of methyl 2-(N-(4-(4-(2-(4,4-difluoropiperidin-1-yl)-6-methylpyrimidin-4-yl)-1H-1,2,3-triazol-1-yl)-3-(6-azaspiro[2.5]octan-6-yl)phenyl)sulfamoyl)acetate

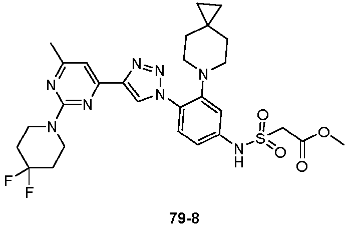

To a solution of 4-(4-(2-(4,4-difluoropiperidin-1-yl)-6-methylpyrimidin-4-yl)-1*H*-1,2,3-triazol-1-yl)-3-(6-azaspiro [2.5]octan-6-yl)aniline (200 mg, 416 µmol) in acetonitrile (5.00 mL) were added slowly methyl 2-(chlorosulfonyl)acetate (86.2 mg, 499 µmol) and potassium phosphate (265 mg, 1.25 mmol), and the reactants were allowed to react at 20 °C for 2 h. The reaction liquid was poured into water (20 mL), and the mixture was extracted with ethyl acetate (20 mL × 3). The organic phase was collected, dried over anhydrous sodium sulfate, filtered, and concentrated to give methyl 2-(N-(4-(4-(2-(4,4-difluoropiperidin-1-yl)-6-methylpyrimidin-4-yl)-1*H*-1,2,3-triazol-1-yl)-3-(6-azaspiro[2.5]octan-6-yl)phenyl)sulfamoyl)acetate (260 mg, crude product).

LC-MS, M/Z (ESI): 617.3 [M+H]⁺.

### Step 10: Synthesis of N-(4-(4-(2-(4,4-difluoropiperidin-1-yl)-6-methylpyrimidin-4-yl)-1H-1,2,3-triazol-1-yl)-3-(6-azaspiro[2.5]octan-6-yl)phenyl)-2-hydroxyethane-1-sulfonamide

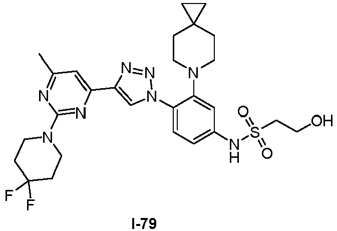

To a solution of methyl *2-(N-(4-(4-(2-(4,4-difluoropiperidin-1-yl)-6-methylpyrimidin-4-yl)-1H-1,2,3-triazol-1-yl)-*3-(6-azaspiro[2.5]octan-6-yl)phenyl)sulfamoyl)acetate (260 mg, 422 µmol) in tetrahydrofuran (5.00 mL) was added lithium borohydride (2 M, 422 µL), and the mixture was stirred under nitrogen atmosphere at 40 °C for 1 h. The reaction liquid was poured into water (20 mL), and then the mixture was extracted with ethyl acetate (20 mL × 3). The organic phase was collected, dried over anhydrous sodium sulfate, filtered, and concentrated, and the residue was separated and purified by high performance liquid chromatography (column: C18 150 × 30 mm; solvents: A = water + 0.05 (by volume) formic acid (99%), B = acetonitrile; gradient: 62%-92%, 7 min), followed by lyophilization to give *N*-(4-(4-(2-(4,4-difluoropiperidin-1-yl)-6-methylpyrimidin-4-yl)-1*H*-1,2,3-triazol-1-yl)-3-(6-azaspiro[2.5] octan-6-yl)phenyl)-2-hydroxyethane-1-sulfonamide (158 mg, yield: 62.7%).

¹H NMR (400 MHz, CDCl₃) δ 9.01 (s, 1H), 7.66 (d, 1H), 7.31 (s, 1H), 7.17 (d, 1H), 7.00 (dd, 1H), 6.64 (s, 1H), 4.18 (br s, 2H), 4.07 (br t, 4H), 3.29-3.43 (m, 2H), 2.80 (t, 4H), 2.45 (s, 3H), 1.99-2.06 (m, 4H), 1.38 (br s, 4H), 0.30 (s, 4H).

LC-MS, M/Z (ESI): 589.2 [M+H]⁺.

### Example 46: Preparation of Target Compound I-131

### N-(4-(2-(2-(4,4-Difluoropiperidin-1-yl)-6-methylpyrimidin-4-yl)-2H-1,2,3-triazol-4-yl)-3-(6-azaspiro[2.5]octan-6-yl)phenyl)-2-hydroxyethane-1-sulfonamide

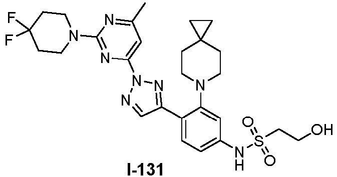

The synthetic route of the target compound **I-131** was as follows:

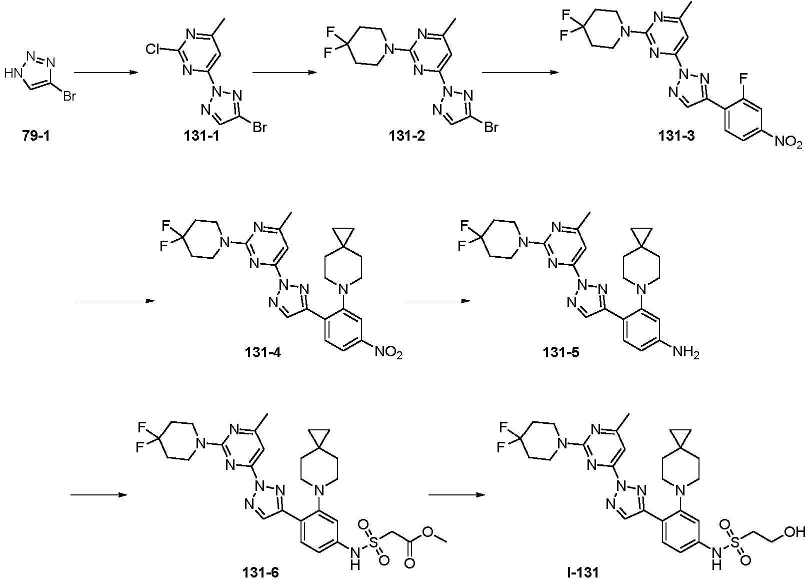

### Step 1: Synthesis of 4-(4-bromo-2H-1,2,3-triazol-2-yl)-2-chloro-6-methylpyrimidine

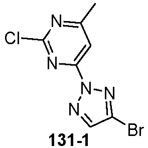

2,4-Dichloro-6-methylpyrimidine (24.0 g, 147 mmol) was dissolved in *N*,*N*-dimethylformamide (300 mL), and then diisopropylethylamine (38.1 g, 294 mmol) and 4-bromo-1H-1,2,3-triazole (21.8 g, 147 mmol) were added. The mixture was stirred at 25 °C for 2 h. The reaction liquid was quenched with water (100 mL) and extracted with ethyl acetate (200 mL × 2). The organic phases were combined, washed with saturated brine (200 mL), dried over anhydrous sodium sulfate, filtered, and concentrated to give 4-(4-bromo-2H-1,2,3-triazol-2-yl)-2-chloro-6-methylpyrimidine (30.0 g, yield: 74.2%).

LC-MS, M/Z (ESI): 274.0 [M+H]⁺.

### Step 2: Synthesis of 4-(4-bromo-2H-1,2,3-triazol-2-yl)-2-(4,4-difluoropiperidin-1-yl)-6-methylpyrimidine

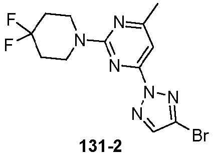

4-(4-Bromo-2*H*-1,2,3-triazol-2-yl)-2-chloro-6-methylpyrimidine (10.0 g, 36.4 mmol) was dissolved in *N,N-*dimethylformamide (100 mL), and then *N*,*N*-diisopropylethylamine (14.1 g, 109 mmol) and 4,4-difluoropiperidine hydrochloride (6.89 g, 43.7 mmol) were added. The mixture was stirred at 25 °C for 2 h. The reaction liquid was quenched with water (100 mL) and extracted with ethyl acetate (200 mL × 2). The organic phases were combined, washed with saturated brine (200 mL), dried over anhydrous sodium sulfate, filtered, and concentrated, and the residue was separated and purified by a silica gel column (petroleum ether:ethyl acetate (V/V) = 50:1-0:1) to give 4-(4-bromo-2*H*-1,2,3-triazol-2-yl)-2-(4,4-difluoropiperidin-1-yl)-6-methylpyrimidine (6.00 g, yield: 45.9%).

¹H NMR (400 MHz, CDCl3) δ 7.85 (s, 1H), 7.08 (s, 1H), 4.06-4.09 (m, 4H), 2.45 (s, 3H), 2.00-2.09 (m, 4H). LC-MS, M/Z (ESI): 359.0 [M+H]⁺.

### Step 3: Synthesis of 2-(4,4-difluoropiperidin-1-yl)-4-(4-(2-fluoro-4-nitrophenyl)-2H-1,2,3-triazol-2-yl)-6-methylpyrimidine

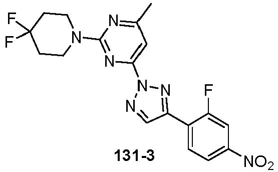

4-(4-Bromo-2*H*-1,2,3-triazol-2-yl)-2-(4,4-difluoropiperidin-1-yl)-6-methylpyrimidine (6.50 g, 18.1 mmol), potassium carbonate (7.50 g, 54.3 mmol), [1,1-bis(diphenylphosphino)ferrocene]palladium chloride (2.65 g, 3.62 mmol), and (2-fluoro-4-nitrophenyl)boronic acid (3.68 g, 19.9 mmol) were added to anhydrous dioxane (50 mL) and water (50 mL), and the mixture was allowed to react under nitrogen atmosphere at 100 °C for 2 h. The reaction liquid was quenched with water (100 mL) and extracted with ethyl acetate (100 mL × 2). The organic phases were combined, washed with saturated brine (100 mL), dried over anhydrous sodium sulfate, filtered, and concentrated, and the residue was separated and purified by a silica gel column (petroleum ether:ethyl acetate (V/V) = 50:1-0:1) to give 2-(4,4-difluoropiperidin-1-yl)-4-(4-(2-fluoro-4-nitrophenyl)-2*H*-1,2,3-triazol-2-yl)-6-methylpyrimidine (6.30 g, yield: 83.0%).

LC-MS, M/Z (ESI): 420.1 [M+H]⁺.

### Step 4: Synthesis of 6-(2-(2-(2-(4,4-difluoropiperidin-1-yl)-6-methylpyrimidin-4-yl)-2H-1,2,3-triazol-4-yl)-5-nitrophenyl)-6-azaspiro[2.5]]octane

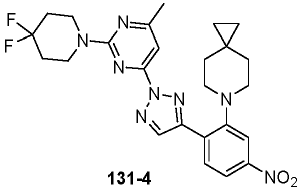

2-(4,4-Difluoropiperidin-1-yl)-4-(4-(2-fluoro-4-nitrophenyl)-2*H*-1,2,3-triazol-2-yl)-6-methylpyrimidine (4.00 g, 9.54 mmol), potassium carbonate (3.95 g, 28.6 mmol), and 6-azaspiro[2.5]octane hydrochloride (1.55 g, 10.5 mmol) were added to *N*,*N*-dimethylformamide (20 mL), and the mixture was allowed to react at 140 °C for 2 h. The reaction liquid was quenched with water (150 mL) and extracted with ethyl acetate (200 mL × 2). The organic phases were combined, washed with saturated brine (100 mL), dried over anhydrous sodium sulfate, filtered, and concentrated, and the residue was separated and purified by a silica gel column (petroleum ether:ethyl acetate (V/V) = 50:1-0:1) to give 6-(2-(2-(2-(4,4-difluoropiperidin-1-yl)-6-methylpyrimidin-4-yl)-2*H*-1,2,3-triazol-4-yl)-5-nitrophenyl)-6-azaspiro [2.5]octane (1.20 g, yield: 24.6%).

LC-MS, M/Z (ESI): 511.3 [M+H]⁺.

### Step 5: Synthesis of 4-(2-(2-(4,4-difluoropiperidin-1-yl)-6-methylpyrimidin-4-yl)-2H-1,2,3-triazol-4-yl)-3-(6-azaspiro[2.5]octan-6-yl)aniline

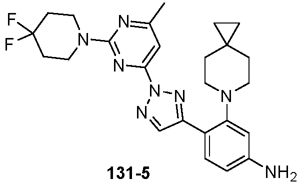

6-(2-(2-(2-(4,4-Difluoropiperidin-1-yl)-6-methylpyrimidin-4-yl)-2*H*-1,2,3-triazol-4-yl)-5-nitrophenyl)-6-azaspiro[2.5]octane (700 mg, 1.37 mmol) was added to tetrahydrofuran (50 mL), and then platinum vanadium on carbon (35.8 mg, 137 mmol) was added. The mixture was purged three times with hydrogen and then allowed to react under hydrogen atmosphere (15 psi) at 25 °C for 6 h. After the reaction was completed, the mixture was filtered, and the filtrate was concentrated to give a crude product, which was then separated by reverse phase high performance liquid chromatography (separation method: column: Waters Xbridge 150 × 25 mm × 5 µm; solvents: A = water + 0.05% (by volume) aqueous ammonia (30.0%), B = acetonitrile; gradient: 38%-68%, 13 min), followed by lyophilization to give 4-(2-(2-(4,4-difluoropiperidin-1-yl)-6-methylpyrimidin-4-yl)-2*H*-1,2,3-triazol-4-yl)-3-(6-azaspiro[2.5]octan-6-yl)aniline (620 mg, yield: 76.2%).

¹H NMR (400 MHz, DMSO_d₆) *δ* 8.60 (s, 1H), 7.51 (d, 1H), 7.15 (s, 1H), 6.46 (s, 1H), 6.36 (d, 1H), 5.44 (br. s, 2H), 3.96-4.05 (m, 8H), 2.47 (s, 3H), 2.07-2.11 (m, 4H), 1.38-1.46 (m, 4H), 0.34 (s, 4H).

LC-MS, M/Z (ESI): 481.3 [M+H]⁺.

### Step 6: Synthesis of methyl 2-(N-(4-(2-(2-(4,4-difluoropiperidin-1-yl)-6-methylpyrimidin-4-yl)-2H-1,2,3-triazol-4-yl)-3-(6-azaspiro[2.5]octan-6-yl)phenyl)sulfamoyl)acetate

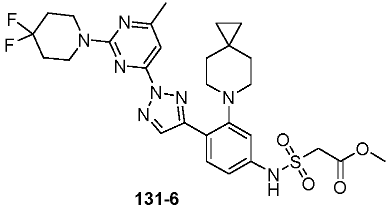

4-(2-(2-(4,4-Difluoropiperidin-1-yl)-6-methylpyrimidin-4-yl)-2*H*-1,2,3-triazol-4-yl)-3-(6-azaspiro[2.5]octan-6-yl)aniline (620 mg, 1.05 mmol), potassium phosphate (665 mg, 3.14 mmol), and methyl 2-(chlorosulfonyl)acetate (180 mg, 1.05 mmol) were added to acetonitrile (20 mL), and the mixture was allowed to react at 25 °C for 6 h. The reaction liquid was quenched with water (50 mL) and extracted with ethyl acetate (25 mL × 2). The organic phases were combined, washed with saturated brine (50 mL), dried over anhydrous sodium sulfate, filtered, and concentrated to give methyl 2-(*N*-(4-(2-(2-(4,4-difluoropiperidin-1-yl)-6-methylpyrimidin-4-yl)-2*H*-1,2,3-triazol-4-yl)-3-(6-azaspiro[2.5]octan-6-yl)phenyl)sulfamoyl)acetate (430 mg, yield: 66.7%).

LC-MS, M/Z (ESI): 617.3 [M+H]⁺.

### Step 7: Synthesis of N-(4-(2-(2-(4,4-difluoropiperidin-1-yl)-6-methylpyrimidin-4-yl)-2H-1,2,3-triazol-4-yl)-3-(6-azaspiro[2.5]octan-6-yl)phenyl)-2-hydroxyethane-1-sulfonamide

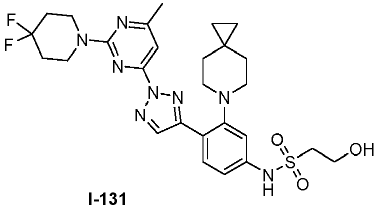

Methyl 2-(*N*-(4-(2-(2-(4,4-difluoropiperidin-1-yl)-6-methylpyrimidin-4-yl)-2*H*-1,2,3-triazol-4-yl)-3-(6-azaspiro [2.5]octan-6-yl)phenyl)sulfamoyl)acetate (430 mg, 697 µmol) was dissolved in tetrahydrofuran (20 mL), and then lithium borohydride (2 M, 697 µL) was added dropwise under nitrogen atmosphere at 0 °C. The mixture was allowed to react at 35 °C for 2 h. After the reaction was completed, the reaction liquid was quenched with a saturated ammonium chloride solution (50 mL) and extracted with ethyl acetate (30 mL × 3). The organic phase was washed with saturated brine (50 mL), dried over anhydrous sodium sulfate, filtered, and concentrated to give a crude product, which was then separated by reverse phase high performance liquid chromatography (separation method: column: Phenomenex luna C18 150 × 25 mm × 5 µm; solvents: A = water + 0.05% (by volume) formic acid (99.0%), B = acetonitrile; gradient: 65%-95%, 12 min), followed by lyophilization to give *N*-(4-(2-(2-(4,4-difluoropiperidin-1-yl)-6-methylpyrimidin-4-yl)-2*H*-1,2,3-triazol-4-yl)-3-(6-azaspiro [2.5] octan-6-yl)phenyl)-2-hydroxyethane-1-sulfonamide (121 mg, 198 µmol, purity: 96.8%, yield: 28.4%).

¹H NMR (400 MHz, CDCl₃) *δ* 8.66 (s, 1H), 7.89 (d, 1H), 7.19 (s, 1H), 7.15 (s, 1H), 7.00 (d, 1H), 6.81 (br. s, 1H), 4.10-4.15 (m, 6H), 3.35 (t, 2H), 2.89-2.94 (m, 4H), 2.47 (s, 3H), 2.03-2.11 (m, 4H), 1.43-1.58 (m, 4H), 0.38 (s, 4H). LC-MS, M/Z (ESI): 589.3 [M+H]⁺.

### Example 47: Preparation of Target Compound I-116

### N-(4-(4-(6-Methyl-2-(pyrrolidin-1-yl)pyrimidin-4-yl)-1H-pyrazol-1-yl)-3-(6-azaspiro[2.5]octan-6-yl)phenyl)-2-hydroxyethane-1-sulfonamide

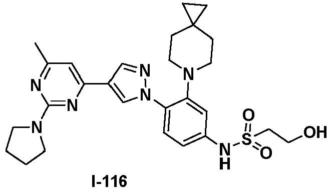

The synthetic route of the target compound **I-116** was as follows:

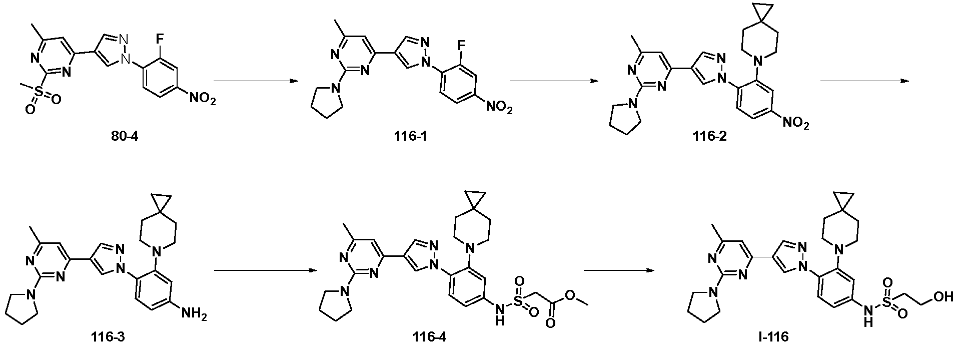

### Step 1: Synthesis of 4-(1-(2-fluoro-4-nitrophenyl)-1H-pyrazol-4-yl)-6-methyl-2-(pyrrolidin-1-yl)pyrimidine

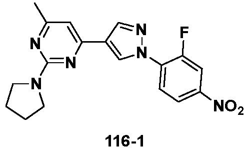

4-(1-(2-Fluoro-4-nitrophenyl)-1H-pyrazol-4-yl)-6-methyl-2-(methylsulfonyl)pyrimidine (2.00 g, 5.30 mmol) and potassium carbonate (1.47 g, 10.60 mmol) were dissolved in DMSO (20 mL), and then tetrahydropyrrole (565 mg, 7.95 mmol) was added slowly. The mixture was stirred at 25 °C for 1 h. After the reaction was completed, the mixture was diluted with water (20 mL) and extracted three times with dichloromethane (20 mL). The organic phases were combined, washed with saturated brine (30 mL), dried over anhydrous sodium sulfate, filtered, and concentrated to give a crude product. The crude product was subjected to column chromatography (PE/EA (v/v) = 3/1) to give 4-(1-(2-fluoro-4-nitrophenyl)-1*H*-pyrazol-4-yl)-6-methyl-2-(pyrrolidin-1-yl)pyrimidine (1.81 g, yield: 93%).

LC-MS, M/Z (ESI): 368.1 [M+H]⁺.

### Step 2: Synthesis of 6-(2-(4-(6-methyl-2-(pyrrolidin-1-yl)pyrimidin-4-yl)-1H-pyrazol-1-yl)-5-nitrophenyl)-6-azaspiro[2.5]octane

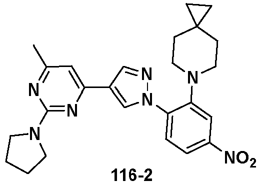

4-(1-(2-Fluoro-4-nitrophenyl)-1*H*-pyrazol-4-yl)-6-methyl-2-(pyrrolidin-1-yl)pyrimidine (1.80 g, 4.9 mmol) and potassium carbonate (2.03 g, 14.7 mmol) were dissolved in DMSO (15 mL), and then 6-azaspiro[2.5]octane hydrochloride (1.44 g, 9.8 mmol) was added slowly. The mixture was stirred at 140 °C for 8 h. After the reaction was completed, the mixture was diluted with water (20 mL) and extracted three times with dichloromethane (20 mL). The organic phases were combined, washed with saturated brine (30 mL), dried over anhydrous sodium sulfate, filtered, and concentrated to give a crude product. The crude product was subjected to column chromatography (PE/EA (v/v) = 3/1) to give 6-(2-(4-(6-methyl-2-(pyrrolidin-1-yl)pyrimidin-4-yl)-1*H*-pyrazol-1-yl)-5-nitrophenyl)-6-azaspiro[2.5]octane (1.65 g, yield: 74%).

LC-MS, M/Z (ESI): 460.1 [M+H]⁺.

### Step 3: Synthesis of 4-(4-(6-methyl-2-(pyrrolidin-1-yl)pyrimidin-4-yl)-1H-pyrazol-1-yl)-3-(6-azaspiro[2.5]octan-6-yl)aniline

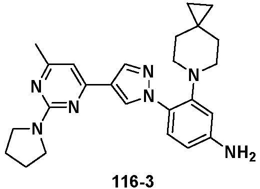

6-(2-(4-(4-(6-Methyl-2-(pyrrolidin-1-yl)pyrimidin-4-yl)-1*H*-pyrazol-1-yl)-5-nitrophenyl)-6-azaspiro[2.5]octane (1.00 g, 2.18 mmol) was dissolved in ethanol (10 mL) and water (2 mL), and then zinc powder (711 mg, 10.88 mmol) was added. The mixture was stirred at 90 °C for 1 h. After the reaction was completed, the mixture was diluted with water (30 mL) and extracted three times with dichloromethane (20 mL). The organic phases were combined, washed with saturated brine (30 mL), dried over anhydrous sodium sulfate, filtered, and concentrated to give a crude product. The crude product was subjected to column chromatography (PE/EA (v/v) = 2/1) to give 4-(4-(6-methyl-2-(pyrrolidin-1-yl)pyrimidin-4-yl)-1*H*-pyrazol-1-yl)-3-(6-azaspiro[2.5]octan-6-yl)aniline (530 mg, yield: 57%).

LC-MS, M/Z (ESI): 430.1 [M+H]⁺.

### Step 4: Synthesis of methyl 2-(N-(4-(4-(6-methyl-2-(pyrrolidin-1-yl)pyrimidin-4-yl)-1H-pyrazol-1-yl)-3-(6-azaspiro[2.5]octan-6-yl)phenyl)sulfamoyl)acetate

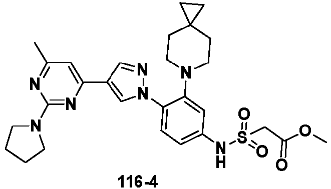

4-(4-(6-Methyl-2-(pyrrolidin-1-yl)pyrimidin-4-yl)-1*H*-pyrazol-1-yl)-3-(6-azaspiro[2.5]octan-6-yl)aniline (500 mg, 1.16 mmol) was dissolved in acetonitrile (10 mL), and then potassium phosphate (741 mg, 3.49 mmol) and methyl 2-(chlorosulfonyl)acetate (402 mg, 2.333 mmol) were added. The mixture was stirred at room temperature for 1 h. After the reaction was completed, the mixture was diluted with water (10 mL) and extracted three times with dichloromethane (10 mL). The organic phases were combined, washed with saturated brine (20 mL), dried over anhydrous sodium sulfate, filtered, and concentrated to give a crude product. The crude product was subjected to column chromatography (DCM/MeOH (v/v) = 10/1) to give methyl 2-(N-(4-(4-(6-methyl-2-(pyrrolidin-1-yl)pyrimidin-4-yl)-1*H* pyrazol-1-yl)-3-(6-azaspiro[2.5]octan-6-yl)phenyl)sulfamoyl)acetate (527 mg, yield: 80%).

LC-MS, M/Z (ESI): 566.1 [M+H]⁺.

### Step 5: Synthesis of N-(4-(4-(6-methyl-2-(pyrrolidin-1-yl)pyrimidin-4-yl)-1H-pyrazol-1-yl)-3-(6-azaspiro[2.5] octan-6-yl)phenyl)-2-hydroxyethane-1-sulfonamide

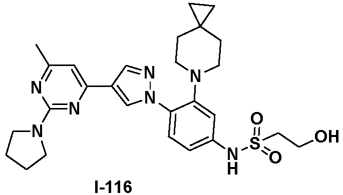

Methyl 2-(*N*-(4-(4-(6-methyl-2-(pyrrolidin-1-yl)pyrimidin-4-yl)-1*H*-pyrazol-1-yl)-3-(6-azaspiro[2.5]octan-6-yl)phenyl) sulfamoyl)acetate (480 mg, 849 µmol) was dissolved in tetrahydrofuran (20 mL), and then lithium borohydride (2 M, 509 µL) was added dropwise under nitrogen atmosphere at 0 °C. The mixture was allowed to react at 25 °C for 1 h. After the reaction was completed, the reaction liquid was quenched with a saturated ammonium chloride solution (25 mL) and extracted with ethyl acetate (30 mL × 3). The organic phase was washed with saturated brine (50 mL), dried over anhydrous sodium sulfate, filtered, and concentrated to give a crude product, which was then separated by reverse phase high performance liquid chromatography (separation method: column: Phenomenex luna C18 150 × 25 mm × 5 µm; solvents: A = water + 0.05% (by volume) formic acid (99.0%), B = acetonitrile; gradient: 25%-55%, 7 min), followed by lyophilization to give *N*-(4-(4-(6-methyl-2-(pyrrolidin-1-yl)pyrimidin-4-yl)-1*H*-pyrazol-1-yl)-3-(6-azaspiro[2.5]octan-6-yl)phenyl)-2-hydroxyethane-1-sulfonamide (137 mg, yield: 29.1%).

¹H NMR (400 MHz, CDCl3) *δ* 8.91 (s, 1H), 8.07 (s, 1H), 7.49 (d, 1H), 7.08 (s, 1H), 6.91 (d, 1H), 6.53 (s, 1H), 3.77 (t, 2H), 3.62-3.69 (m, 4H), 3.25 (t, 2H), 2.53-2.56 (m, 4H), 2.43-2.49 (m, 4H), 1.96-2.07 (m, 4H), 1.34-1.42 (m, 4H), 0.22 (s, 4H).

LC-MS, M/Z (ESI): 538.4 [M+H]⁺.

### Example 48: Preparation of Target Compound I-130

### N-(4-(4-(4-(4,4-Difluoropiperidin-1-yl)-1,3,5-triazin-2-yl)-1H-pyrazol-1-yl)-3-(6-azaspiro[2.5]octan-6-yl)phenyl)-2-hydroxyethanesulfonamide

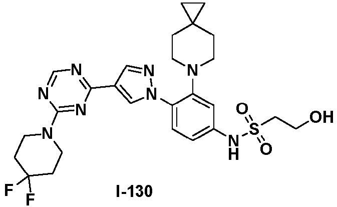

The synthetic route of the target compound **I-130** was as follows:

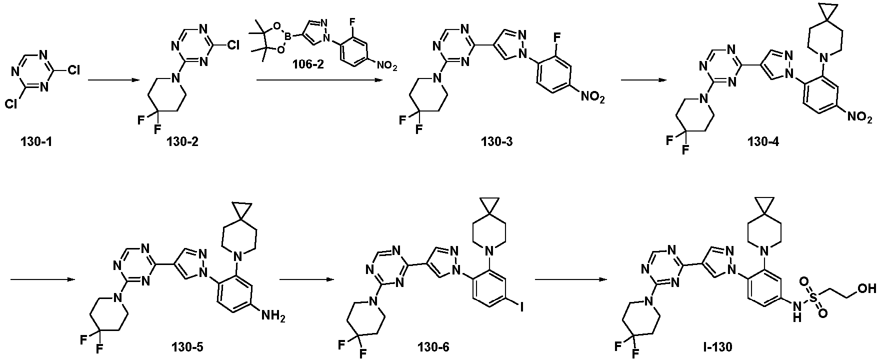

### Step 1: Synthesis of 2-chloro-4-(4,4-difluoropiperidin-1-yl)-1,3,5-triazine

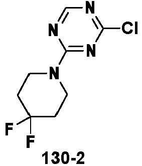

To a solution of 2,4-dichloro-1,3,5-triazine (1 g, 6.7 mmol) in dichloromethane (10 mL) was added *N,N-*diisopropylethylamine (2.6 g, 20.1 mmol), and then 4,4-difluoropiperidine (1.2 g, 10.5 mmol) was added dropwise and slowly. The mixture was stirred at 25 °C for 1 h. The reaction liquid was concentrated to give a crude product. The crude product was purified by column chromatography (petroleum ether/ethyl acetate (V/V) = 1/0-1/1) to give 2-chloro-4-(4,4-difluoropiperidin-1-yl)-1,3,5-triazine (1.4 g, yield: 89.5%).

LC-MS, M/Z (ESI): 235.3 [M+H]⁺.

### Step 2: Synthesis of 2-(4,4-difluoropiperidin-1-yl)-4-(1-(2-fluoro-4-nitrophenyl)-1H-pyrazol-4-yl)-1,3,5-triazine

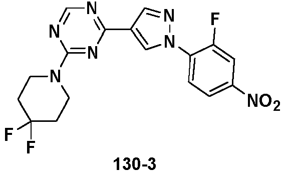

To a solution of 2-chloro-4-(4,4-difluoropiperidin-1-yl)-1,3,5-triazine (1.4 g, 6 mmol) and 1-(2-fluoro-4-nitrophenyl)-4-(4,4,5,5-tetramethyl-1,3,2-dioxaborolan-2-yl)-1*H*-pyrazole (4.0 g, 12 mmol) in *N,N-*dimethylformamide (12 mL) and water (3 mL) were added [1,1'-bis(diphenylphosphino)ferrocene]dichloropalladium(II) (439 mg, 0.6 mmol) and potassium carbonate (2.5 g, 18 mmol), and the mixture was stirred at 90 °C for 18 h. The reaction liquid was added to water (100 mL), and the mixture was extracted with ethyl acetate (50 mL × 3). The organic phase was washed with saturated brine (50 mL × 2), dried over anhydrous sodium sulfate, filtered, and concentrated to give a crude product. The crude product was purified by column chromatography (petroleum ether/ethyl acetate (V/V) = 1/0-1/3) to give 2-(4,4-difluoropiperidin-1-yl)-4-(1-(2-fluoro-4-nitrophenyl)-1H-pyrazol-4-yl)-1,3,5-triazine (1 g, yield: 41.2%).

LC-MS, M/Z (ESI): 406.3 [M+H]⁺.

### Step 3: Synthesis of 6-(2-(4-(4-(4,4-difluoropiperidin-1-yl)-1,3,5-triazin-2-yl)-1H-pyrazol-1-yl)-5-nitrophenyl)-6-azaspiro[2.5]octane

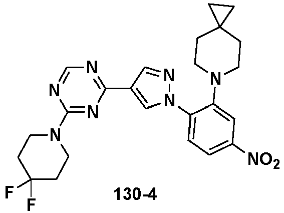

To a solution of 2-(4,4-difluoropiperidin-1-yl)-4-(1-(2-fluoro-4-nitrophenyl)-1H-pyrazol-4-yl)-1,3,5-triazine (1 g, 2.5 mmol) in dimethylsulfoxide (10 mL) were added 6-azaspiro[2.5]octane (556 mg, 5 mmol) and potassium carbonate (1.0 g, 7.5 mmol), and the mixture was allowed to react under microwave conditions at 140 °C for 3 h. The reaction liquid was poured into water (50 mL), and the mixture was extracted with ethyl acetate (20 mL × 3). The organic phase was washed with saturated brine (20 mL × 2), dried over anhydrous sodium sulfate, filtered, and concentrated to give a crude product. The crude product was purified by column chromatography (petroleum ether/ethyl acetate (V/V) = 1/0-1/1) to give 6-(2-(4-(4-(4,4-difluoropiperidin-1-yl)-1,3,5-triazin-2-yl)-1*H*-pyrazol-1-yl)-5-nitrophenyl)-6-azaspiro[2.5]octane (600 mg, yield: 49%).

LC-MS, M/Z (ESI): 497.4 [M+H]⁺.

### Step 4: Synthesis of 4-(4-(4-(4,4-difluoropiperidin-1-yl)-1,3,5-triazin-2-yl)-1H-pyrazol-1-yl)-3-(6-azaspiro[2.5] octan-6-yl)aniline

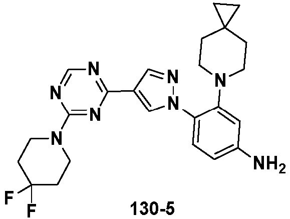

To a solution of 6-(2-(4-(4-(4,4-difluoropiperidin-1-yl)-1,3,5-triazin-2-yl)-1*H*-pyrazol-1-yl)-5-nitrophenyl)-6-azaspiro[2.5]octane (600 mg, 1.2 mmol) in ethanol (9 mL) were added zinc powder (392 g, 6 mmol) and ammonium chloride (642 g, 12 mmol), and the mixture was stirred at 85 °C for 2 h. Water (50 mL) and ethyl acetate (20 mL) were added to the reaction liquid, and the mixture was filtered. The filtrate was extracted with ethyl acetate (20 mL × 3). The organic phase was washed with saturated brine (10 mL × 2), dried over anhydrous sodium sulfate, filtered, and concentrated to give a crude product. The crude product was purified by column chromatography (petroleum ether/ethyl acetate (V/V) = 1/0-1/2) to give 4-(4-(4-(4,4-difluoropiperidin-1-yl)-1,3,5-triazin-2-yl)-1*H*-pyrazol-1-yl)-3-(6-azaspiro[2.5]octan-6-yl)aniline (400 mg, yield: 71%).

LC-MS, M/Z (ESI): 467.3 [M+H]⁺.

### Step 5: Synthesis of 6-(2-(4-(4-(4,4-difluoropiperidin-1-yl)-1,3,5-triazin-2-yl)-1H-pyrazol-1-yl)-5-iodophenyl)-6-azaspiro[2.5]octane

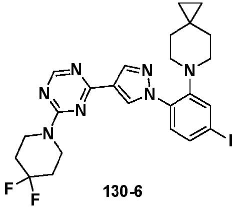

To a solution of 4-(4-(4-(4,4-difluoropiperidin-1-yl)-1,3,5-triazin-2-yl)-1*H*-pyrazol-1-yl)-3-(6-azaspiro[2.5]octan-6-yl)aniline (0.4 g, 0.9 mmol) in acetonitrile (4 mL) was added copper(I) iodide (857 mg, 4.5 mmol), and then isoamyl nitrite (316 mg, 2.7 mmol) was added dropwise and slowly to the reaction liquid at 0 °C. The mixture was then stirred at 25 °C for 18 h. Water (50 mL) was added to the reaction liquid, and the mixture was extracted with ethyl acetate (20 mL × 3). The organic phase was washed with saturated brine (20 mL × 3), dried over anhydrous sodium sulfate, filtered, and concentrated to give a crude product. The crude product was purified by column chromatography (petroleum ether/ethyl acetate (V/V) = 1/0-1/1) to give 6-(2-(4-(4-(4,4-difluoropiperidin-1-yl)-1,3,5-triazin-2-yl)-1*H-*pyrazol-1-yl)-5-iodophenyl)-6-azaspiro[2.5]octane (200 mg, yield: 40%).

LC-MS, M/Z (ESI): 578.4 [M+H]⁺.

### Step 6: Synthesis of N-(4-(4-(4-(4,4-difluoropiperidin-1-yl)-1,3,5-triazin-2-yl)-1H-pyrazol-1-yl)-3-(6-azaspiro[2.5] octan-6-yl)phenyl)-2-hydroxyethanesulfonamide

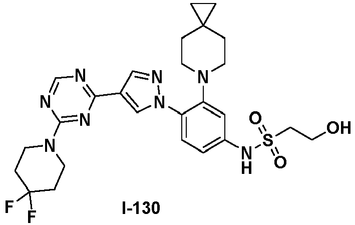

To a solution of 6-(2-(4-(4-(4,4-difluoropiperidin-1-yl)-1,3,5-triazin-2-yl)-1*H*-pyrazol-1-yl)-5-iodophenyl)-6-azaspiro[2.5]octane (0.2 g, 0.35 mmol) and 2-hydroxyethanesulfonamide (87 mg, 0.70 mmol) in *N,N-*dimethylformamide (2 mL) were added 2-(dimethylamino)acetic acid (7 mg, 0.07 mmol), copper(I) iodide (6.6 mg, 0.035 mmol), and potassium carbonate (145 mg, 1.05 mmol), and then the mixture was stirred at 130 °C for 2 h. The reaction liquid was poured into water (20 mL), and the mixture was extracted with ethyl acetate (10 mL × 3). The organic phase was washed with saturated brine (10 mL × 2), dried over anhydrous sodium sulfate, filtered, and concentrated to give a crude product. The crude product was subjected to preparative reverse phase chromatography (column: Phenomenex Synergi C18 100 × 25 mm × 4 µm; solvents: A = water + 0.1 vol% formic acid (99%), B = acetonitrile; gradient: 5%-95%, 7 min) to give *N*-(4-(4-(4-(4,4-difluoropiperidin-1-yl)-1,3,5-triazin-2-yl)-1*H-*pyrazol-1-yl)-3-(6-azaspiro[2.5]octan-6-yl)phenyl)-2-hydroxyethanesulfonamide (56.6 mg, yield: 28%).

¹H NMR (400 MHz, dmso) δ 9.07 (s, 1H), 8.63 (s, 1H), 8.30 (s, 1H), 7.50 (d,1H), 7.08 (d, 1H), 6.98 (dd, 1H), 4.00 (d, 4H), 3.76 (dd, 2H), 3.29 (s, 2H), 2.76-2.61 (m, 4H), 2.14-1.96 (m, 4H), 1.34 (s, 4H), 0.26 (s, 4H).

LC-MS, M/Z (ESI): 575.1 [M+H]⁺.

### Example 49: Preparation of Target Compound I-123

### N-(4-(4-(1-(4,4-Difluorocyclohexyl)-6-oxo-1,6-dihydropyridazin-3-yl)-1H-pyrazol-1-yl)-3-(6-azaspiro[2.5]octan-6-yl)phenyl)-2-hydroxyethane-1-sulfonamide

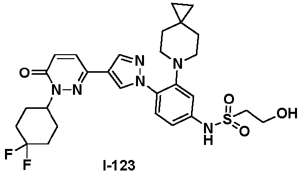

The synthetic route of the target compound **I-123** was as follows:

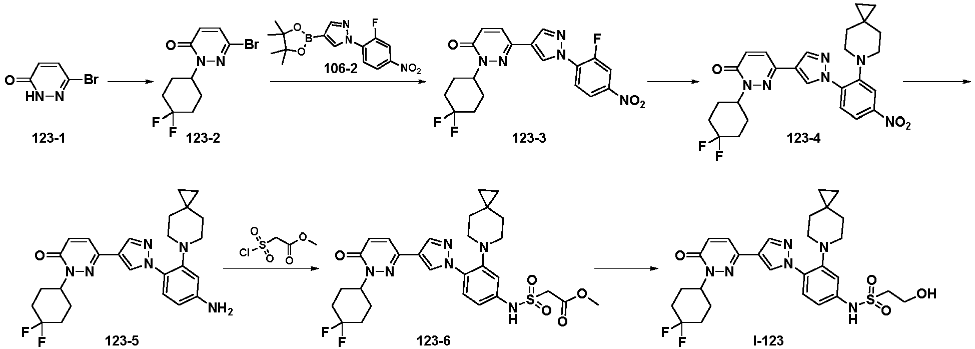

### Step 1: Synthesis of 6-bromo-2-(4,4-difluorocyclohexyl)pyridazin-3(2H)-one

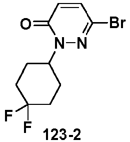

To a solution of 4-bromo-1,1-difluorocyclohexane (1 g, 5.0 mmol) in N,N-dimethylformamide (10 mL) were added 6-bromopyridazin-3(2H)-one (900 mg, 5.0 mmol) and potassium carbonate (2.4 g, 17.4 mmol), and the mixture was stirred at 50 °C for 18 h. The reaction liquid was poured into water (100 mL), and the mixture was extracted with ethyl acetate (30 mL × 3). The organic phase was washed with saturated brine (20 mL × 2), dried over anhydrous sodium sulfate, filtered, and concentrated to give a crude product. The crude product was purified by column chromatography (petroleum ether/ethyl acetate (V/V) = 1/0-1/1) to give 6-bromo-2-(4,4-difluorocyclohexyl) pyridazin-3(2H)-one (300 mg, yield: 24%).

LC-MS, M/Z (ESI): 293.2 [M+H]⁺.

### Step 2: Synthesis of 2-(4,4-difluorocyclohexyl)-6-(1-(2-fluoro-4-nitrophenyl)-1H-pyrazol-4-yl)pyridazin-3(2H)-one

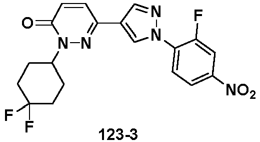

To a solution of 6-bromo-2-(4,4-difluorocyclohexyl)pyridazin-3(2H)-one (300 mg, 0.80 mmol) and 1-(2-fluoro-4-nitrophenyl)-4-(4,4,5,5-tetramethyl-1,3,2-dioxaborolan-2-yl)-1*H*-pyrazole (533 mg, 1.50 mmol) in *N,N-*dimethylformamide (2 mL) and water (0.4 mL) were added [1,1'-bis(diphenylphosphino)ferrocene]dichloropalladium(II) (54 mg, 0.075 mmol) and potassium carbonate (332 mg, 2.25 mmol), and the mixture was stirred at 90 °C for 18 h. The reaction liquid was added to water (20 mL), and the mixture was extracted with ethyl acetate (5 mL × 3). The organic phase was washed with saturated brine (5 mL × 2), dried over anhydrous sodium sulfate, filtered, and concentrated to give a crude product. The crude product was purified by column chromatography (petroleum ether/ethyl acetate (V/V) = 1/0-1/3) to give 2-(4,4-difluorocyclohexyl)-6-(1-(2-fluoro-4-nitrophenyl)-1H-pyrazol-4-yl)pyridazin-3(2*H*)-one (250 mg, yield: 75%).

LC-MS, M/Z (ESI): 420.2 [M+H]⁺.

### Step 3: Synthesis of 2-(4,4-difluorocyclohexyl)-6-(1-(4-nitro-2-(6-azaspiro[2.5]octan-6-yl)phenyl)-1H-pyrazol-4-yl)pyridazin-3(2H)-one

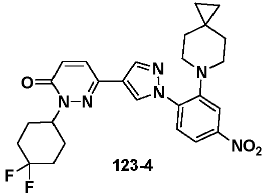

To a solution of 2-(4,4-difluorocyclohexyl)-6-(1-(2-fluoro-4-nitrophenyl)-1*H*-pyrazol-4-yl)pyridazin-3(2*H*)-one (200 mg, 0.45 mmol) in dimethylsulfoxide (2 mL) were added 6-azaspiro[2.5]octane (111 mg, 1 mmol) and potassium carbonate (207 mg, 1.5 mmol), and the mixture was allowed to react under microwave conditions at 140 °C for 8 h. The reaction liquid was poured into water (20 mL), and the mixture was extracted with ethyl acetate (5 mL × 3). The organic phase was washed with saturated brine (5 mL × 2), dried over anhydrous sodium sulfate, filtered, and concentrated to give a crude product. The crude product was purified by column chromatography (petroleum ether/ethyl acetate (V/V) = 1/0-1/1) to give 2-(4,4-difluorocyclohexyl)-6-(1-(4-nitro-2-(6-azaspiro[2.5]octan-6-yl)phenyl)-1*H*-pyrazol-4-yl)pyridazin-3(2*H*)-one (220 mg, yield: 90%).

LC-MS, M/Z (ESI): 511.2 [M+H]⁺.

### Step 4: Synthesis of 6-(1-(4-amino-2-(6-azaspiro[2.5]octan-6-yl)phenyl)-1H-pyrazol-4-yl)-2-(4,4-difluorocyclohexyl) pyridazin-3(2H)-one

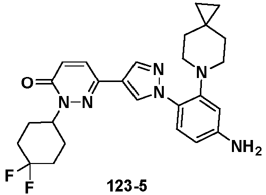

To a solution of 2-(4,4-difluorocyclohexyl)-6-(1-(4-nitro-2-(6-azaspiro[2.5]octan-6-yl)phenyl)-1*H*-pyrazol-4-yl)pyridazin-3(2*H*)-one (220 mg, 0.4 mmol) in ethanol (5 mL) were added zinc powder (131 g, 2 mmol) and ammonium chloride (212 g, 4 mmol), and the mixture was stirred at 85 °C for 2 h. Water (20 mL) and ethyl acetate (10 mL) were added to the reaction liquid, and the mixture was filtered. The filtrate was extracted with ethyl acetate (5 mL × 3). The organic phase was washed with saturated brine (10 mL × 2), dried over anhydrous sodium sulfate, filtered, and concentrated to give a crude product. The crude product was purified by column chromatography (petroleum ether/ethyl acetate (V/V) = 1/0-1/2) to give 6-(1-(4-amino-2-(6-azaspiro[2.5]octan-6-yl)phenyl)-1*H-*pyrazol-4-yl)-2-(4,4-difluorocyclohexyl)pyridazin-3(2*H*)-one (100 mg, yield: 50%).

LC-MS, M/Z (ESI): 481.2 [M+H]⁺.

### Step 5: Synthesis of methyl 2-(N-(4-(4-(1-(4,4-difluorocyclohexyl)-6-oxo-1,6-dihydropyridazin-3-yl)-1H-pyrazol-1-yl)-3-(6-azaspiro[2.5]octan-6-yl)phenyl)sulfamoyl)acetate

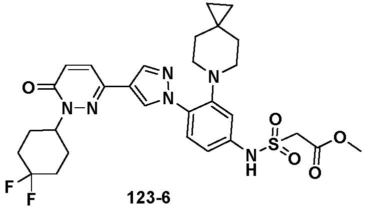

To a solution of 6-(1-(4-amino-2-(6-azaspiro[2.5]octan-6-yl)phenyl)-1*H*-pyrazol-4-yl)-2-(4,4-difluorocyclohexyl) pyridazin-3(2*H*)-one (100 mg, 0.2 mmol) in acetonitrile (1.5 mL) were added methyl 2-(chlorosulfonyl)acetate (57 mg, 0.33 mmol) and potassium phosphate (127 mg, 0.66 mmol), and then the mixture was stirred at 25 °C for 3 h. The reaction liquid was poured into water (20 mL), and the mixture was extracted with ethyl acetate (5 mL × 3). The organic phase was washed with saturated brine (5 mL × 2), dried over anhydrous sodium sulfate, filtered, and concentrated to give a crude product. The crude product was purified by column chromatography (petroleum ether/ethyl acetate (V/V) = 1/0-0/1) to give methyl 2-(N-(4-(4-(1-(4,4-difluorocyclohexyl)-6-oxo-1,6-dihydropyridazin-3-yl)-1*H*-pyrazol-1-yl)-3-(6-azaspiro[2.5]octan-6-yl)phenyl)sulfatnoyl)acetate (80 mg, yield: 60%).

LC-MS, M/Z (ESI): 617.3 [M+H]+.

### Step 6: Synthesis of N-(4-(4-(1-(4,4-difluorocyclohexyl)-6-oxo-1,6-dihydropyridazin-3-yl)-1H-pyrazol-1-yl)-3-(6-azaspiro[2.5]octan-6-yl)phenyl)-2-hydroxyethane-1-sulfonamide

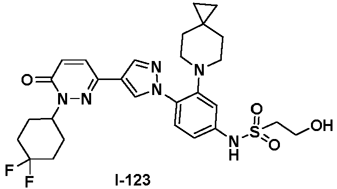

To a solution of methyl 2-(*N*-(4-(4-(1-(4,4-difluorocyclohexyl)-6-oxo-1,6-dihydropyridazin-3-yl)-1*H*-pyrazol-1-yl)-3-(6-azaspiro[2.5]octan-6-yl)phenyl)sulfamoyl)acetate (80 mg, 0.14 mmol) in tetrahydrofuran (1 mL) was added calcium chloride (151 mg, 1.4 mmol), and then sodium borohydride (51 mg, 1.4 mmol) was added slowly. The mixture was stirred at 25 °C for 2 h. The reaction liquid was added dropwise and slowly to a saturated aqueous ammonium chloride solution (10 mL), and ethyl acetate (5 mL) was added. The mixture was then filtered. The filtrate was extracted with ethyl acetate (5 mL × 3), and the organic phase was washed with saturated brine (5 mL × 2), dried over anhydrous sodium sulfate, filtered, and concentrated to give a crude product. The crude product was subjected to preparative reverse phase chromatography (column: Phenomenex Synergi C18 100 × 25 mm × 4 µm; solvents: A = water + 0.1 vol% aqueous ammonia (99%), B = acetonitrile; gradient: 5%-95%, 7 min) to give *N*-(4-(4-(1-(4,4-difluorocyclohexyl)-6-oxo-1,6-dihydropyridazin-3-yl)-1*H*-pyrazol-1-yl)-3-(6-azaspiro[2.5]octan-6-yl)phenyl)-2-hydroxyethane-1-sulfonamide (25 mg, yield: 27%).

1H NMR (400 MHz, DMSO) δ 8.87 (s, 1H), 8.15 (s, 1H), 7.85 (d, 1H), 7.45 (d, 1H), 7.03 - 6.93 (m, 3H), 5.00 (m, 1H), 3.76 - 3.72 (m, 2H), 3.34 - 3.26 (m, 2H), 2.66 - 2.48 (m, 3H), 2.12 - 1.87 (m, 9H), 1.34 (s, 5H), 0.23 (s, 4H). LC-MS, M/Z (ESI): 589.2 [M+H]⁺.

### Example 50: Preparation of Target Compound I-125

### N-(4-(4-(2-(4,4-Difluoropiperidin-1-yl)-6-methylpyrimidin-4-yl)-1H-pyrazol-1-yl)-3-(4-methylpiperidin-1-yl)phenyl)-2-hydroxyethane-1-sulfonamide

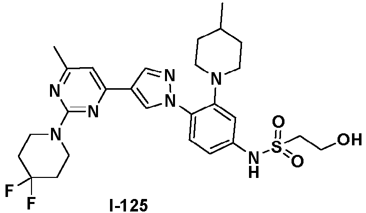

The synthetic route of the target compound **I-125** was as follows:

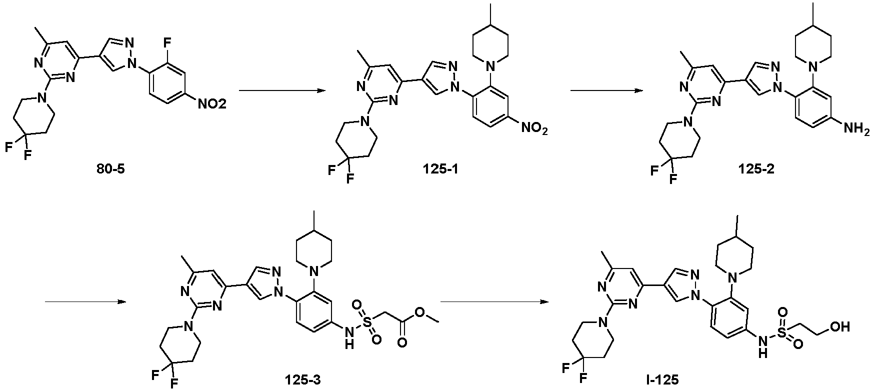

### Step 1: Synthesis of 2-(4,4-difluoropiperidin-1-yl)-4-methyl-6-(1-(2-(4-methylpiperidin-1-yl)-4-nitrophenyl)-1H-pyrazol-4-yl)pyrimidine

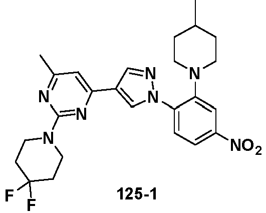

2-(4,4-Difluoropiperidin-1-yl)-4-(1-(2-fluoro-4-nitrophenyl)-1*H*-pyrazol-4-yl)-6-methylpyrimidine (0.5 g, 1.2 mmol), potassium carbonate (0.6 g, 3.6 mmol), and 4-methylpiperidine (0.5 g, 4.2 mmol) were added to dimethylsulfoxide (60 mL), and the mixture was stirred at 130 °C for 8 h. Water (500 mL) was added for dilution, and the mixture was extracted with ethyl acetate (50 mL × 2). The organic phase was concentrated to dryness, and the residue was separated and purified by a silica gel column (petroleum ether:ethyl acetate (V/V) = 1:2) to give 2-(4,4-difluoropiperidin-1-yl)-4-methyl-6-(1-(2-(4-methylpiperidin-1-yl)-4-nitrophenyl)-1*H*-pyrazol-4-yl)pyrimidine (300 mg, yield: 58%).

LC-MS, M/Z (ESI): 498.2 [M+H]⁺.

### Step 2: Synthesis of 4-(4-(2-(4,4-difluoropiperidin-1-yl)-6-methylpyrimidin-4-yl)-1H-pyrazol-1-yl)-3-(4-methylpiperidin-1-yl)aniline

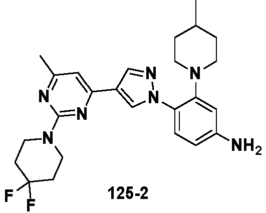

2-(4,4-Difluoropiperidin-1-yl)-4-methyl-6-(1-(2-(4-methylpiperidin-1-yl)-4-nitrophenyl)-1*H*-pyrazol-4-yl)pyrimidine (300 mg, 0.62 mmol), zinc powder (100 mg, 1.8 mmol), and ammonium chloride (310 mg, 5.8 mmol) were added to 3 mL of ethanol and 1 mL of water, and the mixture was heated to 80 °C and allowed to react for 2 h. Water (20 mL) was added for dilution, and the mixture was extracted with ethyl acetate (20 mL × 2). The organic phase was concentrated to dryness, and the residue was separated and purified by a silica gel column (ethyl acetate:methanol (V/V) = 10:1) to give 4-(4-(2-(4,4-difluoropiperidin-1-yl)-6-methylpyrimidin-4-yl)-1*H*-pyrazol-1-yl)-3-(4-methylpiperidin-1-yl)aniline (250 mg, yield: 85%).

LC-MS, M/Z (ESI): 468.2 [M+H]⁺.

### Step 3: Synthesis of methyl 2-(N (4-(4-(2-(4,4-difluoropiperidin-1-yl)-6-methylpyrimidin-4-yl)-1H-pyrazol-1-yl)-3-(4-methylpiperidin-1-yl)phenyl)sulfamoyl)acetate

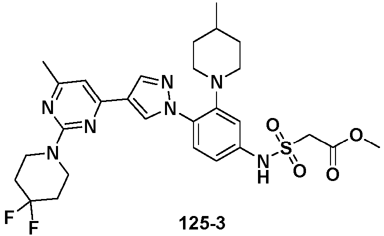

4-(4-(2-(4,4-Difluoropiperidin-1-yl)-6-methylpyrimidin-4-yl)-1*H*-pyrazol-1-yl)-3-(4-methylpiperidin-1-yl)aniline (250 mg, 0.5 mmol), potassium phosphate (318 mg, 1.5 mmol), and methyl 2-(chlorosulfonyl)acetate (104 mg, 0.6 mmol) were added to 2 mL of acetonitrile, and the mixture was allowed to react at room temperature for 1 h. Water (10 mL) was added for dilution, and the mixture was extracted with ethyl acetate (10 mL × 2). The organic phase was concentrated to dryness, and the residue was separated and purified by a silica gel column (ethyl acetate:methanol (V/V) = 10:1) to give 2-(*N*-(4-(4-(2-(4,4-difluoropiperidin-1-yl)-6-methylpyrimidin-4-yl)-1*H*-pyrazol-1-yl)-3-(4-methylpiperidin-1-yl)phenyl)sulfamoyl)acetate (120 mg, yield: 45%).

LC-MS, M/Z (ESI): 604.2 [M+H]⁺.

### Step 4: Synthesis of N-(4-(4-(2-(4,4-difluoropiperidin-1-yl)-6-methylpyrimidin-4-yl)-1H-pyrazol-1-yl)-3-(4-methylpiperidin-1-yl)phenyl)-2-hydroxyethane-1-sulfonamide

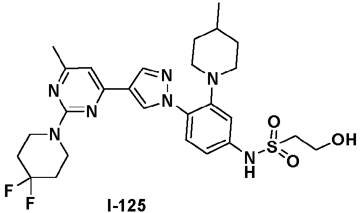

Methyl 2-(N-(4-(4-(2-(4,4-difluoropiperidin-1-yl)-6-methylpyrimidin-4-yl)-1*H*-pyrazol-1-yl)-3-(4-methylpiperidin-1-yl)phenyl)sulfamoyl)acetate (120 mg, 0.22 mmol) was dissolved in tetrahydrofuran (5 mL), and sodium borohydride (500 mg, 4.2 mmol) and calcium chloride (450 mg, 4.2 mmol) were added in batches. The mixture was allowed to react at 20 °C for 1 h. After the reaction was completed, the reaction liquid was quenched with a saturated ammonium chloride solution (25 mL) and extracted with ethyl acetate (20 mL × 3). The organic phase was washed with saturated brine (50 mL), dried over anhydrous sodium sulfate, filtered, and concentrated to give a crude product, which was then separated by reverse phase high performance liquid chromatography (separation method: column: Phenomenex luna C18 150 × 25 mm × 5 µm; solvents: A = water + 0.05% (by volume) trifluoroacetic acid (99.0%), B = acetonitrile; gradient: 38%-68%, 9 min), followed by lyophilization to give *N*-(4-(4-(2-(4,4-difluoropiperidin-1-yl)-6-methylpyrimidin-4-yl)-1*H*-pyrazol-1-yl)-3-(4-methylpiperidin-1-yl)phenyl)-2-hydroxyethane-1-sulfonamide (52 mg, 48%).

LC-MS, M/Z (ESI): 576.2 [M+H]⁺.

¹H NMR (400 MHz, DMSO-d6) δ 9.84 (s, 1H), 8.90 (s, 1H), 8.30 (s, 1H), 7.45 (d, 1H), 7.03 (d, 1H), 6.99 -6.92 (m, 2H), 4.96 (s, 1H), 3.95 (t, 4H), 3.76 (t, 2H), 3.29 (t, 2H), 2.79 (d, 2H), 2.56 - 2.44 (m, 2H), 2.32(s, 3H), 1.99 (td, 4H), 1.61 - 1.50 (m, 2H), 1.48 - 1.30 (m, 1H), 1.19 - 1.06 (m, 2H), 0.85 (d, J = 6.4 Hz, 3H).

### Example 51: Preparation of Target Compound I-129

### N-(4-(4-(2-(4,4-Difluoropiperidin-1-yl)-5-fluoropyrimidin-4-yl)-1H-pyrazol-1-yl)-3-(6-azaspiro[2.5]octan-6-yl)phenyl)-2-hydroxyethane-1-sulfonamide

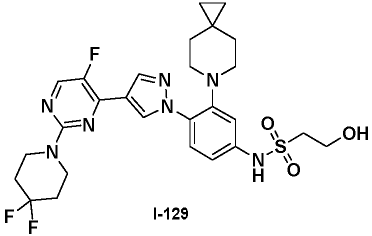

The synthetic route of the target compound **I-129** was as follows:

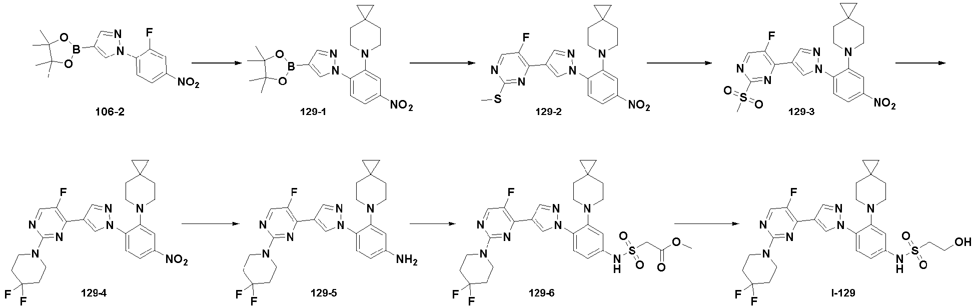

### Step 1: Synthesis of 6-(5-nitro-2-(4-(4,4,5,5-tetramethyl-1,3,2-dioxaborolan-2-yl)-1H-pyrazol-1-yl)phenyl)-6-azaspiro[2.5]octane

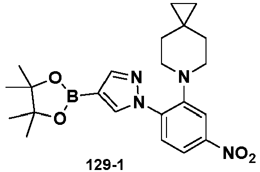

1-(2-Fluoro-4-nitrophenyl)-4-(4,4,5,5-tetramethyl-1,3,2-dioxaborolan-2-yl)-1H-pyrazole (5.1 g, 15.31 mmol) was added to 50 mL of dimethylsulfoxide, and then 6-azaspiro[2.5]octane (5.11 g, 45.9 mmol) and potassium carbonate (15.87 g, 115 mmol) were added. The mixture was heated to 140 °C and allowed to react for 3 h. LCMS showed that the starting materials were completely reacted. The mixture was filtered and then subjected to separation by reverse phase high performance liquid chromatography (separation method: column: Phenomenex luna C18 150 × 70 mm × 10 µm; solvents: A = water + 0.05% (by volume) formic acid (99.0%), B = acetonitrile; gradient: 40%-70%, 20 min), followed by lyophilization to give 6-(5-nitro-2-(4-(4,4,5,5-tetramethyl-1,3,2-dioxaborolan-2-yl)-1H-pyrazol-1-yl)phenyl)-6-azaspiro[2.5]octane (0.4 mg, yield: 6.16%).

LC-MS, M/Z (ESI): 425.2 [M+H]⁺.

### Step 2: Synthesis of 6-(2-(4-(5-fluoro-2-(methylthio)pyrimidin-4-yl)-1H-pyrazol-1-yl)-5-nitrophenyl)-6-azaspiro [2.5]octane

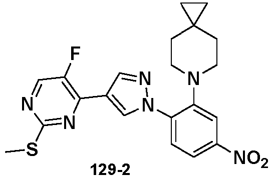

6-(5-Nitro-2-(4-(4,4,5,5-tetramethyl-1,3,2-dioxaborolan-2-yl)-1*H*-pyrazol-1-yl)phenyl)-6-azaspiro[2.5]octane (0.4 g, 0.943 mmol) was added to 8 mL of *N*,*N*-formamide, and then 4-chloro-5-fluoro-2-(methylthio)pyrimidine (0.337 g, 1.885 mmol), potassium carbonate (0.391 g, 2.83 mmol), 2 mL of water, and 1,1'-bis(diphenylphosphino)ferrocene dichloropalladium(II) (0.138 g, 0.189 mmol) were added. The mixture was heated to 105 °C and allowed to react for 4 h. LCMS showed that the reaction was completed. The reaction liquid was poured into 50 mL of water, and the mixture was filtered and lyophilized to give 6-(2-(4-(5-fluoro-2-(methylthio)pyrimidin-4-yl)-1*H*-pyrazol-1-yl)-5-nitrophenyl)-6-azaspiro[2.5]octane (0.4 g, yield: 96%).

LC-MS, M/Z (ESI): 441.1 [M+H]⁺.

### Step 3: Synthesis of 6-(2-(4-(5-fluoro-2-(methylsulfonyl)pyrimidin-4-yl)-1H-pyrazol-1-yl)-5-nitrophenyl)-6-azaspiro[2.5]octane

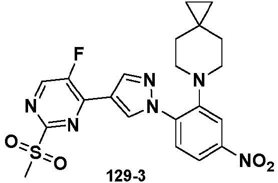

6-(2-(4-(5-Fluoro-2-(methylthio)pyrimidin-4-yl)-1*H*-pyrazol-1-yl)-5-nitrophenyl)-6-azaspiro[2.5]octane (0.4 g, 0.908 mmol) was added to 10 mL of ethanol, and then ammonium molybdate (0.112 g, 0.091 mmol) and 2 mL of 30% hydrogen peroxide were added. The mixture was heated to 80 °C and allowed to react for 48 h. LCMS showed that the starting materials were completely reacted. Saturated sodium chloride (50 mL) was added, and the mixture was stirred for 10 min, filtered, and lyophilized to give a crude product of 6-(2-(4-(5-fluoro-2-(methylsulfonyl)pyrimidin-4-yl)-1H-pyrazol-1-yl)-5-nitrophenyl)-6-azaspiro[2.5]octane (0.466 g, yield: 100%).

LC-MS, M/Z (ESI): 473.1 [M+H]⁺.

### Step 4: Synthesis of 6-(2-(4-(2-(4.4-difluoropiperidin-1-yl)-5-fluoropyrimidin-4-yl)-1H-pyrazol-1-y1)-3-nitrophenyl)-6-azaspiro[2.5]octane

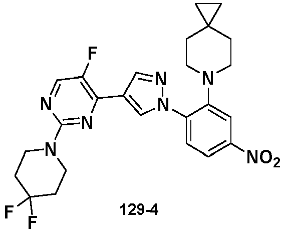

6-(2-(4-(5-Fluoro-2-(methylsulfonyl)pyrimidin-4-yl)-1*H*-pyrazol-1-yl)-5-nitrophenyl)-6-azaspiro[2.5octane (0.466 g, 0.986 mmol) was added to 10 mL of 1,4-dioxane, and then difluoropiperidine hydrochloride (0.933 g, 5.92 mmol) and potassium carbonate (1.363 g, 9.86 mmol) were added. The mixture was heated to 110 °C and allowed to react for 16 h. LCMS showed that the starting materials were completely reacted. The reaction liquid was poured into 200 mL of water, and the mixture was filtered to give 6-(2-(4-(2-(4,4-difluoropiperidin-1-yl)-5-fluoropyrimidin-4-yl)-1*H*-pyrazol-1-yl)-5-nitrophenyl)-6-azaspiro[2.5]octane (0.4 g, yield: 79%).

LC-MS, M/Z (ESI): 514.2 [M+H]⁺.

### Step 5: Synthesis of 4-(4-(2-(4,4-difluoropiperidin-1-yl)-5-fluoropyrimidin-4-yl)-1H-pyrazol-1-yl)-3-(6-azaspiro [2.5]octan-6-yl)aniline

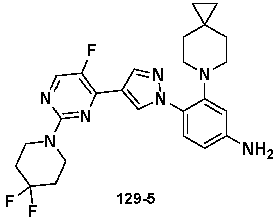

6-(2-(4-(2-(4,4-Difluoropiperidin-1-yl)-5-fluoropyrimidin-4-yl)-1*H*-pyrazol-1-yl)-5-nitrophenyl)-6-azaspiro[2.5]octane (0.4 g, 0.779 mmol) was added to 8 mL of ethanol, and then 2 mL of water, zinc powder (0.764 g, 11.68 mmol), and ammonium chloride (0.417 g, 7.79 mmol) were added. The mixture was heated to 90 °C and allowed to react for 2 h. LCMS showed that the starting materials were completely reacted. The reaction liquid was concentrated to dryness, and the residue was separated and purified by a silica gel column (PE:EA (V/V) = 3:1) to give 4-(4-(2-(4,4-dilluoropiperidin-1-yl)-5-fluoropyrimidin-4-yl)-1*H*-pyrazol-1-yl)-3-(6-azaspiro[2.5]octan-6-yl)aniline (0.25 g, yield: 66.4%).

LC-MS, M/Z (ESI): 484.2 [M+H]⁺.

### Step 6: Synthesis of methyl 2-(N-(4-(4-(2-(4,4-difluoropiperidin-1-yl)-5-fluoropyrimidin-4-yl)-1H-pyrazol-1-yl)-3-(6-azaspiro[2.5]octan-6-yl)phenyl)sulfamoyl)acetate

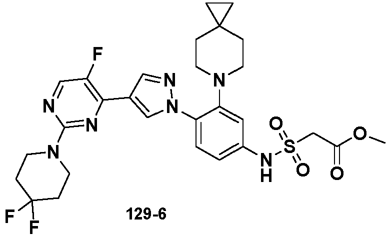

4-(4-(2-(4,4-Difluoropiperidin-1-yl)-5-fluoropyrimidin-4-yl)-1*H*-pyrazol-1-yl)-3-(6-azaspiro[2.5]octan-6-yl)aniline (0.25 g, 0.517 mmol) was added to 5 mL of acetonitrile, and then potassium phosphate (0.406 g, 2.068 mmol) and methyl 2-(chlorosulfonyl)acetate (0.134 g, 0.776 mmol) were added. The mixture was allowed to react at room temperature for 4 h. LCMS showed that the starting materials were completely reacted. The reaction liquid was concentrated to dryness, and the residue was separated and purified by a silica gel column (PE:EA (V/V) = 5:1) to give methyl 2-(*N*-(4-(4-(2-(4,4-difluoropiperidin-1-yl)-5-fluoropyrimidin-4-yl)-1*H*-pyrazol-1-yl)-3-(6-azaspiro[2.5] octan-6-yl)phenyl)sulfamoyl)acetate (0.2 g, yield: 62.4%).

LC-MS, M/Z (ESI): 620.2 [M+H]⁺.

### Step 7: Synthesis of N-(4-(4-(2-(4,4-difluoropiperidin-1-yl)-5-fluoropyrimidin-4-yl)-1H-pyrazol-1-yl)-3-(6-azaspiro[2.5]octan-6-yl)phenyl)-2-hydroxyethane-1-sulfonamide

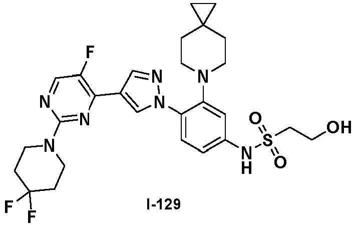

Methyl 2-(*N*-(4-(4-(2-(4,4-difluoropiperidin-1-yl)-5-fluoropyrimidin-4-yl)-1*H*-pyrazol-1-yl)-3-(6-azaspiro[2.5] octan-6-yl)phenyl)sulfamoyl)acetate (0.2 g, 0.329 mmol) was added to 5 mL of ethanol and 5 mL of tetrahydrofuran, and then sodium borohydride (0.124 g, 3.29 mmol) and calcium chloride (0.182 g, 1.643 mmol) were added. The mixture was allowed to react at room temperature for 1 h. LCMS showed that the starting materials were completely reacted. Saturated ammonium chloride (1 mL) was added to quench the reaction. The reaction liquid was then concentrated to dryness, and the residue was separated and purified by a silica gel column (PE:EA (V/V) = 1:1) to give *N*-(4-(4-(2-(4,4-difluoropiperidin-1-yl)-5-fluoropyrimidin-4-yl)-1*H*-pyrazol-1-yl)-3-(6-azaspiro[2.5]octan-6-yl)phenyl)-2-hydroxyethane-1-sulfonamide (95 mg, yield: 49.7%).

LC-MS, M/Z (ESI): 592.2 [M+H]⁺.

¹H NMR (400 MHz,DMSO-d6) δ 9.07 (s, 1H), 8.47 (d, 1H), 8.27 (d, 1H), 7.50 (d, 1H), 7.06(d, 1H), 6.96-6.98(dd, 1H), 3.91 (t, 4H), 3.75 (t, 2H), 3.28 (t, 2H), 2.68 (t, 4H), 1.96-2.05(m, 4H), 1.33 (s, 4H), 0.21 (s, 4H).

**Example 52:** The following compounds were prepared with reference to the preparation methods for the above compounds.

| **No.** | **Structural formula** | **Name** | **LC-MS, M/Z (ESI)** |
|---|---|---|---|
| I-4 | 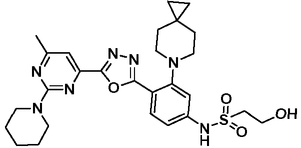 | 2-Hydroxy-*N*-(4-(5-(6-methyl-2-(piperidin-1-yl)pyrimidin-4-yl)-1,3,4-oxadiazol-2-yl)-3-(6-azaspiro[2.5]octan-6-yl)phenyl)ethane-1-sulfonamide | 554.3 |
| I-6 | 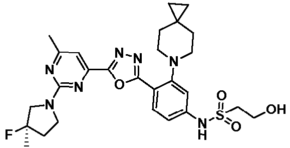 | (*R*)-*N*-(4-(5-(2-(3-Fluoro-3-methylpyrrolidin-1-yl)-6-methylpyrimidin-4-yl)-1,3,4-oxadiazol-2-yl)-3-(6-azaspiro[2.5]octan-6-yl)phenyl)-2-hydroxyethane-1-sulfonamide | 572.2 |
| I-9 | 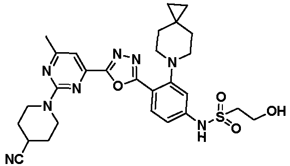 | *N*-(4-(5-(2-(4-Cyanopiperidin-1-yl)-6-methylpyrimidin-4-yl)-1,3,4-oxadiazol-2-yl)-3-(6-azaspiro[2.5]octan-6-yl)phenyl)-2-hydroxyethane-1-sulfonamide | 579.7 |
| I-10 | 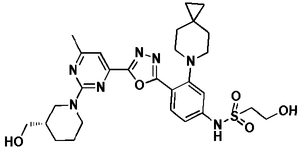 | (*S*)-*N*-(4-(5-(2-(3-(Hydroxymethyl)piperidin-1-yl)-6-methylpyrimidin-4-yl)-1,3,4-oxadiazol-2-yl)-3-(6-azaspiro[2.5]octan-6-yl)phenyl)-2-hydroxyethane-1-sulfonamide | 584.3 |
| I-11 | 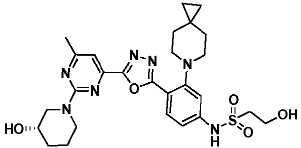 | (*S*)-*N*-(4-(5-(2-(3-Hydroxypiperidin-1-yl)-6-methylpyrimidin-4-yl)-1,3,4-oxadiazol-2-yl)-3-(6-azaspiro[2.5]octan-6-yl)phenyl)-2-hydroxyethane-1-sulfonamide | 570.2 |
| I-12 | 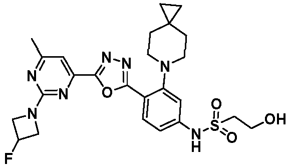 | *N*-(4-(5-(2-(3-Fluoroazetidin-1-yl)-6-methylpyrimidin-4-yl)-1,3,4-oxadiazol-2-yl)-3-(6-azaspiro[2.5]octan-6-yl)phenyl)-2-hydroxyethane-1-sulfonamide | 544.2 |
| I-13 | 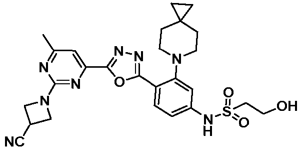 | *N*-(4-(5-(2-(3-Cyanoazetidin-1-yl)-6-methylpyrimidin-4-yl)-1,3,4-oxadiazol-2-yl)-3-(6-azaspiro[2.5]octan-6-yl)phenyl)-2-hydroxyethane-1-sulfonamide | 551.2 |
| I-14 | 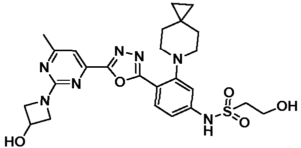 | *N*-(4-(5-(2-(3-Hydroxyazetidin-1-yl)-6-methylpyrimidin-4-yl)-1,3,4-oxadiazol-2-yl)-3-(6-azaspiro[2.5]octan-6-yl)phenyl)-2-hydroxyethane-1-sulfonamide | 542.2 |
| I-15 | 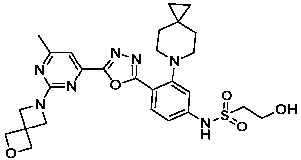 | *N*-(4-(5-(6-Methyl-2-(2-oxa-6-azaspiro[3.3]heptan-6-yl)pyrimidin-4-yl)-1,3,4-oxadiazol-2-yl)-3-(6-azaspiro [2.5] octan-6-yl)phenyl)-2-hydroxyethane-1-sulfonamide | 568.2 |
| I-16 | 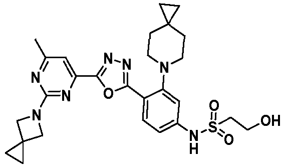 | *N*-(4-(5-(6-Methyl-2-(5-azaspiro[2.3]hexan-5-yl)pyrimidin-4-yl)-1,3,4-oxadiazol-2-yl)-3-(6-azaspiro [2.5] octan-6-yl)phenyl)-2-hydroxyethane-1-sulfonamide | 552.2 |
| I-18 | 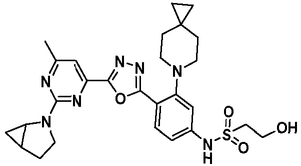 | *N*-(4-(5-(2-(2-Azabicyclo[3.1.0]hexan-2-yl)-6-methylpyrimidin-4-yl)-1,3,4-oxadiazol-2-yl)-3-(6-azaspiro[2.5]octan-6-yl)phenyl)-2-hydroxyethane-1-sulfonamide | 552.2 |
| I-19 | 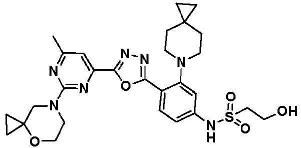 | 2-Hydroxy-*N*-(4-(5-(6-methyl-2-(4-oxa-7-azaspiro[2.5]octan-7-yl)pyrimidin-4-yl)-1,3,4-oxadiazol-2-yl)-3-(6-azaspiro[2.5]octan-6-yl)phenyl)ethane-1-sulfonamide | 582.2 |
| I-20 | 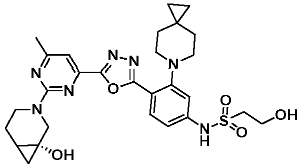 | 2-Hydroxy-*N*-(4-(5-(2-((1R)-1-hydroxy-3-azabicyclo [4.1.0]heptan-3-yl)-6-methylpyrimidin-4-yl)-1,3,4-oxadiazol-2-yl)-3-(6-azaspiro[2.5]octan-6-yl)phenyl)ethane-1-sulfonamide | 582.2 |
| I-21 | 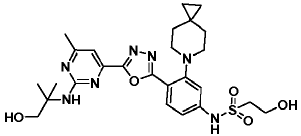 | 2-Hydroxy-*N*-(4-(5-(2-((1-hydroxy-2-methylpropan-2-yl)amino)-6-methylpyrimidin-4-yl)-1,3,4-oxadiazol-2-yl)-3-(6-azaspiro[2.5]octan-6-yl)phenyl)ethane-1-sulfonamide | 558.2 |
| I-22 | 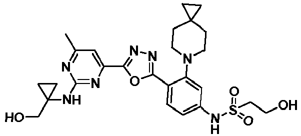 | 2-Hydroxy-*N*-(4-(5-(2-((1-(hydroxymethyl)cyclopropyl)amino)-6-methylpyrimidin-4-yl)-1,3,4-oxadiazol-2-yl)-3-(6-azaspiro[2.5]octan-6-yl)phenyl)ethane-1-sulfonamide | 556.2 |
| I-23 | 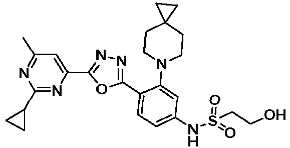 | *N*-(4-(5-(2-Cyclopropyl-6-methylpyrimidin-4-yl)-1,3,4-oxadiazol-2-yl)-3-(6-azaspiro[2.5]octan-6-yl)phenyl)-2-hydroxyethane-1-sulfonamide | 511.2 |
| 1-24 | 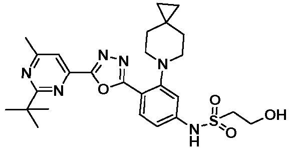 | *N*-(4-(5-(2-(*tert*-Butyl)-6-methylpyrimidin-4-yl)-1,3,4-oxadiazol-2-yl)-3-(6-azaspiro[2.5]octan-6-yl)phenyl)-2-hydroxyethane-1-sulfonamide | 527.3 |
| I-25 | 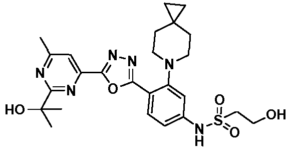 | 2-Hydroxy-*N*-(4-(5-(2-(2-hydroxypropan-2-yl)-6-methylpyrimidin-4-yl)-1,3,4-oxadiazol-2-yl)-3-(6-azaspiro[2.5]octan-6-yl)phenyl)ethane-1-sulfonamide | 529.2 |
| I-26 | 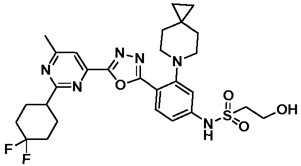 | *N*-(4-(5-(2-(4,4-Difluorocyclohexyl)-6-methylpyrimidin-4-yl)-1,3,4-oxadiazol-2-yl)-3-(6-azaspiro[2.5]octan-6-yl)phenyl)-2-hydroxyethane-1-sulfonamide | 589.2 |
| I-28 | 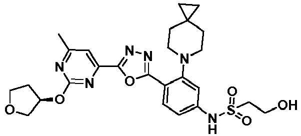 | (*R*)-2-Hydroxy-*N*-(4-(5-(6-methyl-2-((tetrahydrofuran-3-yl)oxy)pyrimidin-4-yl)-1,3,4-oxadiazol-2-yl)-3-(6-azaspiro[2.5]octan-6-yl)phenyl)ethane-1-sulfonamide | 557.2 |
| I-29 | 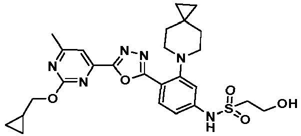 | *N*-(4-(5-(2-(Cyclopropylmethoxy)-6-methylpyrimidin-4-yl)-1,3,4-oxadiazol-2-yl)-3-(6-azaspiro[2.5]octan-6-yl)phenyl)-2-hydroxyethane-1-sulfonamide | 541.2 |
| I-30 | 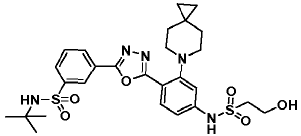 | *N*-(*tert*-Butyl)-3-(5-(4-((2-hydroxyethyl)sulfonamido)-2-(6-azaspiro[2.5]octan-6-yl)phenyl)-1,3,4-oxadiazol-2-yl)benzenesulfonamide | 590.2 |
| I-31 | 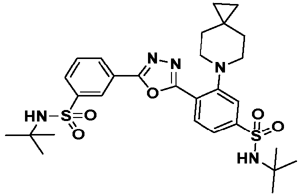 | *N*-(*tert*-Butyl)-4-(5-(3-(N-(tert-butyl)sulfamoyl)phenyl)-1,3,4-oxadiazol-2-yl)-3-(6-azaspiro[2.5]octan-6-yl)benzenesulfonamide | 602.3 |
| I-32 | 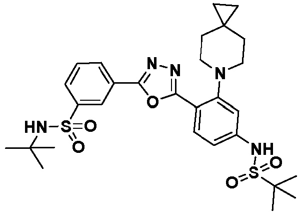 | *N-*(*tert-Butyl*)-3-(5-(4-((1,1-dimethylethyl)sulfonamido)-2-(6-azaspiro[2.5]octan-6-yl)phenyl)-1,3,4-oxadiazol-2-yl)benzenesulfonamide | 602.3 |
| I-33 | 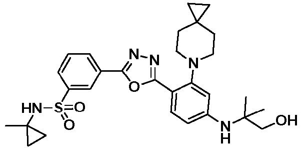 | 3-(5-(4-((1-Hydroxy-2-methylpropan-2-yl)amino)-2-(6-azaspiro[2.5]octan-6-yl)phenyl)-1,3,4-oxadiazol-2-yl)-*N*-(1-methylcyclopropyl)benzenesulfonamide | 552.3 |
| I-34 | 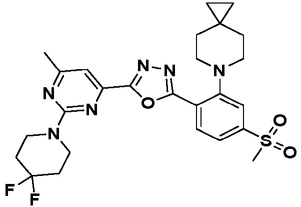 | 2-(2-(4,4-Difluoropiperidin-1-yl)-6-methylpyrimidin-4-yl)-5-(4-(methylsulfonyl)-2-(6-azaspiro[2.5]octan-6-yl)phenyl)-1,3,4-oxadiazole | 545.2 |
| I-35 | 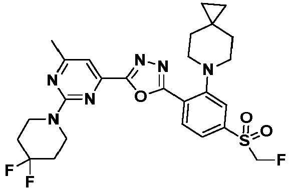 | 2-(2-(4,4-Difluoropiperidin-1-yl)-6-methylpyrimidin-4-yl)-5-(4-((fluoromethyl)sulfonyl)-2-(6-azaspiro[2.5]octan-6-yl)phenyl)-1,3,4-oxadiazole | 563.2 |
| I-36 | 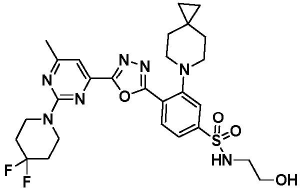 | 4-(5-(2-(4,4-Difluoropiperidin-1-yl)-6-methylpyrimidin-4-yl)-1,3,4-oxadiazol-2-yl)-*N*-(2-hydroxyethyl)-3-(6-azaspiro[2.5]octan-6-yl)benzenesulfonamide | 590.2 |
| I-37 | 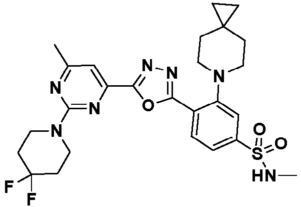 | 4-(5-(2-(4,4-Difluoropiperidin-1-yl)-6-methylpyrimidin-4-yl)-1,3,4-oxadiazol-2-yl)-*N*-methyl-3-(6-azaspiro[2.5]octan-6-yl)benzenesulfonamide | 560.2 |
| I-38 | 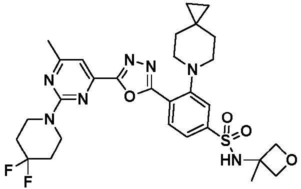 | 4-(5-(2-(4,4-Difluoropiperidin-1-yl)-6-methylpyrimidin-4-yl)-1,3,4-oxadiazol-2-yl)-*N*-(3-methyloxetan-3-yl)-3-(6-azaspiro[2.5]octan-6-yl)benzenesulfonamide | 616.3 |
| I-39 | 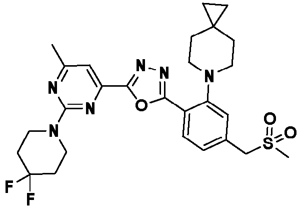 | 2-(2-(4,4-Difluoropiperidin-1-yl)-6-methylpyrimidin-4-yl)-5-(4-((methylsulfonyl)methyl)-2-(6-azaspiro[2.5]octan-6-yl)phenyl)-1,3,4-oxadiazole | 559.2 |
| I-42 | 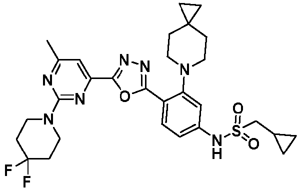 | 1-Cyclopropyl-*N*-(4-(5-(2-(4,4-difluoropiperidin-1-yl)-6-methylpyrimidin-4-yl)-1,3,4-oxadiazol-2-yl)-3-(6-azaspiro[2.5]octan-6-yl)phenyl)methanesulfonamide | 600.3 |
| I-43 | 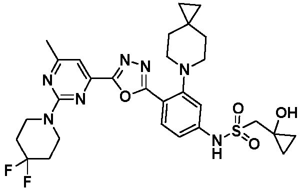 | *N*-(4-(5-(2-(4,4-Difluoropiperidin-1-yl)-6-methylpyrimidin-4-yl)-1,3,4-oxadiazol-2-yl)-3-(6-azaspiro[2.5]octan-6-yl)phenyl)-1-(1-hydroxycyclopropyl)methanesulfonamid e | 616.3 |
| I-44 | 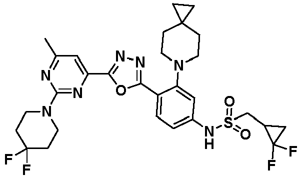 | 1-(2,2-Difluorocyclopropyl)-*N*-(4-(5-(2-(4,4-difluoropiperidin-1-yl)-6-methylpyrimidin-4-yl)-1,3,4-oxadiazol-2-yl)-3-(6-azaspiro[2.5]octan-6-yl)phenyl)methanesulfonamide | 636.2 |
| I-45 | 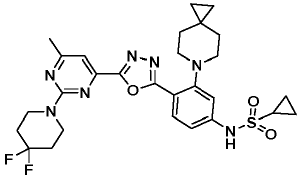 | *N*-(4-(5-(2-(4,4-Difluoropiperidin-1-yl)-6-methylpyrimidin-4-yl)-1,3,4-oxadiazol-2-yl)-3-(6-azaspiro[2.5]octan-6-yl)phenyl)cyclopropanesulfonamide | 586.2 |
| I-46 | 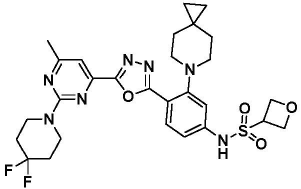 | *N*-(4-(5-(2-(4,4-Difluoropiperidin-1-yl)-6-methylpyrimidin-4-yl)-1,3,4-oxadiazol-2-yl)-3-(6-azaspiro[2.5]octan-6-yl)phenyl)oxetane-3-sulfonamide | 602.2 |
| I-47 | 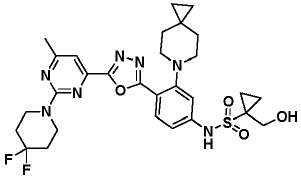 | *N*-(4-(5-(2-(4,4-Difluoropiperidin-1-yl)-6-methylpyrimidin-4-yl)-1,3,4-oxadiazol-2-yl)-3-(6-azaspiro[2.5]octan-6-yl)phenyl)-1-(hydroxymethyl)cyclopropane-1-sulfonamide | 616.2 |
| I-48 | 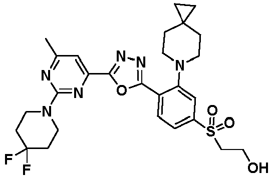 | 2-((4-(5-(2-(4,4-Difluoropiperidin-1-yl)-6-methylpyrimidin-4-yl)-1,3,4-oxadiazol-2-yl)-3-(6-azaspiro[2.5]octan-6-yl)phenyl)sulfonyl)ethan-1-ol | 575.2 |
| I-49 | 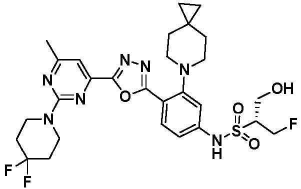 | (*R*)-*N*-(4-(5-(2-(4,4-Difluoropiperidin-1-yl)-6-methylpyrimidin-4-yl)-1,3,4-oxadiazol-2-yl)-3-(6-azaspiro[2.5]octan-6-yl)phenyl)-1-fluoro-3-hydroxypropane-2-sulfonamide | 622.2 |
| I-50 | 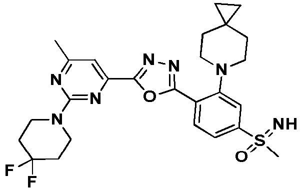 | (4-(5-(2-(4,4-Difluoropiperidin-1-yl)-6-methylpyrimidin-4-yl)-1,3,4-oxadiazol-2-yl)-3-(6-azaspiro[2.5]octan-6-yl)phenyl)(imino)(methyl)-16-sulfanone | 544.2 |
| I-51 | 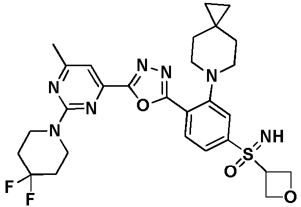 | (4-(5-(2-(4,4-Difluoropiperidin-1-yl)-6-methylpyrimidin-4-yl)-1,3,4-oxadiazol-2-yl)-3-(6-azaspiro[2.5]octan-6-yl)phenyl)(imino)(oxetan-3-yl)-16-sulfanone | 586.2 |
| I-54 | 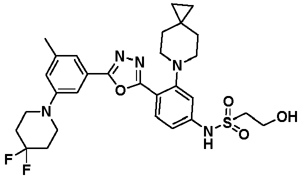 | *N*-(4-(5-(3-(4,4-Difluoropiperidin-1-yl)-5-methylphenyl)-1,3,4-oxadiazol-2-yl)-3-(6-azaspiro[2.5]octan-6-yl)phenyl)-2-hydroxyethane-1-sulfonamide | 588.2 |
| I-55 | 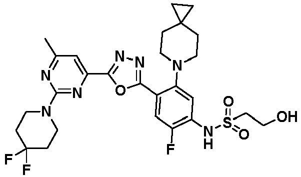 | *N*-(4-(5-(2-(4,4-Difluoropiperidin-1-yl)-6-methylpyrimidin-4-yl)-1,3,4-oxadiazol-2-yl)-2-fluoro-5-(6-azaspiro [2.5]octan-6-yl)phenyl)-2-hydroxyethane-1-sulfonamide | 608.2 |
| I-56 | 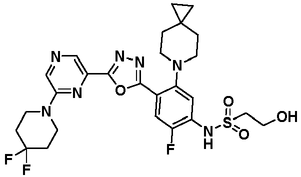 | *N*-(4-(5-(6-(4,4-Difluoropiperidin-1-yl)pyrazin-2-yl)-1,3,4-oxadiazol-2-yl)-2-fluoro-5-(6-azaspiro[2.5]octan-6-yl)phenyl)-2-hydroxyethane-1-sulfonamide | 594.2 |
| I-57 | 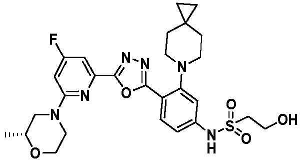 | (*R*)-*N*-(4-(5-(4-Fluoro-6-(2-methylmorpholino)pyridin-2-yl)-1,3,4-oxadiazol-2-yl)-3-(6-azaspiro[2.5]octan-6-yl)phenyl)-2-hydroxyethane-1-sulfonamide | 573.2 |
| I-58 | 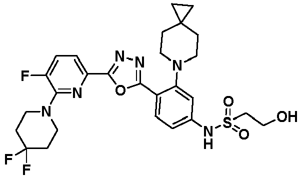 | *N*-(4-(5-(6-(4,4-Difluoropiperidin-1-yl)-5-fluoropyridin-2-yl)-1,3,4-oxadiazol-2-yl)-3-(6-azaspiro[2.5]octan-6-yl)phenyl)-2-hydroxyethane-1-sulfonamide | 593.2 |
| I-59 | 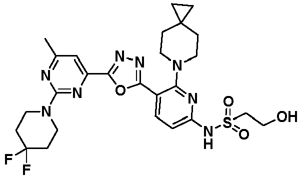 | *N*-(5-(5-(2-(4,4-Difluoropiperidin-1-yl)-6-methylpyrimidin-4-yl)-1,3,4-oxadiazol-2-yl)-6-(6-azaspiro[2.5]octan-6-yl)pyridin-2-yl)-2-hydroxyethane-1-sulfonamide | 591.2 |
| I-60 | 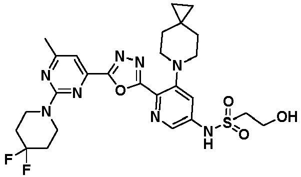 | *N*-(6-(5-(2-(4,4-Difluoropiperidin-1-yl)-6-methylpyrimidin-4-yl)-1,3,4-oxadiazol-2-yl)-5-(6-azaspiro[2.5]octan-6-yl)pyridin-3-yl)-2-hydroxyethane-1-sulfonamide | 591.2 |
| I-61 | 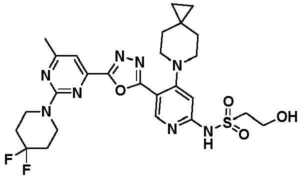 | *N*-(5-(5-(2-(4,4-Difluoropiperidin-1-yl)-6-methylpyrimidin-4-yl)-1,3,4-oxadiazol-2-yl)-4-(6-azaspiro [2.5]octan-6-yl)pyridin-2-yl)-2-hydroxyethane-1-sulfonamide | 591.2 |
| I-62 | 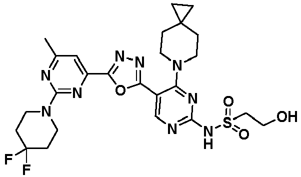 | *N*-(5-(5-(2-(4,4-Difluoropiperidin-1-yl)-6-methylpyrimidin-4-yl)-1,3,4-oxadiazol-2-yl)-4-(6-azaspiro [2.5]octan-6-yl)pyrimidin-2-yl)-2-hydroxyethane-1-sulfonamide | 592.1 |
| I-63 | 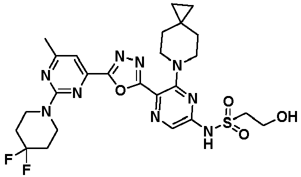 | *N*-(5-(5-(2-(4,4-Difluoropiperidin-1-yl)-6-methylpyrimidin-4-yl)-1,3,4-oxadiazol-2-yl)-6-(6-azaspiro[2.5]octan-6-yl)pyrazin-2-yl)-2-hydroxyethane-1-sulfonamide | 592.1 |
| I-64 | 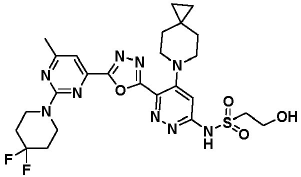 | *N*-(6-(5-(2-(4,4-Difluoropiperidin-1-yl)-6-methylpyrimidin-4-yl)-1,3,4-oxadiazol-2-yl)-5-(6-azaspiro[2.5]octan-6-yl)pyridazin-3-yl)-2-hydroxyethane-1-sulfonamide | 592.1 |
| I-66 | 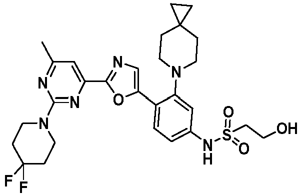 | *N*-(4-(2-(2-(4,4-Difluoropiperidin-1-yl)-6-methylpyrimidin-4-yl)oxazol-5-yl)-3-(6-azaspiro[2.5]octan-6-yl)phenyl)-2-hydroxyethane-1-sulfonamide | 589.2 |
| I-67 | 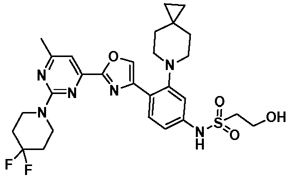 | *N*-(4-(2-(2-(4,4-Difluoropiperidin-1-yl)-6-methylpyrimidin-4-yl)oxazol-4-yl)-3-(6-azaspiro[2.5]octan-6-yl)phenyl)-2-hydroxyethane-1-sulfonamide | 589.2 |
| I-68 | 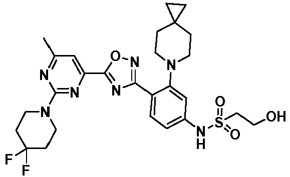 | *N*-(4-(5-(2-(4,4-Difluoropiperidin-1-yl)-6-methylpyrimidin-4-yl)-1,2,4-oxadiazol-3-yl)-3-(6-azaspiro[2.5]octan-6-yl)phenyl)-2-hydroxyethane-1-sulfonamide | 590.2 |
| I-69 | 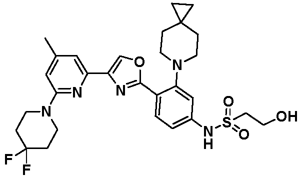 | *N*-(4-(4-(6-(4,4-Difluoropiperidin-1-yl)-4-methylpyridin-2-yl)oxazol-2-yl)-3-(6-azaspiro [2.5] octan-6-yl)phenyl)-2-hydroxyethane-1-sulfonamide | 588.3 |
| I-70 | 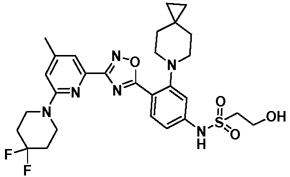 | *N*-(4-(3-(6-(4,4-Difluoropiperidin-1-yl)-4-methylpyridin-2-yl)-1,2,4-oxadiazol-5-yl)-3-(6-azaspiro[2.5]octan-6-yl)phenyl)-2-hydroxyethane-1-sulfonamide | 589.2 |
| I-71 | 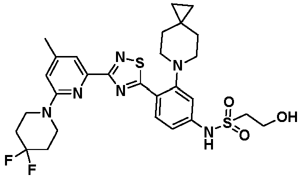 | *N*-(4-(3-(6-(4,4-Difluoropiperidin-1-yl)-4-methylpyridin-2-yl)-1,2,4-thiadiazol-5-yl)-3-(6-azaspiro[2.5]octan-6-yl)phenyl)-2-hydroxyethane-1-sulfonamide | 605.2 |
| I-72 | 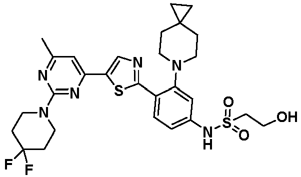 | *N*-(4-(5-(2-(4,4-Difluoropiperidin-1-yl)-6-methylpyrimidin-4-yl)thiazol-2-yl)-3-(6-azaspiro[2.5]octan-6-yl)phenyl)-2-hydroxyethane-1-sulfonamide | 605.2 |
| I-73 | 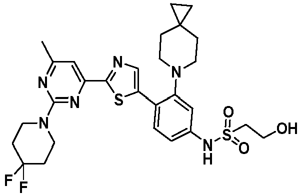 | *N*-(4-(2-(2-(4,4-Difluoropiperidin-1-yl)-6-methylpyrimidin-4-yl)thiazol-5-yl)-3-(6-azaspiro[2.5]octan-6-yl)phenyl)-2-hydroxyethane-1-sulfonamide | 605.2 |
| I-74 | 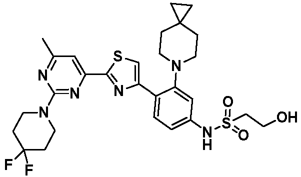 | *N*-(4-(2-(2-(4,4-Difluoropiperidin-1-yl)-6-methylpyrimidin-4-yl)thiazol-4-yl)-3-(6-azaspiro[2.5]octan-6-yl)phenyl)-2-hydroxyethane-1-sulfonamide | 605.2 |
| I-75 | 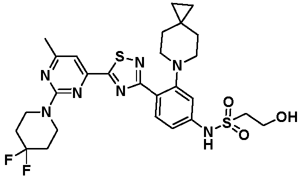 | *N*-(4-(5-(2-(4,4-Difluoropiperidin-1-yl)-6-methylpyrimidin-4-yl)-1,2,4-thiadiazol-3-yl)-3-(6-azaspiro[2.5]octan-6-yl)phenyl)-2-hydroxyethane-1-sulfonamide | 606.2 |
| I-76 | 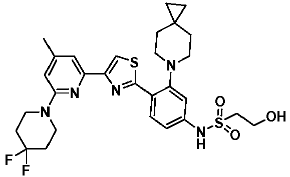 | *N*-(4-(4-(6-(4,4-Difluoropiperidin-1-yl)-4-methylpyridin-2-yl)thiazol-2-yl)-3-(6-azaspiro[2.5]octan)-6-yl)phenyl)-2-hydroxyethane-1-sulfonamide | 604.2 |
| I-78 | 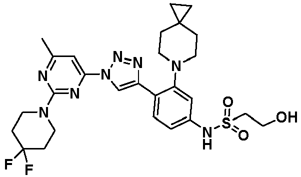 | *N*-(4-(1-(2-(4,4-Difluoropiperidin-1-yl)-6-methylpyrimidin-4-yl)-1*H*-1,2,3-triazol-4-yl)-3-(6-azaspiro[2.5]octan-6-yl)phenyl)-2-hydroxyethane-1-sulfonamide | 589.2 |
| I-89 | 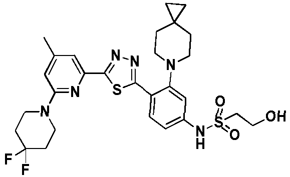 | *N*-(4-(5-(6-(4,4-Difluoropiperidin-1-yl)-4-methylpyridin-2-yl)-1,3,4-thiadiazol-2-yl)-3-(6-azaspiro[2.5]octan-6-yl)phenyl)-2-hydroxyethane-1-sulfonamide | 605.2 |
| | 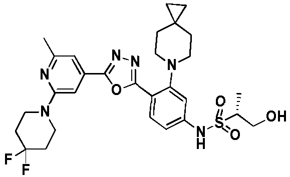 | (*R*)-*N*-(4-(5-(2-(4,4-Difluoropiperidin-1-yl)-6-methylpyridin-4-yl)-1,3,4-oxadiazol-2-yl)-3-(6-azaspiro[2.5]octan-6-yl)phenyl)-1-hydroxypropane-2-sulfonamide | 603.3 |
| | 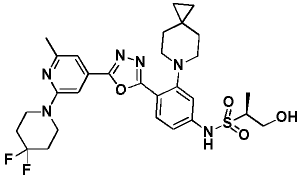 | (*S*)-*N*-(4-(5-(2-(4,4-Difluoropiperidin-1-yl)-6-methylpyridin-4-yl)-1,3,4-oxadiazol-2-yl)-3-(6-azaspiro[2.5]octan-6-yl)phenyl)-1-hydroxypropane-2-sulfonamide | 603.3 |
| I-93 | 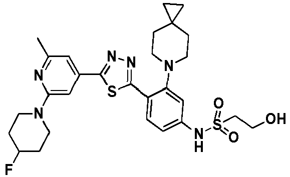 | *N*-(4-(5-(2-(4-Fluoropiperidin-1-yl)-6-methylpyridin-4-yl)-1,3,4-thiadiazol-2-yl)-3-(6-azaspiro[2.5]octan-6-yl)phenyl)-2-hydroxyethane-1-sulfonamide | 587.2 |
| I-94 | 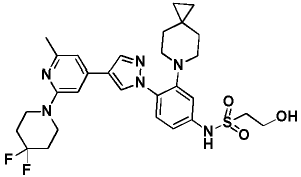 | *N*-(4-(4-(2-(4,4-Difluoropiperidin-1-yl)-6-methylpyridin-4-yl)-1*H*-pyrazol-1-yl)-3 -(6-azaspiro [2.5]octan-6-yl)phenyl)-2-hydroxyethane-1-sulfonamide | 587.3 |
| I-108 | 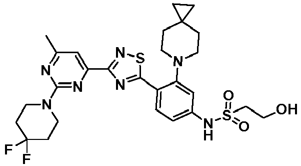 | *N*-(4-(3-(2-(4,4-Difluoropiperidin-1-yl)-6-methylpyrimidin-4-yl)-1,2,4-thiadiazol-5-yl)-3-(6-azaspiro[2.5]octan-6-yl)phenyl)-2-hydroxyethane-1-sulfonamide | 606.2 |
| I-119 | 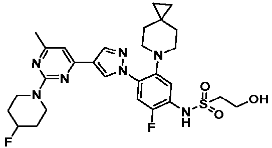 | *N*-(2-Fluoro-4-(4-(2-(4-fluoropiperidin-1-yl)-6-methylpyrimidin-4-yl)-1*H-*pyrazol-1-yl)-5-(6-azaspiro[2.5]octan-6-yl)phenyl)-2-hydroxyethane-1-sulfonamide | 588.3 |
| I-120 | 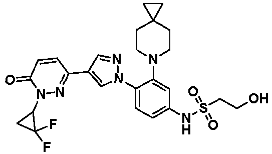 | *N*-(4-(4-(1-(2,2-Difluorocyclopropyl)-6-oxo-1,6-dihydropyridazin-3-yl)-1*H-*pyrazol-1-yl)-3-(6-azaspiro[2.5]octan-6-yl)phenyl)-2-hydroxyethane-1-sulfonamide | 547.2 |
| I-122 | 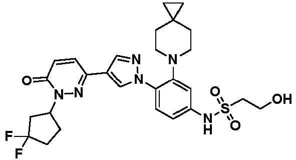 | *N*-(4-(4-(1-(3,3-Difluorocyclopentyl)-6-oxo-1,6-dihydropyridazin-3-yl)-1*H-*pyrazol-1-yl)-3-(6-azaspiro[2.5]octan-6-yl)phenyl)-2-hydroxyethane-1-sulfonamide | 575.2 |
| I-124 | 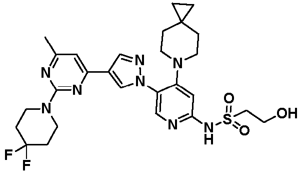 | *N*-(5-(4-(2-(4,4-Difluoropiperidin-1-yl)-6-methylpyrimidin-4-yl)-1*H*-pyrazol-1-yl)-4-(6-azaspiro [2.5]octan-6-yl)pyridin-2-yl)-2-hydroxyethane-1-sulfonamide | 589.2 |
| I-126 | 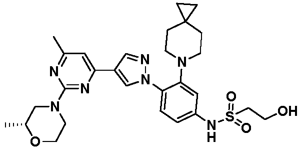 | (*R*)-2-Hydroxy-N-(4-(4-(6-methyl-2-(2-methylmorpholinyl)pyrimidin-4-yl)-1*H-*pyrazol-1-yl)-3-(6-azaspiro[2.5]octan-6-yl)phenyl)-2-hydroxyethane-1-sulfonamide | 568.3 |
| I-127 | 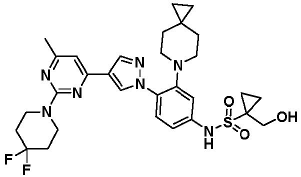 | *N*-(4-(4-(2-(4,4-Difluoropiperidin-1-yl)-6-methylpyrimidin-4-yl)-1H-pyrazol-1-yl)-3-(6-azaspiro[2.5]octan-6-yl)phenyl)-1-(hydroxymethyl)cyclopropane-1-sulfonamide | 614.3 |
| I-128 | 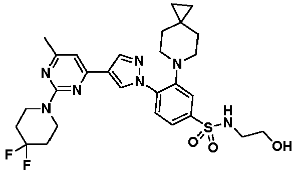 | 4-(4-(2-(4,4-Difluoropiperidin-1-yl)-6-methylpyrimidin-4-yl)-1*H*-pyrazol-1-yl)-N-(2-hydroxyethyl)-3-(6-azaspiro[2.5]octan-6-yl)benzenesulfonamide | 588.3 |
| I-132 | 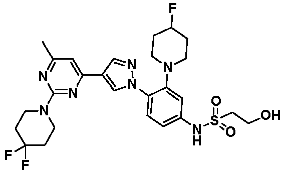 | *N*-(4-(4-(2-(4,4-Difluoropiperidin-1-yl)-6-methylpyrimidin-4-yl)-1*H*-pyrazol-1-yl)-3-(4-fluoropiperidin-1-yl)phenyl)-2-hydroxyethane-1-sulfonamide | 580.2 |

### Test Example 1. Assay on Inhibition of KIF18A Enzymatic Activity by Compounds

The recombinant protein KIF18A (kinesin domain: aa 1-467) used in this assay was expressed by the Bac-to-Bac baculovirus expression system from Invitrogen, and the resulting protein was purified for use in this assay (Seki, M. et al. 2003 Nucleic Acids Res.). Quantitative detection of KIF18A enzymatic activity was completed using an ADP-Glo^{™} kinase assay kit (Promega Inc). Related procedures strictly followed the product instructions, which are briefly described as follows:
The reaction system consisted of a test compound, the recombinant protein KIF18A (aa 1-467), ATP (Promega Inc), and an assay buffer. The test compound was prepared into a 0.5 mM stock solution using DMSO (Sigma Inc) and then serially diluted in DMSO. The assay buffer consisted of an aqueous solution of 15 mM Tris, 10 mM MgCl₂ (Sigma Inc), 0.01% Pluronic F-68 (Life Technologies Inc), 1 µM paclitaxel (Cytoskeleton Inc), 30 µg/mL porcine tubulin (Cytoskeleton Inc), and 2% DMSO. The KIF18A protein (final concentration: 80 nM) and the compound at different concentrations (1 µL) were added to the prepared assay buffer (50 µL), and the mixture was incubated at room temperature for 15 min. Then, ATP (final concentration: 80 µM) was added to the reaction mixture, and the resulting mixture was incubated at room temperature for 3 h. After the reaction was completed, 5 µL of the ADP-Glo^{™} reagent and 2.5 µL of the reaction mixture were added to a 384-well plate (Grenier Inc). The resulting mixture was mixed homogeneously, then sealed by an aluminum foil sealing film, and incubated at room temperature in the dark for 40 min. Finally, 10 µL of the ADP-Glo^{™} assay reagent was added to each reaction well, and the mixture was incubated at room temperature in the dark for 40 min. After all reactions were completed, the luminescence values of the wells were read on a microplate reader (Molecular Device_SpectraMax Id5), and the inhibition ratios were calculated. The calculation formula for the inhibition rates was as follows: inhibition rate = (1 - (compound group - blank group) / (negative control group - blank group)) × 100%. Then, based on the inhibition rates at different compound concentrations, the IC₅₀ value of the test compound was calculated using the GraphPad Prism 8 software.

**Table 1. Inhibitory effects of compounds on KIF18A enzymatic activity**

| Test compound | IC₅₀ (µM) |
|---|---|
| I-1 | 0.033 |
| I-2 | 0.002 |
| I-3 | 0.011 |
| I-5 | 0.007 |
| I-7 | 0.001 |
| I-8 | 0.002 |
| I-17 | 0.010 |
| I-27 | 0.001 |
| I-40 | 0.011 |
| I-52 | 0.001 |
| I-53 | 0.025 |
| I-77 | 0.002 |
| I-79 | 0.004 |
| I-80 | 0.011 |
| I-85 | 0.022 |
| I-86 | 0.003 |
| I-87 | 0.004 |
| I-88 | 0.001 |
| I-95 | 0.002 |
| I-98 | 0.018 |
| I-99 | 0.003 |
| I-100 | 0.010 |
| I-101 | 0.008 |
| I-102 | 0.004 |
| I-103 | 0.012 |
| I-104 | 0.013 |
| I-105 | 0.002 |
| I-106 | 0.011 |
| I-107 | 0.008 |
| I-109 | 0.005 |
| I-110 | 0.002 |
| I-111 | 0.005 |
| I-112 | 0.002 |
| I-113 | 0.007 |
| I-114 | 0.007 |
| I-115 | 0.005 |
| I-116 | 0.013 |
| I-117 | 0.009 |
| I-118 | 0.002 |
| I-121 | 0.003 |
| I-125 | 0.005 |
| I-129 | 0.009 |
| I-130 | 0.009 |

The experimental results indicate that: the assay on inhibition of KIF18A enzymatic activity shows that the compounds of the present disclosure have good inhibitory activity against the KIF18A enzyme.

### Test Example 2. Assay on Inhibition of OVCAR3 Tumor Cell Proliferation by Compounds

NIH:OVCAR-3 cells (ATCC, Cat No. HTB-161, purchased from Co-Bier) were seeded into a T75 culture flask and cultured in an RPMI 1640 medium containing 20% FBS for 3 days in preparation for subsequent culturing and seeding into a 96-well plate for the cell proliferation assay.

The OVCAR3 cells were seeded at a density of 3000 cells/100 µL/well into the 96-well cell plate, and then the plate was incubated in an incubator (37 °C, 5% CO₂) for 18 h. On the second day, drug treatment was started. Serially diluted test compound solutions (each drug starting at a concentration of 10 µM, diluted in DMSO, with a dilution ratio of 1:3, and nine gradient points for each drug) or DMSO (negative control) was added to the medium in the plate at 100 µL/well, and a blank group was set up with only medium, but no seeded cell strains. After the drug addition, the plate was incubated in the incubator for another 3 days (37 °C, 5% CO₂). On the fourth day, plate detection was performed. To each well, 100 µL of CellTiter-Glo reagent (Promega G9243) was added. The plate was shaken for 5 min and then left to stand at room temperature for 5 min. The chemical luminescence signal value of each well was determined using a microplate reader (PerkinElmer EnVision^{®}2104). Cell proliferation inhibition rate = (1 - (compound group - blank group) / (DMSO group - blank group)) × 100%. Then, the IC₅₀ value of each compound was calculated using the GraphPad Prism 8 software.

**Table 2. Inhibitory effects of compounds on tumor cell proliferation**

| Test compound | IC₅₀ (µM) |
|---|---|
| I-1 | 0.045 |
| I-2 | 0.080 |
| I-3 | 0.051 |
| I-5 | 0.186 |
| I-7 | 0.073 |
| I-8 | 0.035 |
| I-17 | 0.560 |
| I-27 | 0.079 |
| I-40 | 0.076 |
| I-52 | 0.045 |
| I-53 | 0.214 |
| I-77 | 0.12 |
| I-79 | 0.038 |
| I-80 | 0.096 |
| I-81 | 0.393 |
| I-85 | 0.284 |
| I-86 | 0.070 |
| I-87 | 0.144 |
| I-88 | 0.015 |
| I-95 | 0.412 |
| I-99 | 0.205 |
| I-100 | 0.115 |
| I-101 | 0.260 |
| I-102 | 0.144 |
| I-105 | 0.033 |
| I-106 | 0.208 |
| I-107 | 0.213 |
| I-109 | 0.066 |
| I-110 | 0.061 |
| I-111 | 0.073 |
| I-112 | 0.107 |
| I-113 | 0.051 |
| I-114 | 0.076 |
| I-115 | 0.167 |
| I-116 | 0.389 |
| I-117 | 0.365 |
| I-118 | 0.184 |
| I-121 | 0.056 |
| I-129 | 0.148 |
| I-130 | 0.142 |

The experimental results indicate that: the results of the assay on OVCAR3 cell proliferation show that the compounds of the present disclosure can significantly inhibit OVCAR3 cell proliferation.

The embodiments of the technical solutions of the present disclosure have been described above by way of example. It should be understood that the protection scope of the present disclosure is not limited to the embodiments described above. Any modification, equivalent replacement, improvement, and the like made by those skilled in the art without departing from the spirit and principle of the present disclosure shall fall within the protection scope of the claims of the present application.

## Claims

1. A compound represented by formula V, or a tautomer, a stereoisomer, a hydrate, a solvate, a pharmaceutically acceptable salt, or a prodrug thereof: 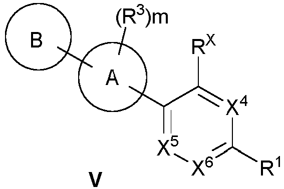 wherein
ring A is a 5- to 6-membered heteroaryl ring;
ring B is selected from 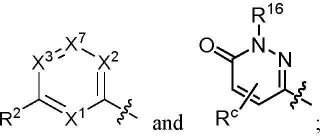
X¹ is N or CR⁵;
X² is N or CR⁶;
X³ is N or CR⁷;
X⁴ is N or CR⁸;
X⁵ is N or CR⁹;
X⁶ is N or CR¹⁰;
X⁷ is N or CR⁴;
R¹ is selected from cyano and a -ZR¹² group, wherein Z is independently selected from -C₀₋₆ alkyl-, -C₀₋₆ alkyl-NR¹¹-C₀₋₆ alkyl-, -C₀₋₆ alkyl-NR¹¹SO₂-C₀₋₆ alkyl-, -C₀₋₆ alkyl-SO₂NR¹¹-C₀₋₆ alkyl-, -C₀₋₆ alkyl-NR¹¹SO₂NR¹¹-C₀₋₆ alkyl-, -C₀₋₆ alkyl-NR¹¹SO₂NR¹¹-C(=O)-O-C₀₋₆ alkyl-, -C₀₋₆ alkyl-NR¹¹-S(=O)(=NH)-C₀₋₆ alkyl-, -C₀₋₆ alkylS(=O)(=NH)-C₀₋₆ alkyl-, -C₀₋₆ alkyl-S-C₀₋₆ alkyl-, -C₀₋₆ alkyl-S(=O)-C₀₋₆ alkyl-, -C₀₋₆ alkyl-SO₂-C₀₋₆ alkyl-, -C₀₋₆ alkyl-O-C₀₋₆ alkyl-, -P-, -C₀₋₆ alkyl-P(=O)(R¹¹)-, -C₀₋₆ alkyl-P(=O)₂, -C₀₋₆ alkyl-(C=O)-C₀₋₆ alkyl-, -C₀₋₆ alkyl-(C=O)-O-C₀₋₆ alkyl-, -C₀₋₆ alkyl-(C=O)NR¹¹-C₀₋₆ alkyl-, -C₀₋₆ alkyl-NR¹¹(C=O)-C₀₋₆ alkyl-, and -C(=N-OH)-; or the -ZR¹² group is -N=S(=O)-(R¹²)₂, wherein two R¹² are alternatively combined with the sulfur atom to which they are attached to form a saturated or partially saturated 3-, 4-, 5-, or 6-membered monocyclic ring comprising 0, 1, 2, or 3 N atoms and 0, 1, or 2 atoms selected from O and S;
R² is halogen or a -Y-R¹³ group, wherein Y is -C₀₋₆ alkyl-, -C₀₋₆ alkyl-NR¹³-C₀₋₆ alkyl-, -C₀₋₆ alkyl-O-C₀₋₆ alkyl-, - C₀₋₆ alkyl-S-C₀₋₆ alkyl-, -C₀₋₆ alkyl-S(=O)-C₀₋₆ alkyl-, -C₀₋₆ alkyl-S(=O)₂-C₀₋₆ alkyl-, -C₀₋₆ alkyl-SO₂NR¹³-C₀₋₆ alkyl-, -C₀₋₆ alkyl-S(=O)(=NH)-, -C₀₋₆ alkyl-NR¹³-SO₂-C₀₋₆ alkyl-, -C₀₋₆ alkyl-NR¹³SO₂NR¹³-C₀₋₆ alkyl-, -C₀₋₆ alkyl-C(=O)-C₀₋₆ alkyl-, -C₀₋₆ alkyl-NR¹³C(=O)-C₀₋₆ alkyl-, -C₀₋₆ alkyl-(C=O)NR¹³-C₀₋₆ alkyl-, or -C₀₋₆ alkyl-C(=O)-O-C₀₋₆ alkyl-; or -Y-R¹³ is -N=S(=O)-(R¹³)₂, wherein two R¹³ are alternatively combined with the sulfur atom to which they are attached to form a saturated or partially saturated 3-, 4-, 5-, or 6-membered monocyclic ring comprising 0, 1, 2, or 3 N atoms and 0, 1, or 2 atoms selected from O and S;
R³ is selected from halogen, hydroxyl, cyano, amino, C₁₋₆ alkyl, C₁₋₆ alkoxy, C₁₋₆ haloalkyl, -OC₁₋₆ alkyl, and -OC₁₋₆ haloalkyl;
m is selected from 0, 1, 2, 3, and 4;
R⁴ is selected from hydrogen, halogen, cyano, C₁₋₈ alkyl, -OC₁₋₈ alkyl, C₁₋₄ haloalkyl, -OR^{4a}, -OR^{4b}, -C₀₋₆ alkyl-(C=O)-N-(C₀₋₆ alkyl)₂, -C₀₋₆ alkyl-SO₂N-(C₀₋₆ alkyl)₂, R^{4a}, and R^{4b};
R⁵ is selected from hydrogen, halogen, cyano, C₁₋₆ alkyl, C₁₄ haloalkyl, C₃₋₈ cycloalkyl, halogenated C₃₋₈ cycloalkyl, -O-C₁₋₈ alkyl, and -O-R^{5a}, wherein R^{5a} is a saturated, partially saturated, or unsaturated 3-, 4-, 5-, 6-, or 7-membered monocyclic ring comprising 0, 1, 2, or 3 N atoms and 0, 1, or 2 atoms selected from O and S; or
alternatively, R² and R⁵ can be combined with the carbon atom to which they are each attached to form a saturated or partially saturated 5- or 6-membered monocyclic ring fused to the phenyl ring, wherein the 5- or 6-membered monocyclic ring comprises 0, 1, 2, or 3 N atoms and 0 or 1 atom selected from O and S, and further wherein the 5- or 6-membered monocyclic ring is substituted with 0, 1, 2, or 3 groups selected from: halogen, C₁₋₆ alkyl, C₁₋₄ haloalkyl, -OR^{a}, -OC₁₋₄ haloalkyl, cyano, -NR^{a}R^{a}, and oxo;
R⁷ is selected from hydrogen, halogen, cyano, C₁₋₈ alkyl, C₃₋₈ cycloalkyl, C₃₋₈ halocycloalkyl, C₁₋₄ haloalkyl, -O-C₁₋₈ alkyl, and R^{7a}; or alternatively, R² and R⁷ may be combined with the carbon atom to which they are each attached to form a saturated or partially saturated 5- or 6-membered monocyclic ring fused to the phenyl ring, wherein the 5- or 6-membered monocyclic ring comprises 0, 1, 2, or 3 N atoms and 0 or 1 atom selected from O and S, and further wherein the 5- or 6-membered monocyclic ring is substituted with 0, 1, 2, or 3 groups selected from: halogen, C₁₋₆ alkyl, C₁₋₄ haloalkyl, -OR^{a}, -OC₁₋₄ haloalkyl, CN, -NR^{a}R^{a}, and oxo;
R⁶, R⁸, and R⁹ are each independently selected from hydrogen, halogen, cyano, C₁₋₈ alkyl, -O-C₁₋₈ alkyl, C₃₋₈ cycloalkyl, C₃₋₈ halocycloalkyl, and C₁₋₄ haloalkyl;
R¹⁰ is hydrogen, halogen, hydroxyl, cyano, C₁₋₈ alkyl, C₃₋₈ cycloalkyl, C₃₋₈ halocycloalkyl, C₁₄ haloalkyl, -O-R^{10a}, or -O-R^{10b};
R¹¹ is hydrogen, R^{11a}, or R^{11b};
R¹² is hydrogen, halogen, hydroxyl, cyano, R^{12a}, or R^{12b};
R¹³ is hydrogen, halogen, cyano, R^{13a}, or R^{13b};
R¹⁶ is hydrogen, R^{16a}, or R^{16b};
R^{4a}, R^{7a}, R^{10a}, R^{11a}, R^{12a}, R^{13a}, and R^{16a} are each independently selected from the group consisting of: a saturated, partially saturated, or unsaturated 3-, 4-, 5-, 6-, or 7-membered monocyclic ring or 4-, 5-, 6-, 7-, 8-, 9-, 10-, 11-, or 12-membered bicyclic ring comprising 0, 1, 2, or 3 N atoms and 0, 1, or 2 atoms selected from O and S, wherein the monocyclic or bicyclic ring is substituted with 0, 1, 2, or 3 groups selected from: halogen, cyano, amino, C₁₋₆ alkyl, C₁₋₆ haloalkyl, -OR^{a}, -OC₁₋₆ haloalkyl, -C(=O)R^{b}, -C(=O)OR^{a}, -C(=O)NR^{a}R^{a}, -C(=NR^{a})NR^{a}R^{a}, -OC(=O)R^{b}, -OC(=O)NR^{a}R^{a}, -OC₂₋₆ alkyl NR^{a}R^{a}, -OC₂₋₆ alkyl OR^{a}, -SR^{a}, -S(=O)R^{b}, -S(=O)₂R^{b}, -S(=O)₂NR^{a}R^{a}, -NR^{a}R^{a}, - N(R^{a})C(=O)R^{b}, -N(R^{a})C(=O)OR^{b}, -N(R^{a})C(=O)NR^{a}R^{a}, -N(R^{a})C(=NR^{a})NR^{a}R^{a}, -N(R^{a})S(=O)₂R^{b}, - N(R^{a})S(=O)₂NR^{a}R^{a}, -NR^{a}C₂₋₆ alkyl NR^{a}R^{a}, -NR^{a}C₂₋₆ alkyl OR^{a}, -C₁₋₆ alkyl NR^{a}R^{a}, -C₁₋₆ alkyl OR^{a}, -C₁₋₆ alkyl N(R^{a})C(=O)R^{b}, -C₁₋₆ alkyl OC(=O)R^{b}, -C₁₋₆ alkyl C(=O)NR^{a}R^{a}, -C₁₋₆ alkyl C(=O)OR^{a}, R¹⁴, and oxo;
R^{4b}, R^{10b}, R^{11b}, R^{12b}, R^{13b}, and R^{16b} are each independently selected from the group consisting of: C₁₋₆ alkyl substituted with 0, 1, 2, 3, 4, or 5 groups selected from the group consisting of: halogen, cyano, C₁₋₆ alkyl, C₁₋₆ haloalkyl, -C₀₋₆NR^{a}R^{a}, -OR^{a}, -C(=O)OR^{a}, and a saturated, partially saturated, or unsaturated 3-, 4-, 5-, or 6-membered monocyclic ring;
R¹⁴ is independently selected from the group consisting of: a saturated, partially saturated, or unsaturated 3-, 4-, 5-, 6-, or 7-membered monocyclic ring or 4-, 5-, 6-, 7-, 8-, 9-, 10-, 11-, or 12-membered bicyclic ring comprising 0, 1, 2, or 3 N atoms and 0, 1, or 2 atoms selected from O and S, wherein the monocyclic or bicyclic ring is substituted with 0, 1, 2, or 3 groups selected from: halogen, cyano, amino, C₁₋₆ alkyl, C₁₋₆ haloalkyl, -OR^{a}, -OC₁₋₆ haloalkyl, - C(=O)R^{b}, -C(=O)OR^{a}, -C(=O)NR^{a}R^{a}, -C(=NR^{a})NR^{a}R^{a}, -OC(=O)R^{b}, -OC(=O)NR^{a}R^{a}, -OC₂₋₆ alkyl NR^{a}R^{a}, -OC₂-C₆ alkyl OR^{a}, -SR^{a}, -S(=O)R^{b}, -S(=O)₂R^{b}, -S(=O)₂NR^{a}R^{a}, -NR^{a}R^{a}, -N(R^{a})C(=O)R^{b}, -N(R^{a})C(=O)OR^{b}, - N(R^{a})C(=O)NR^{a}R^{a}, -N(R^{a})C(=NR^{a})NR^{a}R^{a}, -N(R^{a})S(=O)₂R^{b}, -N(R^{a})S(=O)₂NR^{a}R^{a}, -NR^{a}C₂₋₆ alkyl NR^{a}R^{a}, -NR^{a}C₂₋₆ alkyl OR^{a}, -C₁₋₆ alkyl NR^{a}R^{a}, -C₁₋₆ alkyl OR^{a}, -C₁₋₆ alkyl N(R^{a})C(=O)R^{b}, -C₁₋₆ alkyl OC(=O)R^{b}, -C₁₋₆ alkyl C(=O)NR^{a}R^{a}, -C₁₋₆ alkyl C(=O)OR^{a}, and oxo;
R^{x} is selected from the group consisting of: hydrogen, 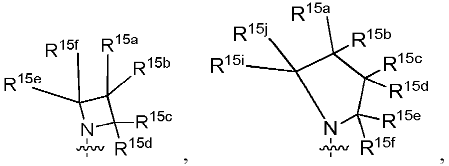 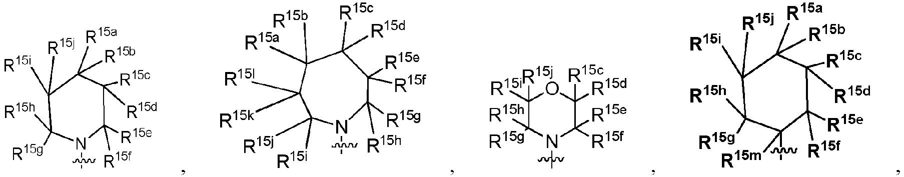 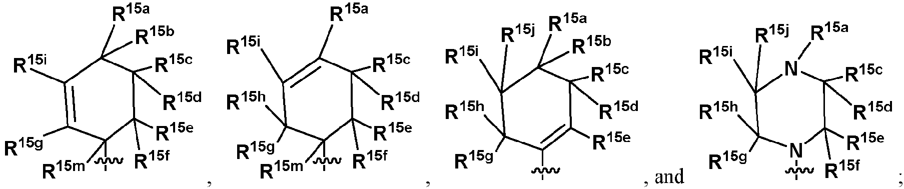 or
R^{x} is C₂₋₈ alkyl substituted with 0, 1, 2, 3, 4, or 5 groups selected from the group consisting of: halogen, cyano, amino, C₁₋₆ alkyl, C₁₋₆ haloalkyl, -OR^{a}, -NR^{a}R^{a}, -C(=O)OR^{a}, -OC₁₋₄ haloalkyl, and R¹⁵ⁿ; or
R^{x} is phenyl, or phenyl or an unsaturated 5-membered monocyclic ring substituted with 0, 1, 2, 3, 4, or 5 groups selected from the group consisting of: halogen, cyano, amino, C₁₋₆ alkyl, C₁₋₆ haloalkyl, -OR^{a}, -NR^{a}R^{a}, -C(=O)OR^{a}, -OC₁₋₄ haloalkyl, and R¹⁵ⁿ;
the 5-membered monocyclic ring comprises 0, 1, 2, or 3 N atoms and 0 or 1 atom selected from O and S;
R^{15a}, R^{15b}, R^{15c}*,* R^{15d}, R^{15e}, R^{15f}, R^{15g}, R^{15h}, R¹⁵ⁱ, R^{15j}, R^{15k}, R^{15l}, and R^{15m} are each independently hydrogen, halogen, R^{15o}, or R^{15p}; or
alternatively, each of the pairs of R^{15a} and R^{15b}, R^{15c} and R^{15d}, R^{15c} and R^{15f}, R^{15g} and R^{15h}, R¹⁵ⁱ and R^{15j}, and R^{15k} and R^{15l} can be independently combined with the carbon atom to which they are attached to form a saturated or partially saturated 3-, 4-, 5-, or 6-membered monocyclic ring spiro-attached to the R^{x} ring, wherein the 3-, 4-, 5-, or 6-membered monocyclic ring comprises 0, 1, 2, or 3 N atoms and 0, 1, or 2 atoms selected from O and S, and further wherein the 3-, 4-, 5-, or 6-membered monocyclic ring is substituted with 0, 1, 2, or 3 groups selected from: halogen, C₁₋₆ alkyl, C₁₋₄ haloalkyl, -OR^{a}, -OC₁₋₄ haloalkyl, CN, -NR^{a}R^{a}, and oxo; or
yet alternatively, each of the pairs of R^{15a} and R^{15b}, R^{15c} and R^{15d}, R^{15c} and R^{15f}, R^{15g} and R^{15h}, R¹⁵ⁱ and R^{15j}, and R^{15k} and R^{15l} can be independently combined to form a double bond;
R¹⁵ⁿ and R^{15o} can be independently selected from a saturated, partially saturated, or unsaturated 3-, 4-, 5-, 6-, or 7-membered monocyclic ring or 8-, 9-, 10-, 11-, or 12-membered bicyclic ring, wherein the monocyclic or bicyclic ring comprises 0, 1, 2, or 3 N atoms and 0, 1, or 2 atoms selected from O and S, and further wherein the monocyclic or bicyclic ring is substituted with 0, 1, 2, 3, or 4 groups selected from: halogen, C₁₋₆ alkyl, C₁₋₄ haloalkyl, -OR^{a}, - OC₁₋₄ haloalkyl, CN, -C(=O)OR^{a}, -C(=O)R^{b}, -C(=O)NR^{a}R^{a}, -C(=NR^{a})NR^{a}R^{a}, -OC(=O)R^{b}, -OC(=O)NR^{a}R^{a}, -OC₂₋₆ alkyl NR^{a}R^{a}, -NR^{a}R^{a}, -OC₂₋₆OR^{a}, -SR^{a}, -S(=O)R^{b}, -S(=O)₂R^{b}, -S(=O)₂NR^{a}R^{a}, -NR^{a}C(=O)R^{b}, -NR^{a}C(=O)OR^{b}, - NR^{a}C(=O)NR^{a}R^{a}, -NR^{a}C(=NR^{a})NR^{a}R^{a}, -NR^{a}S(=O)₂NR^{b}, -NR^{a}S(=O)₂NR^{a}R^{a}, -R^{a}C₂₋₆ alkyl NR^{a}R^{a}, -NR^{a}C₂₋₆ alkyl OR^{a}, -C₁₋₆ alkyl NR^{a}R^{a}, -C₁₋₆ alkyl OR^{a}, -C₁₋₆ alkyl OC(=O)R^{b}, -C₁₋₆ alkyl NR^{a}C(=O)R^{b}, -C₁₋₆ alkyl NR^{a}C(=O)NR^{a}R^{a}, -C₁₋₆ alkyl-C(=O)OR^{a}, and oxo;
R^{15p} can be independently selected from C₁₋₈ alkyl substituted with 0, 1, 2, 3, 4, or 5 groups selected from halogen, C₁₋₄ haloalkyl, CN, -C(=O)OR^{a}, -OR^{a}, -OC₁₋₄ haloalkyl, and -NR^{a}R^{a};
R^{a} and R^{c} may be independently selected from H and R^{b};
R^{b} is independently selected from C₁₋₆ alkyl, phenyl, and benzyl, wherein the C₁₋₆ alkyl is substituted with 0, 1, 2, or 3 substituents selected from: halogen, hydroxyl, -OC₁₋₆ alkyl, -NH₂, -NHC₁₋₆ alkyl, -OC(=O)C₁₋₆ alkyl, and - N(C₁₋₆ alkyl)C₁₋₆ alkyl; and the phenyl or benzyl is substituted with 0, 1, 2, or 3 substituents selected from: halogen, C₁₋₆ alkyl, C₁₋₄ haloalkyl, -OH, -OC₁₋₆ alkyl, -NH₂, -NHC₁₋₆ alkyl, -OC(=O)C₁₋₆ alkyl, and -N(C₁₋₆ alkyl)C₁₋₆ alkyl.

2. The compound represented by formula V, or the tautomer, the stereoisomer, the hydrate, the solvate, the pharmaceutically acceptable salt, or the prodrug thereof as claimed in claim 1, wherein the compound represented by formula V has structural formula II: 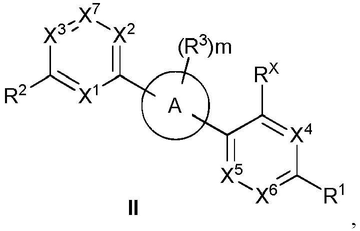 wherein
X¹ is N or CR⁵;
X² is N or CR⁶;
X³ is N or CR⁷;
X⁴ is N or CR⁸;
X⁵ is N or CR⁹;
X⁶ is N or CR¹⁰;
X⁷ is N or CR⁴;
ring A is a 5- to 6-membered heteroaryl ring;
R¹ is selected from cyano and a -ZR¹² group, wherein Z is independently selected from -C₀₋₆ alkyl-, -C₀₋₆ alkyl-NR¹¹-C₀₋₆ alkyl-, -C₀₋₆ alkyl-NR¹¹SO₂-C₀₋₆ alkyl-, -C₀₋₆ alkyl-SO₂NR¹¹-C₀₋₆ alkyl-, -C₀₋₆ alkyl-NR¹¹SO₂NR¹¹-C₀₋₆ alkyl-, -C₀₋₆ alkyl-NR¹¹SO₂NR¹¹-C(=O)-O-C₀₋₆ alkyl-, -C₀₋₆ alkyl-NR¹¹-S(=O)(=NH)-C₀₋₆ alkyl-, -C₀₋₆ alkylS(=O)(=NH)-C₀₋₆ alkyl-, -C₀₋₆ alkyl-S-C₀₋₆ alkyl-, -C₀₋₆ alkyl-S(=O)-C₀₋₆ alkyl-, -C₀₋₆ alkyl-SO₂-C₀₋₆ alkyl-, -C₀₋₆ alkyl-O-C₀₋₆ alkyl-, -P-, -C₀₋₆ alkyl-P(=O)(R¹¹)-, -C₀₋₆ alkyl-P(=O)₂, -C₀₋₆ alkyl-(C=O)-C₀₋₆ alkyl-, -C₀₋₆ alkyl-(C=O)-O-C₀₋₆ alkyl-, -C₀₋₆ alkyl-(C=O)NR¹¹-C₀₋₆ alkyl-, -C₀₋₆ alkyl-NR¹¹(C=O)-C₀₋₆ alkyl-, and -C(=N-OH)-; or the -ZR¹² group is -N=S(=O)-(R¹²)₂, wherein two R¹² are alternatively combined with the sulfur atom to which they are attached to form a saturated or partially saturated 3-, 4-, 5-, or 6-membered monocyclic ring comprising 0, 1, 2, or 3 N atoms and 0, 1, or 2 atoms selected from O and S;
R² is halogen or a -Y-R¹³ group, wherein Y is -C₀₋₆ alkyl-, -C₀₋₆ alkyl-NR¹³-C₀₋₆ alkyl-, -C₀₋₆ alkyl-O-C₀₋₆ alkyl-, - C₀₋₆ alkyl-S-C₀₋₆ alkyl-, -C₀₋₆ alkyl-S(=O)-C₀₋₆ alkyl-, -C₀₋₆ alkyl-S(=O)₂-C₀₋₆ alkyl-, -C₀₋₆ alkyl-SO₂NR¹³-C₀₋₆ alkyl-, -C₀₋₆ alkyl-S(=O)(=NH)-, -C₀₋₆ alkyl-NR¹³-SO₂-C₀₋₆ alkyl-, -C₀₋₆ alkyl-NR¹³SO₂NR¹³-C₀₋₆ alkyl-, -C₀₋₆ alkyl-C(=O)-C₀₋₆ alkyl-, -C₀₋₆ alkyl-NR¹³C(=O)-C₀₋₆ alkyl-, -C₀₋₆ alkyl-(C=O)NR¹³-C₀₋₆ alkyl-, or -C₀₋₆ alkyl-C(=O)-O-C₀₋₆ alkyl-; or -Y-R¹³ is -N=S(=O)-(R¹³)₂, wherein two R¹³ are alternatively combined with the sulfur atom to which they are attached to form a saturated or partially saturated 3-, 4-, 5-, or 6-membered monocyclic ring comprising 0, 1, 2, or 3 N atoms and 0, 1, or 2 atoms selected from O and S;
R³ is selected from halogen, hydroxyl, cyano, amino, C₁₋₆ alkyl, C₁₋₆ alkoxy, C₁₋₆ haloalkyl, -OC₁₋₆ alkyl, and -OC₁₋₆ haloalkyl;
m is selected from 0, 1, 2, 3, and 4;
R⁴ is selected from hydrogen, halogen, cyano, C₁₋₈ alkyl, -OC₁₋₈ alkyl, C₁₋₄ haloalkyl, -OR^{4a}, -OR^{4b}, -C₀₋₆ alkyl-(C=O)-N-(C₀₋₆ alkyl)₂, -C₀₋₆ alkyl-SO₂N-(C₀₋₆ alkyl)₂, R^{4a}, and R^{4b};
R⁵ is selected from hydrogen, halogen, cyano, C₁₋₆ alkyl, C₁₄ haloalkyl, C₃₋₈ cycloalkyl, halogenated C₃₋₈ cycloalkyl, -O-C₁₋₈ alkyl, and -O-R^{5a}, wherein R^{5a} is a saturated, partially saturated, or unsaturated 3-, 4-, 5-, 6-, or 7-membered monocyclic ring comprising 0, 1, 2, or 3 N atoms and 0, 1, or 2 atoms selected from O and S; or
alternatively, R² and R⁵ can be combined with the carbon atom to which they are each attached to form a saturated or partially saturated 5- or 6-membered monocyclic ring fused to the phenyl ring, wherein the 5- or 6-membered monocyclic ring comprises 0, 1, 2, or 3 N atoms and 0 or 1 atom selected from O and S, and further wherein the 5- or 6-membered monocyclic ring is substituted with 0, 1, 2, or 3 groups selected from: halogen, C₁₋₆ alkyl, C₁₋₄ haloalkyl, -OR^{a}, -OC₁₋₄ haloalkyl, cyano, -NR^{a}R^{a}, and oxo;
R⁷ is selected from hydrogen, halogen, cyano, C₁₋₈ alkyl, C₃₋₈ cycloalkyl, C₃₋₈ halocycloalkyl, C₁₋₄ haloalkyl, -O-C₁₋₈ alkyl, and R^{7a}; or alternatively, R² and R⁷ may be combined with the carbon atom to which they are each attached to form a saturated or partially saturated 5- or 6-membered monocyclic ring fused to the phenyl ring, wherein the 5- or 6-membered monocyclic ring comprises 0, 1, 2, or 3 N atoms and 0 or 1 atom selected from O and S, and further wherein the 5- or 6-membered monocyclic ring is substituted with 0, 1, 2, or 3 groups selected from: halogen, C₁₋₆ alkyl, C₁₋₄ haloalkyl, -OR^{a}, -OC₁₋₄ haloalkyl, CN, -NR^{a}R^{a}, and oxo;
R⁶, R⁸, and R⁹ are each independently selected from hydrogen, halogen, cyano, C₁₋₈ alkyl, -O-C₁₋₈ alkyl, C₃₋₈ cycloalkyl, C₃₋₈ halocycloalkyl, and C₁₋₄ haloalkyl;
R¹⁰ is hydrogen, halogen, hydroxyl, cyano, C₁₋₈ alkyl, C₃₋₈ cycloalkyl, C₃₋₈ halocycloalkyl, C₁₄ haloalkyl, -O-R^{10a}, or -O-R^{10b};
R¹¹ is hydrogen, R^{11a}, or R^{11b};
R¹² is hydrogen, halogen, hydroxyl, cyano, R^{12a}, or R^{12b};
R¹³ is hydrogen, halogen, cyano, R^{13a}, or R^{13b};
R^{4a}, R^{7a}, R^{10a}, R^{11a}, R^{12a}, and R^{13a} are each independently selected from the group consisting of: a saturated, partially saturated, or unsaturated 3-, 4-, 5-, 6-, or 7-membered monocyclic ring or 4-, 5-, 6-, 7-, 8-, 9-, 10-, 11-, or 12-membered bicyclic ring comprising 0, 1, 2, or 3 N atoms and 0, 1, or 2 atoms selected from O and S, wherein the monocyclic or bicyclic ring is substituted with 0, 1, 2, or 3 groups selected from: halogen, cyano, amino, C₁₋₆ alkyl, C₁₋₆ haloalkyl, -OR^{a}, -OC₁₋₆ haloalkyl, -C(=O)R^{b}, -C(=O)OR^{a}, -C(=O)NR^{a}R^{a}, -C(=NR^{a})NR^{a}R^{a}, -OC(=O)R^{b}, - OC(=O)NR^{a}R^{a}, -OC₂₋₆ alkyl NR^{a}R^{a}, -OC₂₋₆ alkyl OR^{a}, -SR^{a}, -S(=O)R^{b}, -S(=O)₂R^{b}, -S(=O)₂NR^{a}R^{a}, -NR^{a}R^{a}, - N(R^{a})C(=O)R^{b}, -N(R^{a})C(=O)OR^{b}, -N(R^{a})C(=O)NR^{a}R^{a}, -N(R^{a})C(=NR^{a})NR^{a}R^{a}, -N(R^{a})S(=O)₂R^{b}, - N(R^{a})S(=O)₂NR^{a}R^{a}, -NR^{a}C₂₋₆ alkyl NR^{a}R^{a}, -NR^{a}C₂₋₆ alkyl OR^{a}, -C₁₋₆ alkyl NR^{a}R^{a}, -C₁₋₆ alkyl OR^{a}, -C₁₋₆ alkyl N(R^{a})C(=O)R^{b}, -C₁₋₆ alkyl OC(=O)R^{b}, -C₁₋₆ alkyl C(=O)NR^{a}R^{a}, -C₁₋₆ alkyl C(=O)OR^{a}, R¹⁴, and oxo;
R^{4b}, R^{10b}, R^{11b}, R^{12b}, and R^{13b} are each independently selected from the group consisting of: C₁₋₆ alkyl substituted with 0, 1, 2, 3, 4, or 5 groups selected from the group consisting of: halogen, cyano, C₁₋₆ alkyl, C₁₋₆ haloalkyl, -C₀₋₆NR^{a}R^{a}, -OR^{a}, -C(=O)OR^{a}, and a saturated, partially saturated, or unsaturated 3-, 4-, 5-, or 6-membered monocyclic ring;
R¹⁴ is independently selected from the group consisting of: a saturated, partially saturated, or unsaturated 3-, 4-, 5-, 6-, or 7-membered monocyclic ring or 4-, 5-, 6-, 7-, 8-, 9-, 10-, 11-, or 12-membered bicyclic ring comprising 0, 1, 2, or 3 N atoms and 0, 1, or 2 atoms selected from O and S, wherein the monocyclic or bicyclic ring is substituted with 0, 1, 2, or 3 groups selected from: halogen, cyano, amino, C₁₋₆ alkyl, C₁₋₆ haloalkyl, -OR^{a}, -OC₁₋₆ haloalkyl, - C(=O)R^{b}, -C(=O)OR^{a}, -C(=O)NR^{a}R^{a}, -C(=NR^{a})NR^{a}R^{a}, -OC(=O)R^{b}, -OC(=O)NR^{a}R^{a}, -OC₂₋₆ alkyl NR^{a}R^{a}, -OC₂-C₆ alkyl OR^{a}, -SR^{a}, -S(=O)R^{b}, -S(=O)₂R^{b}, -S(=O)₂NR^{a}R^{a}, -NR^{a}R^{a}, -N(R^{a})C(=O)R^{b}, -N(R^{a})C(=O)OR^{b}, - N(R^{a})C(=O)NR^{a}R^{a}, -N(R^{a})C(=NR^{a})NR^{a}R^{a}, -N(R^{a})S(=O)₂R^{b}, -N(R^{a})S(=O)₂NR^{a}R^{a}, -NR^{a}C₂₋₆ alkyl NR^{a}R^{a}, -NR^{a}C₂₋₆ alkyl OR^{a}, -C₁₋₆ alkyl NR^{a}R^{a}, -C₁₋₆ alkyl OR^{a}, -C₁₋₆ alkyl N(R^{a})C(=O)R^{b}, -C₁₋₆ alkyl OC(=O)R^{b}, -C₁₋₆ alkyl C(=O)NR^{a}R^{a}, -C₁₋₆ alkyl C(=O)OR^{a}, and oxo;
R^{x} is selected from the group consisting of: hydrogen, 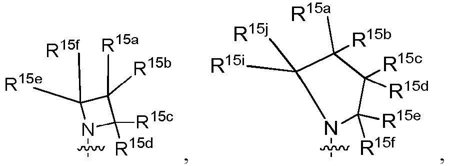 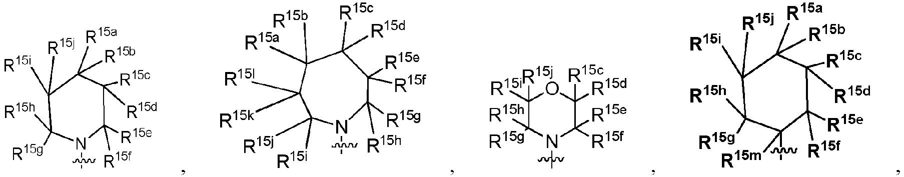 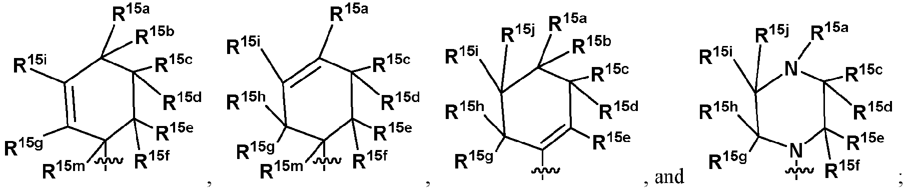 or
R^{x} is C₂₋₈ alkyl substituted with 0, 1, 2, 3, 4, or 5 groups selected from the group consisting of: halogen, cyano, amino, C₁₋₆ alkyl, C₁₋₆ haloalkyl, -OR^{a}, -NR^{a}R^{a}, -C(=O)OR^{a}, -OC₁₋₄ haloalkyl, and R¹⁵ⁿ; or
R^{x} is phenyl, or phenyl or an unsaturated 5-membered monocyclic ring substituted with 0, 1, 2, 3, 4, or 5 groups selected from the group consisting of: halogen, cyano, amino, C₁₋₆ alkyl, C₁₋₆ haloalkyl, -OR^{a}, -NR^{a}R^{a}, -C(=O)OR^{a}, -OC₁₋₄ haloalkyl, and R¹⁵ⁿ;
the 5-membered monocyclic ring comprises 0, 1, 2, or 3 N atoms and 0 or 1 atom selected from O and S;
R^{15a}, R^{15b}, R^{15c}, R^{15d}, R^{15e}, R^{15f}, R^{15g}, R^{15h}, R¹⁵ⁱ, R^{15j}, R^{15k}, R^{15l}, and R^{15m} are each independently hydrogen, halogen, R^{15o}, or R^{15p}; or
alternatively, each of the pairs of R^{15a} and R^{15b}, R^{15c} and R^{15d}, R^{15c} and R^{15f}, R^{15g} and R^{15h}, R¹⁵ⁱ and R^{15j}, and R^{15k} and R^{15l} may be independently combined with the carbon atom to which they are attached to form a saturated or partially saturated 3-, 4-, 5-, or 6-membered monocyclic ring spiro- attached to the R^{x} ring, wherein the 3-, 4-, 5-, or 6-membered monocyclic ring comprises 0, 1, 2, or 3 N atoms and 0, 1, or 2 atoms selected from O and S, and further wherein the 3-, 4-, 5-, or 6-membered monocyclic ring is substituted with 0, 1, 2, or 3 groups selected from: halogen, C₁₋₆ alkyl, C₁₋₄ haloalkyl, -OR^{a}, -OC₁₋₄ haloalkyl, CN, -NR^{a}R^{a}, and oxo; or
yet alternatively, each of the pairs of R^{15a} and R^{15b}, R^{15c} and R^{15d}, R^{15c} and R^{15f}, R^{15g} and R^{15h}, R¹⁵ⁱ and R^{15j}, and R^{15k} and R^{15l} can be independently combined to form a double bond;
R¹⁵ⁿ and R^{15o} can be independently selected from a saturated, partially saturated, or unsaturated 3-, 4-, 5-, 6-, or 7-membered monocyclic ring or 8-, 9-, 10-, 11-, or 12-membered bicyclic ring, wherein the monocyclic or bicyclic ring comprises 0, 1, 2, or 3 N atoms and 0, 1, or 2 atoms selected from O and S, and further wherein the monocyclic or bicyclic ring is substituted with 0, 1, 2, 3, or 4 groups selected from: halogen, C₁₋₆ alkyl, C₁₋₄ haloalkyl, -OR^{a}, - OC₁₋₄ haloalkyl, CN, -C(=O)OR^{a}, -C(=O)R^{b}, -C(=O)NR^{a}R^{a}, -C(=NR^{a})NR^{a}R^{a}, -OC(=O)R^{b}, -OC(=O)NR^{a}R^{a}, -OC₂₋₆ alkyl NR^{a}R^{a}, -NR^{a}R^{a}, -OC₂₋₆OR^{a}, -SR^{a}, -S(=O)R^{b}, -S(=O)₂R^{b}, -S(=O)₂NR^{a}R^{a}, -NR^{a}C(=O)R^{b}, -NR^{a}C(=O)OR^{b}, - NR^{a}C(=O)NR^{a}R^{a}, -NR^{a}C(=NR^{a})NR^{a}R^{a}, -NR^{a}S(=O)₂NR^{b}, -NR^{a}S(=O)₂NR^{a}R^{a}, -R^{a}C₂₋₆ alkyl NR^{a}R^{a}, -NR^{a}C₂₋₆ alkyl OR^{a}, -C₁₋₆ alkyl NR^{a}R^{a}, -C₁₋₆ alkyl OR^{a}, -C₁₋₆ alkyl OC(=O)R^{b}, -C₁₋₆ alkyl NR^{a}C(=O)R^{b}, -C₁₋₆ alkyl NR^{a}C(=O)NR^{a}R^{a}, -C₁₋₆ alkyl-C(=O)OR^{a}, and oxo;
R^{15p} can be independently selected from C₁₋₈ alkyl substituted with 0, 1, 2, 3, 4, or 5 groups selected from halogen, C₁₋₄ haloalkyl, CN, -C(=O)OR^{a}, -OR^{a}, -OC₁₋₄ haloalkyl, and -NR^{a}R^{a};
R^{a} is independently selected from H and R^{b};
R^{b} is independently selected from C₁₋₆ alkyl, phenyl, and benzyl, wherein the C₁₋₆ alkyl is substituted with 0, 1, 2, or 3 substituents selected from: halogen, hydroxyl, -OC₁₋₆ alkyl, -NH₂, -NHC₁₋₆ alkyl, -OC(=O)C₁₋₆ alkyl, and - N(C₁₋₆ alkyl)C₁₋₆ alkyl; and the phenyl or benzyl is substituted with 0, 1, 2, or 3 substituents selected from: halogen, C₁₋₆ alkyl, C₁₋₄ haloalkyl, -OH, -OC₁₋₆ alkyl, -NH₂, -NHC₁₋₆ alkyl, -OC(=O)C₁₋₆ alkyl, and -N(C₁₋₆ alkyl)C₁₋₆ alkyl.

3. The compound represented by formula V, or the tautomer, the stereoisomer, the hydrate, the solvate, the pharmaceutically acceptable salt, or the prodrug thereof as claimed in claim 1, wherein the compound represented by formula V has structural formula II-1: 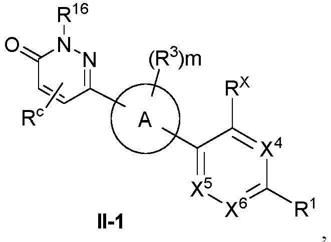 wherein X⁴, X⁵, X⁶, ring A, R¹, R³, R¹⁶, R^{c}, R^{x}, and m are as defined in claim 1.

4. The compound represented by formula V, or the tautomer, the stereoisomer, the hydrate, the solvate, the pharmaceutically acceptable salt, or the prodrug thereof as claimed in claim 1, wherein the compound represented by formula V has structural formula I: 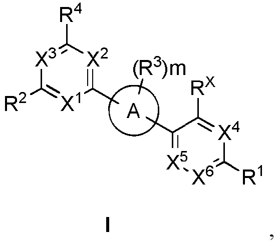 wherein X¹, X², X³, X⁴, X⁵, X⁶, ring A, R¹, R², R³, R⁴, R^{x}, and m are as defined in claim 1.

5. The compound represented by formula V, or the tautomer, the stereoisomer, the hydrate, the solvate, the pharmaceutically acceptable salt, or the prodrug thereof as claimed in claim 1, wherein the ring A is 5- to 6-membered heteroaryl comprising 1-3 heteroatoms;
m is 0, 1, 2, 3, or 4;
R³ is selected from halogen, C₁₋₆ alkyl, and C₁₋₆ haloalkyl;
preferably, the heteroatom is selected from N, O, and S; when a plurality of heteroatoms are present, the heteroatoms are identical or different;
preferably, ring A is selected from pyrrole, pyrazole, imidazole, triazole, furan, oxazole, oxadiazole, thiophene, thiazole, thiadiazole, pyridine, pyrimidine, pyridazine, pyrazine, and triazine;
more preferably, ring A is selected from pyrazole, oxazole, oxadiazole, thiazole, thiadiazole, and triazole.

6. The compound represented by formula V, or the tautomer, the stereoisomer, the hydrate, the solvate, the pharmaceutically acceptable salt, or the prodrug thereof as claimed in claim 1, wherein R^{x} is selected from 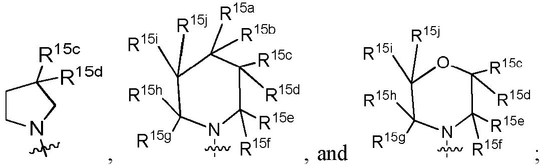 **the** R^{15a}, R^{15b}, R^{15c}, R^{15d}, R^{15e}, R^{15f}, R^{15g}, R^{15h} R¹⁵ⁱ, and R^{15j} are each independently hydrogen, halogen, C₁₋₆ alkyl, or C₁₋₄ haloalkyl; or R^{15a} and R^{15b} are combined with the carbon atom to which they are attached to form a saturated 3-, 4-, or 5-membered monocyclic ring spiro-attached to the R^{x} ring, wherein the monocyclic ring comprises 0, 1, 2, or 3 N atoms and 0, 1, or 2 atoms selected from O and S; or yet alternatively, each of the pairs of R^{15a} and R^{15b}, R^{15c} and R^{15d}, R^{15e} and R^{15f}, R^{15g} and R^{15h}, R¹⁵ⁱ and R^{15j}, and R^{15k} and R¹⁵¹ can be independently combined to form a double bond; the R^{15a}, R^{15b}, R^{15c}, R^{15d}, R^{15e}, R^{15f}, R^{15g}, R^{15h}, R¹⁵ⁱ, R^{15j}, R^{15k}, R^{15l}, and R^{15m} are each independently selected from hydrogen, halogen, and R^{15p}.

7. The compound represented by formula V, or the tautomer, the stereoisomer, the hydrate, the solvate, the pharmaceutically acceptable salt, or the prodrug thereof as claimed in claim 1, wherein the R^{15c}, R^{15d}, R¹⁵ⁱ, and R^{15j} are each independently hydrogen, methyl, or ethyl; and R^{15a} and R^{15b} are combined with the carbon atom to which they are attached to form a cyclopropyl, cyclobutyl, or cyclopentyl ring spiro-attached to the R^{x} ring.

8. The compound represented by formula V, or the tautomer, the stereoisomer, the hydrate, the solvate, the pharmaceutically acceptable salt, or the prodrug thereof as claimed in claim 1, wherein the 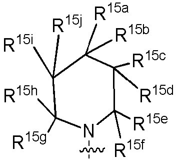 group is selected from 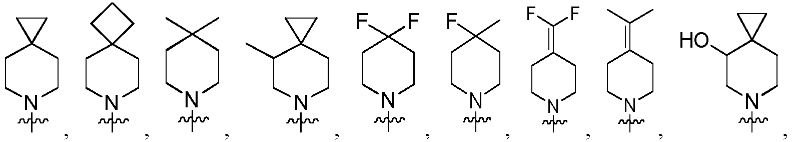 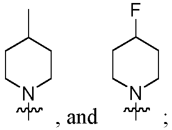 preferably, the 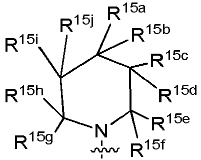 group is selected from 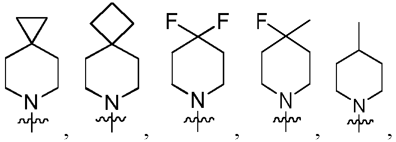 and

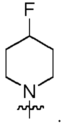

9. The compound represented by formula V, or the tautomer, the stereoisomer, the hydrate, the solvate, the pharmaceutically acceptable salt, or the prodrug thereof as claimed in claim 1, wherein the 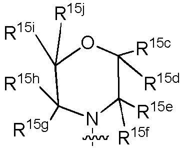 group is selected from

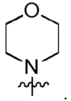

10. The compound represented by formula V, or the tautomer, the stereoisomer, the hydrate, the solvate, the pharmaceutically acceptable salt, or the prodrug thereof as claimed in claim 1, wherein the compound represented by formula V has the following structure: 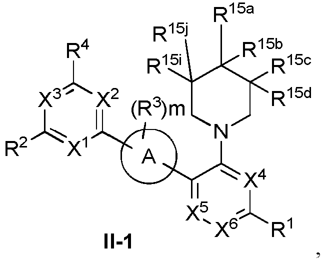
wherein X¹, X², X³, X⁴, X⁵, and X⁶ are as defined in claim 1;
R¹, R², R³, R⁴, and m are as defined in claim 1;
R^{15a}, R^{15b}, R^{15c}, R^{15d}, R¹⁵ⁱ, and R^{15j} are as defined in claim 1;
ring A is as defined in claim 1.

11. The compound represented by formula V, or the tautomer, the stereoisomer, the hydrate, the solvate, the pharmaceutically acceptable salt, or the prodrug thereof as claimed in claim 1, wherein the compound represented by formula V has the following structure: 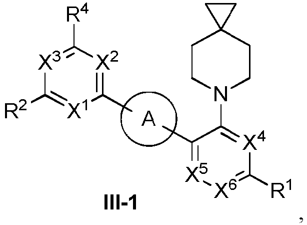
wherein X¹, X², X³, X⁴, X⁵, and X⁶ are as defined in claim 1;
R¹, R², and R⁴ are as defined in claim 1;
ring A is selected from pyrazole, oxazole, oxadiazole, thiazole, thiadiazole, and triazole.

12. The compound represented by formula V, or the tautomer, the stereoisomer, the hydrate, the solvate, the pharmaceutically acceptable salt, or the prodrug thereof as claimed in claim 1, wherein the compound represented by formula V has the following structure: 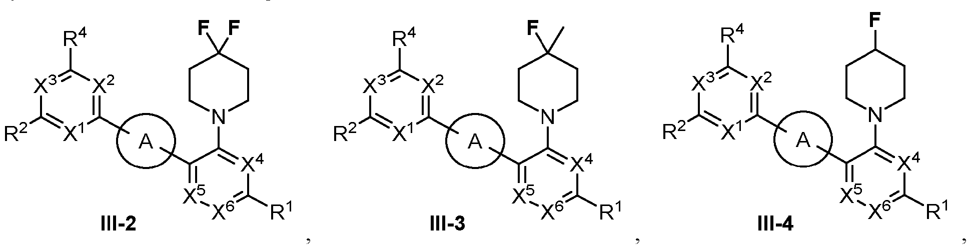 or 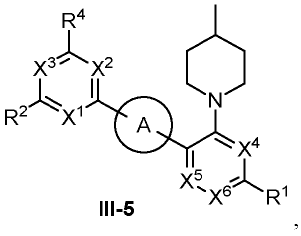
wherein X¹, X², X³, X⁴, X⁵, and X⁶ are as defined in claim 1;
R¹, R², and R⁴ are as defined in claim 1;
ring A is selected from pyrazole, oxazole, oxadiazole, thiazole, thiadiazole, and triazole.

13. The compound represented by formula V, or the tautomer, the stereoisomer, the hydrate, the solvate, the pharmaceutically acceptable salt, or the prodrug thereof as claimed in any one of claims 1, 2, 3, 4, 10, 11, and 12, wherein R¹ is -ZR¹²,
wherein Z is selected from -C₀₋₆ alkyl-NH-C₀₋₆ alkyl-, -C₀₋₆ alkyl-NHSO₂-C₀₋₆ alkyl-, -C₀₋₆ alkyl-SO₂NH-C₀₋₆ alkyl-, -C₀₋₆ alkyl-S(=O)(=NH)-C₀₋₆ alkyl-, and -C₀₋₆ alkyl-SO₂-C₀₋₆ alkyl-;
R¹² is halogen, hydroxyl, R^{12a}, or R^{12b},
wherein the R^{12a} is cyclopropyl or epoxybutane substituted with 0, 1, 2, or 3 groups selected from: fluoro, C₁₋₆ alkyl, C₁₋₆ haloalkyl, -OH, -OC₁₋₆ haloalkyl, and -C₁₋₆ alkyl-OH;
the R^{12b} is C₁₋₆ alkyl substituted with 0, 1, 2, 3, 4, or 5 groups selected from the group consisting of: hydroxyl, fluoro, and methyl;
preferably, Z is selected from -NH-C₀₋₆ alkyl-, -NHSO₂-C₀₋₆ alkyl-, -SO₂NH-C₀₋₆ alkyl-, -S(=O)(=NH)-C₀₋₆ alkyl-, -S0₂-C₀₋₆ alkyl-, and -CH₂-SO₂-C₀₋₆ alkyl-.

14. The compound represented by formula V, or the tautomer, the stereoisomer, the hydrate, the solvate, the pharmaceutically acceptable salt, or the prodrug thereof as claimed in claim 1 or 13, wherein the Z is a bond, -NH-, -NHSO₂-, -SO₂NH-, -S(=O)(=NH)-, -S-, -S(=O)-, -SO₂-, -CH₂-SO₂-, -O-, -P-, -P(=O)CH₃-, -P(=O)₂, -(C=O)-, - (C=O)NH-, or -NH(C=O)-;
preferably, the Z is -NH-, -NHSO₂-, -SO₂NH-, -S(=O)(=NH)-, -SO₂-, or -CH₂-SO₂-.

15. The compound represented by formula V, or the tautomer, the stereoisomer, the hydrate, the solvate, the pharmaceutically acceptable salt, or the prodrug thereof as claimed in claim 1 or 13, wherein the R¹² is selected from R^{12a} and R^{12b};
R^{12a} is selected from: a saturated, partially saturated, or unsaturated 3-, 4-, 5-, 6-, or 7-membered monocyclic ring comprising 0, 1, 2, or 3 N atoms and 0, 1, or 2 atoms selected from O and S, wherein the monocyclic ring is substituted with 0, 1, 2, or 3 groups selected from: halogen, hydroxyl, C₁₋₆ alkyl, and C₁₋₆ haloalkyl;
R^{12b} is C₁₋₆ alkyl unsubstituted or substituted with a substituent selected from: halogen, hydroxyl, C₁₋₆ alkyl, and a saturated, partially saturated, or unsaturated 3-, 4-, 5-, or 6-membered monocyclic ring, wherein preferably, the 3-, 4-, 5-, or 6-membered monocyclic ring is cyclopropyl, cyclobutyl, cyclopentyl, or cyclohexyl;
preferably, R¹² is selected from: C₁₋₆ alkyl, C₃₋₆ cycloalkyl, C₁₋₆ alkyl-C₃₋₆ cycloalkyl, 4- to 6-membered heterocycloalkyl, and C₁₋₆ alkyl-4- to 6-membered heterocycloalkyl,
wherein the C₁₋₆ alkyl, C₃₋₆ cycloalkyl, or 4- to 6-membered heterocycloalkyl is optionally substituted with a substituent selected from: F, Cl, hydroxyl, methyl, ethyl, propyl, and butyl;
more preferably, the R¹² is selected from -CH₂CH₂OH, *tert*-butyl*,* 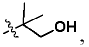 methyl, -CH₂F, 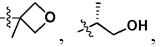 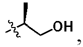 cyclopropyl, -CH₂-cyclopropyl, 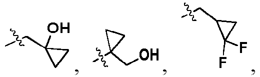 tetrahydrofuranyl, 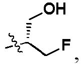 cyclopentyl, oxetanyl, azetidinyl, and 1,3,4-oxathiazinanyl.

16. The compound represented by formula V, or the tautomer, the stereoisomer, the hydrate, the solvate, the pharmaceutically acceptable salt, or the prodrug thereof as claimed in claim 1, wherein the compound represented by formula V has the following structure: 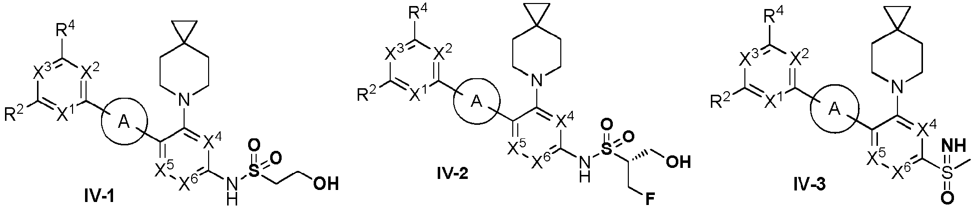 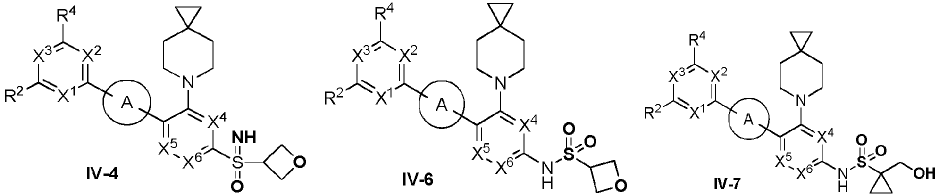 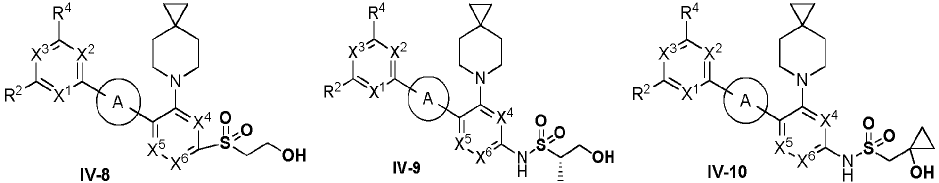 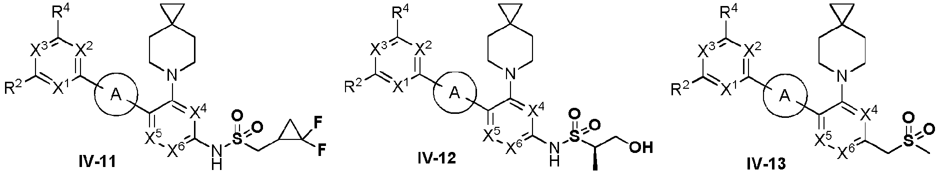 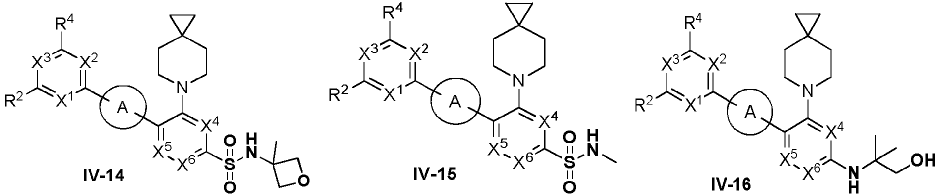 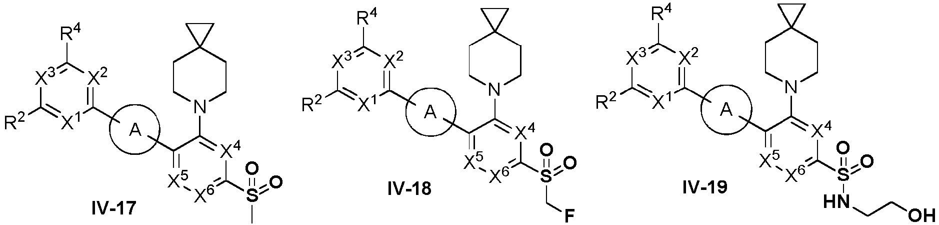 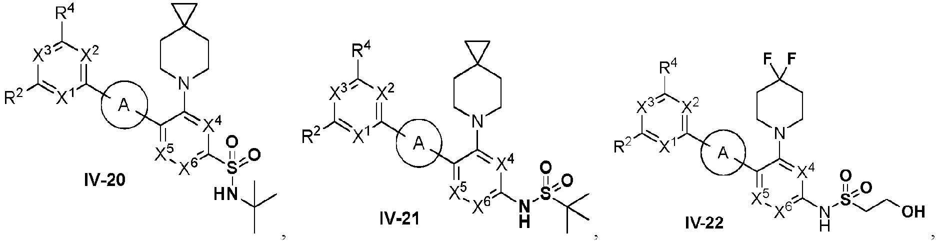 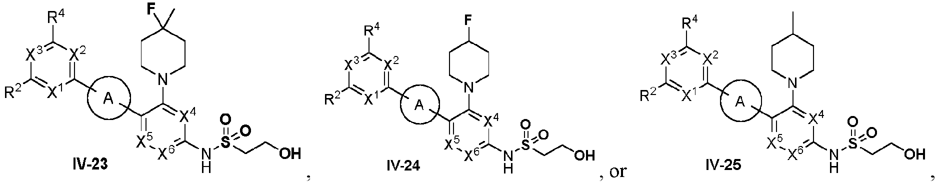
wherein X¹, X², X³, X⁴, X⁵, and X⁶ are as defined in claim 1;
R² and R⁴ are as defined in claim 1;
ring A is selected from pyrazole, oxazole, oxadiazole, thiazole, thiadiazole, and triazole.

17. The compound represented by formula V, or the tautomer, the stereoisomer, the hydrate, the solvate, the pharmaceutically acceptable salt, or the prodrug thereof as claimed in any one of claims 1, 2, 4, 10, 11, 12, and 16, wherein the R² is selected from F, Cl, Br, and a -Y-R¹³ group, wherein Y is a bond, -NH-, -NH-(CH₂)₀₋₄-, -O-(CH₂)₀₋₄-, or -SO₂NH-; and R¹³ is a saturated, partially saturated, or unsaturated 3-, 4-, 5-, 6-, or 7-membered monocyclic ring or 4-, 5-, 6-, 7-, 8-, 9-, 10-, 11-, or 12-membered bicyclic ring comprising 0, 1, 2, or 3 N atoms and 0 or 1 atom selected from O and S, wherein the monocyclic or bicyclic ring is substituted with 0, 1, 2, or 3 identical or different groups selected from: F, Cl, Br, C₁₋₆ alkyl, C₁₋₄ haloalkyl, -OH, -OC₁₋₄ haloalkyl, CN, R¹⁴, and oxo; or R¹³ is C₁₋₆ alkyl substituted with 0, 1, 2, 3, 4, or 5 identical or different substituents selected from F, Cl, Br, -OH, -OC₁₋₄ haloalkyl, and CN; R¹⁴ is as defined in claim 1;
preferably, Y is a bond, and R¹³ is a saturated 3-, 4-, 5-, 6-, or 7-membered monocyclic ring or 4-, 5-, 6-, 7-, 8-, 9-, 10-, 11-, or 12-membered bicyclic ring comprising 0 or 1 N atom and 0 or 1 O atom, wherein the monocyclic or bicyclic ring is substituted with 0, 1, 2, or 3 identical or different groups selected from: F, Cl, Br, -C₁₋₃ alkyl, C₁₋₃ haloalkyl, -OH, CN, and -C₁₋₃ alkyl-OH,
preferably selected from F, Cl, Br, methyl, -OH, -OCH₃, CN, -CH₂OH, and trifluoromethyl; or
Y is -NH-, -NH-(CH₂)₀₋₄-, -O-(CH₂)₀₋₄-, or -SO₂NH-; R¹³ is a saturated 3-, 4-, 5-, 6-, or 7-membered monocyclic ring comprising 0 or 1 N atom and 0 or 1 O atom, wherein the monocyclic ring is substituted with 0, 1, 2, or 3 identical or different groups selected from: fluoro, methyl, trifluoromethyl, -CN, -OH, and CH₂OH; or
R¹³ is C₁₋₆ alkyl substituted with 0, 1, 2, 3, 4, or 5 identical or different substituents, wherein the substituent is -OH, methyl, or F.

18. The compound represented by formula V, or the tautomer, the stereoisomer, the hydrate, the solvate, the pharmaceutically acceptable salt, or the prodrug thereof as claimed in any one of claims 1, 2, 4, 10, 11, 12, and 16, wherein the R² is selected from the following structures:

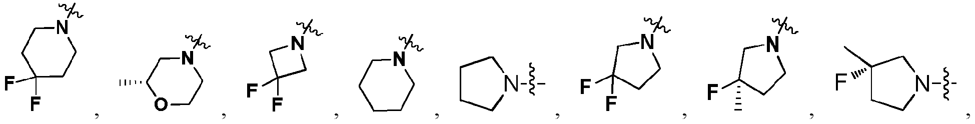

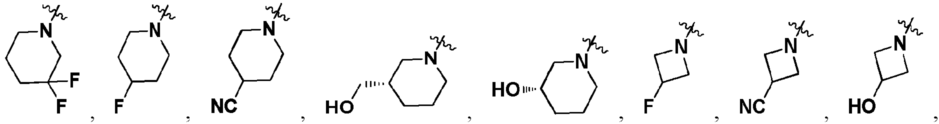

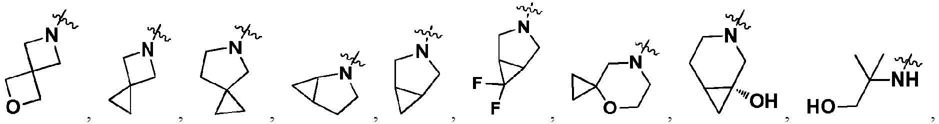

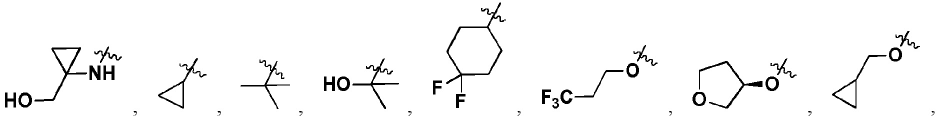

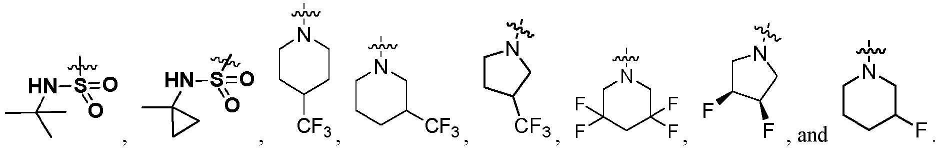

19. The compound, or the tautomer, the stereoisomer, the hydrate, the solvate, the pharmaceutically acceptable salt, or the prodrug thereof as claimed in any one of claim 1 and claim 2, wherein X¹ is N or -CR⁵;
X² is N or CR⁶;
X³ is N or CR⁷;
X⁴ is N or CR⁸;
X⁵ is N or CR⁹;
X⁶ is N or CR¹⁰;
X⁷ is N or CR⁴;
preferably, none, one, or two of the X¹, X², X³, and X⁷ are N;
preferably, none, one, or two of the X⁴, X⁵, and X⁶ are N.

20. The compound represented by formula V, or the tautomer, the stereoisomer, the hydrate, the solvate, the pharmaceutically acceptable salt, or the prodrug thereof as claimed in claim 1, wherein ring B has a structure of 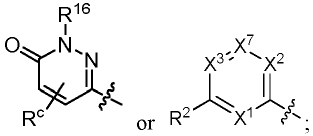
the 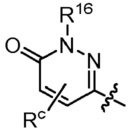 group has a structure of 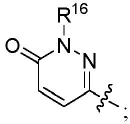
the R¹⁶ is selected from a saturated, partially saturated, or unsaturated 3-, 4-, 5-, 6-, or 7-membered monocyclic ring or 4-, 5-, 6-, 7-, 8-, 9-, 10-, 11-, or 12-membered bicyclic ring comprising 0, 1, 2, or 3 N atoms and 0, 1, or 2 atoms selected from O and S, wherein the monocyclic or bicyclic ring is substituted with 0, 1, 2, or 3 groups selected from: halogen, cyano, amino, C₁₋₆ alkyl, C₁₋₆ haloalkyl, -OR^{a}, and -OC₁₋₆ haloalkyl;
preferably, R¹⁶ is selected from a saturated 3-, 4-, 5-, 6-, or 7-membered monocyclic ring, wherein the monocyclic ring is substituted with 0, 1, 2, or 3 groups selected from: halogen, cyano, amino, hydroxyl, C₁₋₆ alkyl, C₁₋₆ haloalkyl, and -OC₁₋₆ haloalkyl;
preferably, the 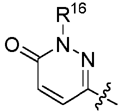 group has a structure of 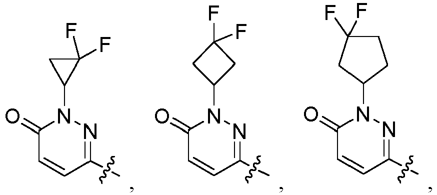 or 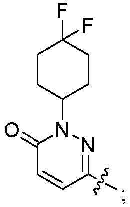
the 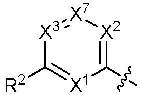
group has a structure of 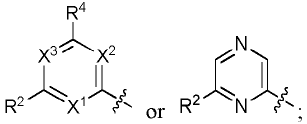
preferably, X¹ is N or CH, X² is N or CH, and X³ is N or CH;
preferably, none, one, or two of the X¹, X², X³, and X⁷ are N;
preferably, the 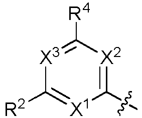
has a structure of

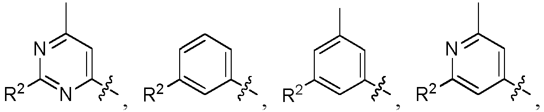

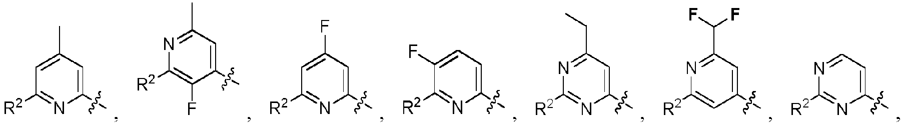

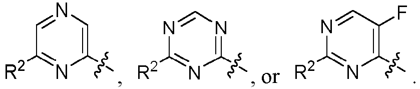

21. The compound, or the tautomer, the stereoisomer, the hydrate, the solvate, the pharmaceutically acceptable salt, or the prodrug thereof as claimed in claim 1, claim 2, or any one of claims 10-12, wherein the R⁴ is selected from (a) hydrogen, F, Cl, Br, CN, -C₀₋₆ alkyl-SO₂N-(C₀₋₆ alkyl)₂, or -C₀₋₆ alkyl-(C=O)-N-(C₀₋₆ alkyl)₂; (b) C₁₋₆ alkyl or - OC₁₋₆ alkyl substituted with 0, 1, 2, or 3 OH groups; (c) 3- to 8-membered heterocycloalkyl comprising 0, 1, 2, or 3 N atoms and 0, 1, or 2 atoms selected from O and S; and (d) C₁₋₃ haloalkyl;
preferably, R⁴ is selected from hydrogen, methyl, ethyl, difluoromethyl, halogen, cyano, methoxy, cyclopropyl, cyclobutyl, -(C=O)NH₂, -CF₃, furanyl, pyridinyl, morpholinyl, -SO₂NHC(CH₃)₃, aziridinyl, and azetidinyl;
preferably, R⁴ is hydrogen, methyl, ethyl, fluoro, or difluoromethyl.

22. The compound represented by formula V, or the tautomer, the stereoisomer, the hydrate, the solvate, the pharmaceutically acceptable salt, or the prodrug thereof as claimed in claim 1, wherein the compound comprises:

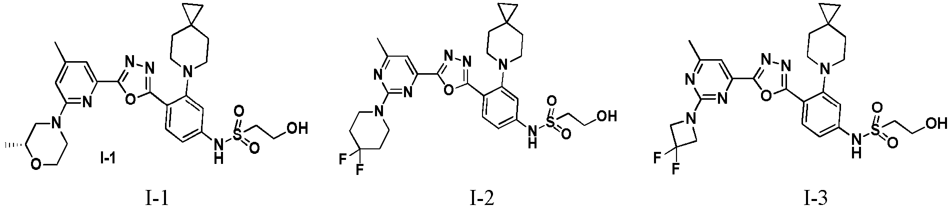

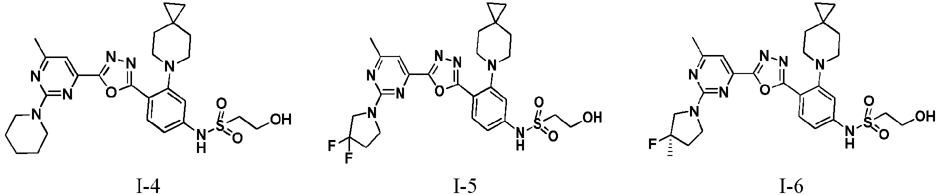

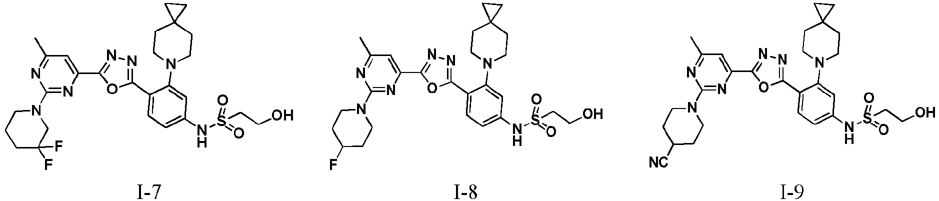

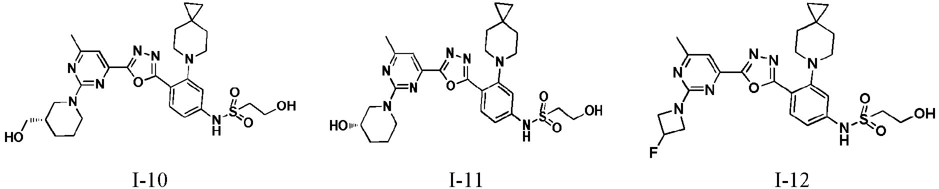

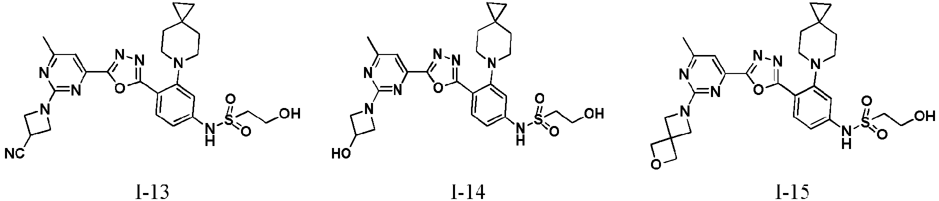

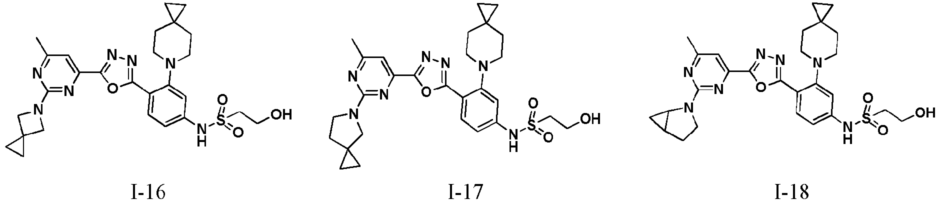

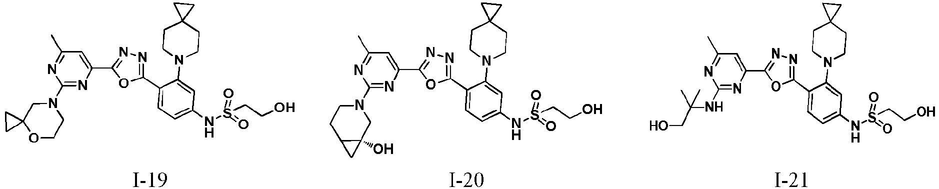

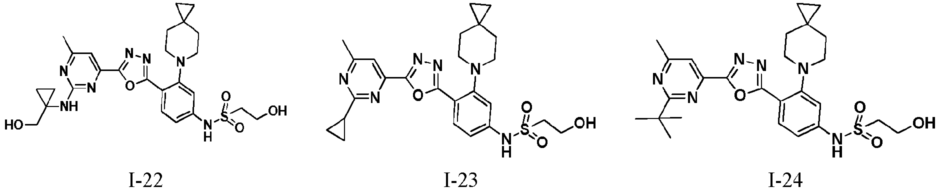

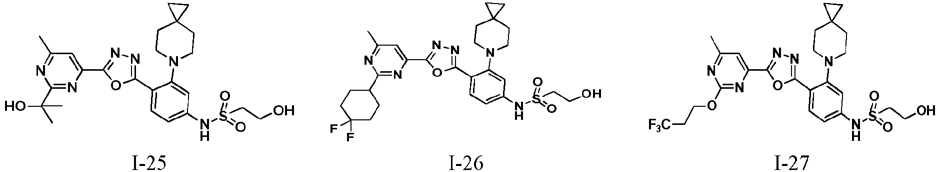

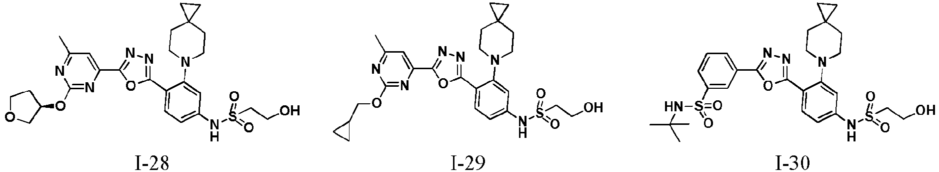

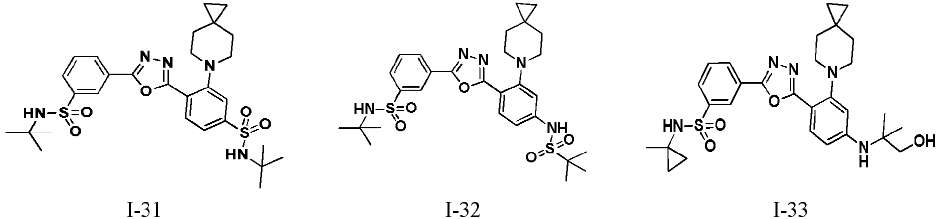

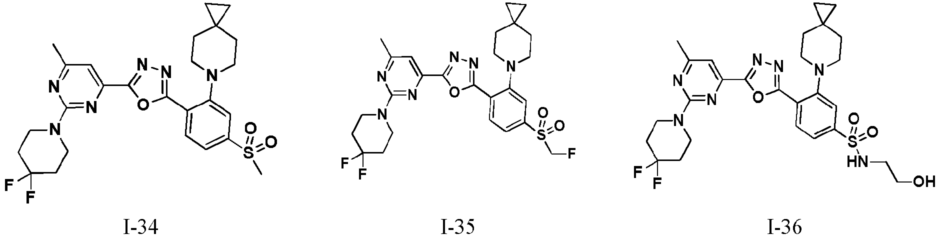

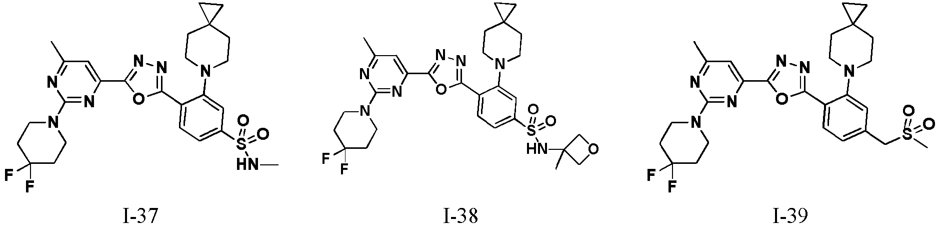

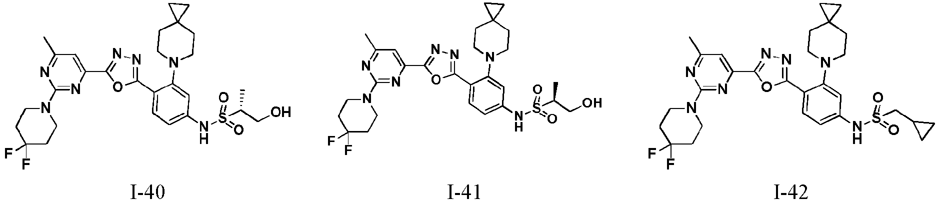

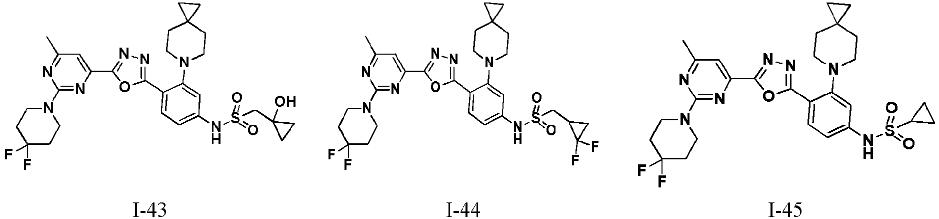

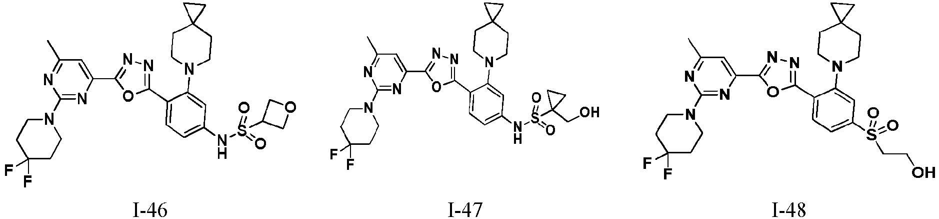

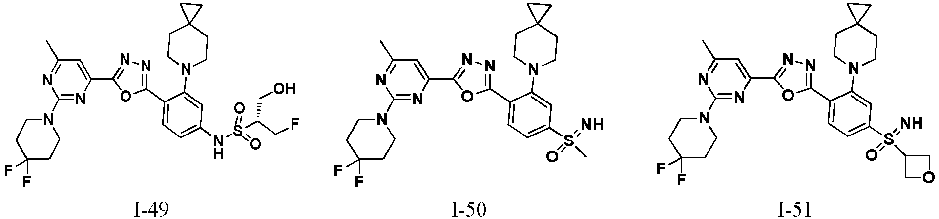

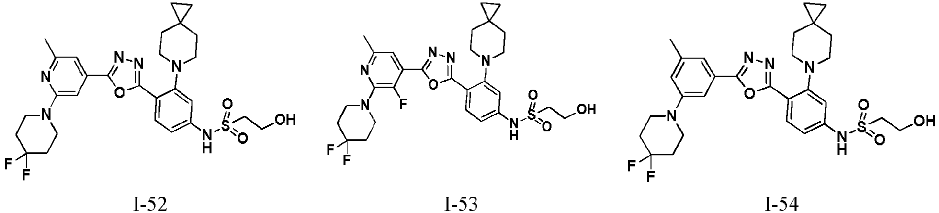

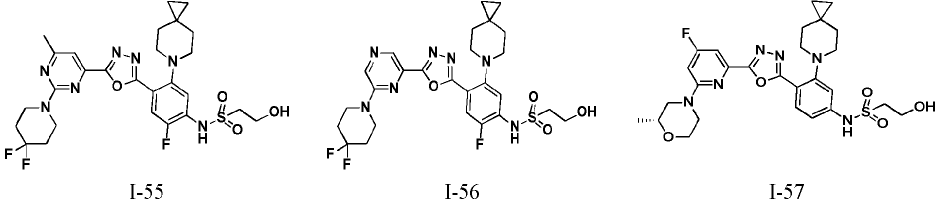

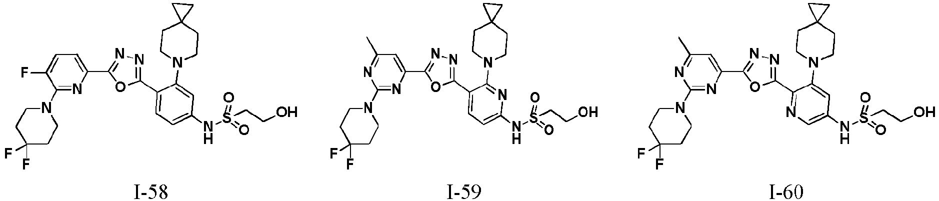

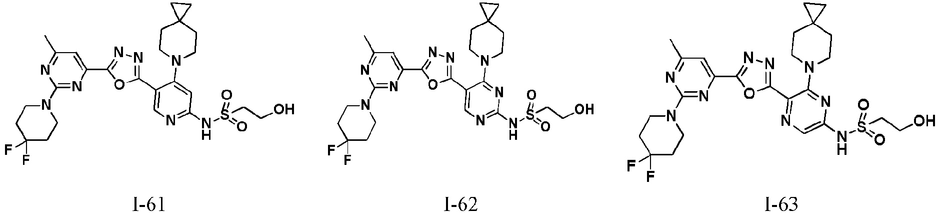

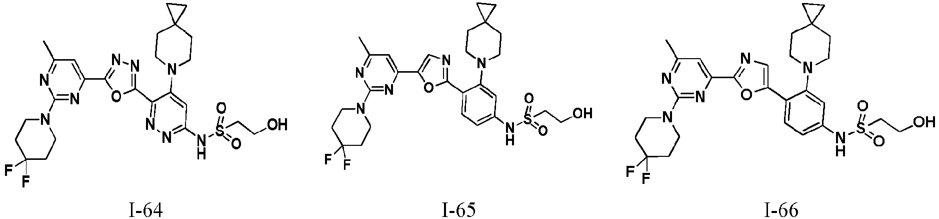

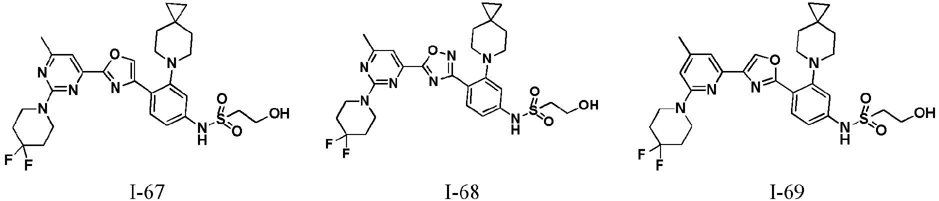

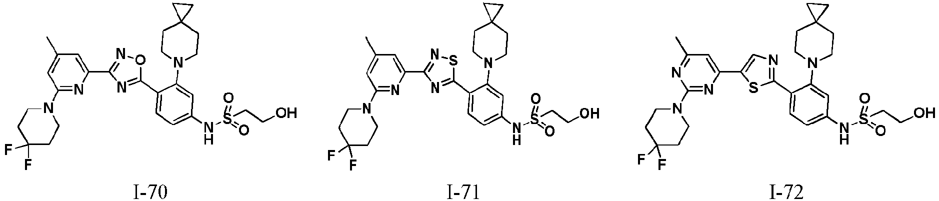

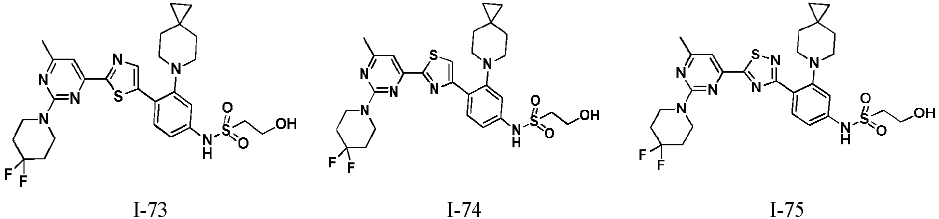

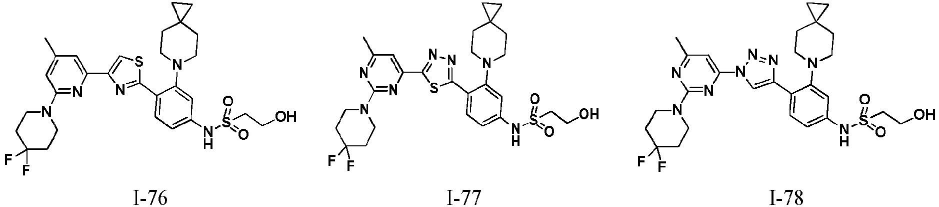

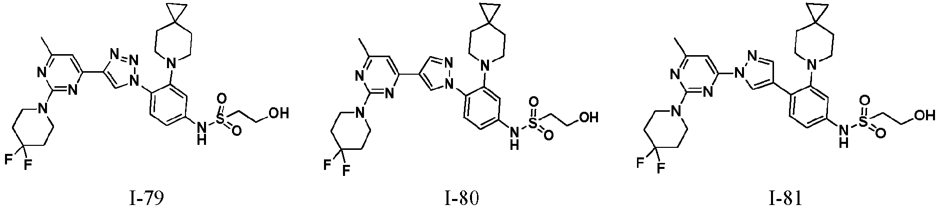

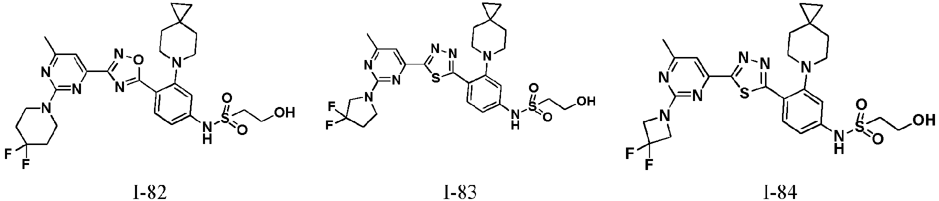

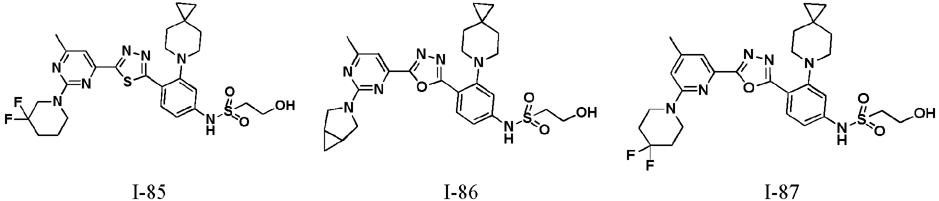

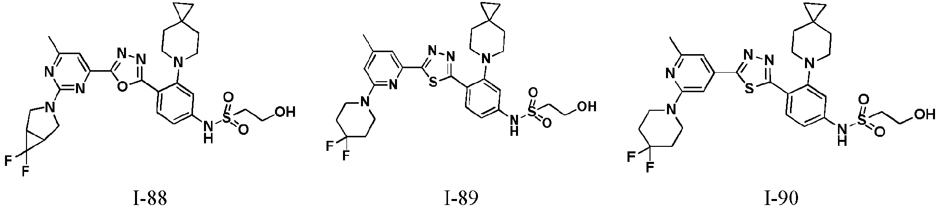

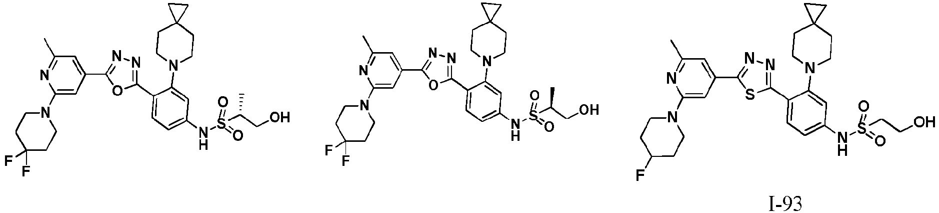

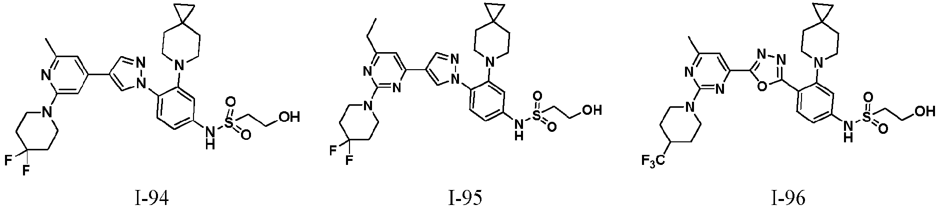

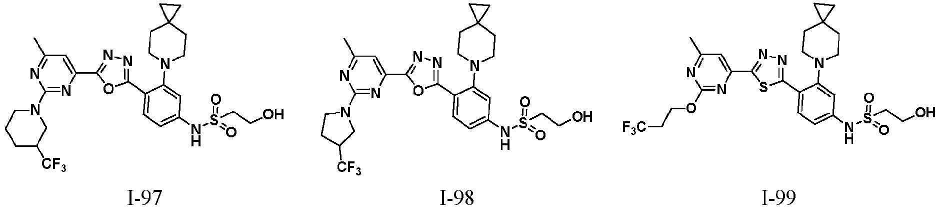

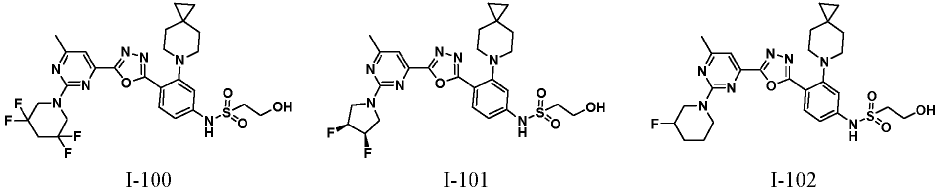

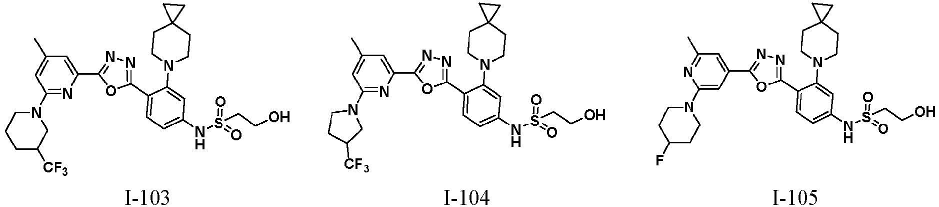

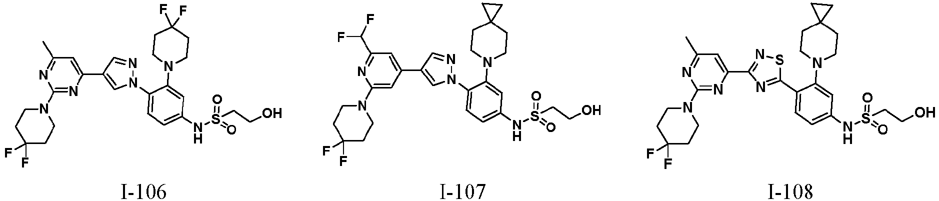

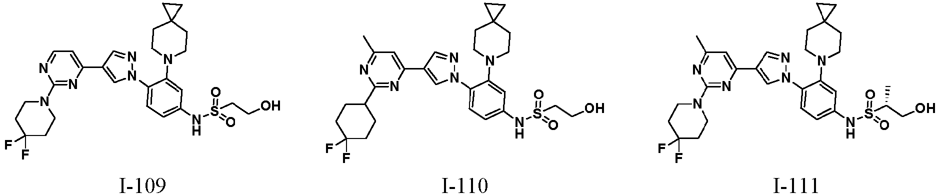

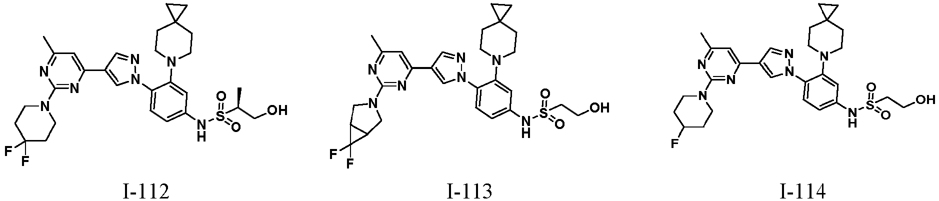

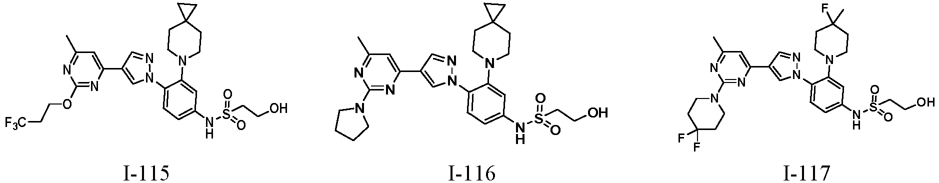

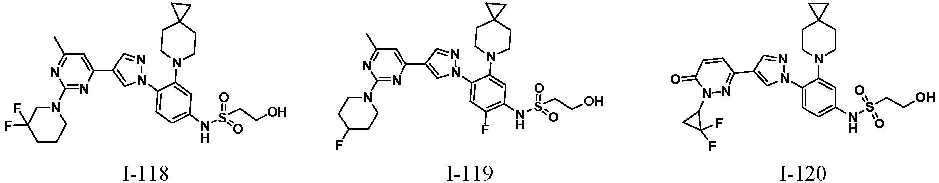

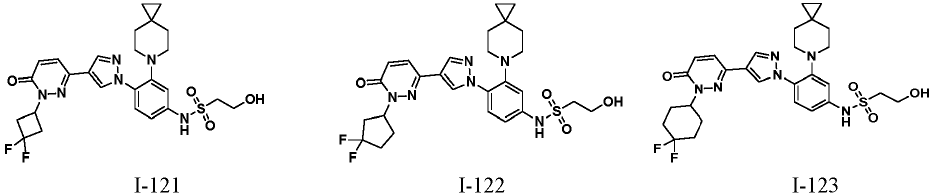

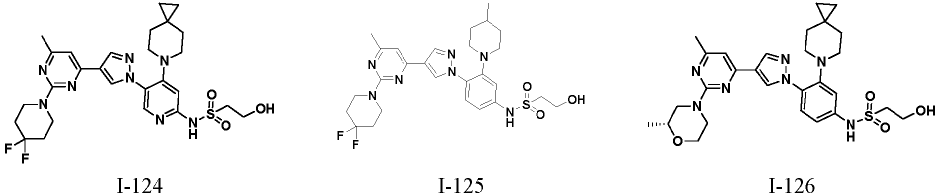

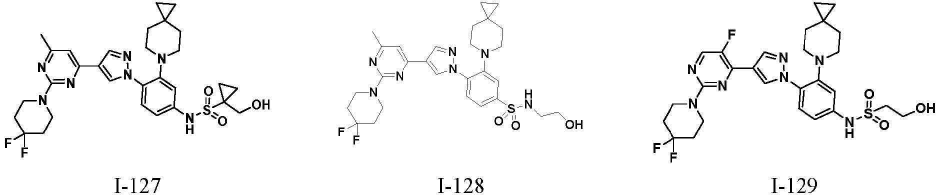

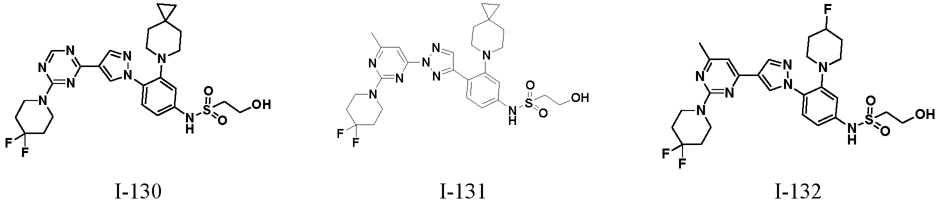

23. A pharmaceutical composition, comprising: the compound represented by formula V, or the tautomer, the stereoisomer, the hydrate, the solvate, the pharmaceutically acceptable salt, or the prodrug thereof as claimed in claims 1-22, and a pharmaceutically acceptable carrier.

24. Use of the compound represented by formula V, or the tautomer, the stereoisomer, the hydrate, the solvate, the pharmaceutically acceptable salt, or the prodrug thereof as claimed in claims 1-22, or use of the pharmaceutical composition as claimed in claim 23, comprising:
inhibiting KIF18A; and/or
preventing and/or treating a disease associated with KIF18A; and/or
manufacturing a medicament, a pharmaceutical composition, or a preparation for inhibiting KIF18A and/or for preventing and/or treating a disease associated with KIF18A.

25. The use as claimed in claim 24, wherein the disease associated with KIF18A comprises: a proliferative disorder of cancer, psoriasis, atopic dermatitis, an autoimmune disease, or an inflammatory bowel disease.

26. The use as claimed in claim 25, wherein the cancer is selected from mesothelioma, neuroblastoma, rectal cancer, colon cancer, familial adenomatous polyposis and hereditary non-polyposis colorectal cancer, esophageal cancer, lip cancer, laryngeal cancer, hypopharyngeal cancer, tongue cancer, salivary gland cancer, gastric cancer, adenocarcinoma, medullary thyroid cancer, papillary thyroid cancer, renal cancer, renal parenchymal carcinoma, ovarian cancer, cervical cancer, corpus uteri cancer, endometrial cancer, choriocarcinoma, pancreatic cancer, prostate cancer, bladder cancer, testicular cancer, breast cancer, urinary cancer, melanoma, brain tumor, lymphoma, head and neck cancer, acute lymphoblastic leukemia, chronic lymphoblastic leukemia, acute myeloid leukemia, chronic myeloid leukemia, hepatocellular carcinoma, gallbladder cancer, bronchial cancer, small cell lung cancer, non-small cell lung cancer, multiple myeloma, basal sarcoma, teratoma, retinoblastoma, choroidal melanoma, seminoma, rhabdomyosarcoma, osteosarcoma, chondrosarcoma, myoma, liposarcoma, fibrosarcoma, Ewing's sarcoma, and plasmacytoma.

27. The use as claimed in claim 25, wherein the autoimmune disease is selected from rheumatoid arthritis, systemic lupus erythematosus, Sjogren's syndrome, scleroderma, mixed connective tissue disease, dermatomyositis, polymyositis, Reiter's syndrome, autoimmune lymphoproliferative syndrome, multiple sclerosis, myasthenia gravis, and encephalomyelitis.

28. The use as claimed in claim 25, wherein the inflammatory bowel disease is selected from ulcerative colitis and Crohn's disease.
